(19) Europäisches Patentamt
European Patent Office
Office européen des brevets

(11) EP 4 257 196 A1

(12) EUROPEAN PATENT APPLICATION

(43) Date of publication:
**11.10.2023 Bulletin 2023/41**

(21) Application number: **23162698.7**

(22) Date of filing: **17.03.2023**

(51) International Patent Classification (IPC):
***A61P 35/00*** (2006.01) ***A61K 38/08*** (2019.01)
***A61K 38/04*** (2006.01)

(52) Cooperative Patent Classification (CPC):
**A61P 35/00; A61K 38/04; A61K 38/08**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.03.2022 US 202263269511 P**

(71) Applicant: **The University of Hong Kong**
**Hong Kong (HK)**

(72) Inventors:
- **LI, Xiang David**
  **Hong Kong (CN)**
- **LI, Xin**
  **Hong Kong (CN)**
- **LIU, Sha**
  **Hong Kong (CN)**

(74) Representative: **HGF**
**HGF Limited**
**1 City Walk**
**Leeds LS11 9DX (GB)**

# (54) DISCOVERY OF YEATS2 YEATS DOMAIN INHIBITORS AS NOVEL ANTI-CANCER AGENTS

(57) The subject invention pertains to novel inhibitors of the YEATS2 YEATS domain and compositions thereof and methods of using said compositions to treat cancer in a subject. The subject inhibitors and compositions thereof can target the Kac binding pocket. Compounds of the subject invention can exhibit strong inhibition on cancerous cell growth, including NSCLC cell growth and proliferation.

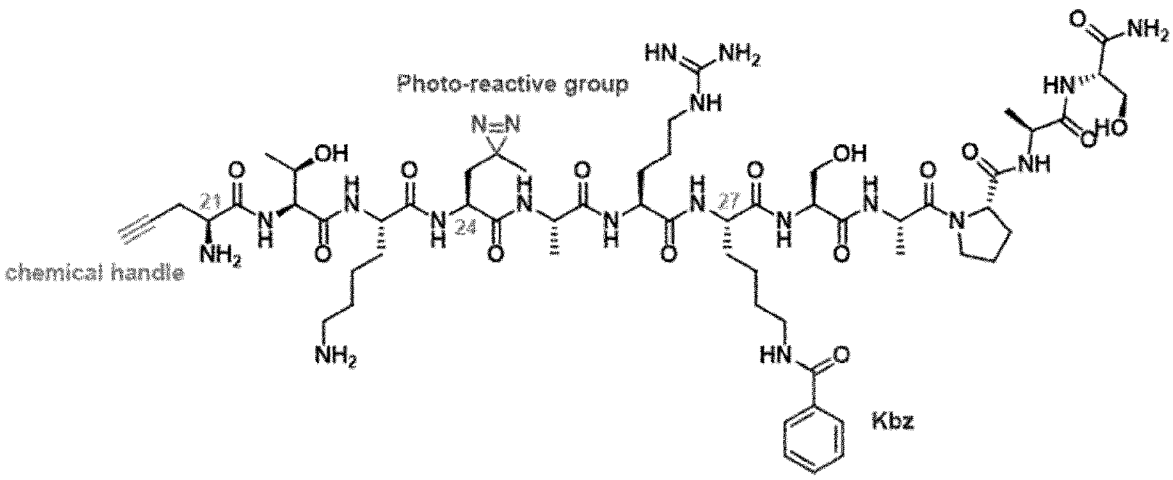


FIG. 1A

EP 4 257 196 A1

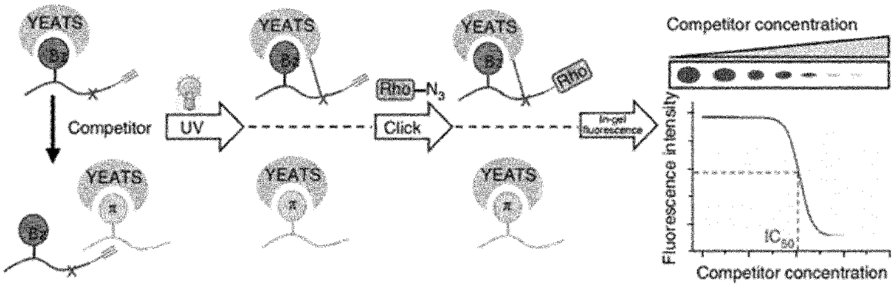


FIG. 1B

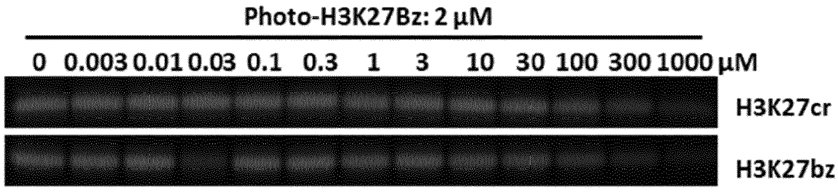


FIG. 1D

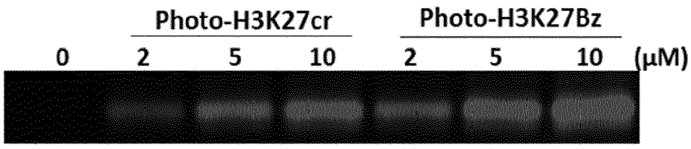


FIG. 1C

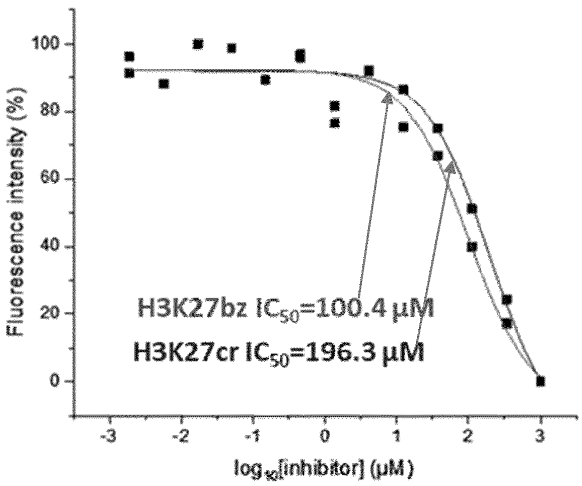


FIG. 1E

## Description

CROSS-REFERENCE TO RELATED APPLICATION

This application claims the benefit of U.S. Provisional Application Serial No. 63/269,511, filed March 17, 2022, which is hereby incorporated by reference in its entirety including any tables, figures, or drawings.

BACKGROUND

Histone post-translational modifications (PTMs), such as lysine acylation, arginine methylation and serine phosphorylation, play critical roles in some biological processes. By neutralizing the positive charge of histone residues (Lysine or Arginine), Histone PTMs disturb the physicochemical environment around modification sites. These PTMs are modified by "writers" and "erasers", and also serve as the docking site to recruit specific proteins ("readers") to the chromatin to signal the downstream cellular events, such as gene expression, DNA replication and damage repair. Given these numerous links to essential cellular processes, aberrant "reading" of histone PTMs has been closely associated with human diseases, such as cancers. Histone PTMs "readers", therefore, have been recognized as promising targets for drug discovery.

YEATS domains have been recently identified as novel "readers" of histone acylation, such as histone lysine acetylation (Kac) and lysine crotonylation (Kcr). The human genome encodes 4 YEATS domain-containing proteins (YCPs), including AF9, ENL, YEATS2, and GAS41, which are pivotal members of chromatin-modifying and transcription complexes, participating in chromatin remodeling and transcriptional regulation. In support of their functional importance, dysregulation of these YEATS family proteins is often linked to human disease.

YEATS2 is an emerging oncogene highly amplified in human cancers, including NSCLC, pancreatic cancers, and ovarian cancers. It's reported that depletion of YEATS2 could reduce NSCLC cell (A549 and H1299) growth and transformation activity, suggesting its indispensable role for cell survival (see Mi, W., Guan, H., Lyu, J. et al. YEATS2 links histone acetylation to tumorigenesis of non-small cell lung cancer. Nat Commun 8, 1088 (2017)). YEATS domain of YEATS2 recognizes histone lysine acylation by forming unique -$\pi$-$\pi$-$\pi$ stacking with its two aromatic residues (Y262 and W282). A Y262A or W282A mutation can disrupt the interaction between YEATS2 and histone lysine acylation. When reintroducing -wide-type YEATS2 to YEATS2-depleted NSCLC cells, cell proliferation restored, whereas the mutant (containing Y262A and W282A) YEATS2 did not. YEATS2 YEATS domain, therefore, emerged as attractive therapeutic target for NSCLC treatment. However, to date, no YEATS2 YEATS inhibitors have been developed.

Therefore, there remains a need for inhibitors of YEATS2 YEATS.

BRIEF SUMMARY

The subject invention pertains to novel YEATS2 YEATS inhibitors and methods of using said inhibitors. In certain embodiments, the inhibitors can target YEATS2 YEATS via a unique "sandwich" cage. The inhibitors can occupy the Kac-binding pocket of YEATS2 YEATS, which is an aromatic cage composed of residues Y262 and W282. The acetyl group of the inhibitor can be intercalated between the two aromatic rings of Y262 and W282 to form the unique "sandwich" cage. In certain embodiments, inhibitors HC25b and HC26b can efficiently inhibit YEATS2 YEATS and also cell growth and cell proliferation. In certain embodiments, YEATS2 YEATS domain preferentially recognizes H3K27ac by in the aromatic 'sandwich' cage. In certain embodiments, The YEATS2 YEATS inhibitors can be used to treat cancer in a subject. In certain embodiments, YEATS2 is linked to tumorigenesis of non-small cell lung cancer (NSCLC) by reading histone acetylation; therefore, the YEATS2 YEATS inhibitors can be used to treat NSCLC.

In certain embodiments, the peptide-derived inhibitor has the highest affinity of 89 nanomolar, targeting the unique $\pi$-$\pi$-$\pi$ stacking interaction of Kbz binding region of YEATS2 YEATS domain. In certain embodiments, an inhibitor, such as, for example, LS-1-124 (formula (II)), can efficiently reduce the chromatin occupancy of YEATS2-associated complexes, downregulate the oncogenes, and/or block NSCLC cell proliferation.

BRIEF DESCRIPTION OF DRAWINGS

**FIGs. 1A-1E.** (**FIG. 1A**). Structure of photoaffinity probe to labeling YEATS2, H3K27bz-Dzyne. (**FIG. 1B**). Schematic diagram demonstrating competitive photo-cross-linking to evaluating the potency of inhibitors. (**FIG. 1C**). Comparison of labeling efficiency between Photo-H3K27cr and Photo-H3K27bz. (**FIGs. 1D-1E**). The $IC_{50}$ curve of H3K27cr and H3K27bz determined by competitive photo-cross-linking assay.

**FIGs. 2A-2C.** (**FIG. 2A**). Chemical structures of developed peptides derived from $H3_{22-32}$K27cr; (**FIG. 2B**) Three-

spot competitive photo-cross-linking assay to roughly estimate the inhibitory activity of peptides inhibitors with different modification at H3K27, probe is H3K27bz-Dzyne. (**FIG. 2C**) Histogram showing the inhibitory effects of peptides. Fluorescence intensity data were quantified from gels shown in (**FIG. 2B**).

**FIGs. 3A-3C.** (**FIG. 3A**). Crystal structure of YEATS2 and H3K27Bz. (**FIGs. 3B-3C**). $IC_{50}$ comparison of the inhibitory effects of inhibitors toward YEATS2 YEATS by photo-cross-linking competition assay.

**FIGs. 4A-4D.** (**FIG. 4A**) Three spot screening by competitive photo-cross-linking assay. (**FIG. 4B**) The structures for 4-1 to 4-19. (**FIG. 4C**) the structures of benzofuran derivatives. (**FIG. 4D**) Heatmap showing the inhibitory effects of peptides (CA1-CA39), fluorescence intensity (%) data were quantified from gels.

**FIGs. 5A-5I.** (**FIGs. 5A, 5D, and 5G**) Structure of photo-H3K27bf; (**FIGs. 5B-5C**, 5E-5F, and 5H-5I) Photo-cross-linking competition assay measuring the $IC_{50}$ of inhibitors.

**FIGs. 6A-6H.** ITC determinations for binding affinity of YEATS2 YEATS towards H3K27cr (**FIG. 6B**), H3K27bz (**FIG. 6C**), H3K27bf (**FIG. 6D**), $AcK_{bf}SAP$ (**FIG. 6E**), $MsK_{bf}SAP$ (**FIG. 6F**), $MsK_{cl\text{-}bf}SAP$ (**FIG. 6G**) and $MsK_{br\text{-}bf}SAP$ (**FIG. 6H**).

**FIGs. 7A-7D.** (**FIG. 7A**) CETSA to show $MsK_{bf}SAP$ targets the endogenous YEATS2 in H1299 cells; FRAP curves (**FIG. 7B**) and recovery $t_{1/2}$ (**FIG. 7C**) in FRAP assay. Scale bar = 4 $\mu$m. Data are reported as mean $\pm$s.e.m., n $\geq$ 20. P values are based on the two-tailed Student's t-test. **P < 0.01, ****P < 0.0001; (**FIG. 7D**) CHIP-qPCR analysis of YEATS2 enriched genes in H1299 cells treated with inhibitors.

**FIGs. 8A-8B.** (**FIG. 8A**) colony formation assay of A549 with or without inhibitor treatment; (**FIG. 8B**) colony number of (**FIG. 8A**).

**FIGs. 9A-9G.** $IC_{50}$ of HC24b, HC25b and HC26b determined by competitive photo-cross-linking assay.

**FIGs. 10A-10C.** Luminescence ATP detection system shows the survival rate (%) in H1299 (**FIG. 10A**) and A549 (**FIG. 10B**) after incubating with inhibitors; (**FIG. 10C**) colony formation assay of A549 with or without inhibitors treatment.

**FIGs. 11A-11B. FIG. 11A** provides a comparison of inhibitory activity between N-methyl Ala and Ser containing inhibitors against YEATS2 YEATS by competitive photo-cross-linking assay; **FIG. 11B** provides a comparison of the potency of six- and seven- member ring coupled at C-terminus toward YEATS2 YEATS by competitive photo-cross-linking assay.

**FIGs. 12A-12B. FIG. 12A** shows a luminescence ATP detection system shows the survival rate (%) in H1299 and A549 after treating with inhibitors; **FIG. 12B** shows colony formation assay of H1299 and A549 with or without inhibitors treatment.

**FIGs. 13A-13B.** FRAP assay using tantem-YEATS2 YEATS (**FIG. 13A**) and FRAP curves and recovery $t_{1/2}$ (**FIG. 13B**) Scale bar = 4 $\mu$m. Data are reported as mean $\pm$s.e.m., n $\geq$ 20. P values are based on the two-tailed Student's t-test. (*P<0.05, **P < 0.01,***<0.001, ****P < 0.0001, n.s.:not significant.)

**FIGs. 14A-14B.** LS-1-124 engaged with endogenous AF9. **FIG. 14A** shows the structure of Probe 1 used for labelling of YEATS2 **(FIG. 14B** shows the photo-crosslinking pull-down in whole cell lysate (2 mg/mL) with probe 1 in the presence or absence of LS-1-124.

**FIGs. 15A-15D.** (**FIG. 15A**) Tumor growth chart; (**FIG. 15B**) Mouse body weight chart; (**FIG. 15C**) Volumes of tumors of the H1299 cells as in (**FIG. 15A**) subcutaneously transplanted into immunodeficient nude mice. Statistical evaluation by paired Student's test (mean $\pm$ S.E.M.). (**FIG. 15D**) H&E staining of tumors after 26 days of treatment with either DMSO or LS-1-124 (upper panel); Representative images of Ki-67 immunohistochemical staining of tumor tissue after 26 days of treatment with either DMSO or LS-1-124 (lower panel).

DETAILED DESCRIPTION

## Selected Definitions

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising". The transitional terms/phrases (and any grammatical variations thereof) "comprising", "comprises", "comprise", "consisting essentially of', "consists essentially of", "consisting" and "consists" can be used interchangeably.

The phrases "consisting essentially of" or "consists essentially of" indicate that the claim encompasses embodiments containing the specified materials or steps and those that do not materially affect the basic and novel characteristic(s) of the claim.

The term "about" means within an acceptable error range for the particular value as determined by one of ordinary skill in the art, which depends in part on how the value is measured, *i.e.,* the limitations of the measurement system. In the context of compositions containing amounts of ingredients where the terms "about" is used, these compositions contain the stated amount of the ingredient with a variation (error range) of 0-10% around the value (X ± 10%). In other contexts the term "about" is provides a variation (error range) of 0-10% around a given value (X ± 10%). As is apparent, this variation represents a range that is up to 10% above or below a given value, for example, X ± 1%, X ± 2%, X ± 3%, X ± 4%, X ± 5%, X ± 6%, X ± 7%, X ± 8%, X ± 9%, or X ± 10%.

In the present disclosure, ranges are stated in shorthand to avoid having to set out at length and describe each and every value within the range. Any appropriate value within the range can be selected, where appropriate, as the upper value, lower value, or the terminus of the range. For example, a range of 0.1-1.0 represents the terminal values of 0.1 and 1.0, as well as the intermediate values of 0.2, 0.3, 0.4, 0.5, 0.6, 0.7, 0.8, 0.9, and all intermediate ranges encompassed within 0.1-1.0, such as 0.2-0.5, 0.2-0.8, 0.7-1.0, etc. Values having at least two significant digits within a range are envisioned, for example, a range of 5-10 indicates all the values between 5.0 and 10.0 as well as between 5.00 and 10.00 including the terminal values. When ranges are used herein, combinations and subcombinations of ranges (e.g., subranges within the disclosed range) and specific embodiments therein are explicitly included.

As used herein, the term "subject" refers to an animal, needing or desiring delivery of the benefits provided by a therapeutic compound. The animal may be for example, humans, pigs, horses, goats, cats, mice, rats, dogs, apes, fish, chimpanzees, orangutans, guinea pigs, hamsters, cows, sheep, birds, chickens, as well as any other vertebrate or invertebrate. These benefits can include, but are not limited to, the treatment of a health condition, disease or disorder; prevention of a health condition, disease or disorder; immune health; enhancement of the function of an organ, tissue, or system in the body. The preferred subject in the context of this invention is a human. The subject can be of any age or stage of development, including infant, toddler, adolescent, teenager, adult, or senior.

As used herein, the terms "therapeutically-effective amount," "therapeutically-effective dose," "effective amount," and "effective dose" are used to refer to an amount or dose of a compound or composition that, when administered to a subject, is capable of treating or improving a condition, disease, or disorder in a subject or that is capable of providing enhancement in health or function to an organ, tissue, or body system. In other words, when administered to a subject, the amount is "therapeutically effective." The actual amount will vary depending on a number of factors including, but not limited to, the particular condition, disease, or disorder being treated or improved; the severity of the condition; the particular organ, tissue, or body system of which enhancement in health or function is desired; the weight, height, age, and health of the patient; and the route of administration.

As used herein, the term "treatment" refers to eradicating, reducing, ameliorating, or reversing a sign or symptom of a health condition, disease or disorder to any extent, and includes, but does not require, a complete cure of the condition, disease, or disorder. Treating can be curing, improving, or partially ameliorating a disorder. "Treatment" can also include improving or enhancing a condition or characteristic, for example, bringing the function of a particular system in the body to a heightened state of health or homeostasis.

As used herein, "preventing" a health condition, disease, or disorder refers to avoiding, delaying, forestalling, or minimizing the onset of a particular sign or symptom of the condition, disease, or disorder. Prevention can, but is not required, to be absolute or complete; meaning, the sign or symptom may still develop at a later time. Prevention can include reducing the severity of the onset of such a condition, disease, or disorder, and/or inhibiting the progression of the condition, disease, or disorder to a more severe condition, disease, or disorder.

In some embodiments of the invention, the method comprises administration of multiple doses of the compounds of the subject invention. The method may comprise administration of 1, 2, 3, 4, 5, 6, 7, 8, 9, 10, 15, 20, 25, 30, 35, 40, or more therapeutically effective doses of a composition comprising the compounds of the subject invention as described herein. In some embodiments, doses are administered over the course of 1 day, 2 days, 3 days, 4 days, 5 days, 6 days, 7 days, 10 days, 14 days, 21 days, 30 days, or more than 30 days. Moreover, treatment of a subject with a therapeutically effective amount of the compounds of the invention can include a single treatment or can include a series of treatments. It will also be appreciated that the effective dosage of a compound used for treatment may increase or decrease over the course of a particular treatment. Changes in dosage may result and become apparent from the results of diagnostic assays or imaging techniques for detecting tumor sizes known in the art. In some embodiments of the invention, the method comprises administration of the compounds at several time per day, including but not limiting to 2 times per day, 3 times per day, and 4 times per day.

As used herein, an "isolated" or "purified" compound is substantially free of other compounds. In certain embodiments, purified compounds are at least 60% by weight (dry weight) of the compound of interest. Preferably, the preparation is at least 75%, more preferably at least 90%, and most preferably at least 99%, by weight of the compound of interest. For example, a purified compound is one that is at least 90%, 91%, 92%, 93%, 94%, 95%, 98%, 99%, or 100% (w/w) of the desired compound by weight. Purity is measured by any appropriate standard method, for example, by column chromatography, thin layer chromatography, or high-performance liquid chromatography (HPLC) analysis.

By "reduces" is meant a negative alteration of at least 1%, 5%, 10%, 25%, 50%, 75%, or 100%.

By "increases" is meant as a positive alteration of at least 1%, 5%, 10%, 25%, 50%, 75%, or 100%.

As used herein, a "pharmaceutical" refers to a compound manufactured for use as a medicinal and/or therapeutic drug.

As used herein, "protein readers" or "readers" are specific proteins that can be recruited to post-translational modification sites on chromatin to signal the downstream cellular events, such as gene expression, DNA replication and damage repair.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

**Preparation of YEATS2 YEAT domain inhibitor Compositions**

In certain embodiments, YEATS2 YEAT inhibitor composition is the YEATS2 YEAT domain inhibitor, according to formula (I), or a composition comprising said YEATS2 YEAT domain inhibitor. In certain embodiments, the therapeutic compound can be HC25b and HC26b, according to formulas (II) and (III).

Formula (I)

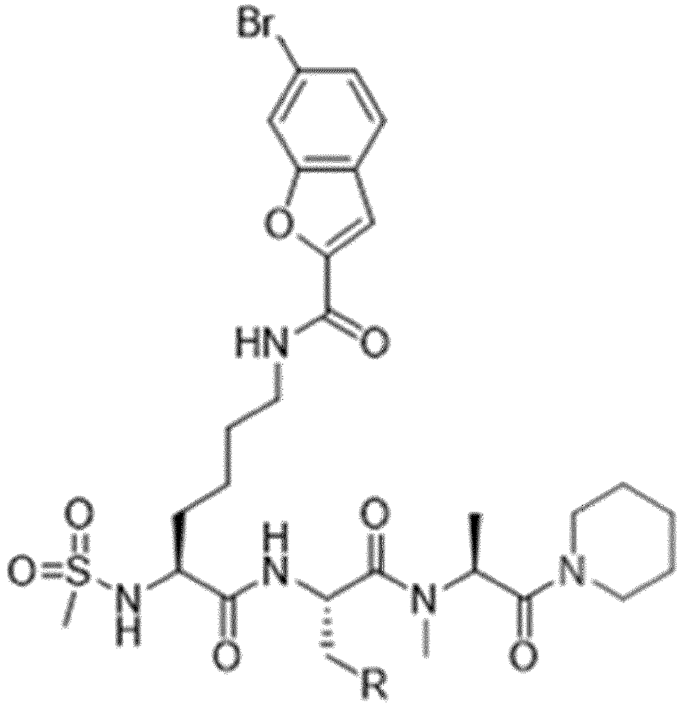

wherein R is selected from

R:

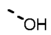 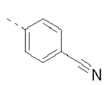 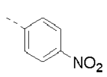 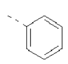 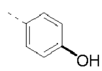 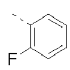 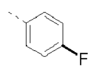

LS100 LS101 LS102 LS103 LS104 LS123 LS124

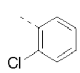 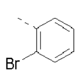 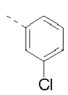 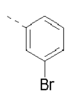 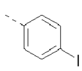 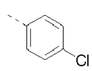 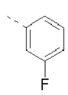 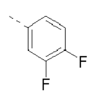 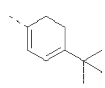 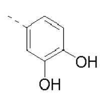

LS130 LS131 LS132 LS133 LS134 LS135 LS136 LS137 LS138 LS139

Formula (II)

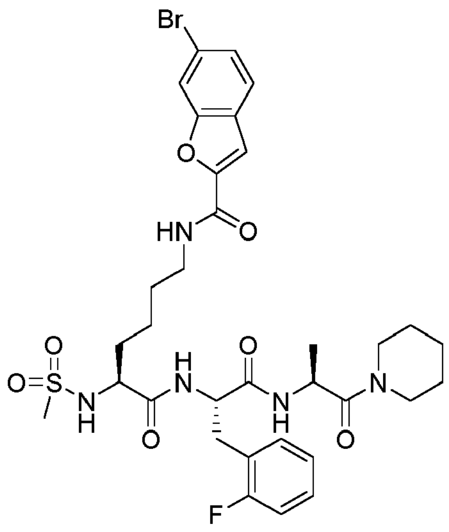

Formula (III)

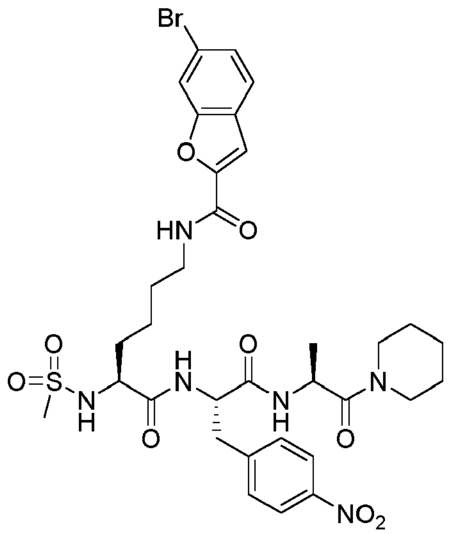

In certain embodiments, YEATS2 YEAT inhibitor composition is the YEATS2 YEAT domain inhibitor, according to formula (IV), formula (V), formula (VI), formula (VII), formula (VIII), formula (IX), formula (X), formula (XI), formula (XII), formula (XIII), formula (XIV), formula (XV), formula (XVI), formula (XVII), formula (XVIII), formula (XIX), formula (XX), formula (XXI), formula (XXII), formula (XXIII), formula (XXIV), formula (XXV), formula (XXVI), formula (XXVII), formula (XXVIII), formula (XXIX), formula (XXX), formula (XXXI), formula (XXXII), formula (XXXIII), formula (XXXIV), formula (XXXV), formula (XXXVI), formula (XXXVII), formula (XXXVIII), formula (XXXIX), formula (XL), formula (XLI), formula (XLII), formula (XLIII), formula (XLIV), formula (XLV), formula (XLVI), formula (XLVII), formula (XLVIII), formula (XLIX), formula (L), formula (LI), formula (LIII), formula (LIV), formula (LV), formula (LVI), formula (LVII), formula (LVIII), or formula (LIX), formula (LX), formula (LXI) or a composition comprising said YEATS2 YEAT domain inhibitors. In certain embodiments, the inhibitor can comprise one or more amino acids or peptides.

Formula (IV)

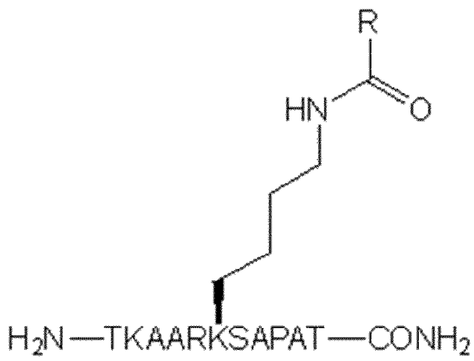

wherein, R is selected from:

Cr 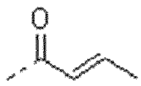

4Py 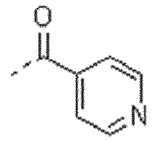

3Tp 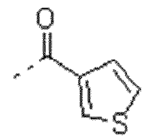

4-oxa 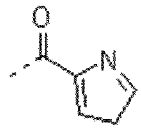

Bz 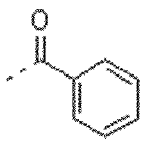

Pm 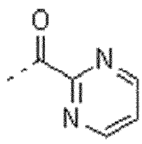

N-Pr 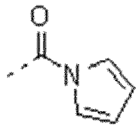

Benfu 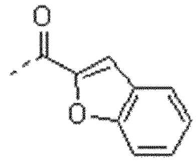

TF 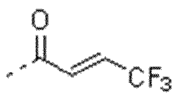

2fu 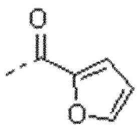

N-Pr-2H 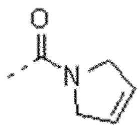

Benthio 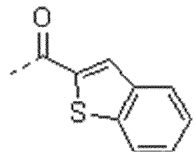

2Py 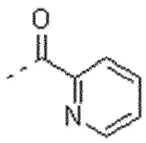

3fu 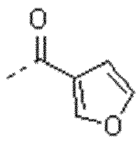

isoxa 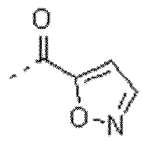

cp 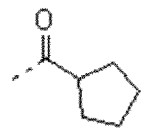

3Py 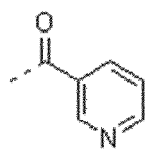

2Tp 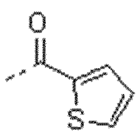

5-oxa 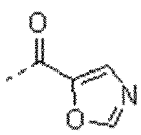

Formula (V)

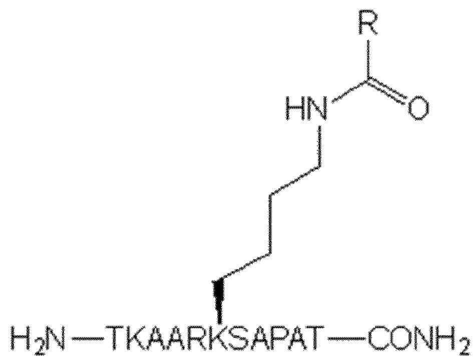

wherein R is selected from

R=

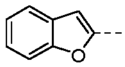

benfu

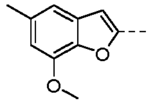

CA-1

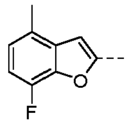

CA-2

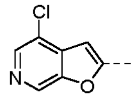

CA-3

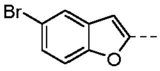

CA-8

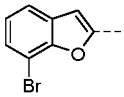

CA-9

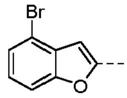

CA-10

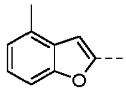

CA-11

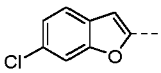

CA-16

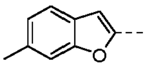

CA-17

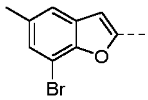

CA-18

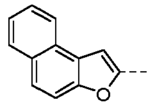

CA-19

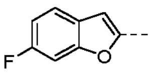

CA-24

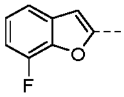

CA-25

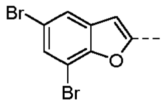

CA-26

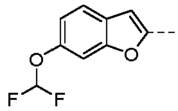

CA-27

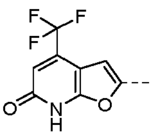

CA-32

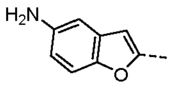

CA-33

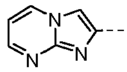

CA-34

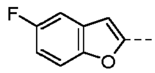

CA-35

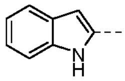

CA-40

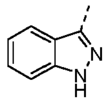

CA-41

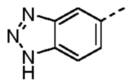

CA-42

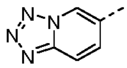

CA-43

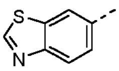

CA-48

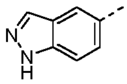

CA-49

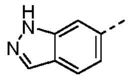

CA-50

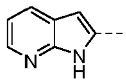

CA-51

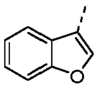

CA-56

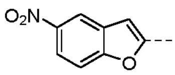

CA-57

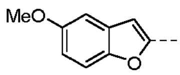

CA-58

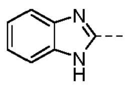

CA-59

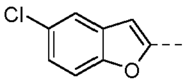
CA-4

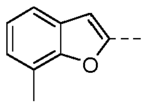
CA-5

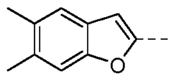
CA-6

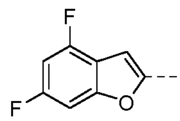
CA-7

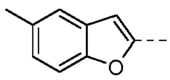
CA-12

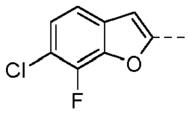
CA-13

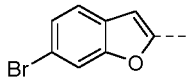
CA-14

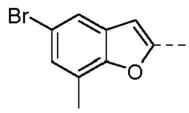
CA-15

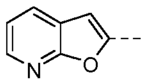
CA-20

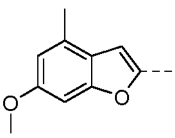
CA-21

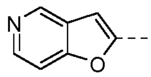
CA-22

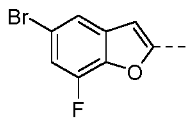
CA-23

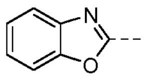
CA-28

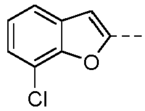
CA-29

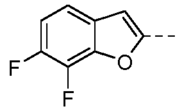
CA-30

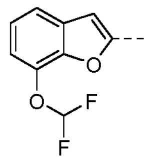
CA-31

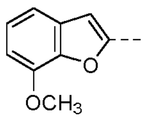
CA-36

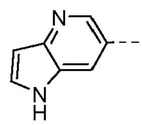
CA-37

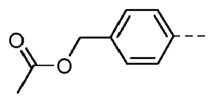
CA-38

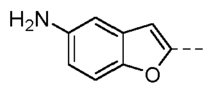
CA-39

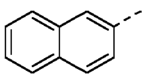
CA-44

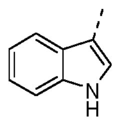
CA-45

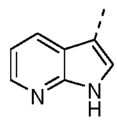
CA-46

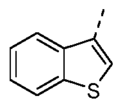
CA-47

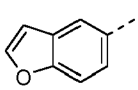
CA-52

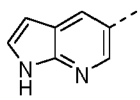
CA-53

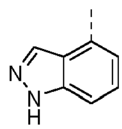
CA-54

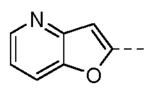
CA-55

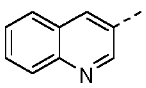
CA-60

Formula (VI)

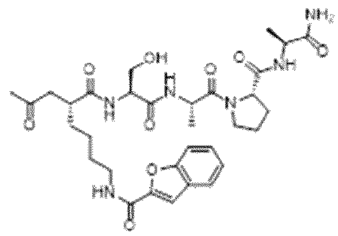

AcKbfSAPA

Formula (VII)

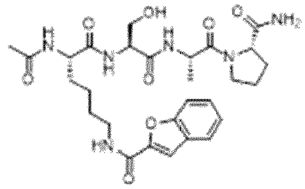

AcKbfSAP

Formula (VIII)

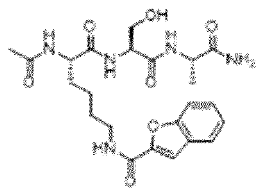

AcKbfSA

Formula (IX)

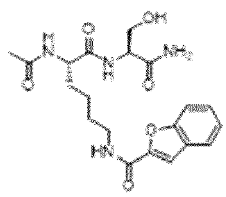

AcKbfS

Formula (X)

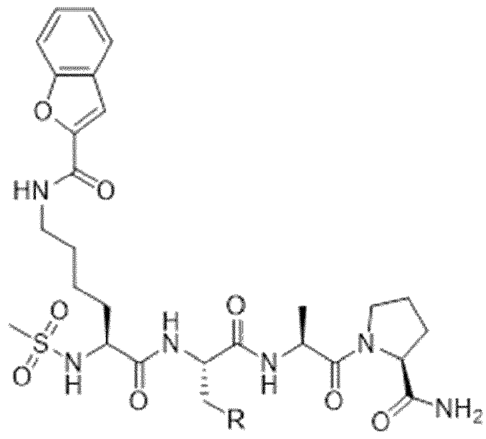

wherein R is selected from

| | | | | | | |
|---|---|---|---|---|---|---|
| 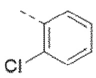 | 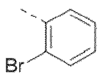 | 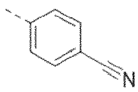 | 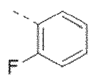 | 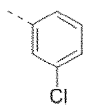 | 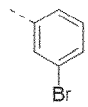 | 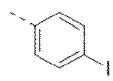 |
| HC1 | HC2 | HC3 | HC4 | HC5 | HC6 | HC7 |
| 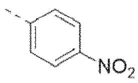 | 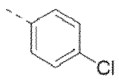 | 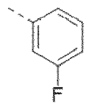 | 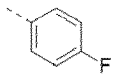 | 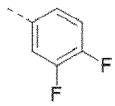 | 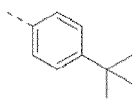 | 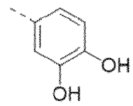 |
| HC8 | HC9 | HC10 | HC11 | HC12 | HC13 | HC14 |

Formula (XI)

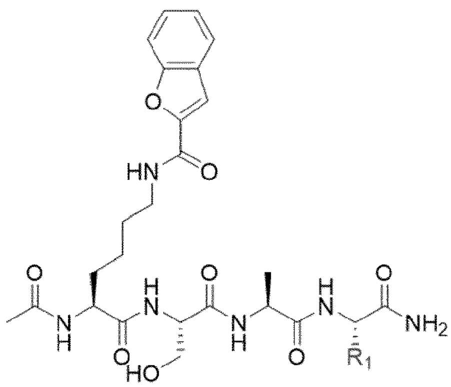

Wherein $R_1$ is selected from:

| | | | | | | | |
|---|---|---|---|---|---|---|---|
| $R_1$ | 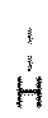 | 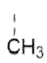 | 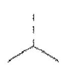 | 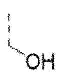 | 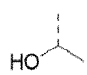 | 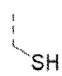 | 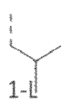 |
| | 1-G | 1-A | 1-V | 1-S | 1-T | 1-C | 1-L |

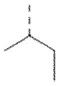 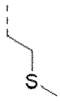 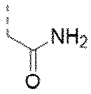 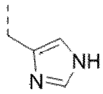 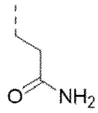 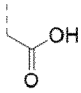 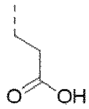

1-I 1-M 1-N 1-H 1-Q 1-D 1-E

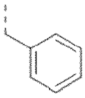 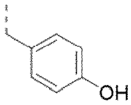 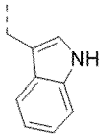 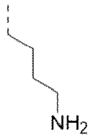 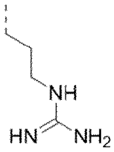

1-F 1-Y 1-W 1-K 1-R

Formula (XII)

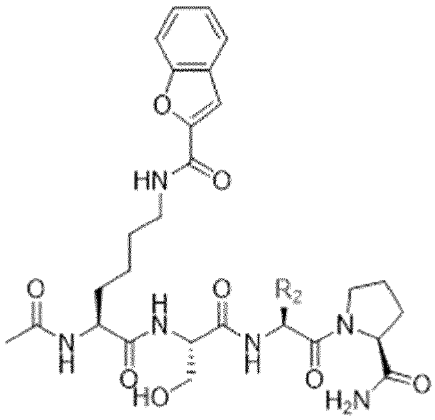

wherein $R_2$ is selected from

$R_2$ 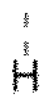 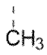 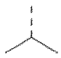 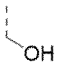 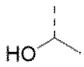 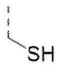 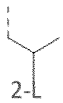

2-G 2-A 2-V 2-S 2-T 2-C 2-L

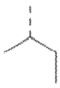 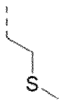 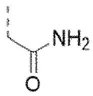 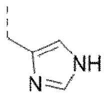 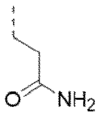 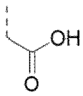 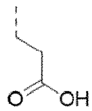

2-I 2-M 2-N 2-H 2-Q 2-D 2-E

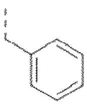 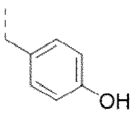 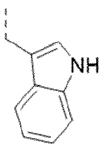 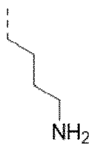 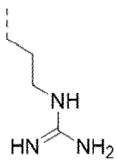

2-F 2-Y 2-W 2-K 2-R

Formula (XIII)

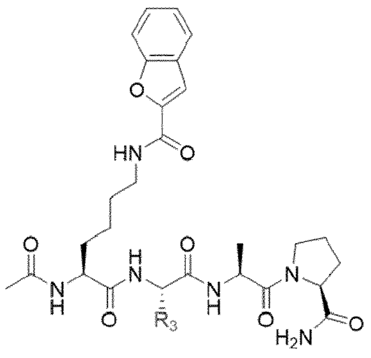

wherein $R_3$ is selected from:

$R_3$

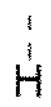
3-G

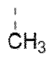
3-A

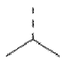
3-V

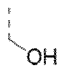
3-S

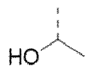
3-T

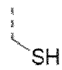
3-C

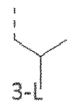
3-L

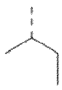
3-I

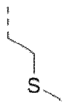
3-M

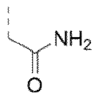
3-N

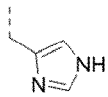
3-H

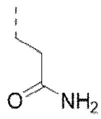
3-Q

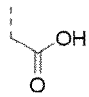
3-D

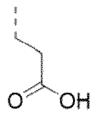
3-E

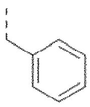
3-F

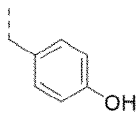
3-Y

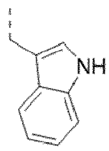
3-W

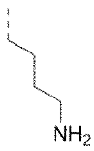
3-K

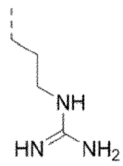
3-R

Formula (XIV)

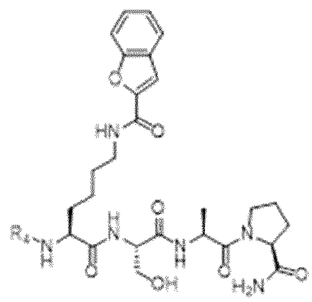

wherein $R_4$ is selected from:

$R_4$

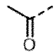
4-1
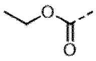
4-2
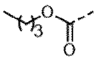
4-3
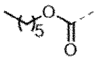
4-4
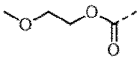
4-5
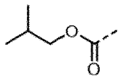
4-6
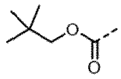
4-7

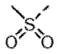
4-12
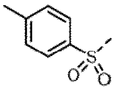
4-13
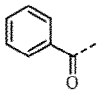
4-15
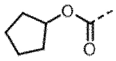
4-8
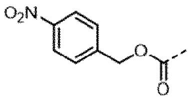
4-9
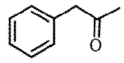
4-16
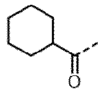
4-18

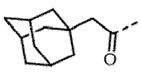
4-19
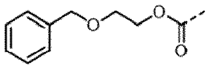
4-10
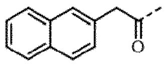
4-17
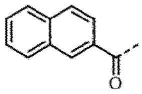
4-14
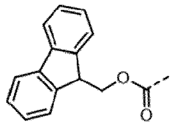
4-11

Formula (XV)

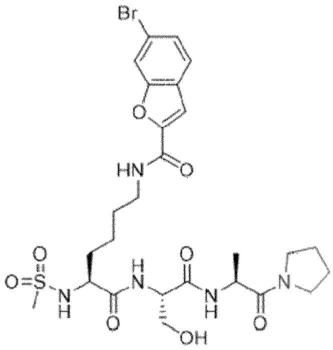

HC15a

Formula (XVI)

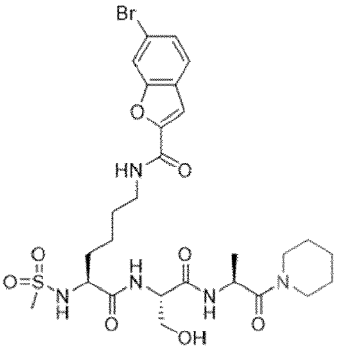

HC15b

Formula (XVII)

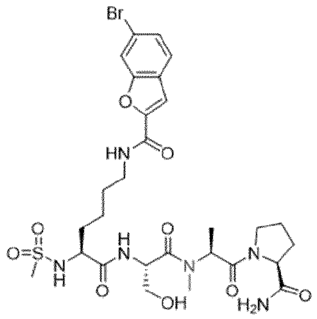

HC18

Formula (XVIII)

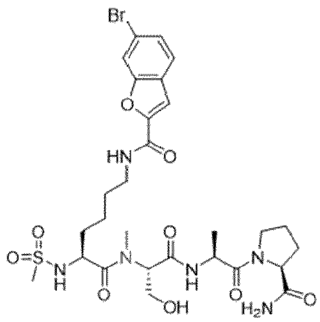

HC19

Formula (XIX)

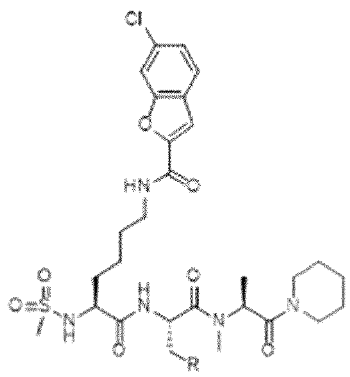

wherein R is selected from

R:

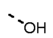
LS118

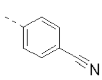
LS119

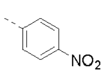
LS120

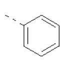
LS121

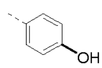
LS122

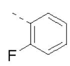
LS116

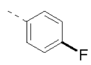
LS125

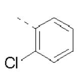
LS140

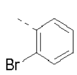
LS141

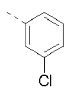
LS142

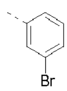
LS143

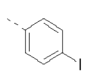
LS144

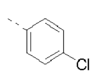
LS145

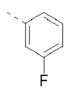
LS146

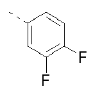
LS147

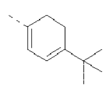
LS148

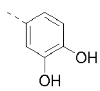
LS149

Formula (XX)

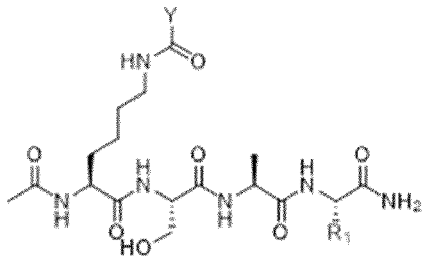

wherein $R_1$ is selected from

$R_1$ 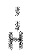 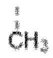 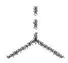 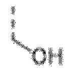 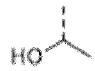 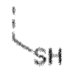 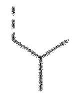

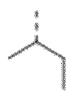 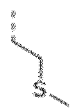 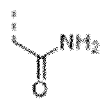 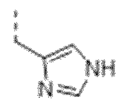 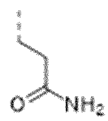 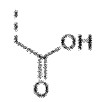 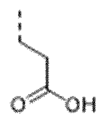

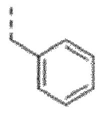 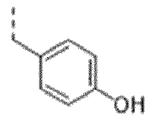 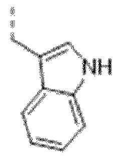 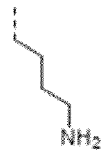 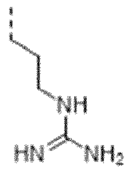

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXI)

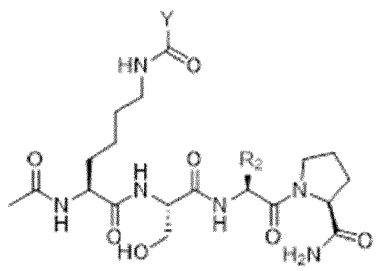

wherein $R_2$ is selected from

$R_2$ 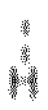 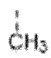 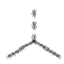 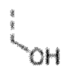 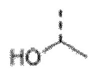 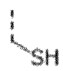 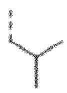

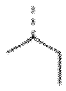 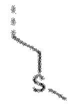 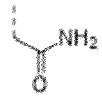 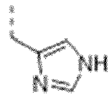 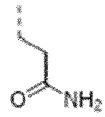 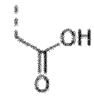 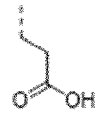

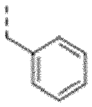 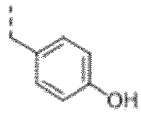 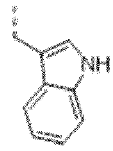 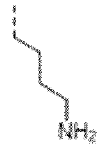 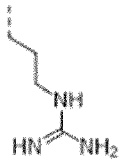

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXII)

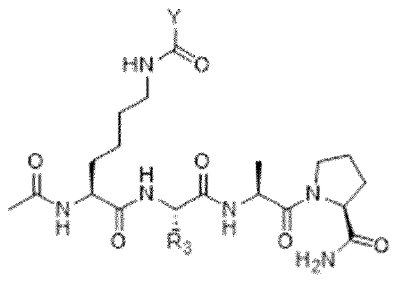

wherein $R_3$ is selected from

$R_3$ 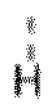 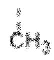 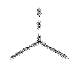 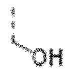 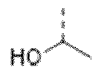 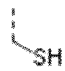 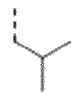

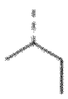 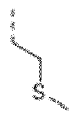 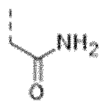 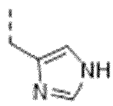 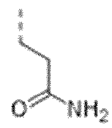 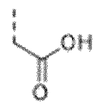 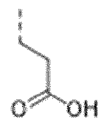

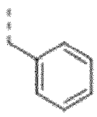 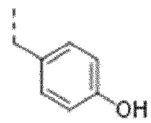 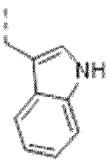 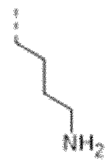 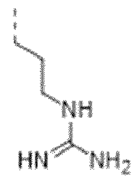

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIII)

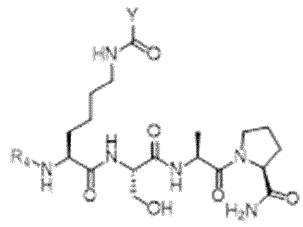

wherein $R_4$ is selected from

$R_4$

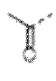 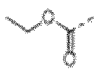 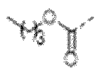 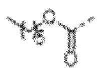 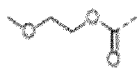 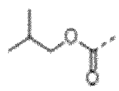 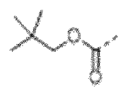

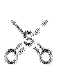 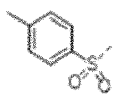 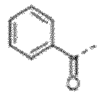 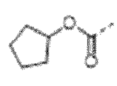 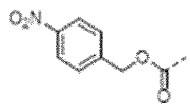 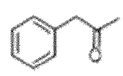 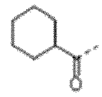

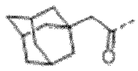 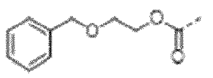 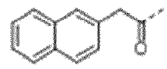 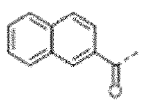 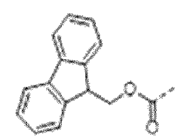

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIV)

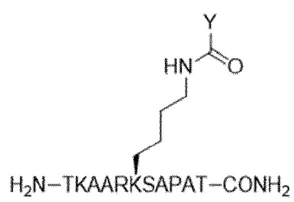

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXV)

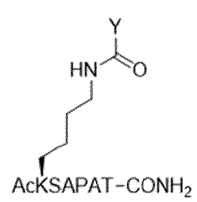

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVI)

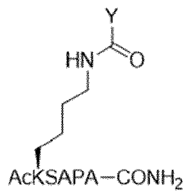

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVII)

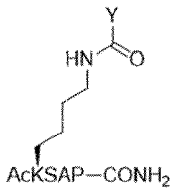

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVIII)

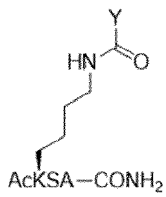

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIX)

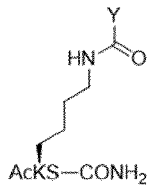

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXX)

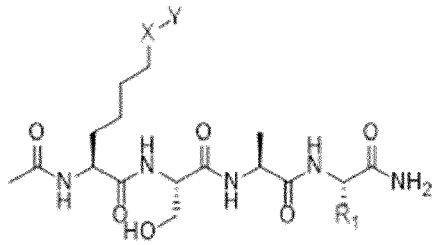

wherein $R_1$ is selected from

$R_1$ 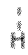 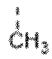 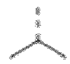 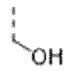 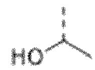 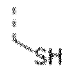 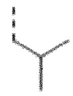

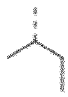 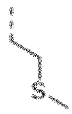 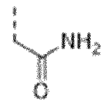 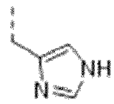 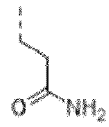 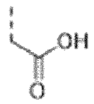 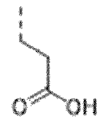

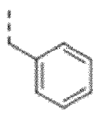 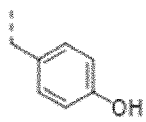 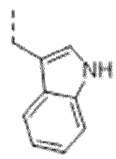 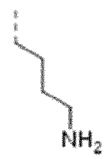 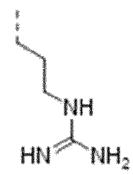

X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXI)

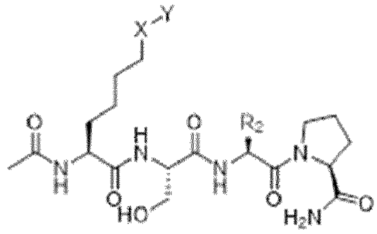

wherein $R_2$ is selected from

$R_2$ 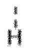 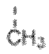 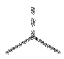 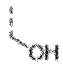 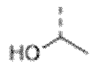 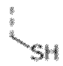 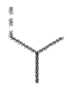

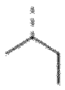 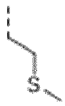 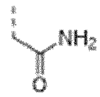 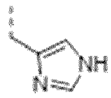 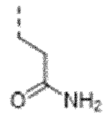 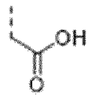 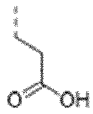

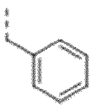 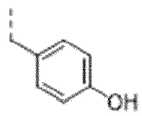 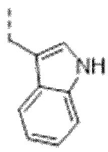 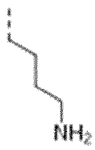 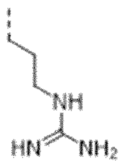

X is selected from NH-C=O, NH-C=S, O-C=O, C=O-O, NH-$SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXII)

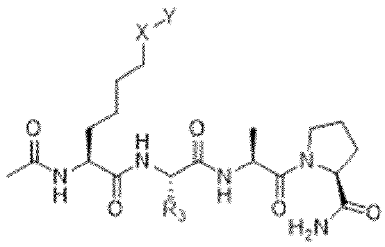

wherein $R_3$ is selected from

$R_3$ 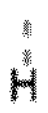 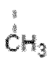 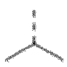 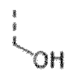 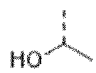 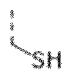 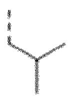

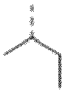 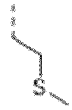 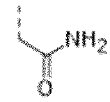 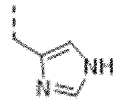 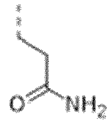 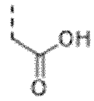 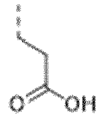

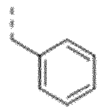 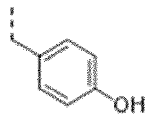 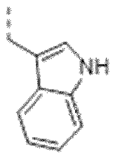 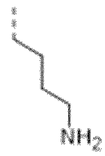 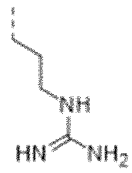

X is selected from NH-C=O, NH-C=S, O-C=O, C=O-O, $NH-SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIII)

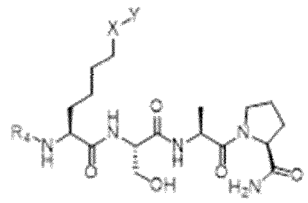

wherein $R_4$ is selected from

$R_4$ 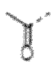 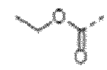 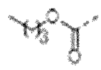 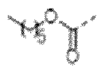 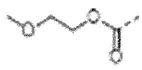 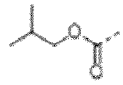 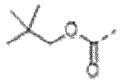

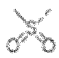 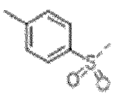 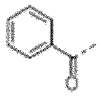 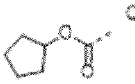 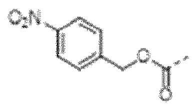 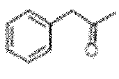 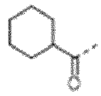

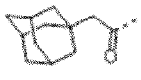 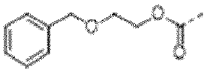 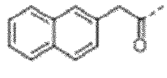 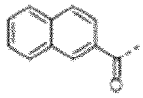 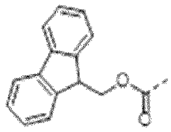

X is selected from NH-C=O, NH-C=S, O-C=O, C=O-O, $NH-SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIV)

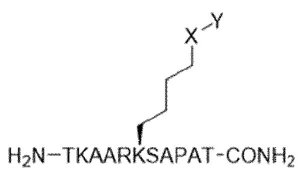

wherein X is selected from NH-C=O, NH-C=S, O-C=O, C=O-O, NH-$SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXV)

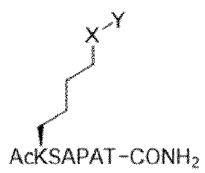

wherein X is selected from NH-C=O, NH-C=S, O-C=O, C=O-O, NH-$SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVI)

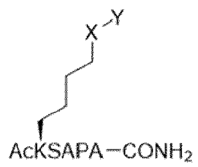

wherein X is selected from NH-C=O, NH-C=S, O-C=O, C=O-O, NH-$SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVII)

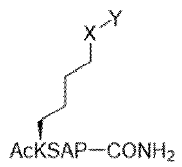

wherein X is selected from NH-C=O, NH-C=S, O-C=O, C=O-O, NH-$SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVIII)

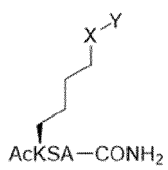

wherein X is selected from NH-C=O, NH-C=S, O-C=O, C=O-O, $NH-SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIX)

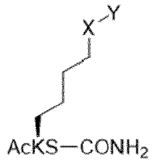

wherein X is selected from NH-C=O, NH-C=S, O-C=O, C=O-O, $NH-SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XL)

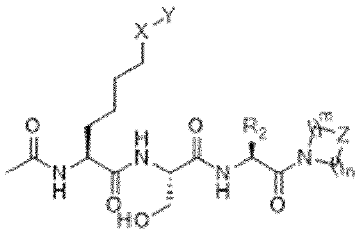

wherein $R_2$ is selected from

$R_2$ 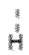 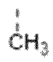 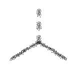 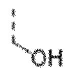 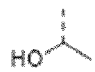 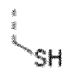 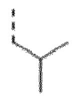

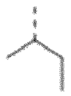 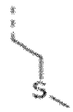 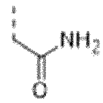 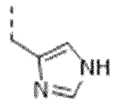 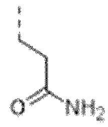 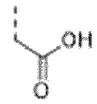 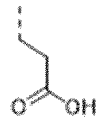

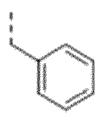 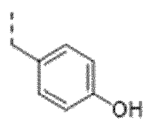 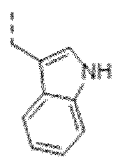 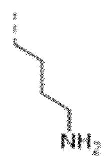 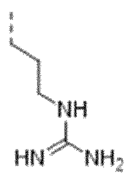

X is selected from NH-C=O, NH-C=S, O-C=O, C=O-O, $NH-SO_2$;

Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

Z is selected from $CH_2$, O, NH, S, N-Me; and

m and n are each independently integers from 0 to 8, wherein at least one of m or n is not 0

Formula (XLI)

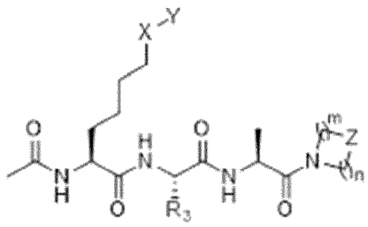

wherein $R_3$ is selected from

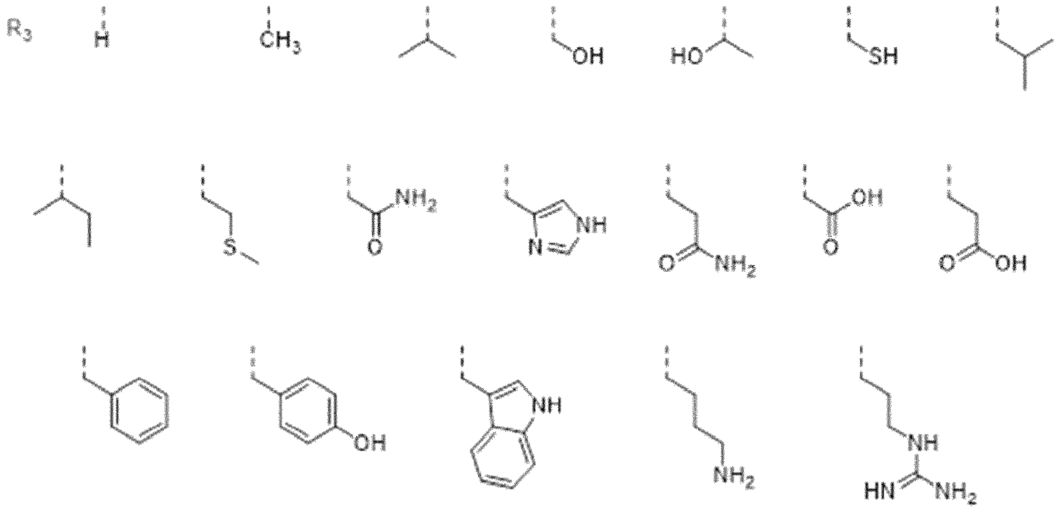

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
Z is selected from $CH_2$, O, NH, S, N-Me; and
m and n are each independently integers from 0 to 8, wherein at least one of m or n is not 0

Formula (XLII)

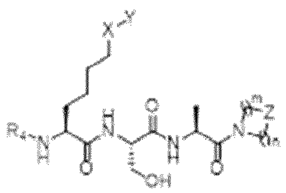

wherein $R_4$ is selected from

$R_4$

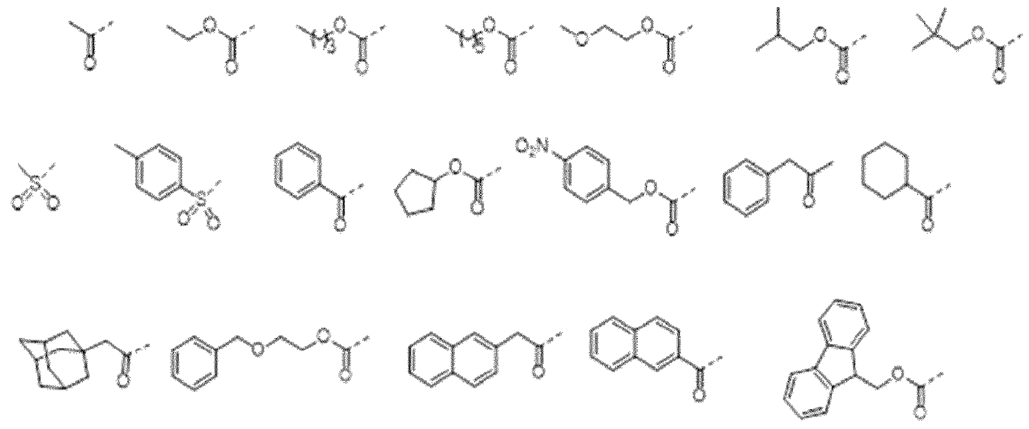

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
Z is selected from $CH_2$, O, NH, S, N-Me; and
m and n are each independently integers from 0 to 8, wherein at least one of m or n is not 0;

Formula (XLIII)

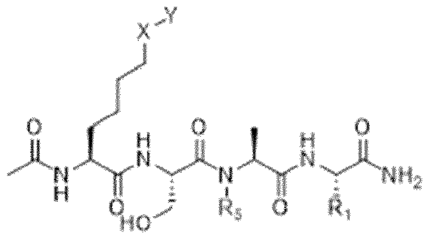

wherein $R_1$ is selected from

$R_1$

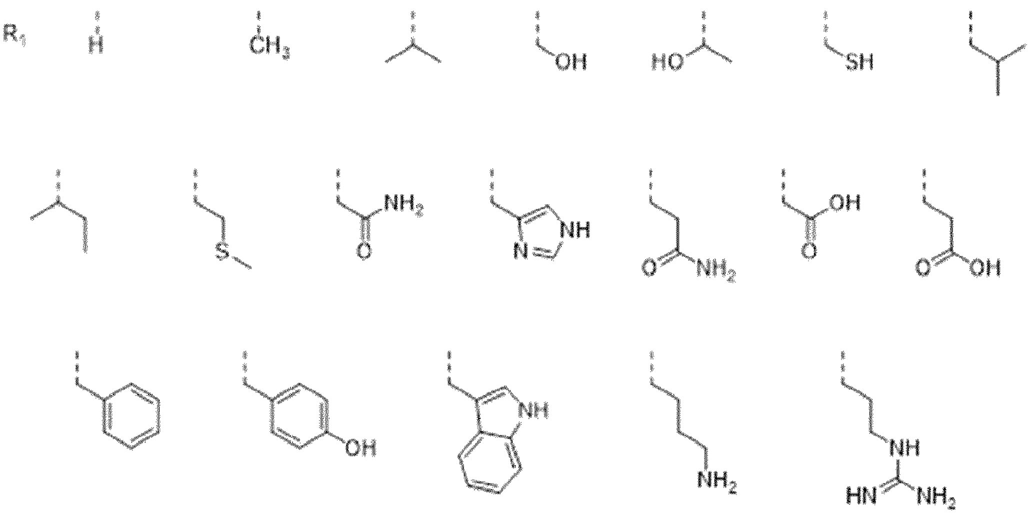

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0 to 10;
Z is selected from $CH_2$, O, NH, S, C=O, N; and
$R_5$ is selected from H, Me, Et, Pr, or iPr

Formula (XLIV)

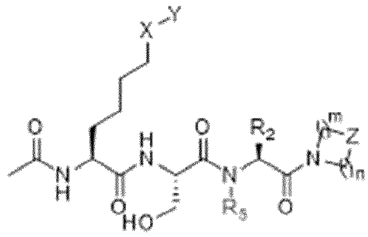

wherein $R_2$ is selected from

$R_2$

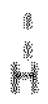 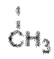 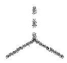 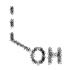 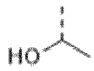 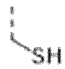 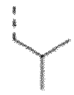

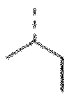 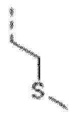 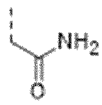 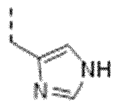 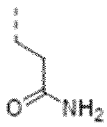 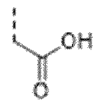 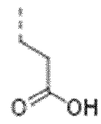

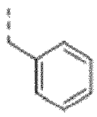 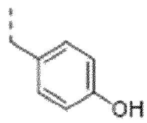 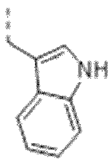 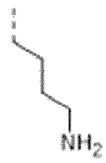 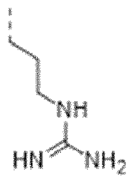

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N; and
$R_5$ is selected from H, Me, Et, Pr, or iPr

Formula (XLV)

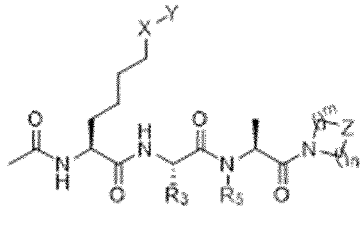

wherein $R_3$ is selected from

R3 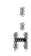 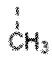 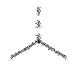 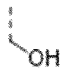 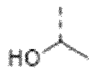 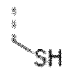 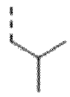

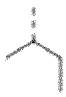 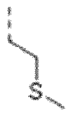 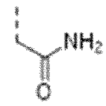 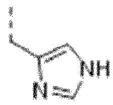 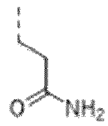 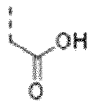 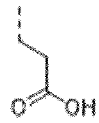

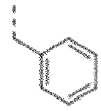 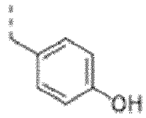 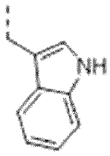 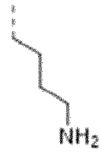 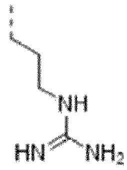

X is selected from NH-C=O, NH-C=S, O-C=O, C=O-O, $NH\text{-}SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N; and
$R_5$ is selected from H, Me, Et, Pr, or iPr

Formula (XLVI)

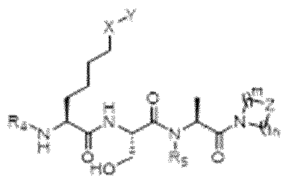

wherein $R_4$ is selected from

R4 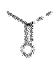 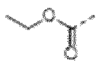 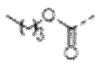 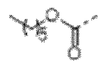 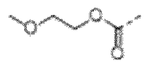 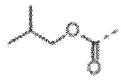 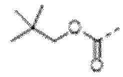

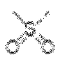 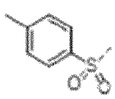 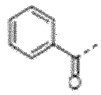 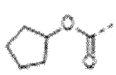 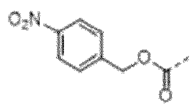 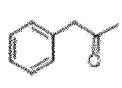 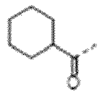

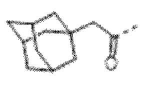 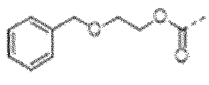 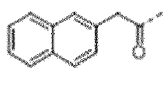 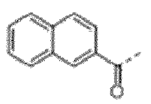 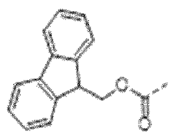

X is selected from NH-C=O, NH-C=S, O-C=O, C=O-O, $NH\text{-}SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;

Z is selected from $CH_2$, O, NH, S, C=O, N; and
$R_5$ is selected from H, Me, Et, Pr, or iPr

Formula (XLVII)

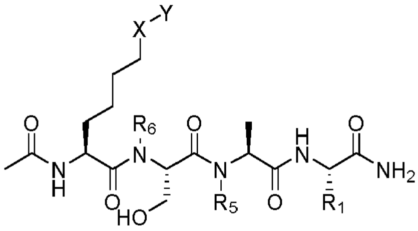

wherein $R_1$ is selected from

$R_1$ 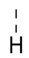 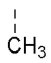 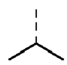 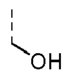 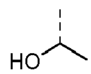 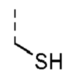 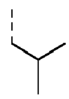

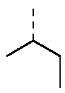 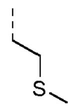 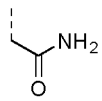 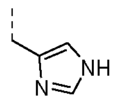 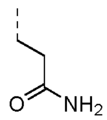 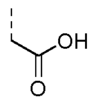 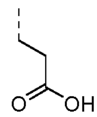

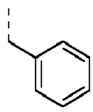 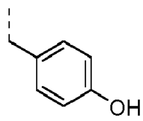 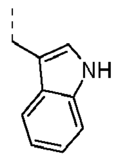 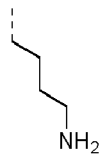 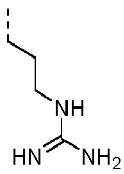

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (XLVIII)

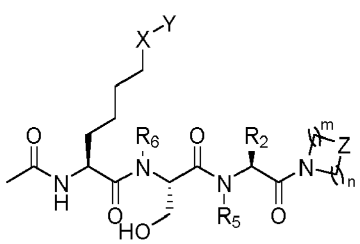

wherein $R_2$ is selected from

R2 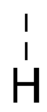 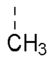 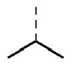 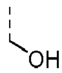 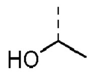 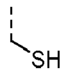 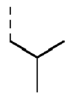

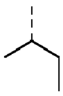 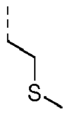 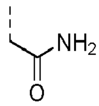 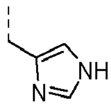 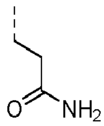 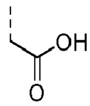 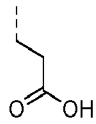

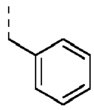 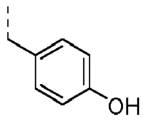 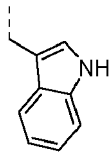 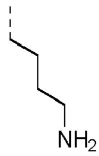 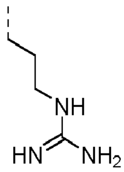

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (XLIX)

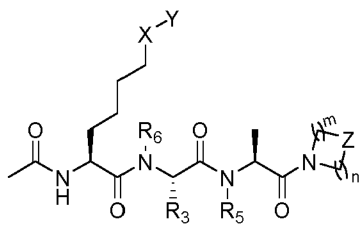

wherein $R_3$ is selected from

R3 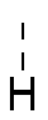 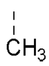 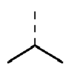 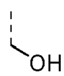 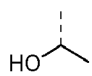 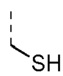 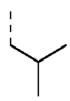

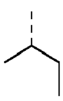 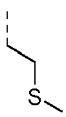 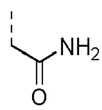 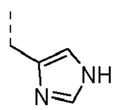 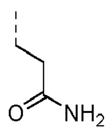 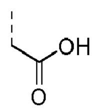 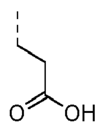

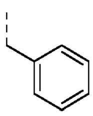 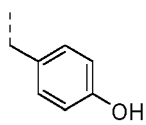 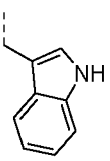 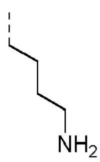 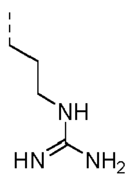

X is selected from NH-C=O, NH-C=S, O-C=O, C=O-O, $NH-SO_2$;

Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (L)

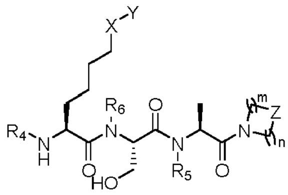

Wherein $R_4$ is selected from

$R_4$

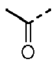 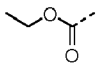 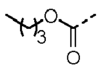 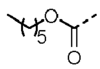 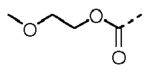 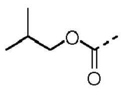 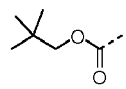

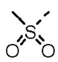 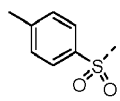 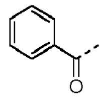 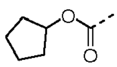 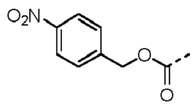 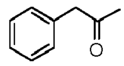 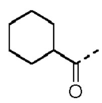

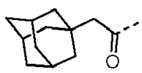 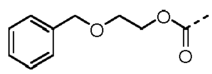 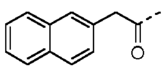 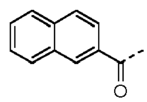 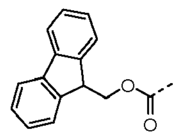

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (LI)

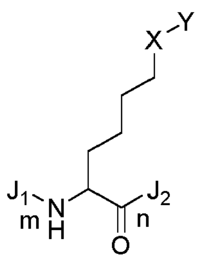

wherein X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

J1 and J2 is independently any α amino acid; and

m and n are each independently integers from 0 to 10, wherein at least one of m or n is not 0.

Formula (LIII)

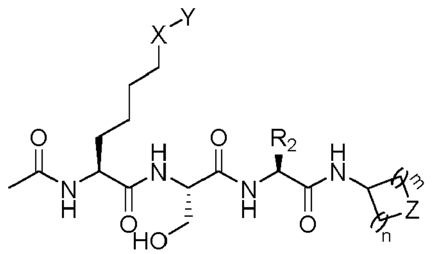

wherein X is NH-C=O, NH-C=S, O-C=O, C=O-O, NH-$SO_2$;

Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0 to 8, wherein at least one of m or n is not 0; and

Z is $CH_2$, O, NH, S, N-Me

$R_2$ 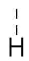 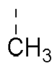 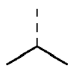 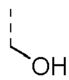 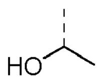 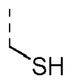 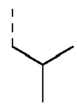

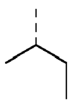 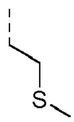 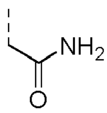 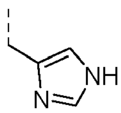 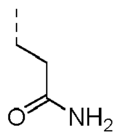 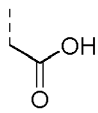 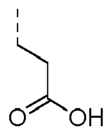

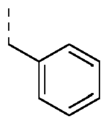 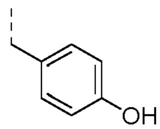 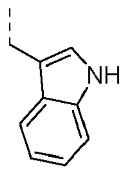 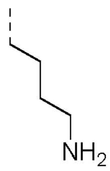 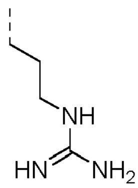

Formula (LIV)

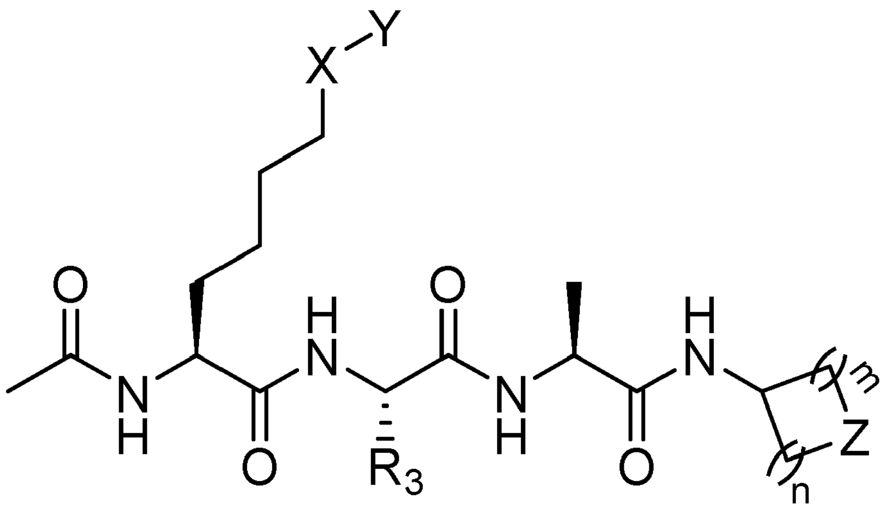

wherein X is NH-C=O, NH-C=S, O-C=O, C=O-O, NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 8, wherein at least one of m or n is not 0; and
Z is $CH_2$, O, NH, S, N-Me

$R_3$

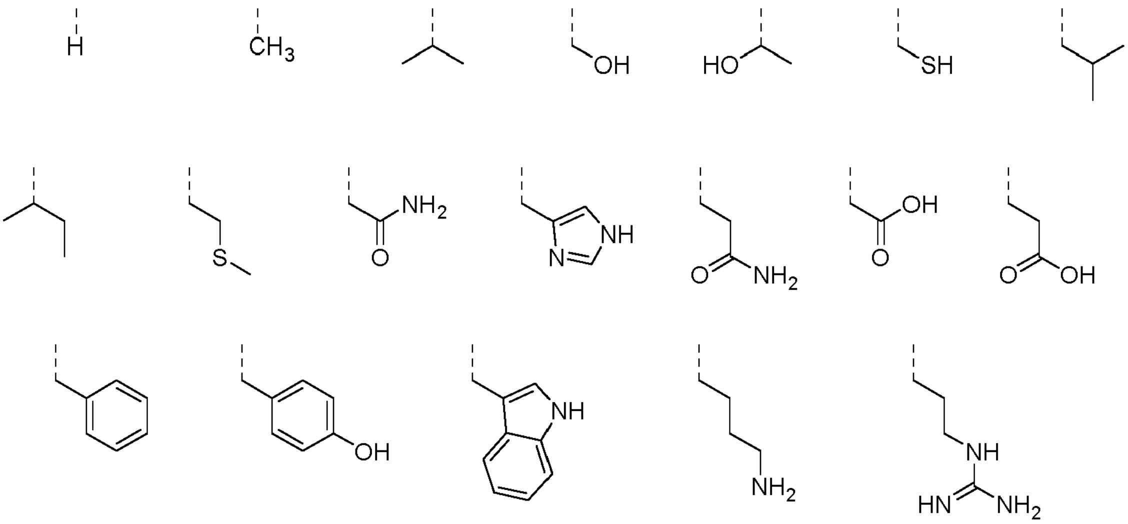

Formula (LV)

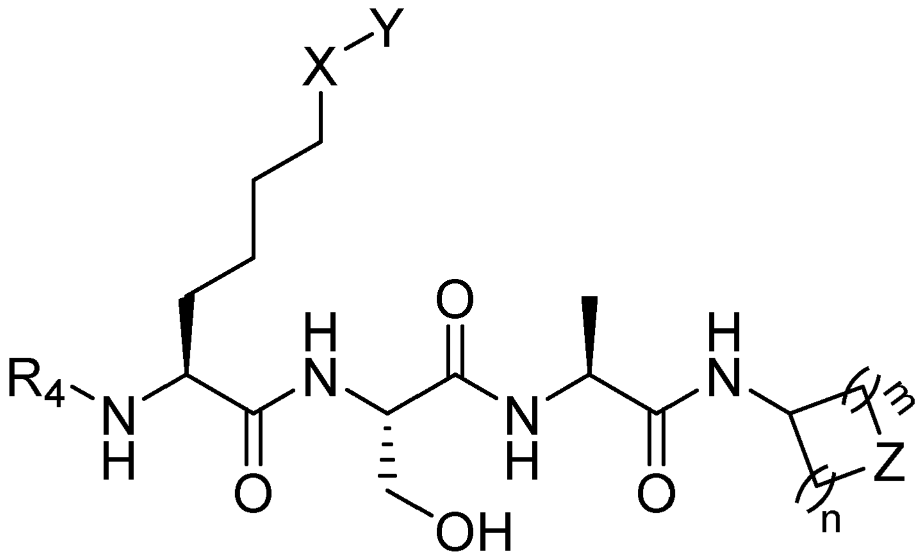

wherein X is NH-C=O, NH-C=S, O-C=O, C=O-O, $NH-SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 8, wherein at least one of m or n is not 0; and
Z is $CH_2$, O, NH, S, N-Me

$R_4$

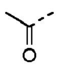 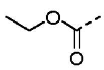 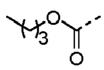 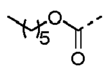 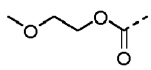 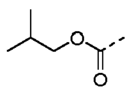 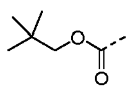

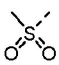 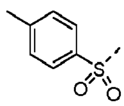 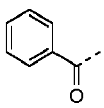 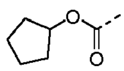 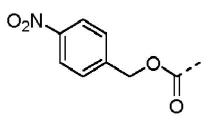 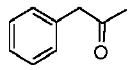 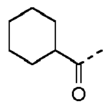

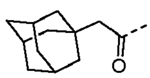 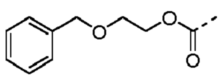 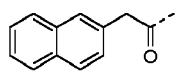 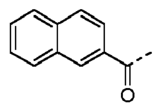 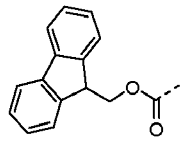

Formula (LVI)

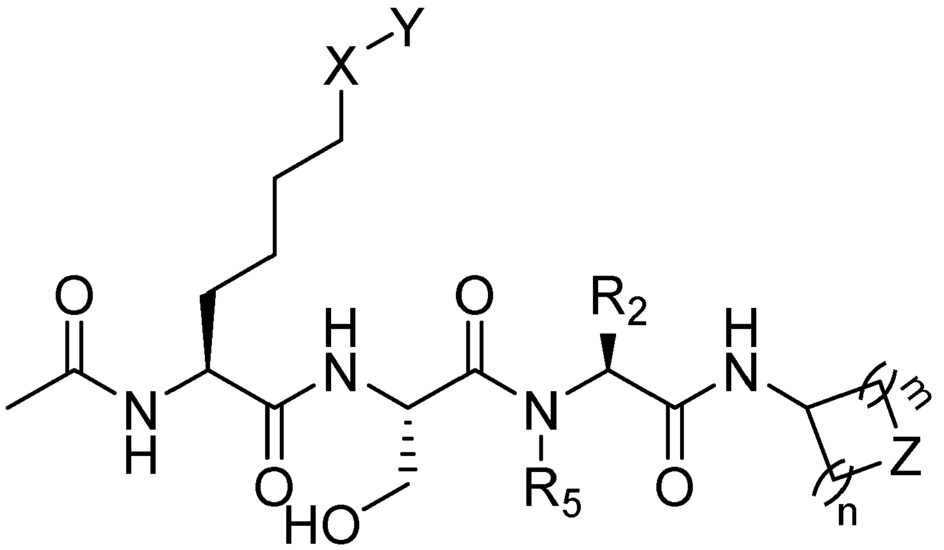

wherein X is NH-C=O, NH-C=S, O-C=O, C=O-O, $NH\text{-}SO_2$;

Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0-10;

Z is $CH_2$, O, NH, S, C=O, N; and

$R_5$ is H, Me, Et, Pr, or iPr

$R_2$ 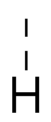 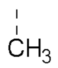 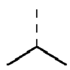 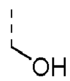 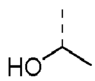 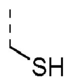 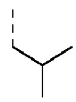

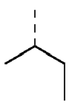 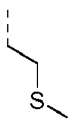 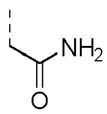 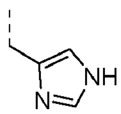 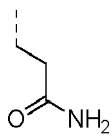 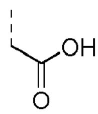 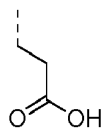

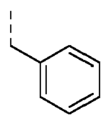 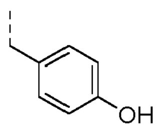 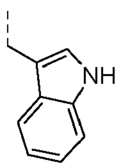 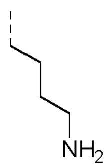 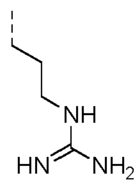

Formula (LVII)

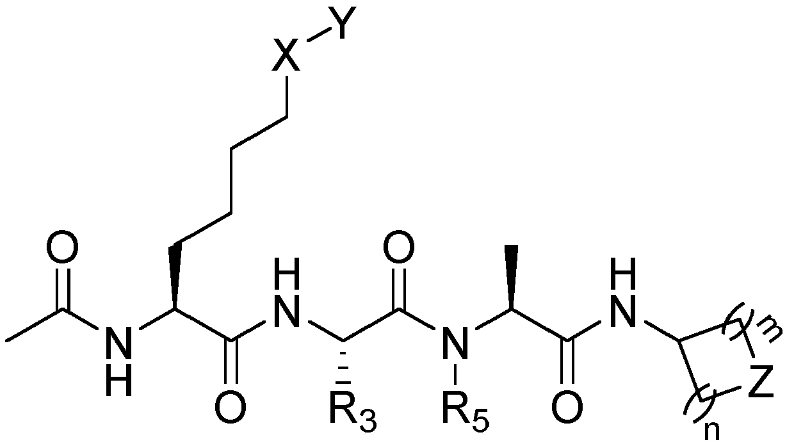

wherein X is NH-C=O, NH-C=S, O-C=O, C=O-O, $NH-SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S, C=O, N; and
$R_5$ is H, Me, Et, Pr, or iPr

$R_3$ 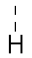 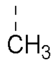 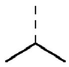 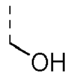 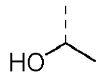 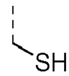 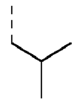

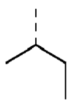 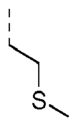 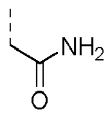 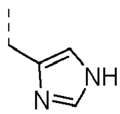 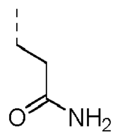 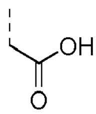 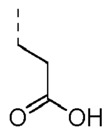

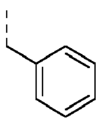 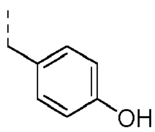 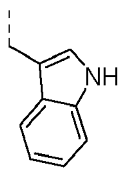 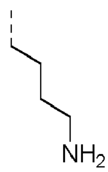 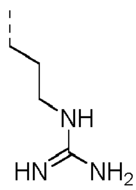

Formula (LVIII)

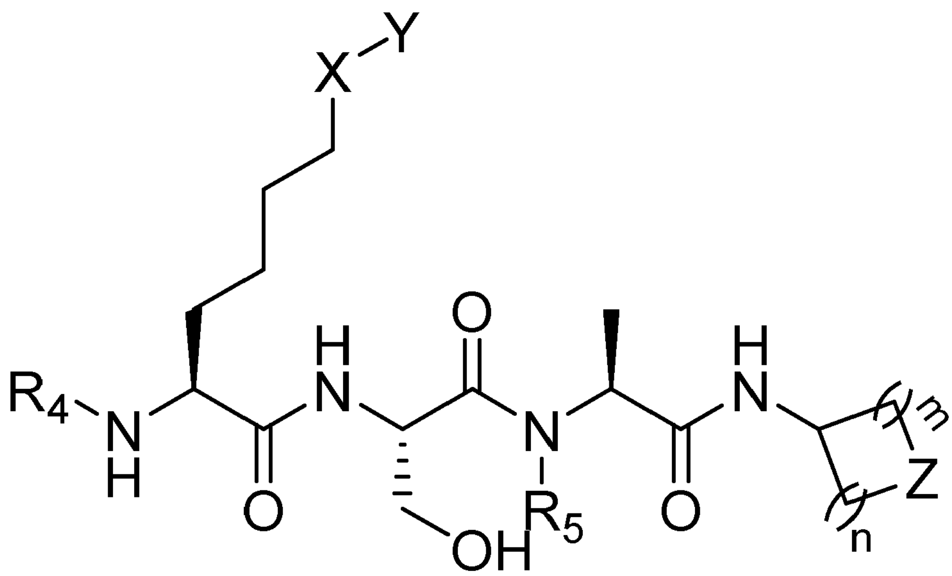

wherein X is NH-C=O, NH-C=S, O-C=O, C=O-O, $NH-SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S, C=O, N; and
$R_5$ is H, Me, Et, Pr, or iPr

$R_4$

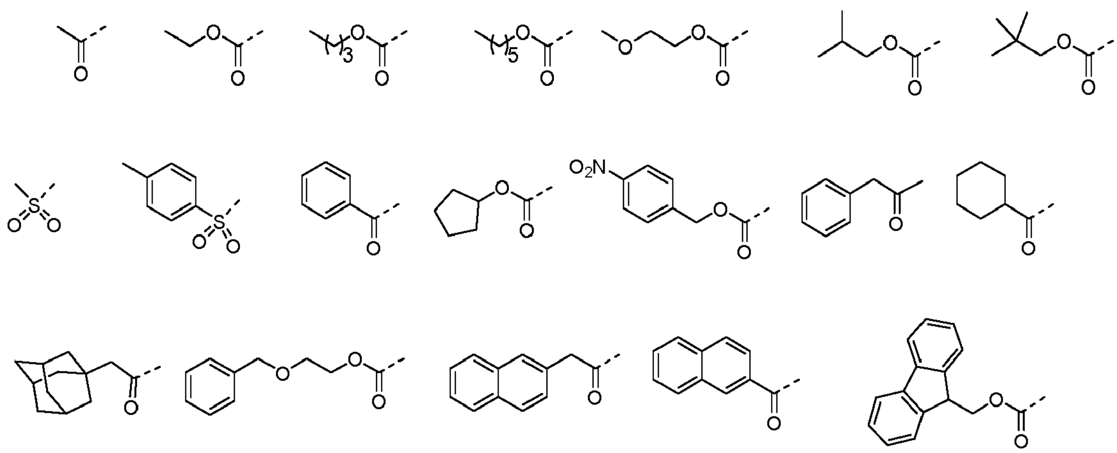

Formula (LIX)

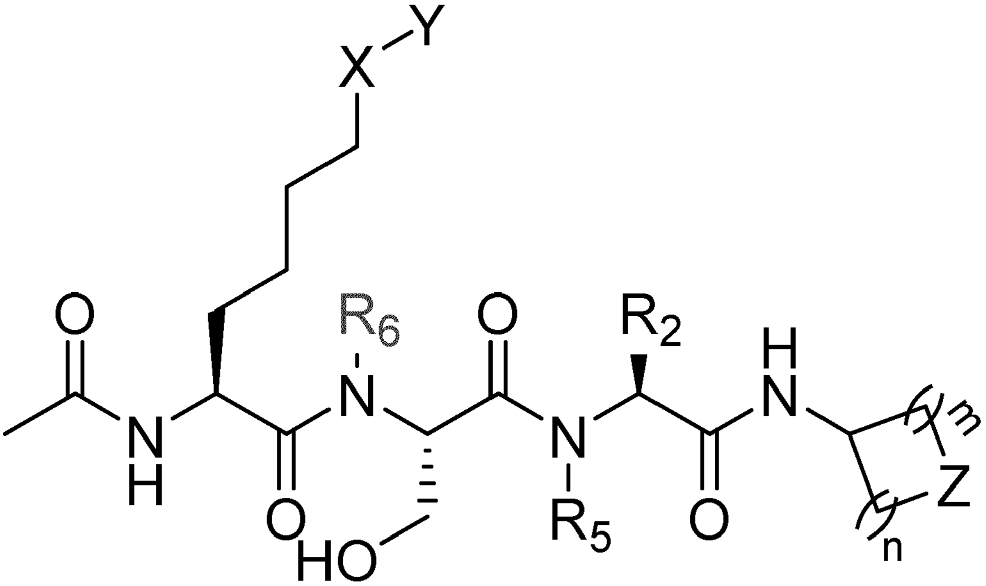

wherein X is NH-C=O, NH-C=S, O-C=O, C=O-O, $NH-SO_2$;

Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0-10;

Z is $CH_2$, O, NH, S,C=O, N;

$R_5$ is H, Me, Et, Pr, or iPr; and

$R_6$ is H, Me, Et, Pr, or iPr

$R_2$ 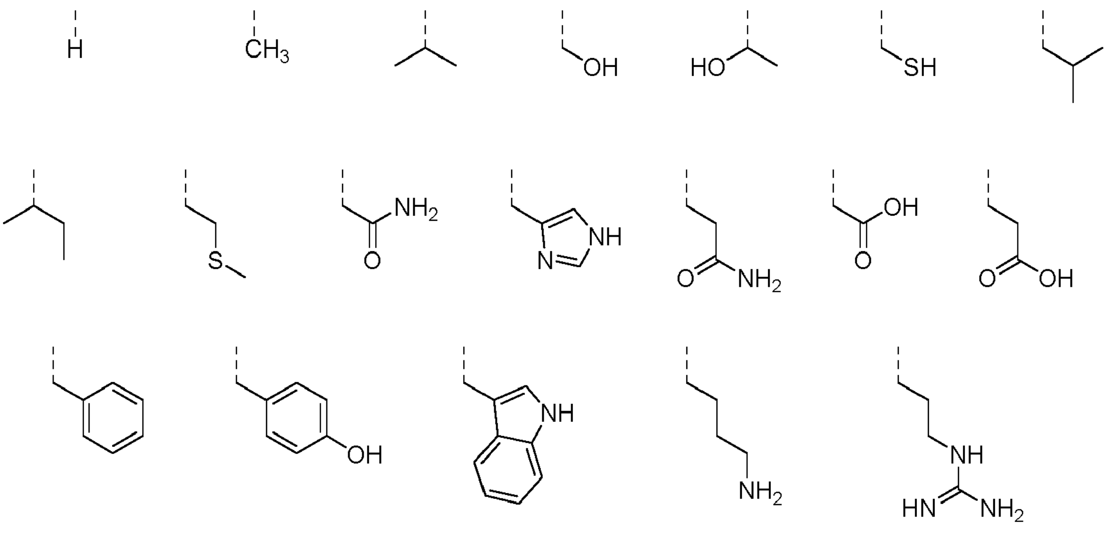

Formula (LX)

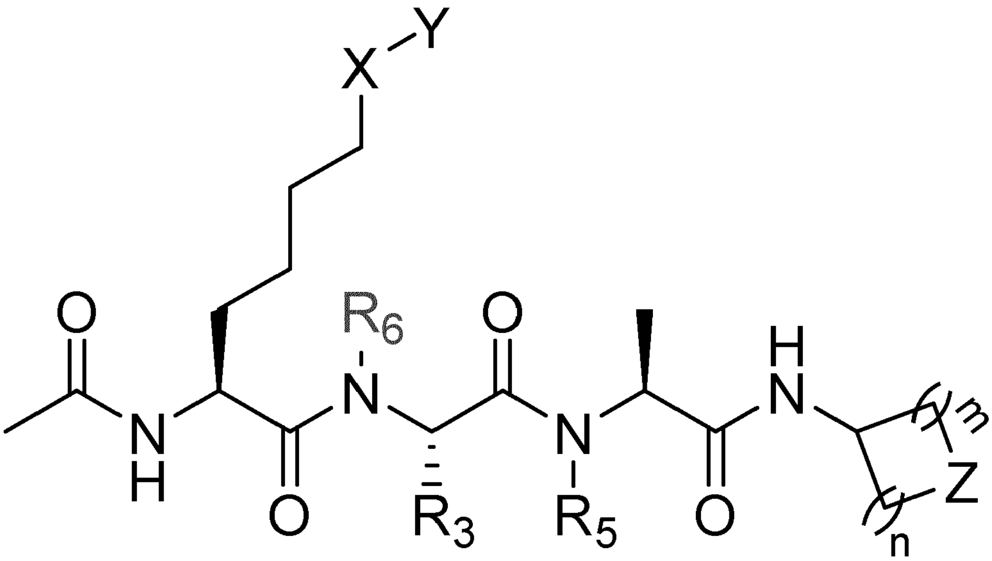

wherein X is NH-C=O, NH-C=S, O-C=O, C=O-O, NH-$SO_2$;

Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0-10;

Z is $CH_2$, O, NH, S,C=O, N;

$R_5$ is H, Me, Et, Pr, or iPr; and

$R_6$ is H, Me, Et, Pr, or iPr

$R_3$ 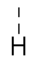 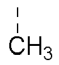 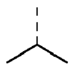 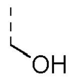 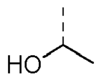 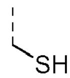 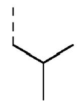

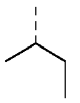 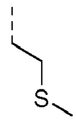 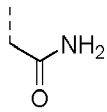 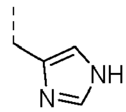 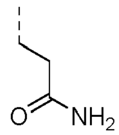 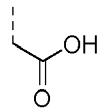 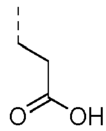

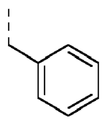 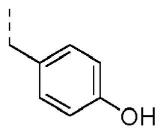 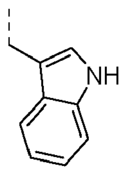 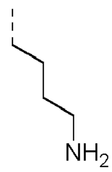 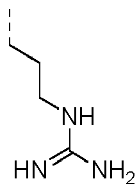

Formula (LXI)

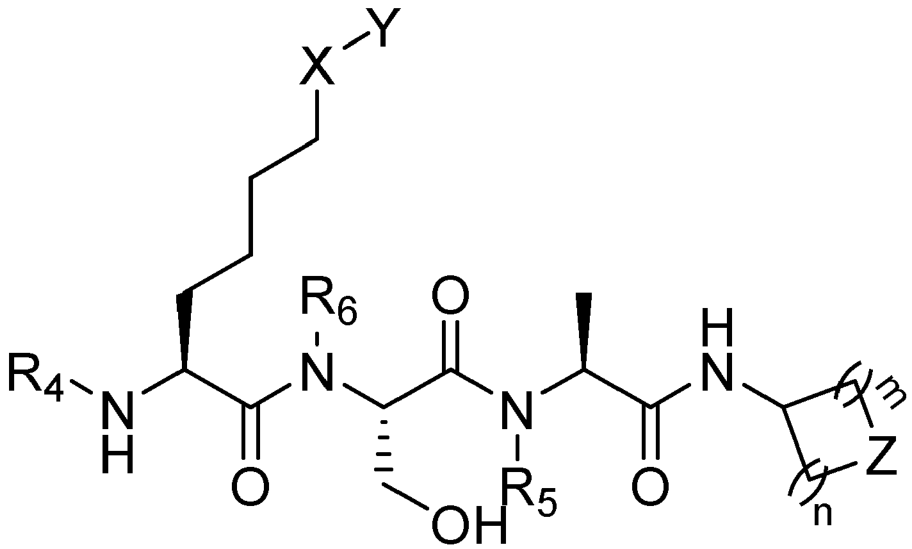

wherein X is NH-C=O, NH-C=S, O-C=O, C=O-O, NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S,C=O, N;
$R_5$ is H, Me, Et, Pr, or iPr; and
$R_6$ is H, Me, Et, Pr, or iPr

$R_4$ 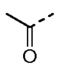 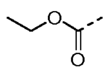 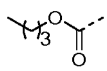 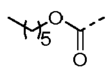 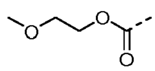 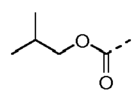 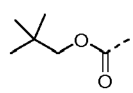

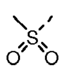 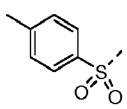 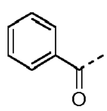 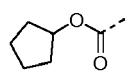 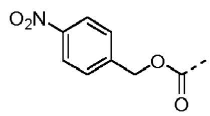 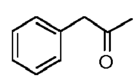 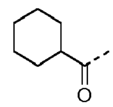

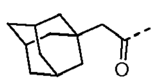 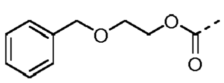 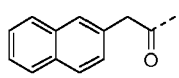 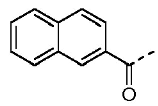 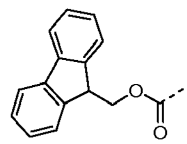

In preferred embodiments, the compositions and methods according to the subject invention utilize a YEATS2 YEAT inhibitor, such as, for example, an inhibitor according to formula (I). YEATS2 YEAT domain inhibitor compounds may be added to compositions at concentrations of 0.01 to 50% by weight (wt %), preferably 0.1 to 20 wt %, and more preferably 0.1 to 10 wt %. In certain embodiments, the YEATS2 YEAT inhibitor can be administered to a subject at a dosage of about 1 mg/kg to about 200 mg/kg.

In one embodiment, the subject compositions are formulated as an orally-consumable product, such as, for example a food item, capsule, pill, or drinkable liquid. An orally deliverable pharmaceutical is any physiologically active substance delivered via initial absorption in the gastrointestinal tract or into the mucus membranes of the mouth. The topic compositions can also be formulated as a solution that can be administered via, for example, injection, which includes intravenously, intraperitoneally, intramuscularly, intrathecally, or subcutaneously. In other embodiments, the subject compositions are formulated to be administered via the skin through a patch or directly onto the skin for local or systemic effects. The compositions can be administered sublingually, buccally, rectally, or vaginally. Furthermore, the compositions can be sprayed into the nose for absorption through the nasal membrane, nebulized, inhaled via the mouth

or nose, or administered in the eye or ear.

Orally consumable products according to the invention are any preparations or compositions suitable for consumption, for nutrition, for oral hygiene, or for pleasure, and are products intended to be introduced into the human or animal oral cavity, to remain there for a certain period of time, and then either be swallowed (e.g., food ready for consumption or pills) or to be removed from the oral cavity again (e.g., chewing gums or products of oral hygiene or medical mouth washes). While an orally-deliverable pharmaceutical can be formulated into an orally consumable product, and an orally consumable product can comprise an orally deliverable pharmaceutical, the two terms are not meant to be used interchangeably herein.

Orally consumable products include all substances or products intended to be ingested by humans or animals in a processed, semi-processed, or unprocessed state. This also includes substances that are added to orally consumable products (particularly food and pharmaceutical products) during their production, treatment, or processing and intended to be introduced into the human or animal oral cavity.

Orally consumable products can also include substances intended to be swallowed by humans or animals and then digested in an unmodified, prepared, or processed state; the orally consumable products according to the invention therefore also include casings, coatings, or other encapsulations that are intended to be swallowed together with the product or for which swallowing is to be anticipated.

In one embodiment, the orally consumable product is a capsule, pill, syrup, emulsion, or liquid suspension containing a desired orally deliverable substance. In one embodiment, the orally consumable product can comprise an orally deliverable substance in powder form, which can be mixed with water or another liquid to produce a drinkable orally-consumable product.

In some embodiments, the orally-consumable product according to the invention can comprise one or more formulations intended for nutrition or pleasure. These particularly include baking products (e.g., bread, dry biscuits, cake, and other pastries), sweets (e.g., chocolates, chocolate bar products, other bar products, fruit gum, coated tablets, hard caramels, toffees and caramels, and chewing gum), alcoholic or non-alcoholic beverages (e.g., cocoa, coffee, green tea, black tea, black or green tea beverages enriched with extracts of green or black tea, Rooibos tea, other herbal teas, fruit-containing lemonades, isotonic beverages, soft drinks, nectars, fruit and vegetable juices, and fruit or vegetable juice preparations), instant beverages (e.g., instant cocoa beverages, instant tea beverages, and instant coffee beverages), meat products (e.g., ham, fresh or raw sausage preparations, and seasoned or marinated fresh meat or salted meat products), eggs or egg products (e.g., dried whole egg, egg white, and egg yolk), cereal products (e.g., breakfast cereals, muesli bars, and pre-cooked instant rice products), dairy products (e.g., whole fat or fat reduced or fat-free milk beverages, rice pudding, yoghurt, kefir, cream cheese, soft cheese, hard cheese, dried milk powder, whey, butter, buttermilk, and partly or wholly hydrolyzed products containing milk proteins), products from soy protein or other soy bean fractions (e.g., soy milk and products prepared thereof, beverages containing isolated or enzymatically treated soy protein, soy flour containing beverages, preparations containing soy lecithin, fermented products such as tofu or tempeh products prepared thereof and mixtures with fruit preparations and, optionally, flavoring substances), fruit preparations (e.g., jams, fruit ice cream, fruit sauces, and fruit fillings), vegetable preparations (e.g., ketchup, sauces, dried vegetables, deep-freeze vegetables, pre-cooked vegetables, and boiled vegetables), snack articles (e.g., baked or fried potato chips (crisps) or potato dough products and extrudates on the basis of maize or peanuts), products on the basis of fat and oil or emulsions thereof (e.g., mayonnaise, remoulade, and dressings), other ready-made meals and soups (e.g., dry soups, instant soups, and pre-cooked soups), seasonings (e.g., sprinkle-on seasonings), sweetener compositions (e.g., tablets, sachets, and other preparations for sweetening or whitening beverages or other food). The present compositions may also serve as semi-finished products for the production of other compositions intended for nutrition or pleasure.

The subject composition can further comprise one or more pharmaceutically acceptable carriers, and/or excipients, and can be formulated into preparations, for example, solid, semisolid, liquid, or gaseous forms, such as tablets, capsules, powders, granules, ointments, solutions, suppositories, injections, inhalants, and aerosols.

The term "pharmaceutically acceptable" as used herein means compatible with the other ingredients of a pharmaceutical composition and not deleterious to the recipient thereof.

Carriers and/or excipients according the subject invention can include any and all solvents, diluents, buffers (such as, e.g., neutral buffered saline, phosphate buffered saline, or optionally Tris-HCl, acetate or phosphate buffers), oil-in-water or water-in-oil emulsions, aqueous compositions with or without inclusion of organic co-solvents suitable for, e.g., IV use, solubilizers (e.g., Polysorbate 65, Polysorbate 80), colloids, dispersion media, vehicles, fillers, chelating agents (e.g., EDTA or glutathione), amino acids (e.g., glycine), proteins, disintegrants, binders, lubricants, wetting agents, emulsifiers, sweeteners, colorants, flavorings, aromatizers, thickeners (e.g. carbomer, gelatin, or sodium alginate), coatings, preservatives (e.g., Thimerosal, benzyl alcohol, polyquaterium), antioxidants (e.g., ascorbic acid, sodium metabisulfite), tonicity controlling agents, absorption delaying agents, adjuvants, bulking agents (e.g., lactose, mannitol) and the like. The use of carriers and/or excipients in the field of drugs and supplements is well known. Except for any conventional media or agent that is incompatible with the target health-promoting substance or with the composition, carrier or excipient use in the subject compositions may be contemplated.

In one embodiment, the compositions of the subject invention can be made into aerosol formulations so that, for example, it can be nebulized or inhaled. Suitable pharmaceutical formulations for administration in the form of aerosols or sprays are, for example, powders, particles, solutions, suspensions or emulsions. Formulations for oral or nasal aerosol or inhalation administration may also be formulated with carriers, including, for example, saline, polyethylene glycol or glycols, DPPC, methylcellulose, or in mixture with powdered dispersing agents or fluorocarbons. Aerosol formulations can be placed into pressurized propellants, such as dichlorodifluoromethane, propane, nitrogen, fluorocarbons, and/or other solubilizing or dispersing agents known in the art. Illustratively, delivery may be by use of a single-use delivery device, a mist nebulizer, a breath-activated powder inhaler, an aerosol metered-dose inhaler (MDI), or any other of the numerous nebulizer delivery devices available in the art. Additionally, mist tents or direct administration through endotracheal tubes may also be used.

In one embodiment, the compositions of the subject invention can be formulated for administration via injection, for example, as a solution or suspension. The solution or suspension can comprise suitable non-toxic, parenterally-acceptable diluents or solvents, such as mannitol, 1,3-butanediol, water, Ringer's solution, or isotonic sodium chloride solution, or suitable dispersing or wetting and suspending agents, such as sterile, non-irritant, fixed oils, including synthetic mono- or diglycerides, and fatty acids, including oleic acid. One illustrative example of a carrier for intravenous use includes a mixture of 10% USP ethanol, 40% USP propylene glycol or polyethylene glycol 600 and the balance USP Water for Injection (WFI). Other illustrative carriers for intravenous use include 10% USP ethanol and USP WFI; 0.01-0.1% triethanolamine in USP WFI; or 0.01-0.2% dipalmitoyl diphosphatidylcholine in USP WFI; and 1-10% squalene or parenteral vegetable oil-in-water emulsion. Water or saline solutions and aqueous dextrose and glycerol solutions may be preferably employed as carriers, particularly for injectable solutions. Illustrative examples of carriers for subcutaneous or intramuscular use include phosphate buffered saline (PBS) solution, 5% dextrose in WFI and 0.01-0.1% triethanolamine in 5% dextrose or 0.9% sodium chloride in USP WFI, or a 1 to 2 or 1 to 4 mixture of 10% USP ethanol, 40% propylene glycol and the balance an acceptable isotonic solution such as 5% dextrose or 0.9% sodium chloride; or 0.01-0.2% dipalmitoyl diphosphatidylcholine in USP WFI and 1 to 10% squalene or parenteral vegetable oil-in-water emulsions.

In one embodiment, the compositions of the subject invention can be formulated for administration via topical application onto the skin, for example, as topical compositions, which include rinse, spray, or drop, lotion, gel, ointment, cream, foam, powder, solid, sponge, tape, vapor, paste, tincture, or using a transdermal patch. Suitable formulations of topical applications can comprise in addition to any of the pharmaceutically active carriers, for example, emollients such as carnauba wax, cetyl alcohol, cetyl ester wax, emulsifying wax, hydrous lanolin, lanolin, lanolin alcohols, microcrystalline wax, paraffin, petrolatum, polyethylene glycol, stearic acid, stearyl alcohol, white beeswax, or yellow beeswax. Additionally, the compositions may contain humectants such as glycerin, propylene glycol, polyethylene glycol, sorbitol solution, and 1,2,6 hexanetriol or permeation enhancers such as ethanol, isopropyl alcohol, or oleic acid.

In certain embodiments, a probe can be bound to YEATS2 protein. In certain embodiments, the probe is according to formula (LII):

Formula (LII)

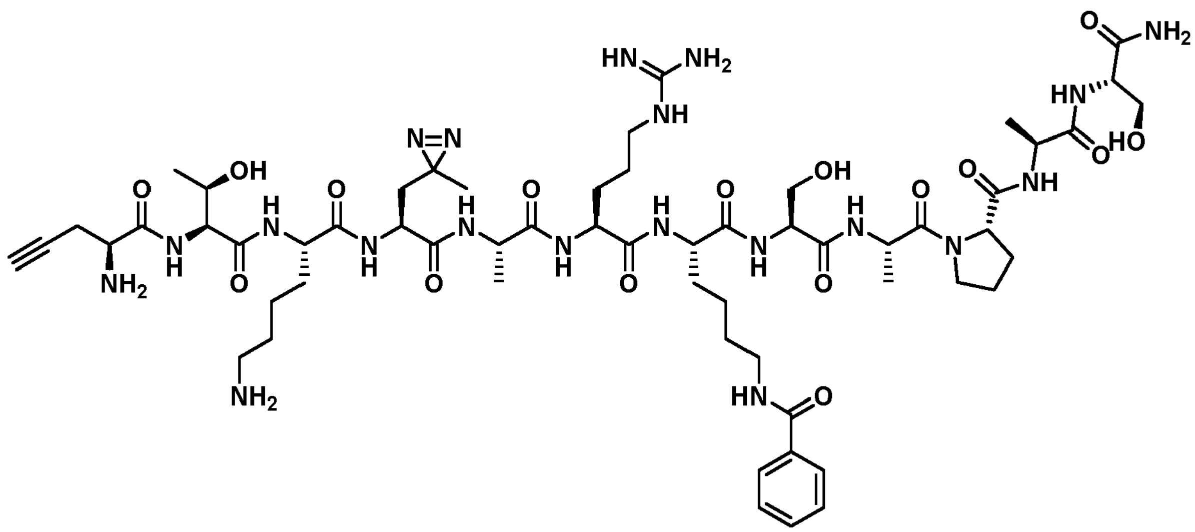

### Methods of Using Compounds of the Subject Invention

In certain embodiments, the YEATS2 YEAT inhibitor, according to formula (I), or derivatives thereof can be administered to a subject. Any means of administration that can permit an inhibitor to contact cells, including, for example, orally, intravenously, intraperitoneally, intramuscularly, intrathecally, or subcutaneously are envisioned in the subject methods.

In certain embodiments, the YEATS2 YEAT inhibitor can contact healthy cells of subject and/or tumor cells or cancerous cells. In certain embodiments, the YEATS2 YEAT inhibitor can contact cells in the lung, including cancerous lung cells. In certain embodiments, the YEATS2 YEAT inhibitor can inhibit non-small cell lung cancer, pancreatic cancer, ovarian cancer, liver cancer, and other solid tumors that have elevated YEATS2 expression levels. The YEATS2 YEAT inhibitor can permeate the cells of a subject, including, for example, cancerous cells.

In certain embodiments, YEATS2 YEAT inhibitor can target the lysine acetylation (Kac) binding pocket of YEATS2. YEATS2 YEATS domain inhibitors can target YEATS2 by occupying the lysine acetylation (Kac) binding pocket.

In certain embodiments, the YEATS2 YEAT inhibitor can recognize epigenetic modifications. YEATS2 YEATS domain inhibitors can disrupt YEATS2-H3K27ac interaction by targeting their binding pocket. In certain embodiments, the modification can be to a histone, including, for example, acetylation of the histone. In preferred embodiments, YEATS2 YEAT inhibitor can recognize the H3K27ac histone modification.

MATERIALS AND METHODS

### Protein expression and purification

$YEATS2_{201-332}$ containing an N-terminal 10×His-SUMO tag was overexpressed in *Escherichia coli* BL21 (DE3). After overnight induction by 0.4mM isopropyl β-D-thiogalactoside at 16 °C, cells were collected and suspended in buffer: 20mM Tris, pH 7.5, 0.5M NaCl, 5% glycerol, 1mM phenylmethylsulfonyl fluoride, and 20mM imidazole. After cell lysis and centrifugation, the recombinant protein was purified to homogeneity over HisTrap, and the 10xHis-SUMO tag was cleaved by ULP1 overnight at 4 °C then removed by reloaded onto the HisTrap column. The free $YEATS2_{201-332}$ protein was finally polished by size-exclusion chromatography on a Superdex G75 column (GE Healthcare, Chicago, IL) in elution buffer: 20mM Tris, pH 7.5, 0.5M sodium citrate, 5% glycerol.

### Photo-cross-linking

The probes in the absence or presence of different concentrations of competitors were incubated with recombinant proteins (5 or 20 ng/μL) in binding buffer (50 mM HEPES, 150 mM NaCl, 2 mM $MgCl_2$, 0.1% Tween-20, 20% glycerol, pH 7.5, with 50 ng/μL BSA) for 10 min at 4 °C. Then, the samples were irradiated at 365 nm using UV lamp for 20 min in 96-well on ice.

### Cu(I)-catalyzed azide-alkyne cycloaddition (click chemistry)

To the prepared photo-cross-linking samples, we added 100 μM rhodamine-N3 for in-gel fluorescence (10 mM stock in DMSO), followed by 1 mM TCEP (freshly prepared 50 mM stock in $H_2O$) and 100 μM TBTA (10 mM stock in DMSO); finally, the reactions were initiated by the addition of 1 mM $CuSO_4$ (freshly prepared 50 mM stock in $H_2O$). The reactions were incubated for 1 h at room temperature (22 to 25 °C) with regular vortexing. The reactions were quenched by adding 5 volumes of ice-cold acetone and placed at - 20 °C overnight to precipitate proteins.

### In-gel fluorescence scanning

Samples after protein precipitation were centrifuged at 13,000 × *g* for 5 min at 4 °C. The supernatant was discarded, and the pellet was washed with ice-cooled acetone and air-dried for 10 min. The proteins were resuspended in 1 × LDS sample loading buffer (Invitrogen, Waltham, MA) with 50 mM DTT and heated at 95 °C for 8 min. Samples were then resolved by SDS-PAGE. The labeled proteins were visualized by scanning the gel on a Typhoon 9410 variable mode imager (excitation 532 nm, emission filter 580 nm).

### Isothermal titration calorimetry measurement

Experiments were performed at 15 °C on a MicroCal iTC200 titration calorimeter (MicroCal, Northampton, MA) in buffer (20mMTris, pH 7.5, 0.5M sodium citrate, 5% glycerol). The reaction cell contained 280 μL of 30-100 μ M proteins, which was titrated with corresponding peptides (ten-fold higher than the protein concentration used). Each of

the titration contained 20 injections. For every first injection, the volume was 0.4 μ L. For the other injections, the volume was 2 μL. The binding isotherm was fit with Origin 7.0 software package (OriginLab, Northampton, MA) using a single set of independent sites to determine the thermodynamic dissociation constants and stoichiometry.

**Cellular thermal shift assay (CETSA)**

H1299 cells were seeded in 150 mm dish at density of 1 × $10^5$ cells/mL. On the next day, cells were washed with PBS and incubated with 20 μM **MsK$_{bf}$SAP** or DMSO in culture medium for 16 h. Cells were then detached by cell scraper and washed with PBS once. The cell pellets were resuspended in PBS with 1× Roche Complete EDTA-free protease inhibitors (approximately 0.5 million cells in 100 μL). The cells (100 μL per PCR tube) were then heated at indicated temperatures for 3 min in a Veriti thermal cycler (Thermo Fisher Scientific, Waltham, MA), followed by incubation at room temperature for 3 min. Samples were subjected to two freeze-thaw cycles to lyse the cells. The cell lysate was clarified by centrifugation at 20,000×g at 4 °C for 30 min, and the resulting supernatant were subjected to immunoblotting analysis.

**Fluorescence recovery after photobleaching (FRAP)**

U2OS cells were seeded in Nunc™ Glass Bottom Dishes (35 mm, ThermoFisher) and transfected with plasmids for the expression of AF9- or ENL-sfGFP fusion protein, using Effectene Transfection Reagent (QIAGEN, Hilden, Germany). Suberanilohydroxamic acid (SAHA, 2.5 μM) was added where required during the transfection and the FRAP analysis was conducted 24 h after the transfection. Right before FRAP, medium was replaced to phenol red-free DMEM medium, supplemented with 10% FBS, 25 mM HEPES and the inhibitors at indicated concentration. The FRAP analysis was performed with Carl Zeiss LSM710 NLO coupled to an inverted Zeiss Axio Observer.Z1 microscope equipped with a high numerical aperture (NA 1.3) 40× oil-immersion objective and a heated chamber set at 37 °C. Bleaching of sfGFP fluorescence and imaging were carried out with argon ion laser (488 nm, 25 mW, 100% power for bleaching, 1% power for imaging at pinhole diameter 3 Airy units) and the photomultiplier tube detector was set to detect fluorescence between 500 and 600 nm.

Cells with very low expression levels of sfGFP-tagged protein requiring high gain setting for imaging were particularly susceptible to photobleaching and often resulting noisy images and were therefore, excluded from the selection for FRAP analysis. Cells with very high expression levels of sfGFP-tagged protein often exhibited aberrant morphology and were also excluded from the selection for FRAP analysis. Thus, only cells with moderate sfGFP fluorescence within the gain range of 650 to 800 were chosen for FRAP analysis.

Photobleaching was performed in a circular region with area size of 5 $mm^2$. A time-lapse series was then taken to record sfGFP fluorescence recovery. The time-lapse images were acquired with a frame size of 512 pixels × 512 pixels with bit depth of 12, bidirectional scanning, scanning speed of 14 and a zoom factor of 14, which allowed for a time interval time of approximately 0.15 s and pixel dwelling time of 0.5 ms.

Images were collected and fluorescence intensity of the selected regions was quantified using the Carl Zeiss Zen Black software. Fluorescence intensity was measured for three regions: 1) bleach region ($F(t)_{ROI}$), 2) unbleached region ($F(t)_{REF}$) and 3) a region outside the cell to determine the background signal ($F(t)_{BG}$). The relative fluorescence intensity ($F(t)_{NORM}$) of the bleached region was calculated for each time point (t) with equation 1, where F(i) is the mean intensity of a region in the ten prebleach scans.

$$F(t)_{NORM} = \frac{F(t)_{ROI} - F(t)_{BG}}{F(t)_{REF} - F(t)_{BG}} \times \frac{F(i)_{REF} - F(i)_{BG}}{F(i)_{ROI} - F(i)_{BG}}$$

**Equation 1**

The normalized data were fitted to double exponential association curves using Two phase association function, which has the formula shown in equation 2.

$$y = y0 + A1(1 - e^{-x/t1}) + A2(1 - e^{-x/t2})$$

**Equation 2**

Using parameters returned by the curve-fitting, the fluorescence intensity corresponding to half-recovery was

calculated using equation 3.

$$y_{1/2} = y0 + \frac{A1 + A2}{2}$$

**Equation 3**

An iterative bisection algorithm was then used to determine the value of $t_{1/2}$ (that is the time at which fluorescence recovered by half) in equation 2 when $y = y_{1/2}$.

Cells where curve-fitting gave plateaus >1 or $R^2$ <0.95 were removed from further analysis as imaging artefacts.

### Chromatin immunoprecipitation and quantitative PCR (ChIP-qPCR)

Chromatin immunoprecipitation was performed as previously reported (Erb MA, Scott TG, Li BE, Xie H, Paulk J, Seo HS, Souza A, Roberts JM, Dastjerdi S, Buckley DL, Sanjana NE, Shalem O, Nabet B, Zeid R, Offei-Addo NK, Dhe-Paganon S, Zhang F, Orkin SH, Winter GE, Bradner JE. Transcription control by the ENL YEATS domain in acute leukaemia. Nature. 2017 Mar 9;543(7644):270-274.) with minor modifications. HeLa cells were seeded on 150 mm dish with approximately 60% confluency. The next day, cells were treated with 20 μM $MsK_{bf}SAP$ or DMSO for 24 h. Cells were then cross-linked for 10 min at room temperature by direct addition of 1/10 volume of 10× crosslinking solution (11% formaldehyde, 50 mM HEPES pH 7.3, 100 mM NaCl, 1 mMEDTA, 0.5 mMEGTA), and 0.125 M glycine was added to quench the cross-linking for 5 min. Cells were then washed three times with PBS and were resuspended in ice-cold ChIP lysis buffer 1 (LB1; 1 ml per 10 million cells; 50 mM HEPES pH 7.3, 140 mM NaCl, 1 mM EDTA, 10% glycerol, 0.5% NP-40, 0.25% Trition X-100, 1× Roche Complete EDTA-free protease inhibitors, Roche, Basel, Switzerland) and rotated for 20 min at 4 °C. After centrifugation, the pellet was resuspended in ice-cold ChIP lysis buffer 2 (LB2; 1 ml per 10 million cells; 10 mM Tris-HCl pH 8.0, 200 mM NaCl, 1 mM EDTA, 0.5 mM EGTA, 1 × Roche Complete EDTA-free protease inhibitors) and rotated for 20 min at 4 °C. After centrifugation, the pellet was suspended in ice-cold sonication buffer (0.3 ml per 10 million cells; 50 mM HEPES, pH 7.3, 140 mM NaCl, 1 mM EDTA, 1 mM EGTA, 1% Triton X-100, 0.1% Na-deoxycholate, 0.1% SDS, 1× Roche Complete EDTA-free protease inhibitors). Samples were sonicated to a chromatin ranging from 600 bp to 800 bp using a VCX750 ultrasonic processors (Sonics & Materials; 2 mm stepped microtip; 30% amplitude; 1 second on, 3 seconds off for 15 min). The chromatin was then cleared by centrifugation. For chromatin quantification, an aliquot (around 1/10 volume) of cleared chromatin was reverse cross-linked for 20 min at 95 °C and treated with 0.2 mg/mL RNase (Thermo Fisher Scientific) for 20 min 37 °C. DNA was recovered using QIAquick PCR Purification Kit (QIAGEN) and quantified by NanoDrop spectrophotometers (Thermo Fisher Scientific). 50 μg chromatin (set 5 μg chromatin as input sample) was used for one assay. Pierce™ ChIP-grade Protein A/G Magnetic Beads (Thermo Fisher Scientific; 25 μl suspension per assay) were washed three times with cold blocking buffer (5 mg/mL BSA in PBS pH 7.4). Washed beads were incubated with anti-flag antibody.

### Colony formation assay

A549 and H1299 cells were seeded in 6-well tissue culture plates (200 cells/well) and grown for 14 days. Colonies were fixed with glutaraldehyde (6.0% v/v), stained with crystal violet (0.5% w/v) and photographed.

### Cell Growth Inhibition Assay

A total of 2000 cells were seeded in a 96-well plate in 100 μL of the medium, treated with DMSO or compounds at indicated concentrations for 3 days. Cell viability was measured using the ATPlite 1step Luminescence Assay System (PerkinElmer) according to the manufacturer's instructions. Surviving cells were calculated as % relative to DMSO-treated cells.

### Subcutaneous tumor model

For the subcutaneous injection model, $4\times10^6$ H1299 non-small cell lung carcinoma cell line cells were injected subcutaneously into 5-week BABL/c nude mice. One week after H1299 xenograft tumor implantation, mice were randomly grouped into two groups according to body weight. For drug treatment, two groups of mice were administered with the vehicle with either DMSO or 200mg/kg LS-1-124 daily for 26 days, through intraperitoneal injection. Mouse body weight and tumor size were recorded once every two days. Tumor size was measured with caliper and estimated with the formula:

$$Tumor\ size = \frac{\pi}{6} \times Length \times Width \times Height$$

Animal experiments were all carried out according to the Control of Hong Kong Animal Experiment Ordinance and the Institute's principles on animal works. The protocols of the animal experiments were justified and approved by University of Hong Kong Committee on the Use of Live Animals in Teaching and Research (CULATR) and were done under Centre for Comparative Medicine Research (CCMR) guidance and monitoring.

**Histochemistry analysis**

Tumor tissues were cut and immersion-fixed with 4% formaldehyde overnight. Paraffin-embedded sections were then prepared with standard protocol. For H&E staining, the sectioning slides were stained by The University of Hong Kong, Department of Pathology. In brief, paraffin sections were deparaffinized and rehydrated with xylene and ethanol gradient. After deionized water washing, slide will be stained by Haematoxylin and Eosin, then dehydrated with ethanol gradient and xylene. For Mki67 immunohistochemical analyses were performed using the alkaline phosphatase antigen retrieval method after paraffin section were dewaxed. The Mki67 (Abcam) antibody was used for incubating at diluted 1:200 overnight at 4°C. After washing with TBST, the section slides were incubated with horseradish peroxidase-conjugated secondary antibody (Dako) for 1 hour. The samples were developed with 3,3'-diaminobenzidine (DAB, Sigma), stained with Haematoxylin, and dehydrated with ethanol gradient and xylene before coverslip mounting. Further, the mounted slide will be scanned by Hamamatsu NanoZoomer S210.

All patents, patent applications, provisional applications, and publications referred to or cited herein are incorporated by reference in their entirety, including all figures and tables, to the extent they are not inconsistent with the explicit teachings of this specification.

Following are examples that illustrate procedures for practicing the invention. These examples should not be construed as limiting. All percentages are by weight and all solvent mixture proportions are by volume unless otherwise noted.

EXAMPLES

EXAMPLE 1-YEATS2 YEATS INHIBITOR

YEATS domain of YEATS2 is newly identified as histone benzoylation reader. YEATS2 YEATS preferentially recognizes H3K27bz (Kd = 21.57 μM) with higher binding affinity than H3K27cr (Kd = 37.38 μM) and H3K27ac (Kd = 131.13 μM). Based on the decapeptide ($H3_{22-32}K27bz$), we designed a photoaffinity probe H3K27bz-Dzyne (**FIG. 1A**) in which the Ala24 was replaced by a diazirine-containing photo reactive amino acid (photo-leu). The probe also had a N-terminal alkyne-containing amino acid (propargyl glycine) to bioorthogonally conjugate with fluorescent tags for visualizing the captured proteins. If the inhibitor can occupy the same binding pocket with probe, the fluorescent intensity would decrease **(FIG. 1B).**

As shown in **FIG. 1C,** photo-H3K27bz robustly labeled YEATS2 in a concentration-dependent manner and has higher labeling efficiency than photo-H3K27cr under the same concentration. We then tested the inhibitory activity of native H3K27cr and H3K27bz competitors derived from histone H3 (residue 22-32) towards YEATS2 (**FIGs. 1D-1E**), showed $IC_{50}$ of 196.3 μM and 100.4 μM, respectively.

To identify competitors with higher potency, we tested a series of peptides derived from histone H3 (residues) with lys27 carrying different π system-containing functional groups (**FIG**. **2A**) by three-spot competitive photo-cross-linking assay (**FIG. 2B**). From all the tested π systems, benzofuran stood out with $IC_{50}$ of 3.25 μM, much stronger inhibitory effect than native peptide H3K27bz toward YEATS2 (**FIG. 2C**).

We next sought to optimize the inhibitors with shorter length and higher potency. In YEATS2-H3K27cr complex crystal structure, the Kcr group clamps by Y262 and W282 and the motif "Kcr27-S28-A29-P30-A31" of H3 mainly contributes to their binding. Notably, the H3P30 inserts into a hydrophobic pocket of YEATS2 to facilitate H3K27cr coordination. Therefore, we synthesized four small peptides with one by one deleting amino acid from the C-terminus, capping the N-terminus of peptide with hydrophobic acetyl group (**FIG. 3A**). Compared with H3K27bf, $AcK_{bf}SA$ (**FIGs. 3B-3C** $IC_{50}$=32.1μM) and $AcK_{bf}S$ ($IC_{50}$=58.8 μM) showed a dramatically reduced inhibitory activity, while $AcK_{bf}SAPA$ ($IC_{50}$=7.4μM) and $AcK_{bf}SAP$ ($IC_{50}$=6μM) exhibited the similar effect toward YEATS2, only 2-fold decrease than H3K27bf, which also uncovered the N-terminal residues "TKAAR" slightly impact their interaction.

Based on the tetrapeptide "AcKbfSAP", we sought to optimize the sequence by replacing the amino acid at position **1-3** (**FIG. 4A**) and carrying different protecting groups at N-terminus. The 75 unpurified tetrapeptides were screened by three-spot competitive photo-cross-linking assay (**FIG. 4B**). When **P** was replaced by other natural amino

acids, the inhibitory activity dramatically decreased, indicating **P** was conserved at position **1.** For the optimization at position **2** and **3,** the potency was slightly changed. Notably, **4-12** ($IC_{50}$=1.1μM) with methanesulfonyl substitution significantly enhanced inhibitory activity.

Next, dozens of benzofuran derivatives were synthesized and evaluated, 6-bromo-2-benzonfuran and 6-chloro-2-benzofuran derivatives stood out from the heatmap (**FIG. 4D**). We then generated $MsK_{bf}SAP$, $MsK_{br\text{-}bf}SAP$ and $MsK_{cl\text{-}bf}SAP$ **(FIGs. 5A-5I),** $MsK_{br\text{-}bf}SAP$ ($IC_{50}$=0.23 μM) showed 2.7-fold increase than $MsK_{bf}SAP$ ($IC_{50}$=0.61 μM). $MsK_{cl\text{-}bf}SAP$ ($IC_{50}$=0.61 μM) was comparable with $MsK_{bf}SAP$.

The dissociation constant determined by isothermal titration calorimetry **(FIGs. 6A-6H)** exhibited the consistent result with their corresponding potency against YEATS2 YEATS domain. Two native peptides H3K27cr ($K_d$= 32 μM) and H3K27bz ($K_d$= 30 μM) showed the similar binding affinity. The replacement of the benzoyl group by benzofuran group ($K_d$= 2.54 μM) led to 12.6-fold binding enhancement, shortening to $AcK_{bf}SAP$ ($K_d$= 1.2 μM) also slightly increased the binding potency. Capping with methanesulfonyl group ($K_d$= 0.19 μM) at N-terminus largely tightened the interaction, 168.4-fold binding enhancement than native peptide H3K27cr. Bromo ($K_d$= 0.089 μM) and chloro ($K_d$= 0.093 μM) substituted on benzofuran produced higher binding affinity toward YEATS2 YEATS domain.

Encouraged by the *in-vitro* activity, we next applied $MsK_{br\text{-}bf}SAP$ into living cells. The cellular thermal shift assay was performed to test whether the inhibitor can target the endogenous YEATS2 protein. H1299 cells treated with or without 20 μM $MsK_{br\text{-}bf}SAP$ were heated at different temperatures, then the soluble portion were extracted and analyzed by western blot. As shown in **FIG. 7A,** $MsK_{br\text{-}bf}SAP$ obviously enhanced the stability of YEATS2 at several temperatures (such as 46.5°C and 47 °C).

To evaluate the ability of inhibitor to disrupt the chromatin association of YEATS2, a fluorescence recovery after photo-bleaching (FRAP) assay was performed. 3-YEATS2 YEATS domains fusing with GFP protein overexpressed in U2OS cells. Compared with DMSO treated sample, 20 μM $MsK_{br\text{-}bf}SAP$ treated one allows significant increase of recovery time after photo bleaching, suggesting the mobility of 3-YEATS2-YEATS-GFP rose by replacing acylated chromatin with our inhibitor(FIGs. 7BC).

To explore the effect of inhibitor on gene regulation, we carried out chromatin immunoprecipitation and quantitative PCR (ChIP-qPCR) in H1299 (stably expressing 3×Flag-YEATS2 protein). It is known that YEATS2 is enriched on individual ribosomal genes (RP7, RPL8, RPL35 and RPL38). The gene abundance was reduced upon $MsK_{br\text{-}bf}SAP$ treatment, indicating the inhibitor could block YEATS2-dependent enrichment on target genes(FIG.7D).

Cancer cells usually loss of growth control by unlimited division. We next sought to test whether our inhibitor could inhibit the proliferation in NSCLC cells. In clonogenic assay of A549 cells (**FIG. 8A**), compound $MsK_{br\text{-}bf}SAP$ and $MsK_{cl\text{-}bf}SAP$ incubated cells for 14 days, then colonies are fixed by glutaraldehyde and stained by crystal violet. From the result (**FIG. 8B**), treated cells formed fewer colonies compared to the control cells

Peptide-based inhibitors typically have low cell permeability. Comparing $MsK_{bf}SAP$ with $MsK_{bf}FAP$, they showed similar inhibitory activity but huge difference on LogP. Converting **S** to **F** derivatives could contribute to better cell permeability without losing potency. 14 analogues (**Table 1**) were estimated, several compounds (**HC-3, HC-4, HC-8 and HC-14**) exhibited comparable potency and ideal LogP value.

**Table 1**. Structures of HC-1 to HC-14 and the inhibitory activity against YEATS2 YEATS.

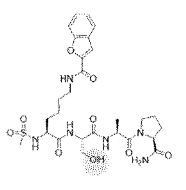

$MsK_{bf}SAP$

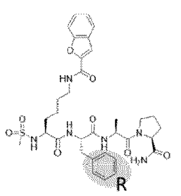

$MsK_{bf}FAP$

| | $IC_{50}$ (μM) | Clogp |
|---|---|---|
| $MsK_{bf}SAP$ | 0.6 | -0.64 |
| $MsK_{bf}FAP$ | 1.4 | 1.6 |

| ID | R | $IC_{50}$(μM) | LogP | ID | R | $IC_{50}$(μM) | LogP |
|---|---|---|---|---|---|---|---|
| HC-1 | 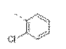 | 1.46 | - | HC-8 | 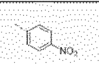 | 0.7 | 2.99 |
| HC-2 | 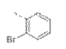 | 1.94 | - | HC-9 | 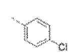 | 1.6 | - |
| HC-3 | 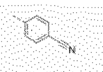 | 0.66 | 2.89 | HC-10 | 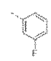 | 1.1 | 2.98 |
| HC-4 | 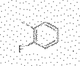 | 0.83 | 2.93 | HC-11 | 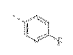 | 0.96 | 2.98 |
| HC-5 | 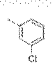 | 1.63 | - | HC-12 | 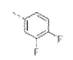 | 1.08 | 3.06 |
| HC-6 | 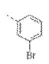 | 1.94 | - | HC-13 | 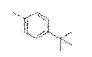 | 1.24 | 3.42 |
| HC-7 | 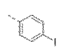 | 2.56 | - | HC-14 | 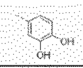 | 0.53 | 2.64 |

The cell permeability can be increased by reducing the hydrogen bond donor of inhibitors. As shown in **FIGs. 9A-9G and FIGs. 11A-11B,** inhibitors involved N-methyl Ala (**HC18**) and N-methyl Ser (**HC19**) were examined, **HC18** maintained the inhibitory activity. Deleting the amide bond at the C-terminus of $MsK_{bf}SAP$ showed no influence on the interaction, what's more, larger ring substituted inhibitor (**HC15b:** $IC_{50}$ = 0.13 $\mu$M) could enhance the potency by occupying more space in the hydrophobic pocket of YEAY2 protein. Altogether, **HC24b, HC25b** and **HC26b** were then synthesized to give the $IC_{50}$ of 0.58 $\mu$M, 0.65 $\mu$M and 0.52 $\mu$M to YEATS2 YEATS domain, respectively.

To assess the cell permeability behavior of inhibitors, the cellular effects were analyzed in H1299 and A549 cells. We treated cells with different concentration of inhibitors for 3 days. Cell viability was measured using the ATPlite 1step Luminescence Assay System (PerkinElmer) according to the manufacturer's instructions. Surviving cells were calculated as % relative to DMSO-treated cells. As shown in **FIGs. 10A-10B,** Compared with the original compounds ($MsK_{br-bf}SAP$ and $MsK_{cl-bf}SAP$), both **HC24b** and **HC26b** displayed potent inhibition at 50 $\mu$M in H1299 and A549 cells, revealing our strategies can successfully improve the cell permeability. More importantly, compound **HC24b, HC25b** and **HC26b** exhibited stronger inhibition on cell proliferation in colony formation assay (**FIG. 10C**).

Encouraged by the *in-vitro* activity, we next sought to optimize the drug-like properties of the peptide-derived inhibitors. Strategies were applied to improve the affinity and cell permeability by reducing the hydrogen donor groups. As shown in **FIG. 11A,** inhibitors involved N-methyl Ala (**HC18**) and N-methyl Ser (**HC19**) were examined to show **HC18** maintained the inhibitory activity, while N-methylation at serine site dramatically decreased the potency. Deleting the amide bond at the C-terminus of $MsK_{bf}SAP$ showed no influence on the interaction. Notably, larger ring substituted inhibitor (**HC15b:** $IC_{50}$ = 0.13 $\mu$M) further enhanced the potency by occupying more space in the hydrophobic pocket of YEAY2 protein (**FIG. 11B**).

The Lipinski Rule of Five shows ClogP is one of the most crucial factors to estimate permeability and absorption of a compound. By analyzing the sequence optimization result, we found $MsK_{bf}SAP$ ($IC_{50}$ = 0.6 $\mu$M, ClogP = -0.64) has the similar affinity but disparate ClogP value with $MsK_{bf}FAP$($IC_{50}$=1.4 $\mu$M, ClogP = 1.6). we next synthesized several F derivatives (**FIG. 12A**) and evaluated the cell viability of those compounds by luminescent ATP detection kit. The cell-active compounds were next subject to determine the cell proliferation using colony formation assay. As shown in **FIG. 12B, LS-1-124** significantly inhibited NSCLC cell growth.

To assess the ability of inhibitor to disrupt the chromatin association of YEATS2, a fluorescence recovery after photo-bleaching (FRAP) assay was performed. Tandem-YEATS2 YEATS domains fusing with GFP protein was overexpressed in U2OS cells. The treatment of **LS-1-124** remarkably decreased the recovery time after photo-bleaching, which phenocopied the loss-of-Kbz-binding YEATS2 mutant (W282A), suggesting the increased mobility of tandem - YEATS2-YEATS-GFP by replacing acylated chromatin with our inhibitor **(FIG. 13A).** When applying **LS-1-124** to FRAP assay by overexpressing AF9-GFP, ENL-GFP and GAS41-GFP **(FIG. 13B),** no difference was observed in the recovery rates, indicating the YEATS2 selectivity of **LS-1-124** in YEATS family in cellular environment.

To examine the engagement of **LS-1-124** with endogenous YEATS2, the whole cell lysate of H1299 was photo-cross-linked with probe **1(FIG. 14A),** then the labeled proteins were conjugated to biotin-N3 and enriched by streptavidin. The eluted protein mixtures were analyzed by immunoblotting. Without inhibitor, the labelling yield is more than 1%, and more than 90% endogenous YEATS2 was blocked in the present of **LS-1-124** at 5 $\mu$M **(FIG. 14B).**

To further demonstrate that YEATS2 inhibition can be useful for treating NSCLC, we tested **LS-1-124** in a mouse xenograft model, in which H1299 cells were injected subcutaneously into immunodeficient mice. After one week, the mice were randomly divided into two groups and treated by intraperitoneal (IP) injection of the control vehicle solvent (DMSO, n=6) or **LS-1-124** (200 mg/kg, n=5) daily. Tumors were collected after 26 days of treatment and monitor. **LS-1-124** treatment significantly suppressed tumor growth compared with the control **(FIG. 15A). LS-1-124** did not cause obvious toxicity in mice and no significant weight loss was observed in **LS-1-124-treated** mice (**FIG. 15B**). The tumor volume and weight both decreased after treating with inhibitor (**FIG. 15C**). The necrosis was detected by H&E staining after treating with **LS-1-124** (**FIG. 15D**). Immunohistochemical staining of Ki67 was performed to assess the effect of LS-1-124 on the proliferation of tumor cells in vivo. As shown in **FIG. 15D,** a significant decrease was observed in **LS-1-124** treatment compared to vehicle treatment. Altogether, these data demonstrates that YEATS2 inhibition with **LS-1-124** reduced tumor growth in vivo.

It should be understood that the examples and embodiments described herein are for illustrative purposes only and that various modifications or changes in light thereof will be suggested to persons skilled in the art and are to be included within the spirit and purview of this application. In addition, any elements or limitations of any invention or embodiment thereof disclosed herein can be combined with any and/or all other elements or limitations (individually or in any combination) or any other invention or embodiment thereof disclosed herein, and all such combinations are contemplated with the scope of the invention without limitation thereto.

## Claims

1. A YEATS2 YEATS inhibitor for use in a method for inhibiting cancer cell growth and/or proliferation of cancer cells.

2. A YEATS2 YEATS inhibitor for use according to claim 1, wherein the YEATS2 YEATS inhibitor is according to formula (I), formula (II), formula (III), formula (V), formula (VI), formula (VII), formula (VIII), formula (IX), formula (X), formula (XI), formula (XII), formula (XIII), formula (XIV), formula (XV), formula (XVI), formula (XVII), formula (XVIII), formula (XIX), formula (XX), formula (XXI), formula (XXII), formula (XXIII), formula (XXIV), formula (XXV), formula (XXVI), formula (XXVII), formula (XXVIII), formula (XXIX), formula (XXX), formula (XXXI), formula (XXXII), formula (XXXIII), formula (XXXIV), formula (XXXV), formula (XXXVI), formula (XXXVII), formula (XXXVIII), formula (XXXIX), formula (XL), formula (XLI), formula (XLII), formula (XLIII), formula (XLIV), formula (XLV), formula (XLVI), formula (XLVII), formula (XLVIII), formula (XLIX), formula (L), formula (LI) formula (LIII), formula (LIV), formula (LV), formula (LVI), formula (LVII), formula (LVIII), formula (LIX), formula (LX), or formula (LXI):
Formula (I)

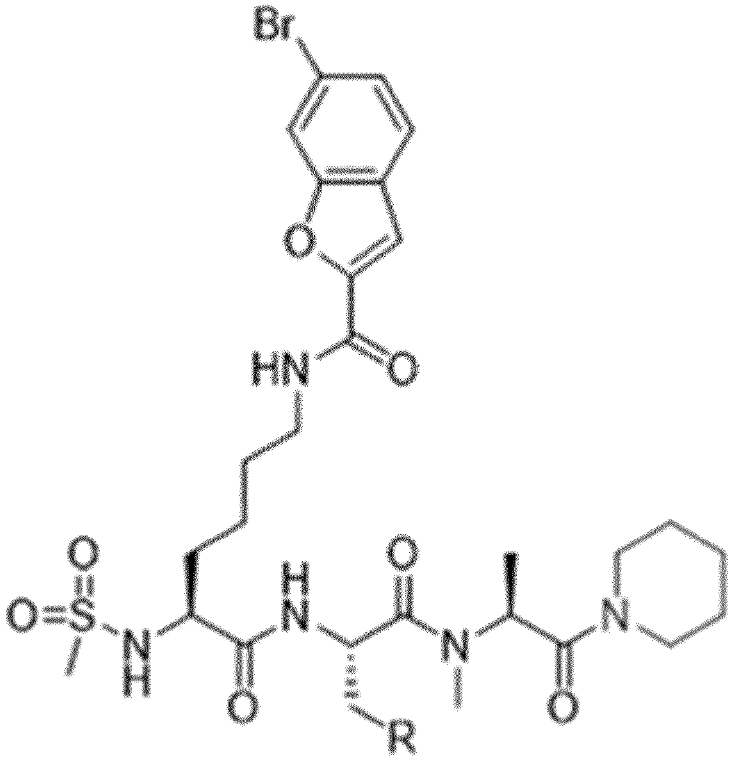

wherein R is selected from

R: 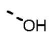 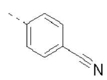 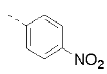 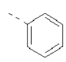 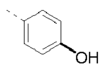 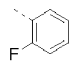 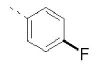

LS100 LS101 LS102 LS103 LS104 LS123 LS124

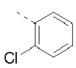 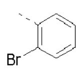 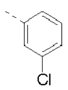 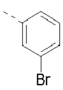 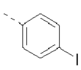 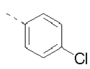 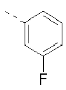 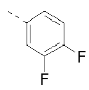 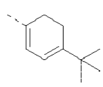 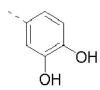

LS130 LS131 LS132 LS133 LS134 LS135 LS136 LS137 LS138 LS139

Formula (II)

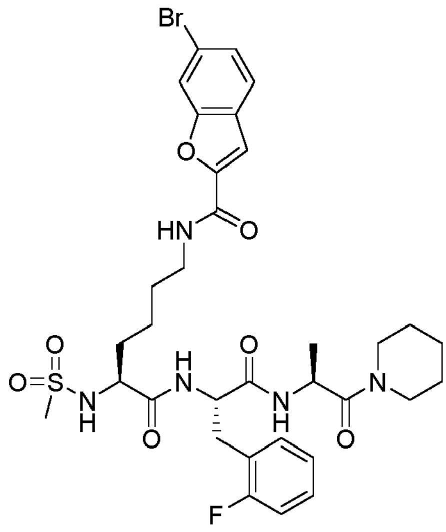

Formula (III)

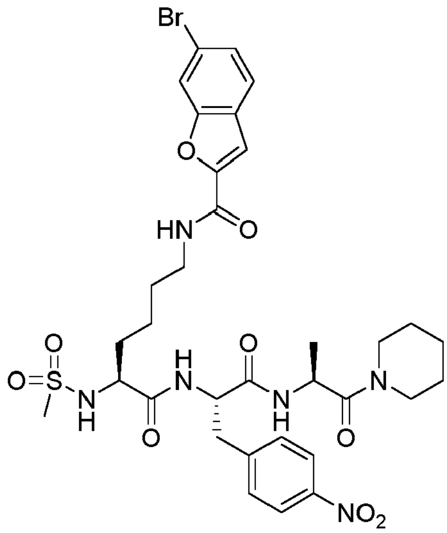

Formula (V)

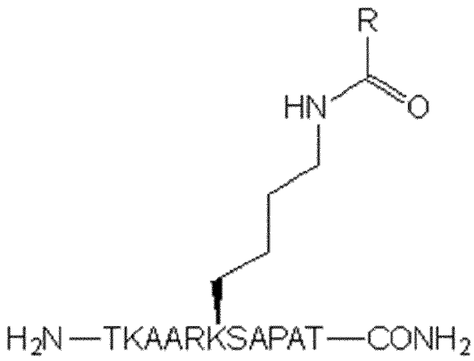

wherein R is selected from

R=

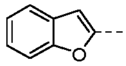
benfu

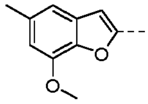
CA-1

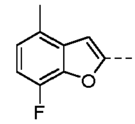
CA-2

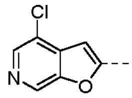
CA-3

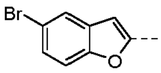
CA-8

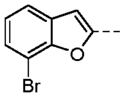
CA-9

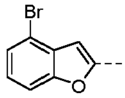
CA-10

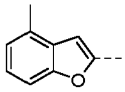
CA-11

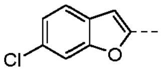
CA-16

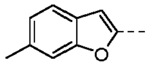
CA-17

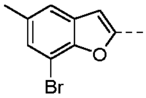
CA-18

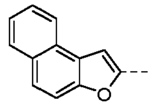
CA-19

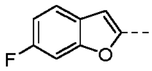
CA-24

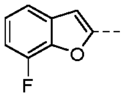
CA-25

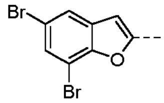
CA-26

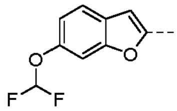
CA-27

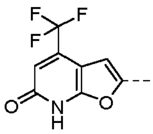
CA-32

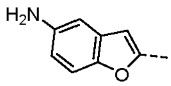
CA-33

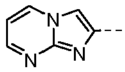
CA-34

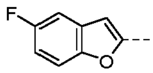
CA-35

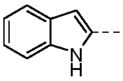
CA-40

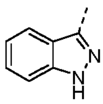
CA-41

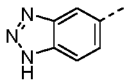
CA-42

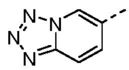
CA-43

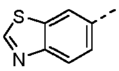
CA-48

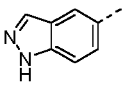
CA-49

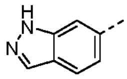
CA-50

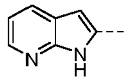
CA-51

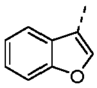
CA-56

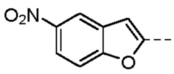
CA-57

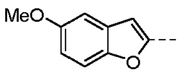
CA-58

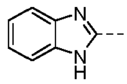
CA-59

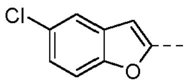

CA-4

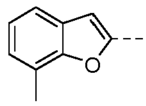

CA-5

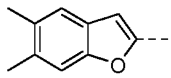

CA-6

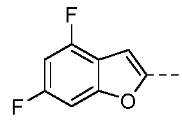

CA-7

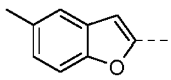

CA-12

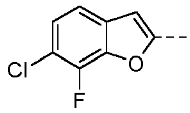

CA-13

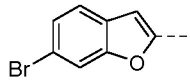

CA-14

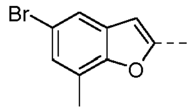

CA-15

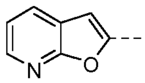

CA-20

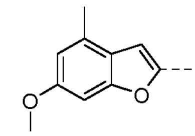

CA-21

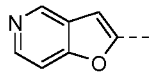

CA-22

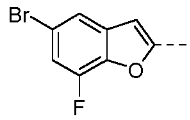

CA-23

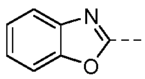

CA-28

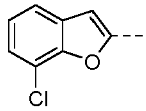

CA-29

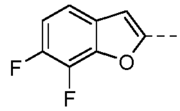

CA-30

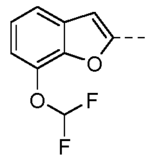

CA-31

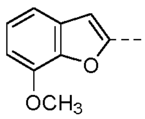

CA-36

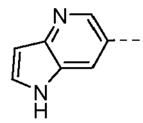

CA-37

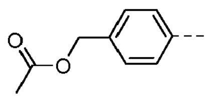

CA-38

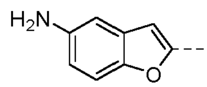

CA-39

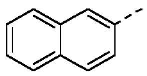

CA-44

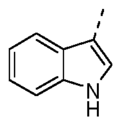

CA-45

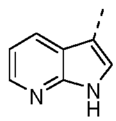

CA-46

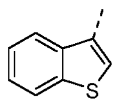

CA-47

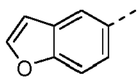

CA-52

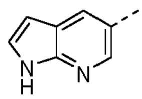

CA-53

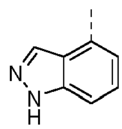

CA-54

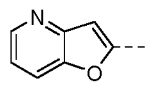

CA-55

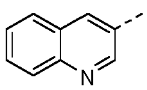

CA-60

Formula (VI)

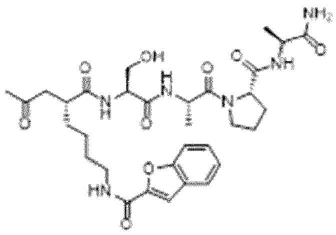

AcKbfSAPA

Formula (VII)

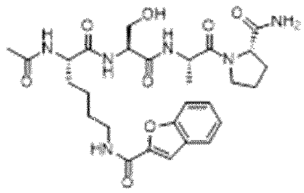

AcKbfSAP

Formula (VIII)

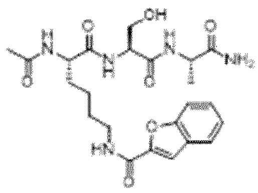

AcKbfSA

Formula (IX)

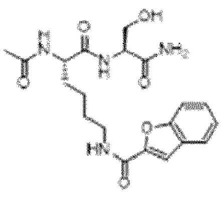

AcKbfS

Formula (X)

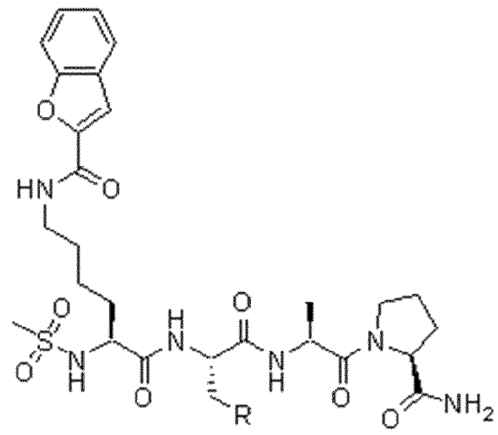

wherein R is selected from

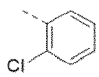
HC1

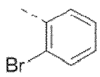
HC2

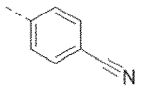
HC3

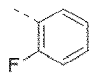
HC4

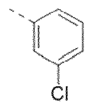
HC5

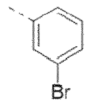
HC6

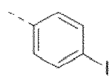
HC7

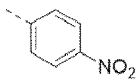
HC8

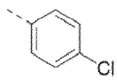
HC9

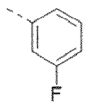
HC10

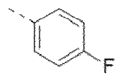
HC11

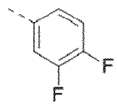
HC12

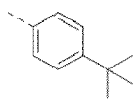
HC13

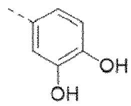
HC14

Formula (XI)

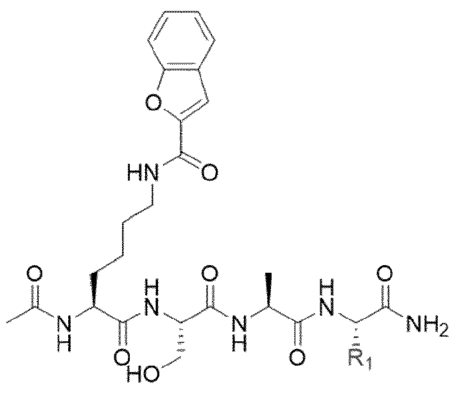

Wherein $R_1$ is selected from:

$R_1$

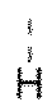
1-G

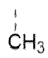
1-A

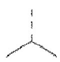
1-V

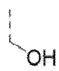
1-S

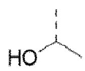
1-T

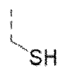
1-C

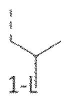
1-L

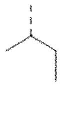 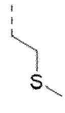 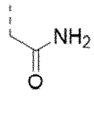 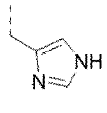 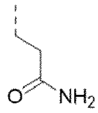 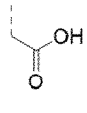 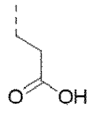

1-I 1-M 1-N 1-H 1-Q 1-D 1-E

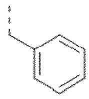 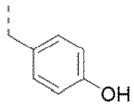 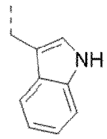 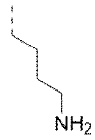 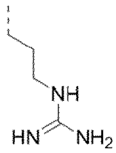

1-F 1-Y 1-W 1-K 1-R

Formula (XII)

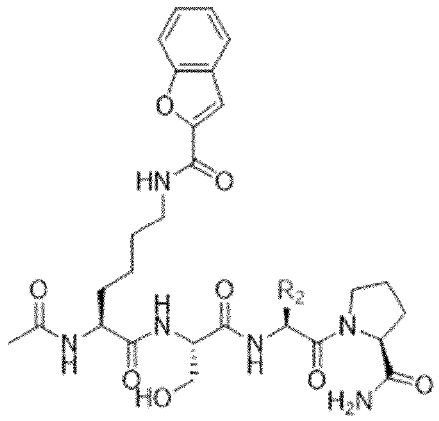

wherein $R_2$ is selected from

$R_2$ 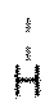 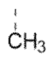 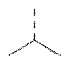 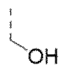 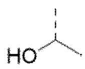 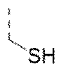 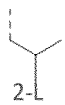

2-G 2-A 2-V 2-S 2-T 2-C 2-L

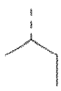 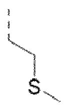 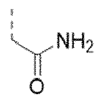 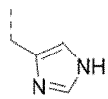 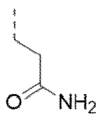 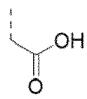 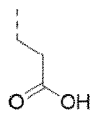

2-I 2-M 2-N 2-H 2-Q 2-D 2-E

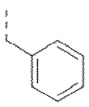 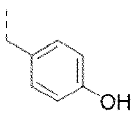 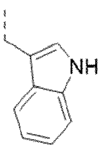 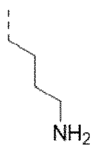 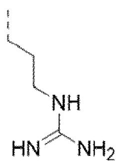

2-F 2-Y 2-W 2-K 2-R

Formula (XIII)

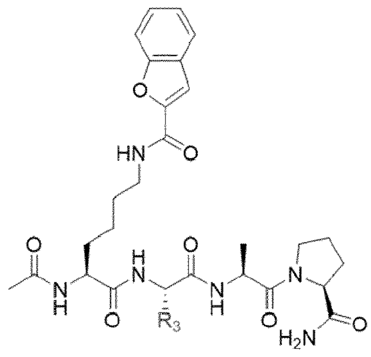

wherein $R_3$ is selected from:

$R_3$

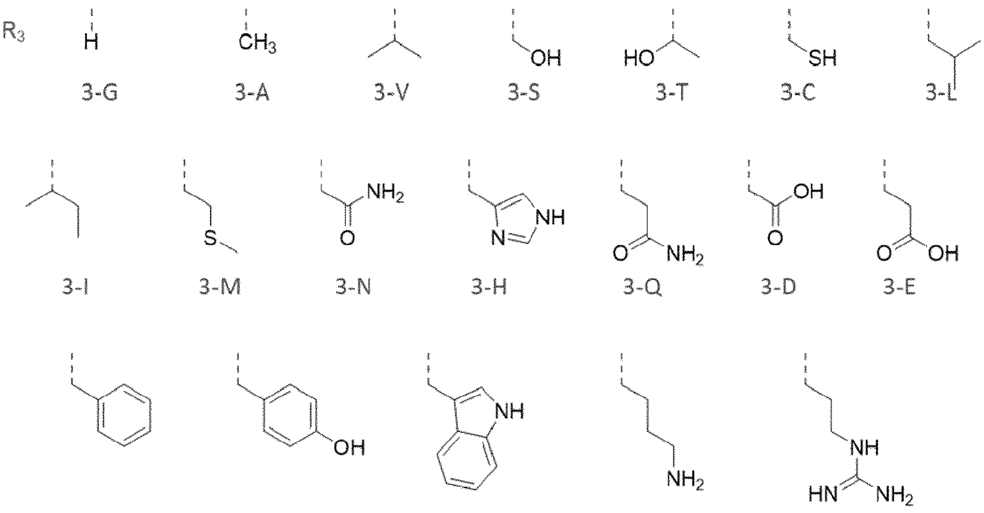

3-G 3-A 3-V 3-S 3-T 3-C 3-L

3-I 3-M 3-N 3-H 3-Q 3-D 3-E

3-F 3-Y 3-W 3-K 3-R

Formula (XIV)

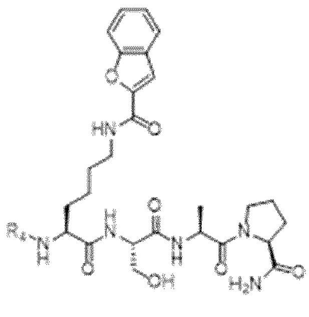

wherein $R_4$ is selected from:

$R_4$

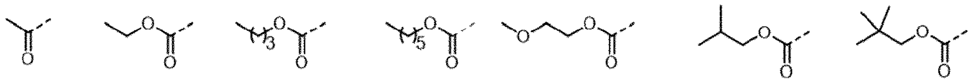

4-1 4-2 4-3 4-4 4-5 4-6 4-7

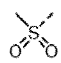
4-12

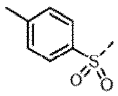
4-13

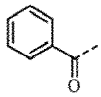
4-15

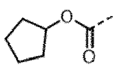
4-8

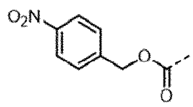
4-9

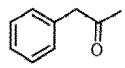
4-16

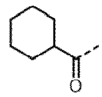
4-18

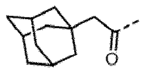
4-19

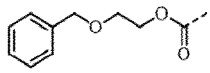
4-10

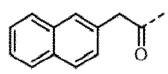
4-17

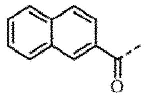
4-14

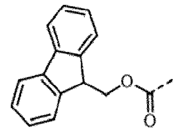
4-11

Formula (XV)

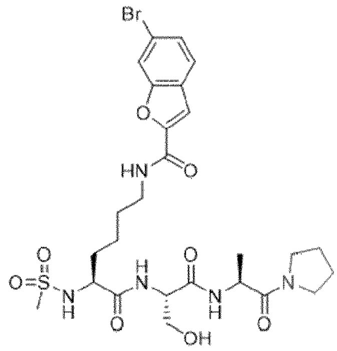
HC15a

Formula (XVI)

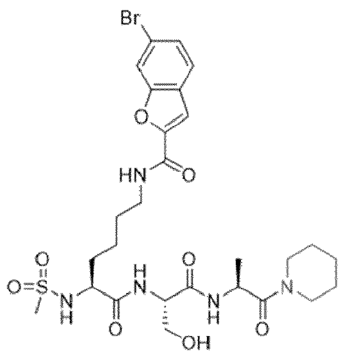
HC15b

Formula (XVII)

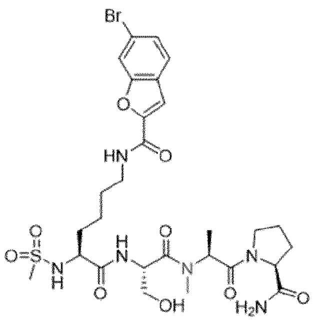

HC18

Formula (XVIII)

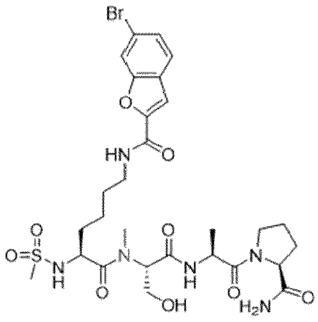

HC19

Formula (XIX)

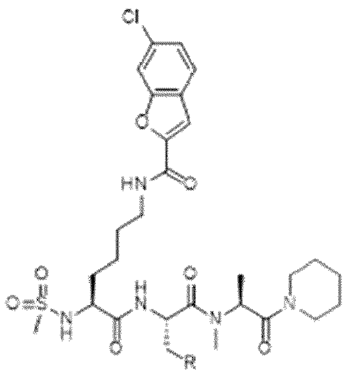

wherein R is selected from

R:

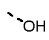
LS118

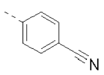
LS119

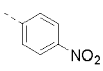
LS120

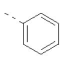
LS121

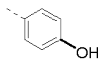
LS122

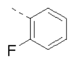
LS116

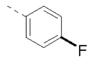
LS125

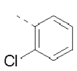
LS140

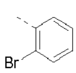
LS141

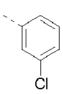
LS142

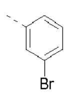
LS143

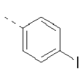
LS144

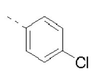
LS145

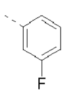
LS146

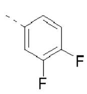
LS147

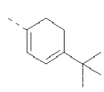
LS148

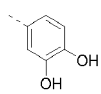
LS149

Formula (XX)

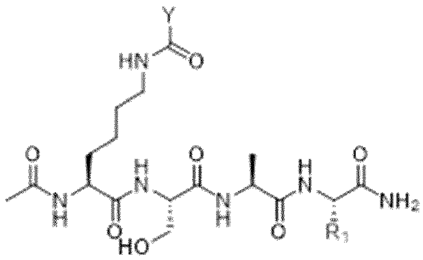

wherein $R_1$ is selected from

$R_1$ 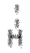 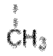 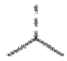 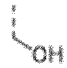 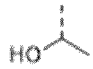 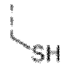 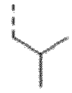

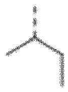 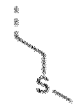 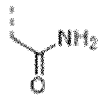 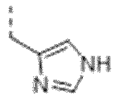 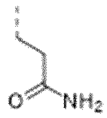 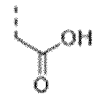 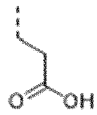

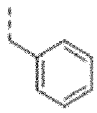 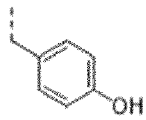 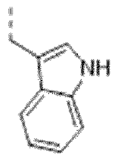 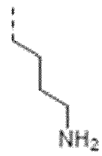 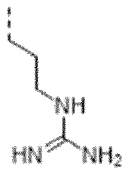

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXI)

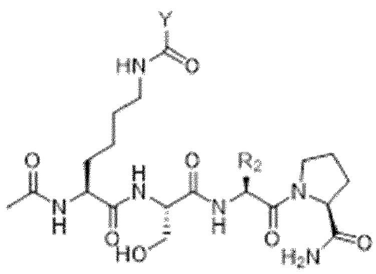

wherein $R_2$ is selected from

$R_2$ 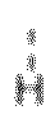 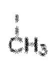 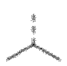 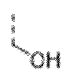 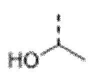 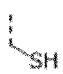 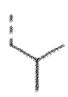

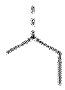 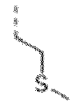 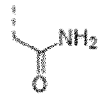 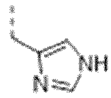 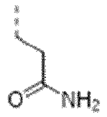 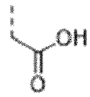 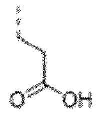

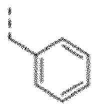 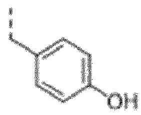 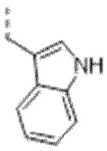 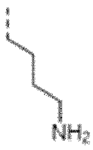 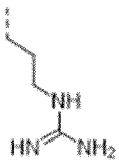

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXII)

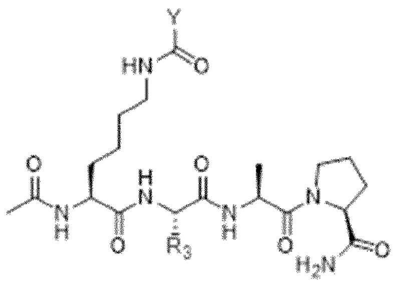

wherein $R_3$ is selected from

$R_3$ 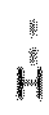 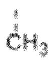 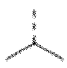 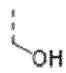 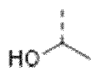 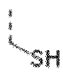 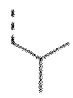

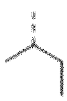 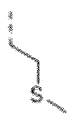 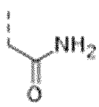 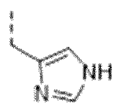 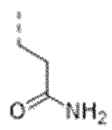 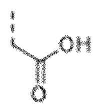 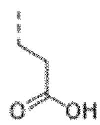

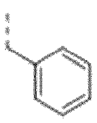 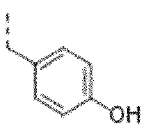 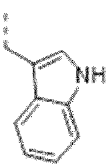 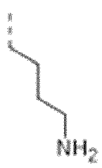 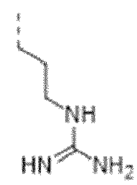

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIII)

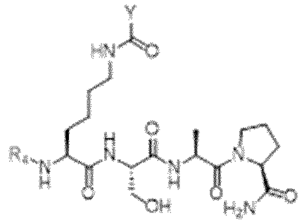

wherein $R_4$ is selected from

$R_4$

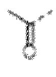 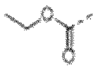 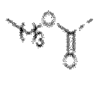 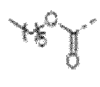 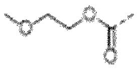 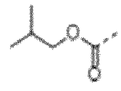 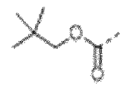

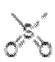 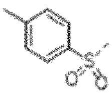 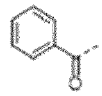 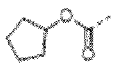 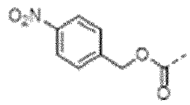 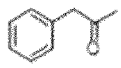 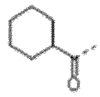

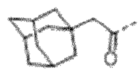 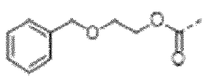 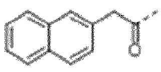 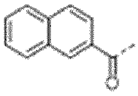 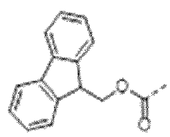

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIV)

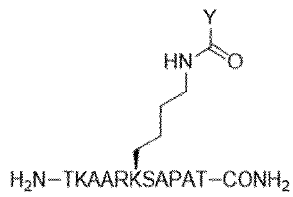

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXV)

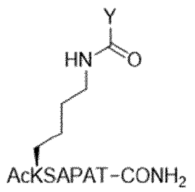

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVI)

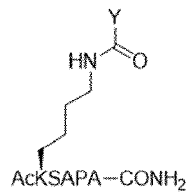

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVII)

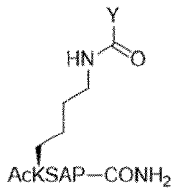

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVIII)

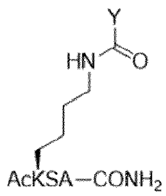

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIX)

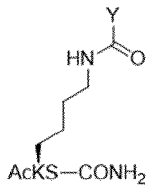

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXX)

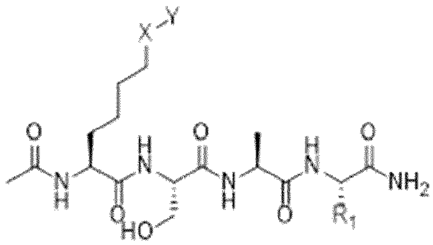

wherein $R_1$ is selected from

$R_1$ 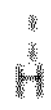 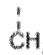 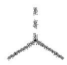 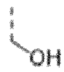 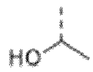 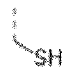 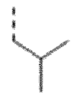

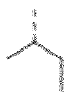 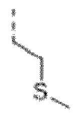 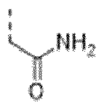 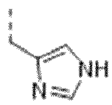 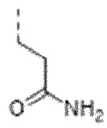 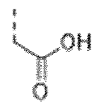 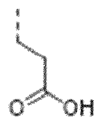

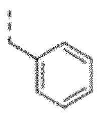 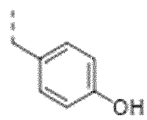 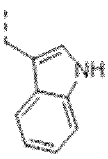 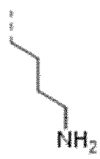 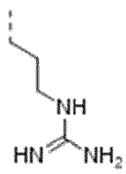

X is selected from NH-C=O, NH-C=S, O-C=O, C=O-O, $NH-SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXI)

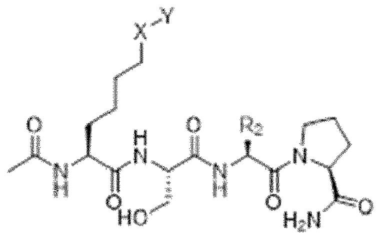

wherein $R_2$ is selected from

R2 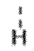 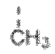 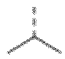 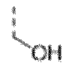 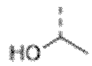 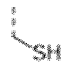 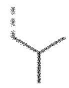

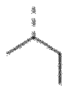 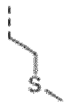 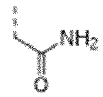 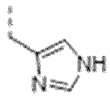 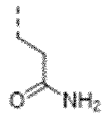 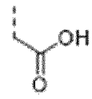 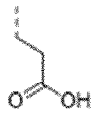

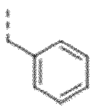 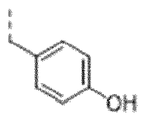 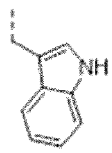 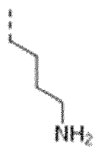 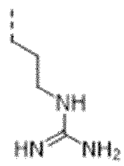

X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXII)

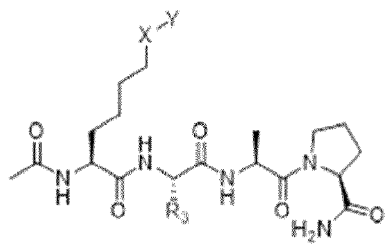

wherein $R_3$ is selected from

R3 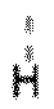 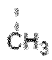 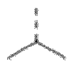 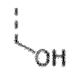 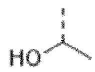 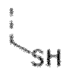 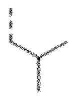

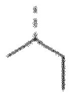 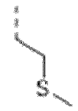 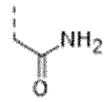 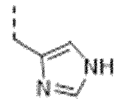 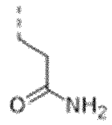 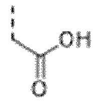 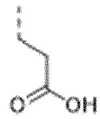

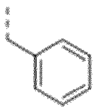 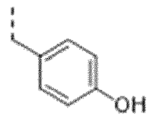 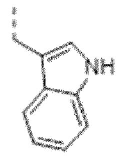 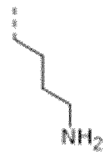 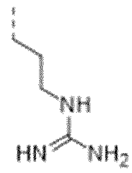

X is selected from NH-C=O, NH-C=S, O-C=O, C=O-O, $NH-SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIII)

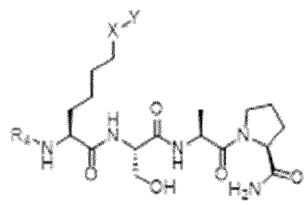

wherein $R_4$ is selected from

$R_4$ 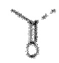 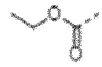 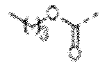 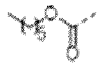 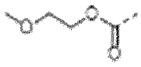 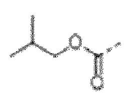 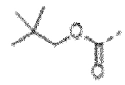

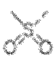 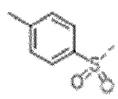 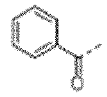 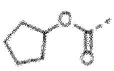 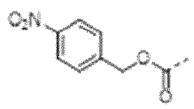 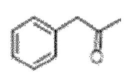 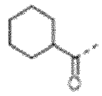

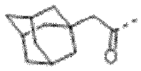 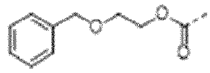 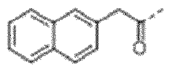 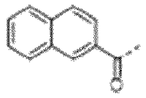 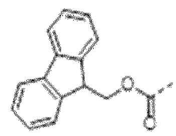

X is selected from NH-C=O, NH-C=S, O-C=O, C=O-O, $NH-SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIV)

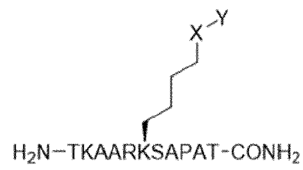

wherein X is selected from NH-C=O, NH-C=S, O-C=O, C=O-O, NH-$SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXV)

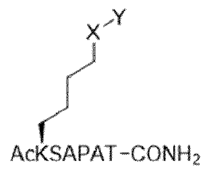

wherein X is selected from NH-C=O, NH-C=S, O-C=O, C=O-O, NH-$SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVI)

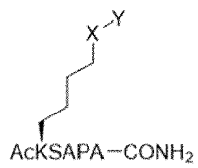

wherein X is selected from NH-C=O, NH-C=S, O-C=O, C=O-O, NH-$SO_2$; and

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVII)

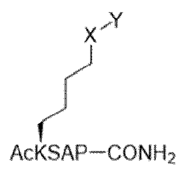

wherein X is selected from NH-C=O, NH-C=S, O-C=O, C=O-O, NH-$SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVIII)

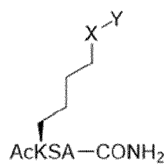

wherein X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIX)

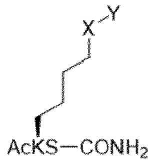

wherein X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XL)

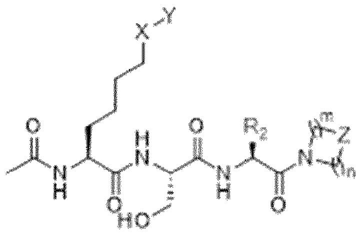

wherein $R_2$ is selected from

$R_2$ 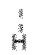 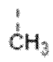 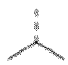 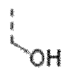 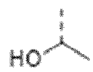 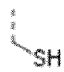 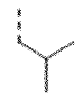

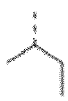 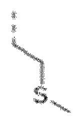 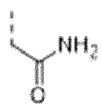 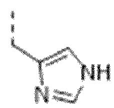 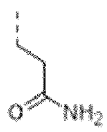 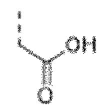 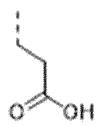

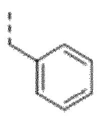 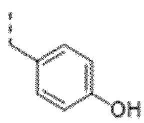 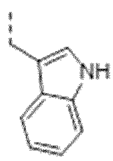 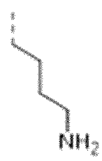 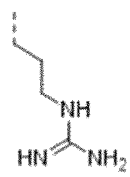

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
Z is selected from $CH_2$, O, NH, S, N-Me; and

m and n are each independently integers from 0 to 8, wherein at least one of m or n is not 0

Formula (XLI)

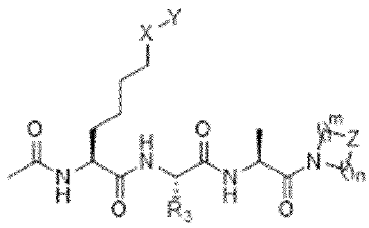

wherein $R_3$ is selected from

$R_3$ 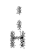 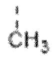 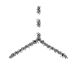 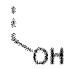 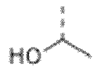 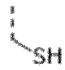 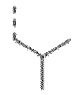

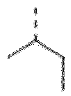 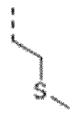 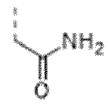 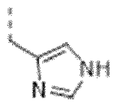 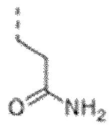 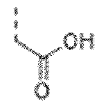 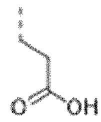

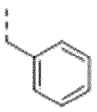 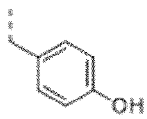 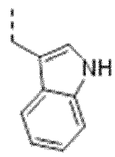 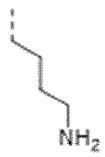 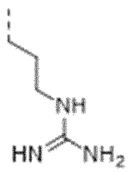

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals allow for pi-electrons to become delocalized;
and Z is selected from $CH_2$, O, NH, S, N-Me; and
m and n are each independently integers from 0 to 8, wherein at least one of m or n is not 0

Formula (XLII)

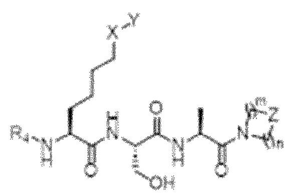

wherein $R_4$ is selected from

$R_4$ 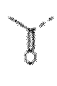 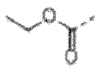 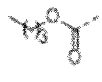 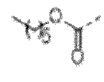 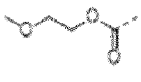 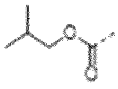 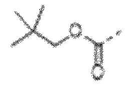

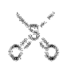 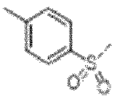 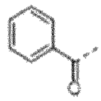 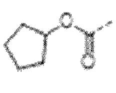 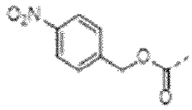 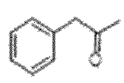 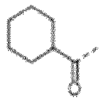

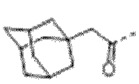 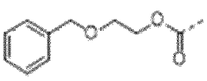 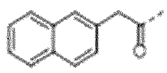 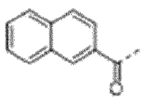 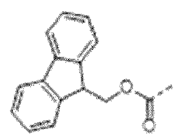

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
Z is selected from $CH_2$, O, NH, S, N-Me; and
m and n are each independently integers from 0 to 8, wherein at least one of m or n is not 0

Formula (XLIII)

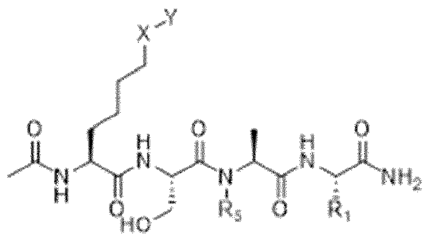

wherein $R_1$ is selected from

$R_1$ 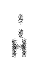 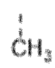 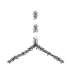 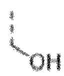 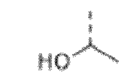 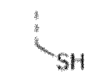 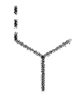

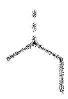 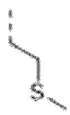 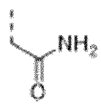 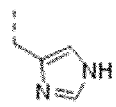 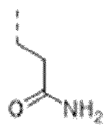 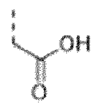 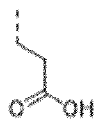

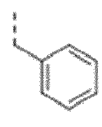 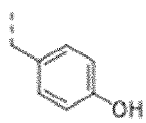 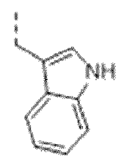 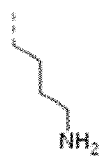 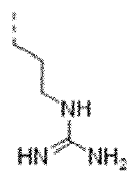

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0 to 10;
Z is selected from $CH_2$, O, NH, S, C=O, N; and
$R_5$ is selected from H, Me, Et, Pr, or iPr

Formula (XLIV)

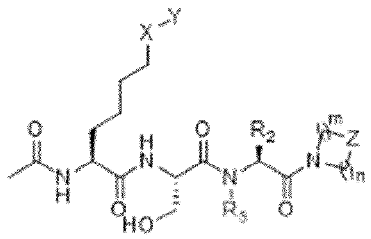

wherein $R_2$ is selected from

$R_2$ 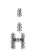 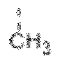 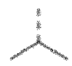 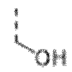 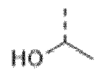 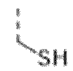 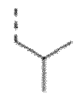

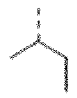 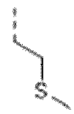 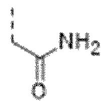 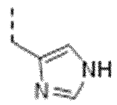 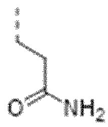 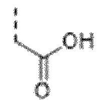 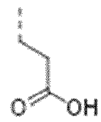

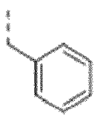 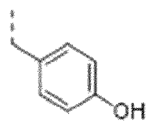 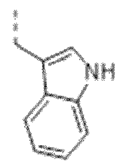 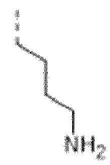 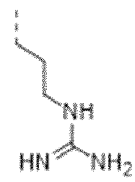

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N; and
$R_5$ is selected from H, Me, Et, Pr, or iPr

Formula (XLV)

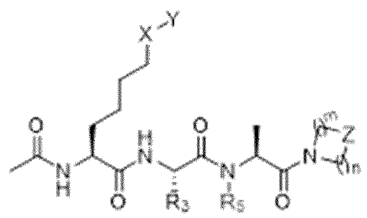

wherein $R_3$ is selected from

$R_3$ 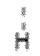 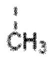 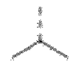 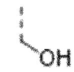 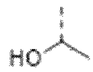 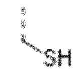 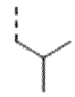

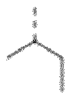 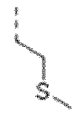 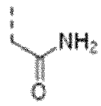 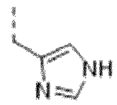 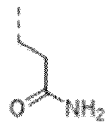 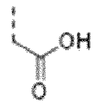 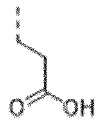

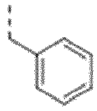 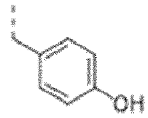 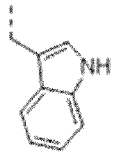 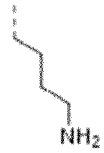 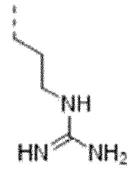

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N; and
$R_5$ is selected from H, Me, Et, Pr, or iPr

Formula (XLVI)

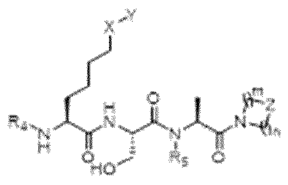

wherein $R_4$ is selected from

$R_4$ 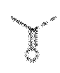 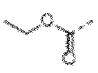 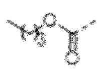 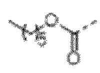 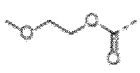 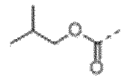 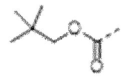

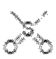 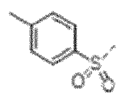 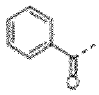 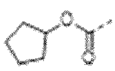 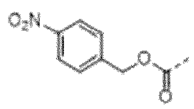 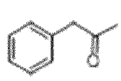 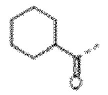

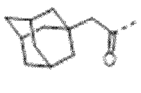 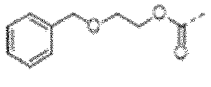 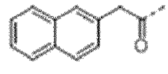 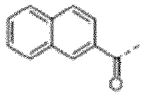 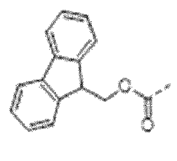

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;

Z is selected from $CH_2$, O, NH, S, C=O, N; and
$R_5$ is selected from H, Me, Et, Pr, or iPr

Formula (XLVII)

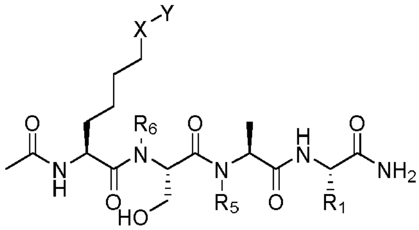

wherein $R_1$ is selected from

$R_1$ 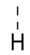 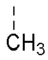 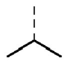 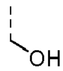 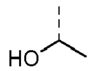 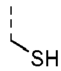 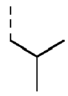

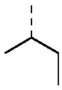 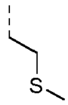 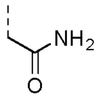 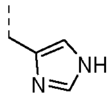 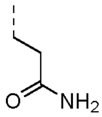 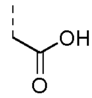 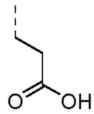

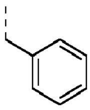 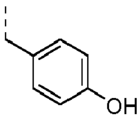 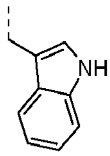 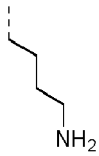 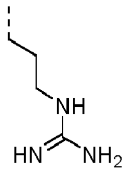

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (XLVIII)

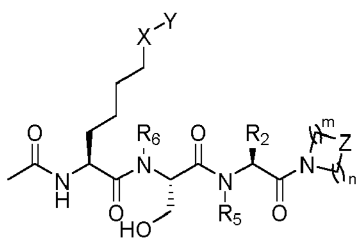

wherein $R_2$ is selected from

$R_2$ 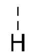 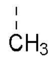 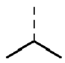 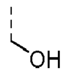 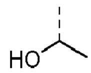 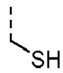 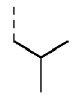

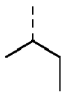 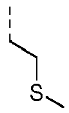 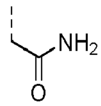 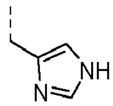 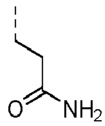 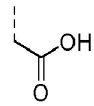 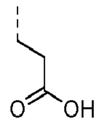

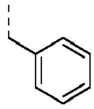 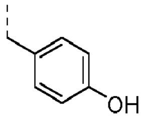 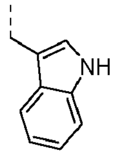 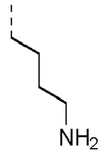 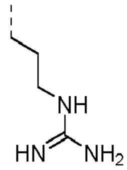

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (XLIX)

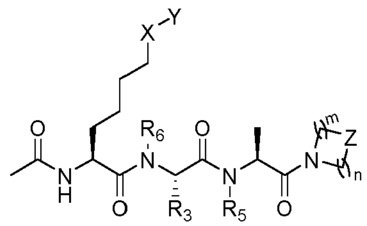

wherein $R_3$ is selected from

$R_3$ 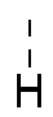 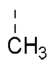 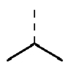 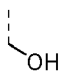 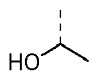 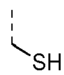 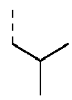

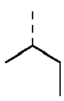 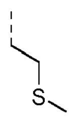 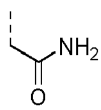 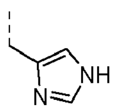 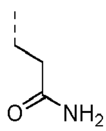 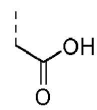 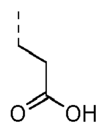

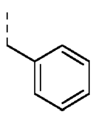 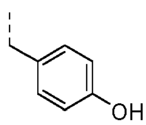 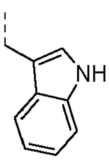 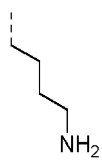 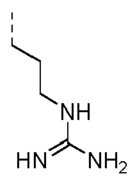

X is selected from NH-C=O, NH-C=S, O-C=O, C=O-O, NH-$SO_2$;

Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (L)

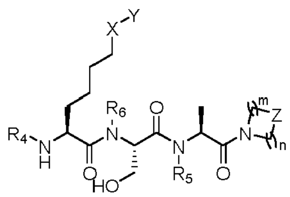

Wherein $R_4$ is selected from

$R_4$ 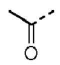 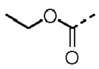 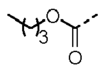 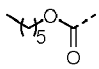 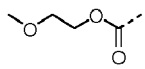 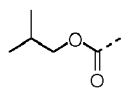 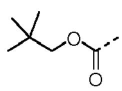

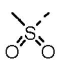 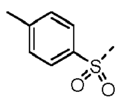 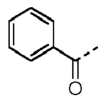 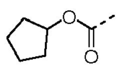 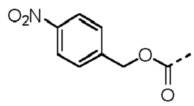 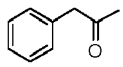 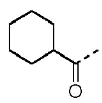

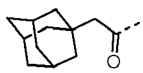 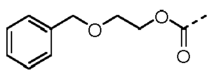 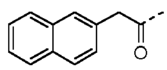 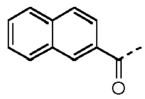 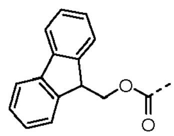

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (LI)

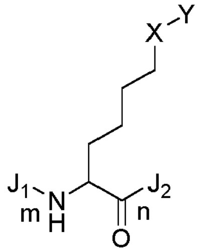

wherein X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

J1 and J2 is independently any $\alpha$ amino acid; and
m and n are each independently integers from 0 to 10, wherein at least one of m or n is not 0

Formula (LIII)

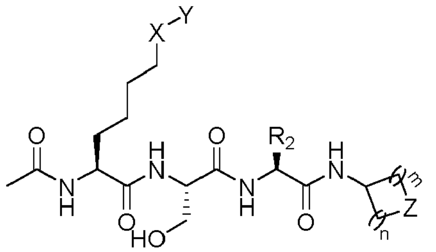

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 8, wherein at least one of m or n is not 0; and
Z is $CH_2$, O, NH, S, or N-Me

$R_2$ 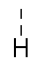 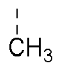 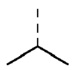 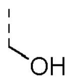 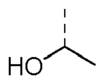 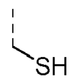 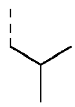

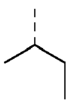 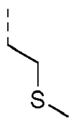 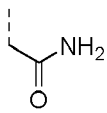 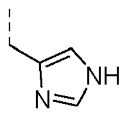 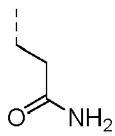 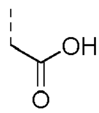 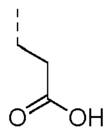

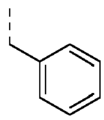 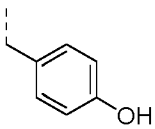 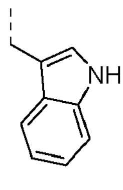 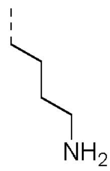 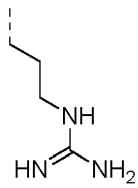

Formula (LIV)

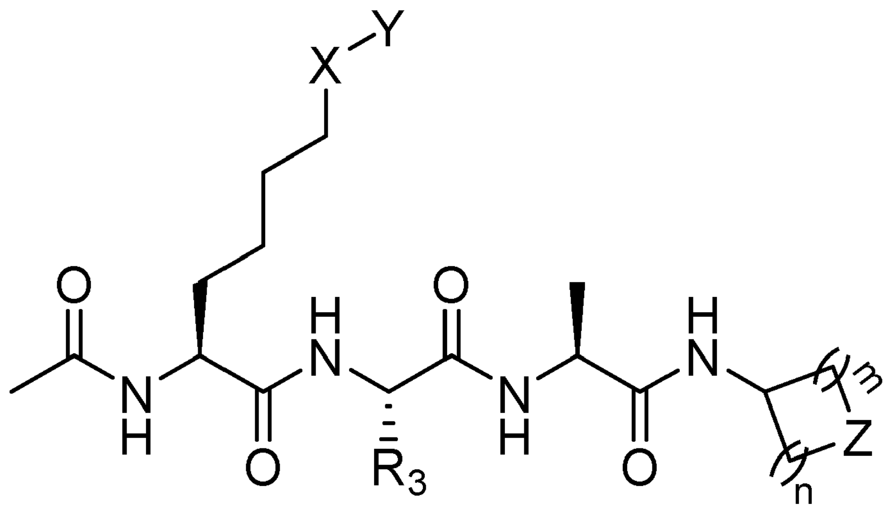

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 8, wherein at least one of m or n is not 0; and
Z is $CH_2$, O, NH, S, or N-Me

$R_3$ 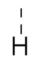 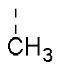 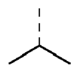 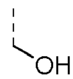 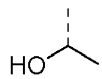 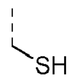 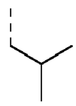

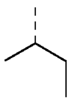 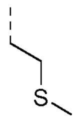 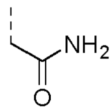 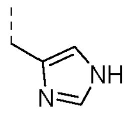 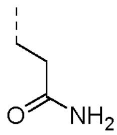 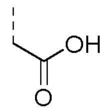 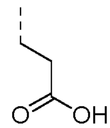

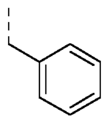 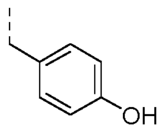 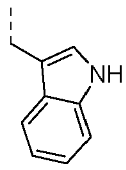 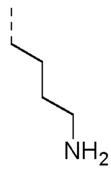 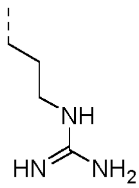

Formula (LV)

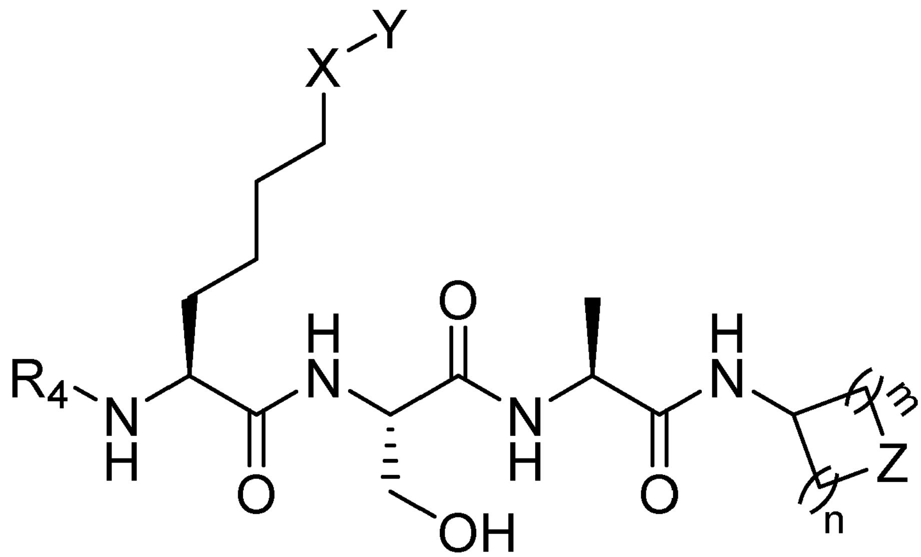

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 8, wherein at least one of m or n is not 0; and
Z is $CH_2$, O, NH, S, or N-Me

$R_4$

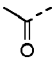 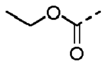 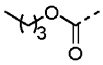 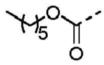 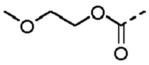 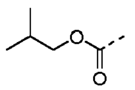 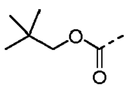

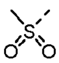 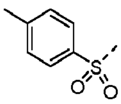 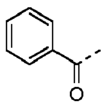 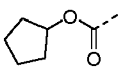 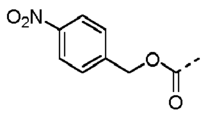 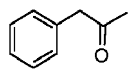 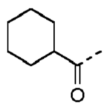

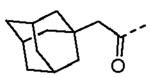 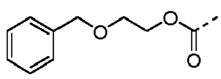 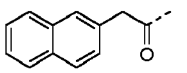 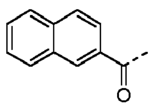 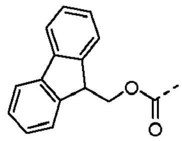

Formula (LVI)

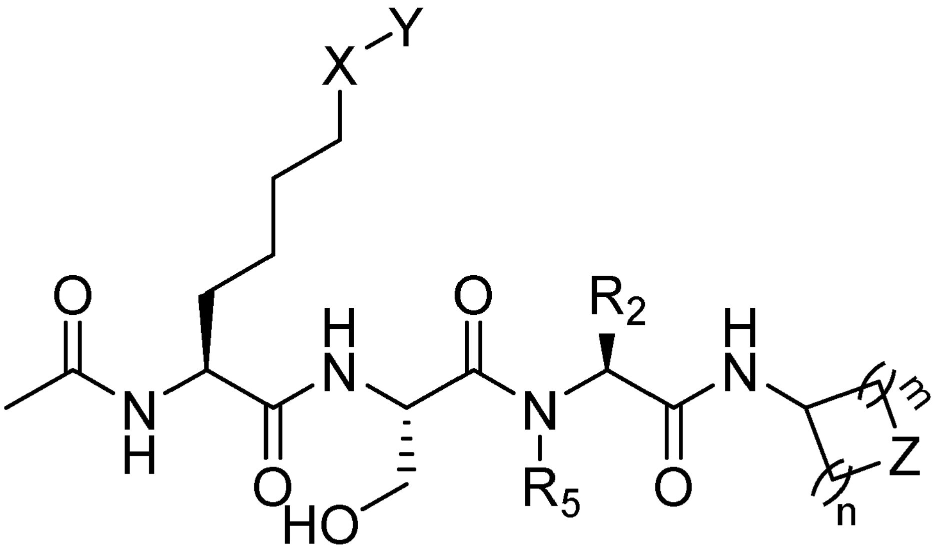

wherein X is NH-C=O, NH-C=S, O-C=O, C=O-O, or NH-$SO_2$;

Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0-10;

Z is $CH_2$, O, NH, S, C=O, or N; and

$R_5$ is H, Me, Et, Pr, or iPr

$R_2$ 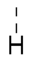 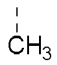 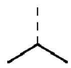 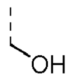 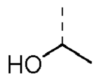 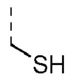 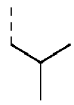

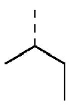 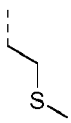 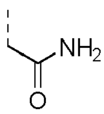 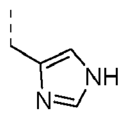 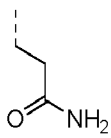 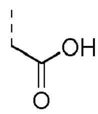 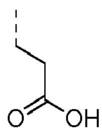

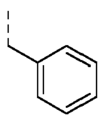 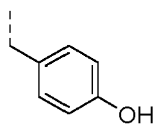 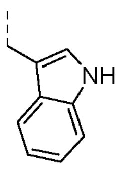 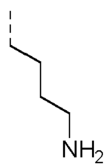 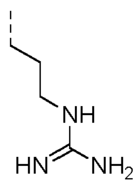

Formula (LVII)

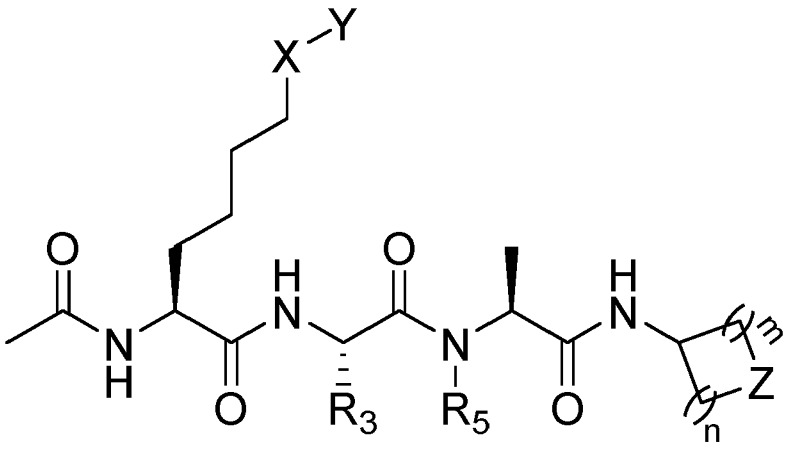

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S, C=O, or N; and
$R_5$ is H, Me, Et, Pr, or iPr

$R_3$ 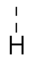 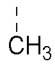 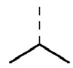 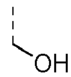 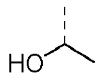 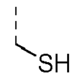 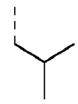

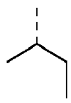 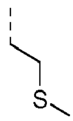 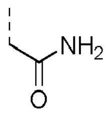 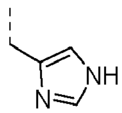 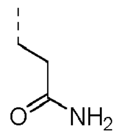 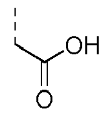 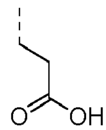

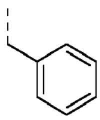 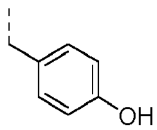 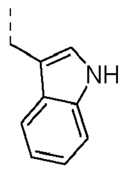 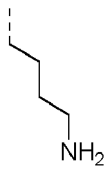 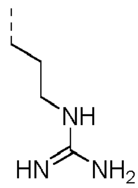

Formula (LVIII)

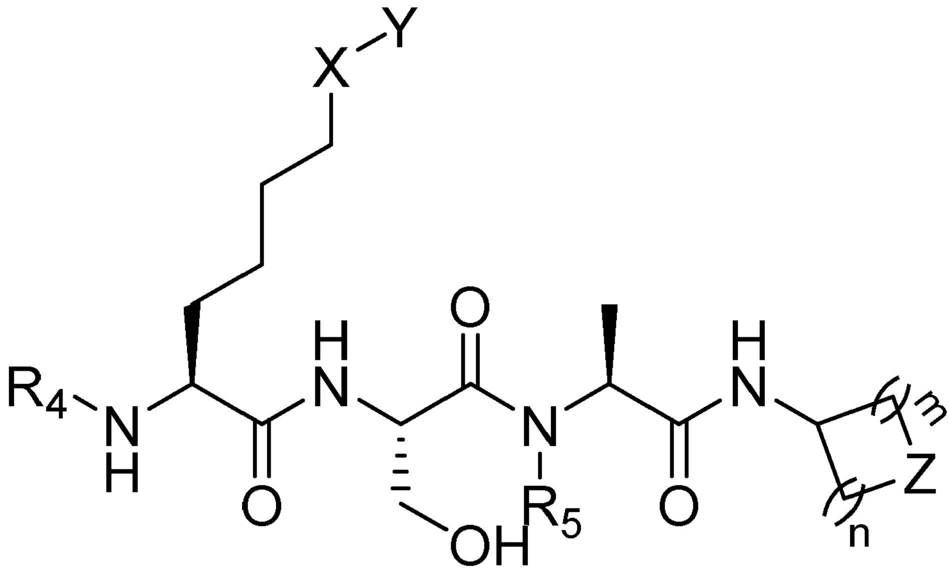

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S, C=O, or N; and
$R_5$ is H, Me, Et, Pr, or iPr

$R_4$

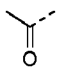 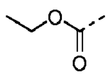 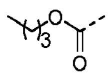 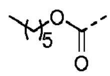 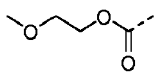 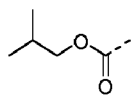 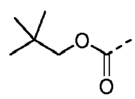

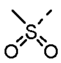 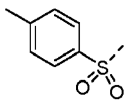 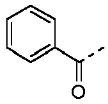 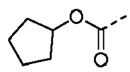 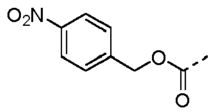 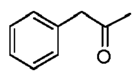 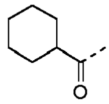

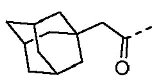 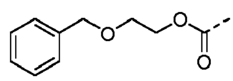 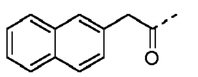 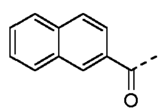 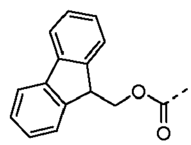

Formula (LIX)

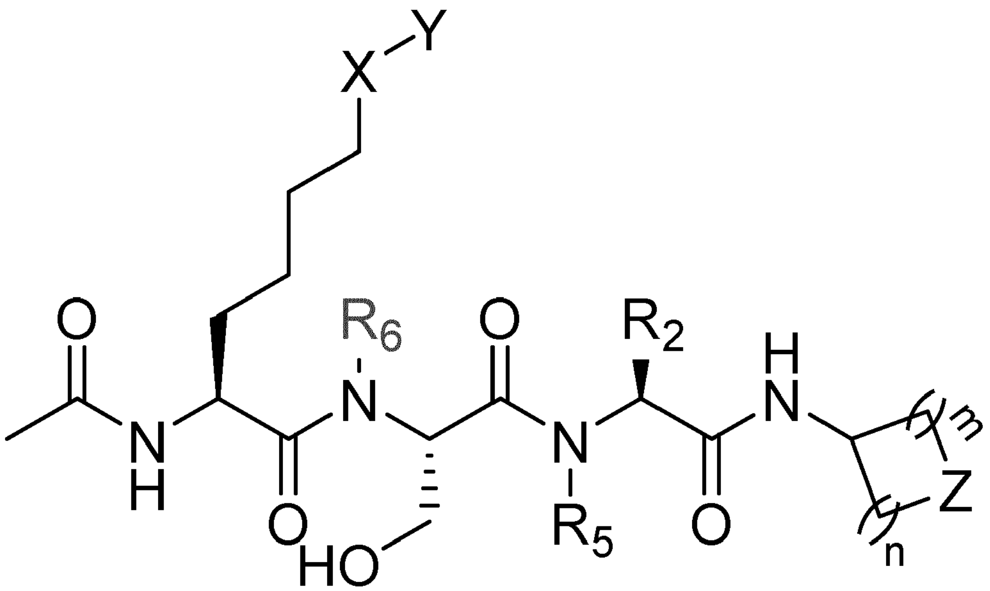

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S,C=O, or N;
$R_5$ is H, Me, Et, Pr, or iPr; and
$R_6$ is H, Me, Et, Pr, or iPr

$R_2$ 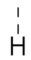 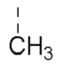 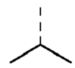 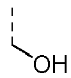 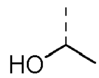 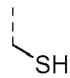 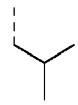

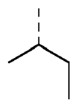 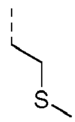 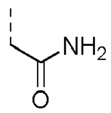 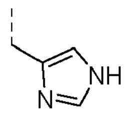 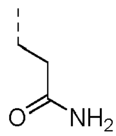 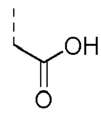 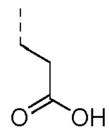

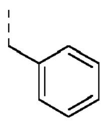 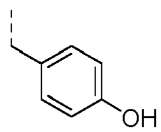 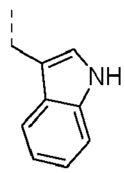 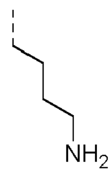 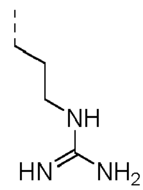

Formula (LX)

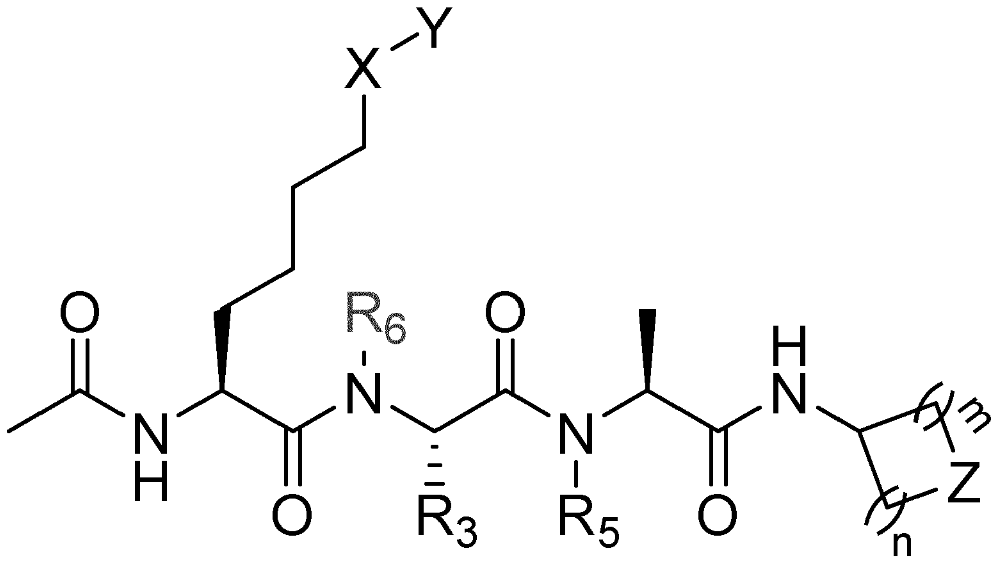

wherein X is NH-C=O, NH-C=S, O-C=O, C=O-O, or NH-$SO_2$;

Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0-10;

Z is $CH_2$, O, NH, S,C=O, or N;

$R_5$ is H, Me, Et, Pr, or iPr; and

$R_6$ is H, Me, Et, Pr, or iPr

$R_3$ 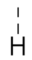 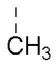 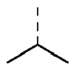 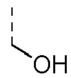 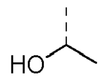 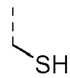 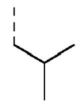

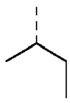 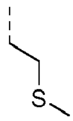 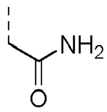 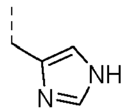 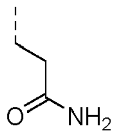 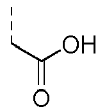 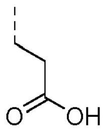

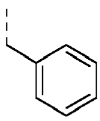 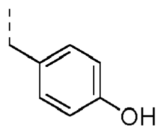 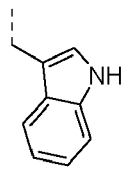 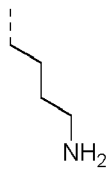 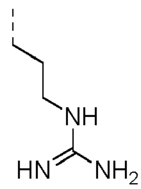

Formula (LXI)

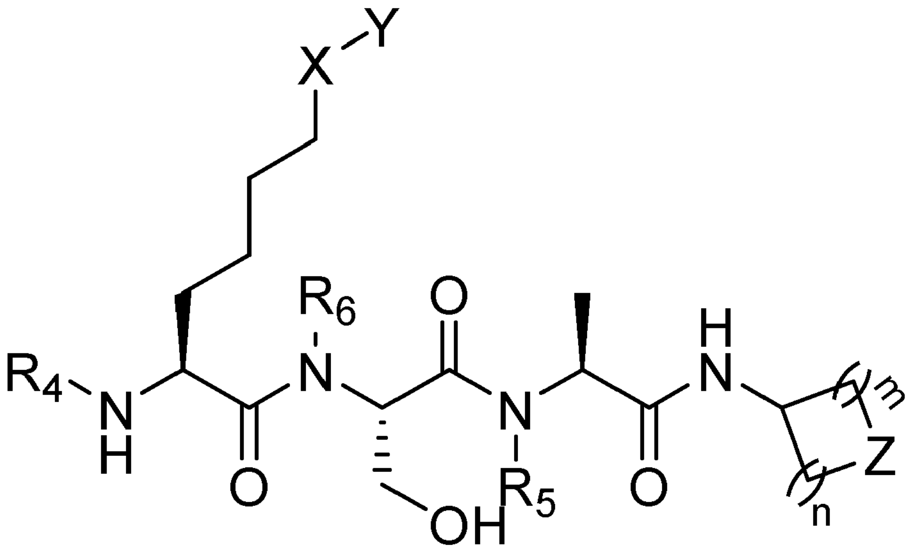

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S,C=O, or N;
$R_5$ is H, Me, Et, Pr, or iPr; and
$R_6$ is H, Me, Et, Pr, or iPr

$R_4$

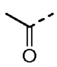 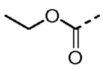 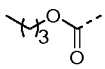 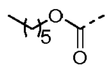 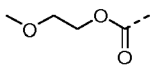 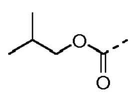 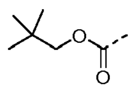

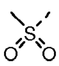 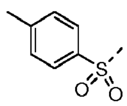 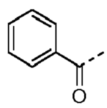 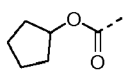 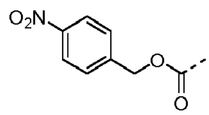 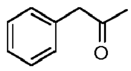 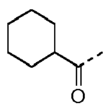

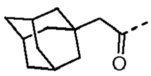 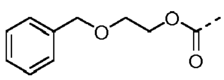 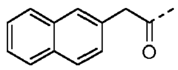 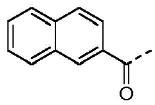 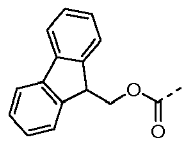

**3.** A YEATS2 YEATS inhibitor for the use of claim 2, wherein the YEATS2 YEATS inhibitor HC25b (formula (II)) or HC26b (formula (III)):

formula (II)

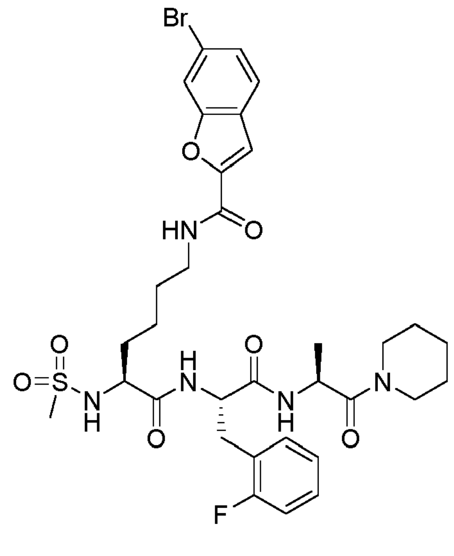

formula (III)

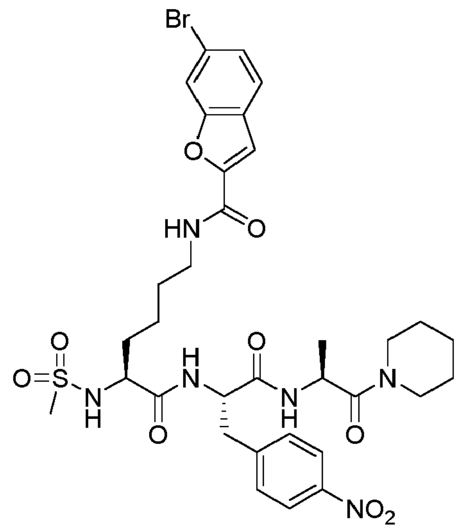

.

4. A YEATS2 YEATS inhibitor for the use of claim 1, wherein the YEATS2 YEATS inhibitor recognizes the H3K27ac epigenetic modification.

5. A YEATS2 YEATS inhibitor for the use of claim 1, wherein the YEATS2 YEATS inhibitor inhibits the Kac binding pocket of YEATS.

6. A YEATS2 YEATS inhibitor for the use of claim 1, wherein the cancer cell is in the lung of a subject, and the YEATS2 YEATS inhibitor is administered to the subject.

7. A YEATS2 YEATS inhibitor for the use of claim 1, wherein the cancer is non-small cell lung cancer (NSCLC), pancreatic cancer, ovarian cancer, or liver cancer.

8. A YEATS2 YEATS inhibitor compound according to formula (I), formula (II), formula (III), formula (V), formula (VI), formula (VII), formula (VIII), formula (IX), formula (X), formula (XI), formula (XII), formula (XIII), formula (XIV), formula (XV), formula (XVI), formula (XVII), formula (XVIII), formula (XIX), formula (XX), formula (XXI), formula (XXII), formula (XXIII), formula (XXIV), formula (XXV), formula (XXVI), formula (XXVII), formula (XXVIII), formula (XXIX), formula (XXX), formula (XXXI), formula (XXXII), formula (XXXIII), formula (XXXIV), formula (XXXV), formula (XXXVI), formula (XXXVII), formula (XXXVIII), formula (XXXIX), formula (XL), formula (XLI), formula (XLII), formula (XLIII), formula (XLIV), formula (XLV), formula (XLVI), formula (XLVII), formula (XLVIII), formula (XLIX), formula (L), formula (LI), formula (LIII), formula (LIV), formula (LV), formula (LVI), formula (LVII), formula (LVIII), formula (LIX), formula (LX),

formula (LXI):
Formula (I)

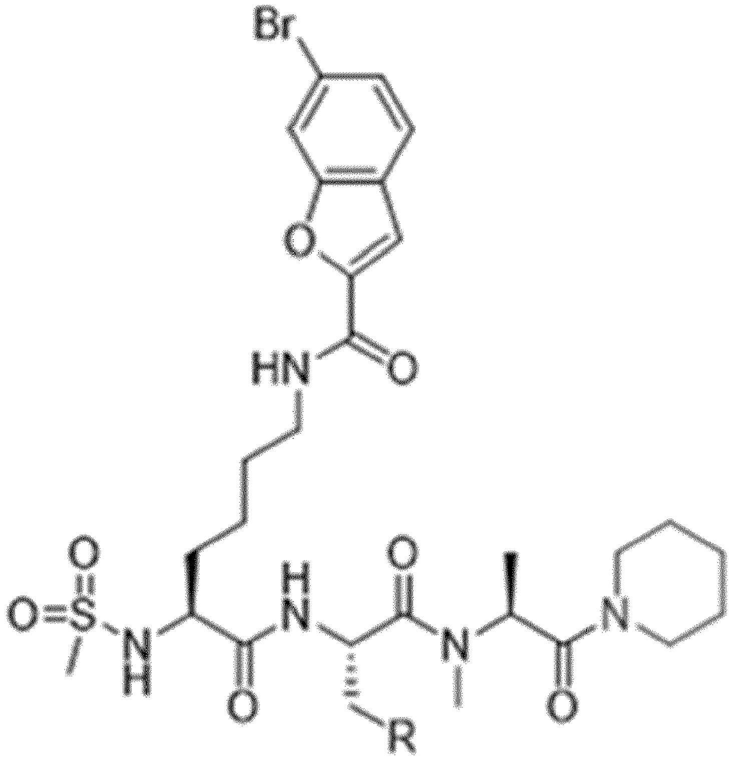

wherein R is selected from

R: 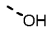 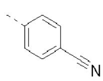 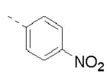 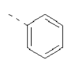 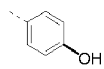 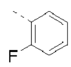 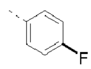

LS100 LS101 LS102 LS103 LS104 LS123 LS124

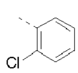 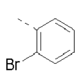 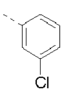 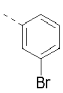 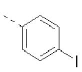 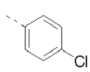 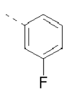 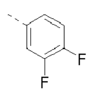 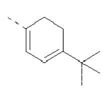 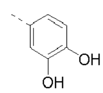

LS130 LS131 LS132 LS133 LS134 LS135 LS136 LS137 LS138 LS139

Formula (II)

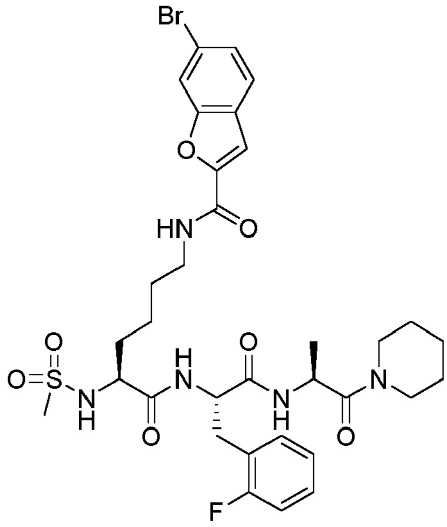

Formula (III)

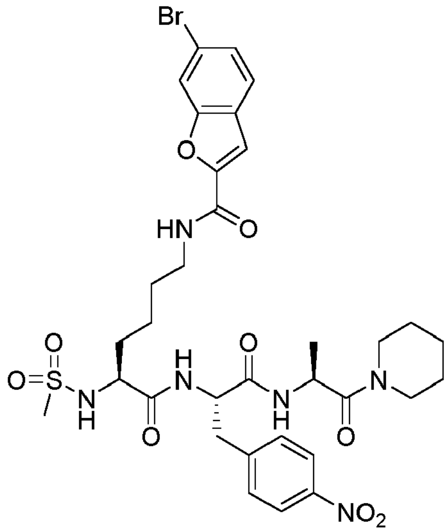

Formula (V)

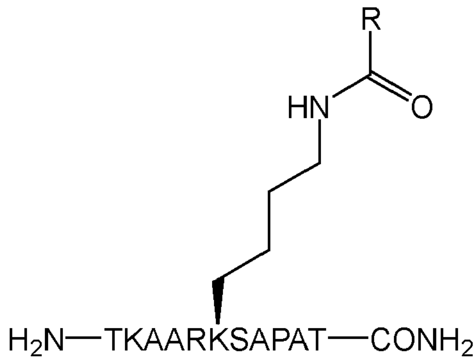

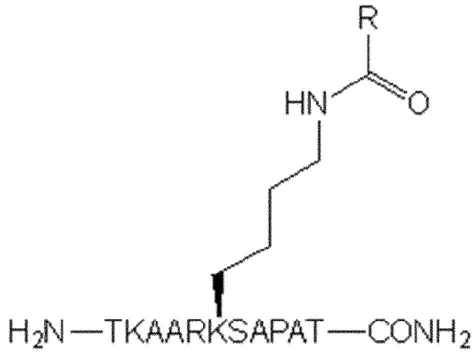

wherein R is selected from

R=

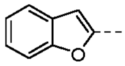

benfu

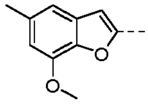

CA-1

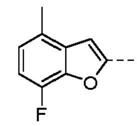

CA-2

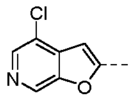

CA-3

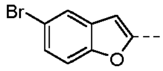

CA-8

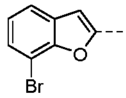

CA-9

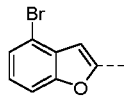

CA-10

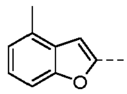

CA-11

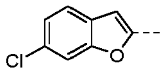

CA-16

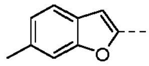

CA-17

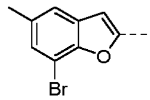

CA-18

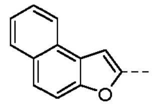

CA-19

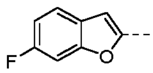

CA-24

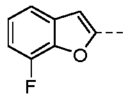

CA-25

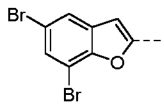

CA-26

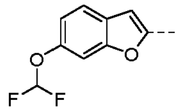

CA-27

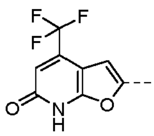

CA-32

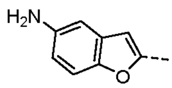

CA-33

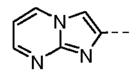

CA-34

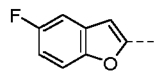

CA-35

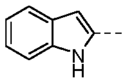

CA-40

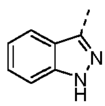

CA-41

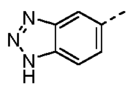

CA-42

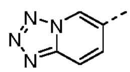

CA-43

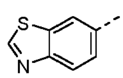

CA-48

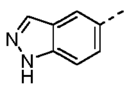

CA-49

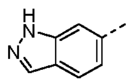

CA-50

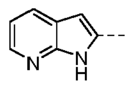

CA-51

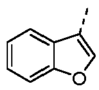

CA-56

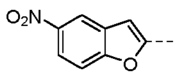

CA-57

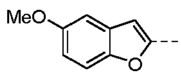

CA-58

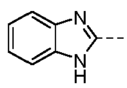

CA-59

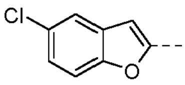
CA-4

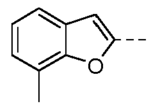
CA-5

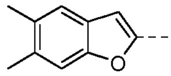
CA-6

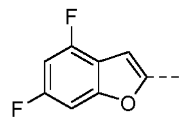
CA-7

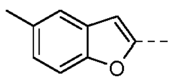
CA-12

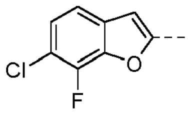
CA-13

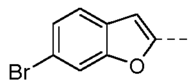
CA-14

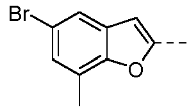
CA-15

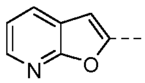
CA-20

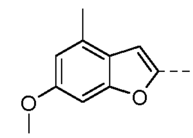
CA-21

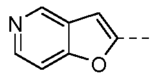
CA-22

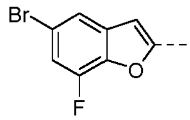
CA-23

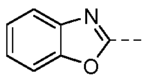
CA-28

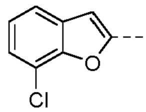
CA-29

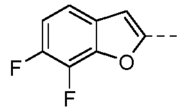
CA-30

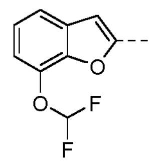
CA-31

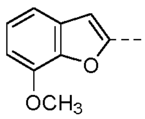
CA-36

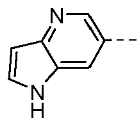
CA-37

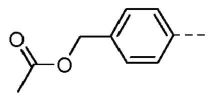
CA-38

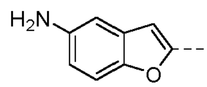
CA-39

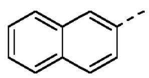
CA-44

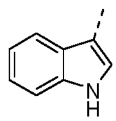
CA-45

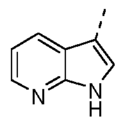
CA-46

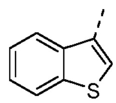
CA-47

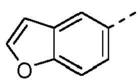
CA-52

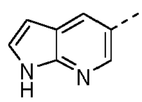
CA-53

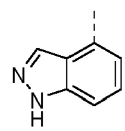
CA-54

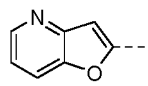
CA-55

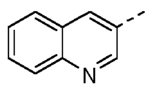
CA-60

Formula (VI)

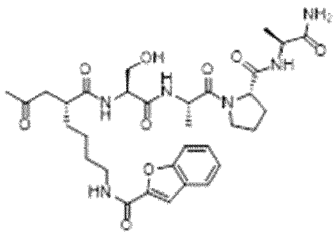

AcKbfSAPA

Formula (VII)

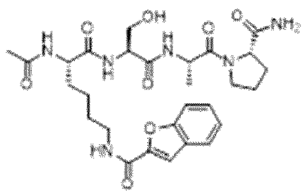

AcKbfSAP

Formula (VIII)

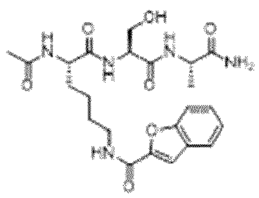

AcKbfSA

Formula (IX)

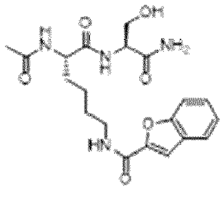

AcKbfS

Formula (X)

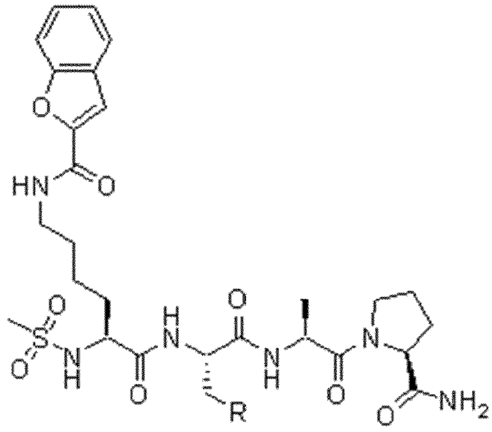

wherein R is selected from

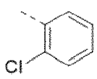
HC1

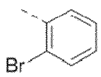
HC2

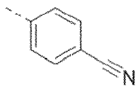
HC3

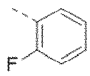
HC4

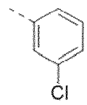
HC5

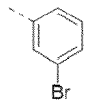
HC6

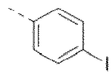
HC7

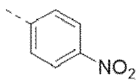
HC8

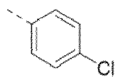
HC9

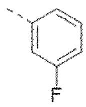
HC10

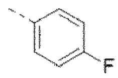
HC11

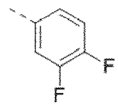
HC12

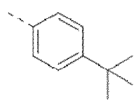
HC13

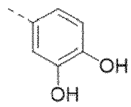
HC14

Formula (XI)

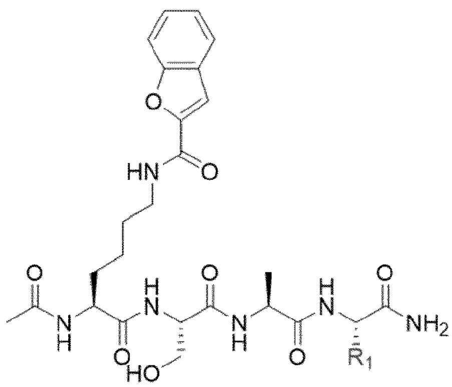

Wherein $R_1$ is selected from:

$R_1$

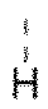
1-G

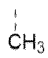
1-A

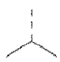
1-V

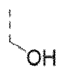
1-S

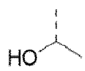
1-T

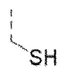
1-C

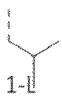
1-L

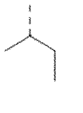 1-I
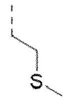 1-M
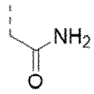 1-N
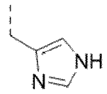 1-H
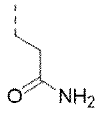 1-Q
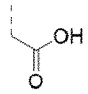 1-D
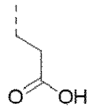 1-E

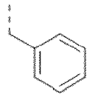 1-F
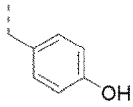 1-Y
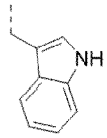 1-W
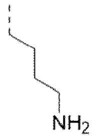 1-K
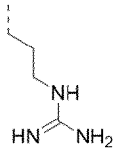 1-R

Formula (XII)

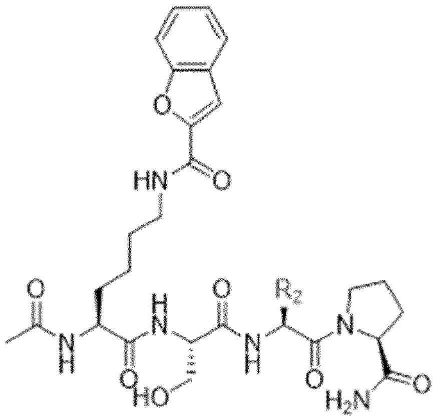

wherein $R_2$ is selected from

$R_2$

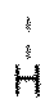 2-G
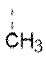 2-A
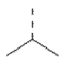 2-V
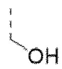 2-S
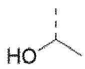 2-T
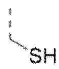 2-C
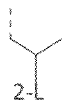 2-L

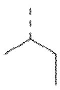 2-I
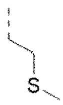 2-M
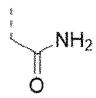 2-N
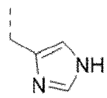 2-H
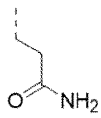 2-Q
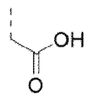 2-D
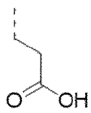 2-E

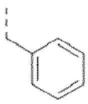 2-F
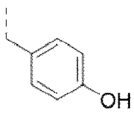 2-Y
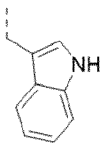 2-W
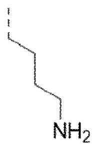 2-K
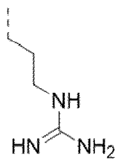 2-R

Formula (XIII)

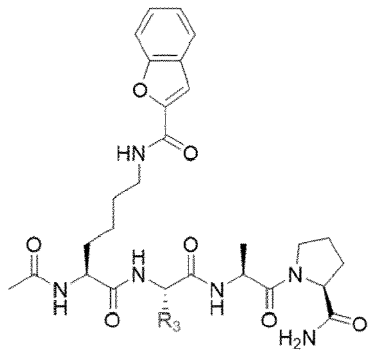

wherein $R_3$ is selected from:

$R_3$

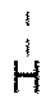
3-G

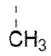
3-A

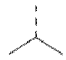
3-V

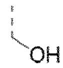
3-S

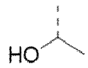
3-T

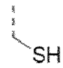
3-C

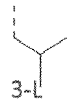
3-L

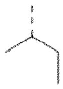
3-I

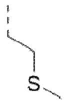
3-M

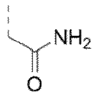
3-N

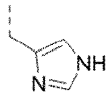
3-H

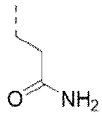
3-Q

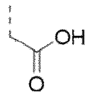
3-D

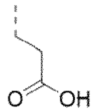
3-E

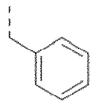
3-F

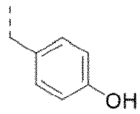
3-Y

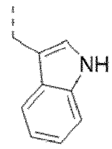
3-W

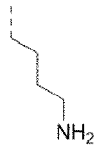
3-K

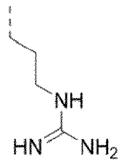
3-R

Formula (XIV)

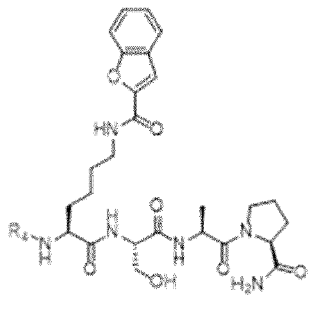

wherein $R_4$ is selected from:

$R_4$

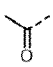
4-1

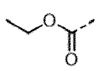
4-2

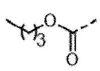
4-3

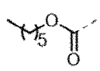
4-4

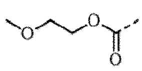
4-5

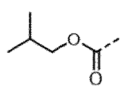
4-6

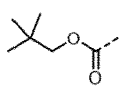
4-7

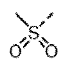
4-12

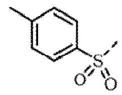
4-13

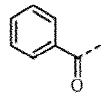
4-15

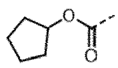
4-8

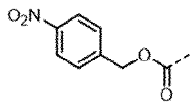
4-9

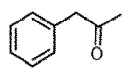
4-16

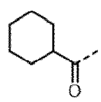
4-18

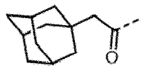
4-19

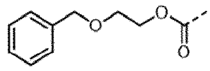
4-10

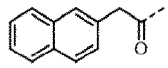
4-17

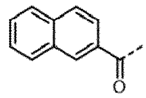
4-14

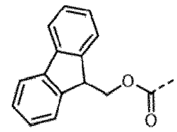
4-11

Formula (XV)

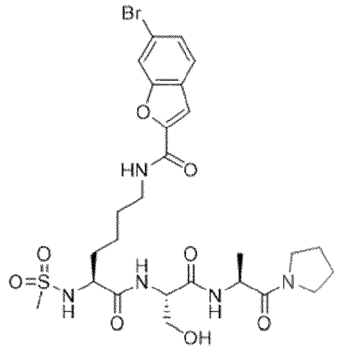
HC15a

Formula (XVI)

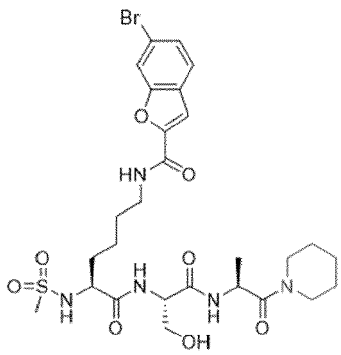
HC15b

Formula (XVII)

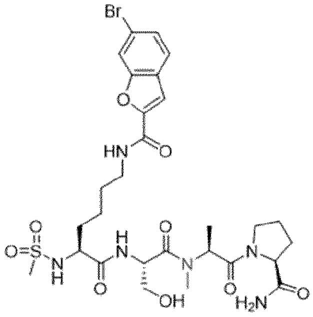

HC18

Formula (XVIII)

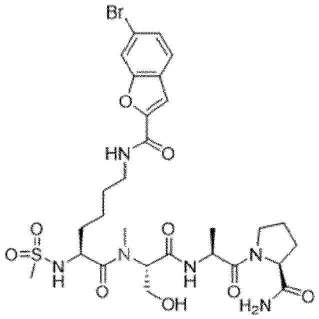

HC19

Formula (XIX)

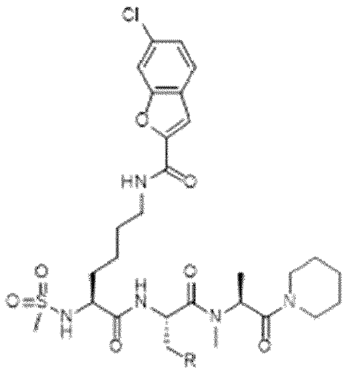

wherein R is selected from

R:

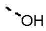
LS118

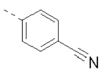
LS119

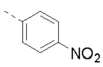
LS120

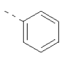
LS121

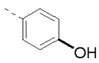
LS122

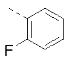
LS116

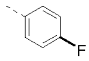
LS125

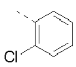
LS140

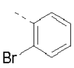
LS141

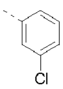
LS142

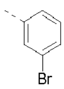
LS143

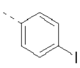
LS144

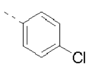
LS145

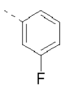
LS146

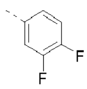
LS147

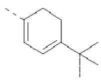
LS148

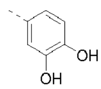
LS149

Formula (XX)

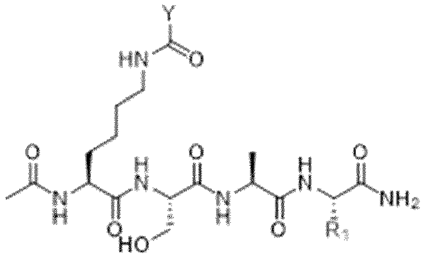

wherein $R_1$ is selected from

$R_1$ 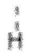 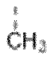 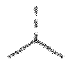 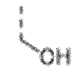 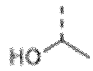 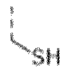 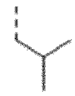

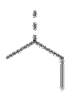 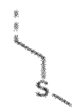 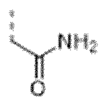 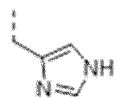 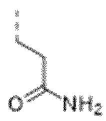 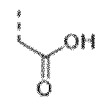 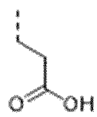

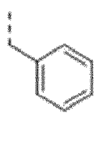 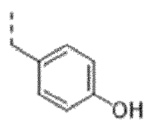 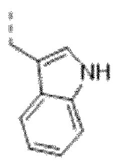 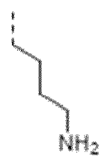 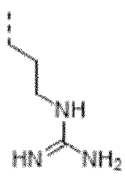

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXI)

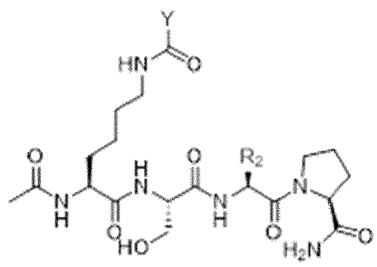

wherein $R_2$ is selected from

$R_2$ 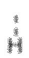 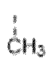 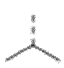 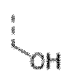 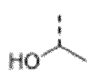 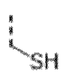 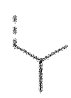

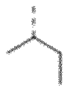 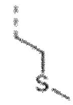 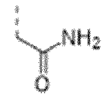 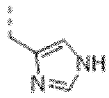 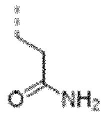 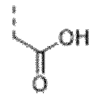 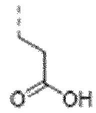

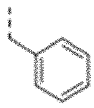 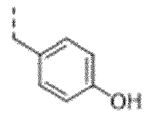 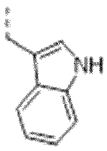 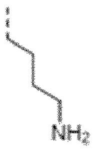 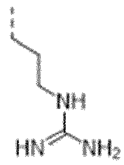

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXII)

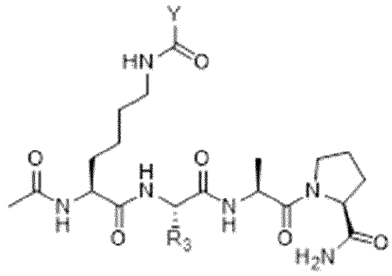

wherein $R_3$ is selected from

$R_3$ 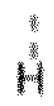 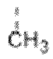 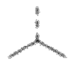 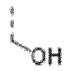 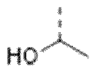 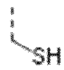 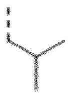

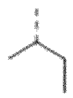 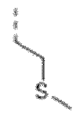 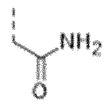 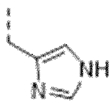 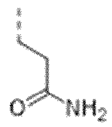 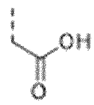 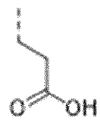

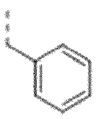 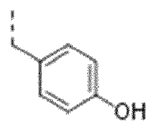 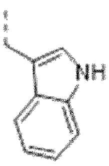 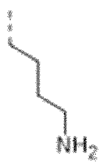 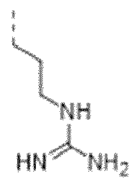

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIII)

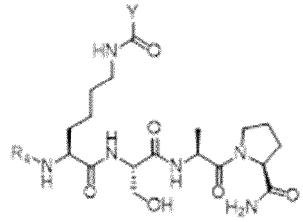

wherein $R_4$ is selected from

$R_4$

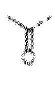 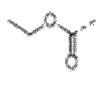 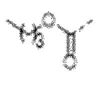 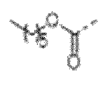 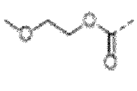 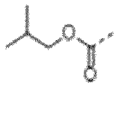 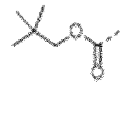

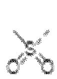 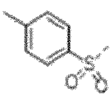 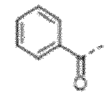 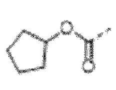 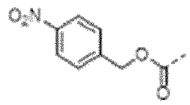 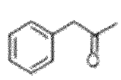 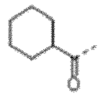

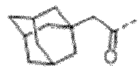 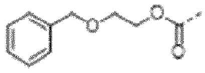 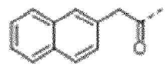 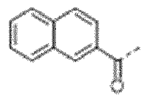 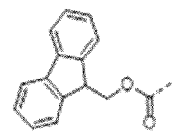

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIV)

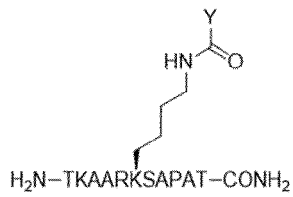

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXV)

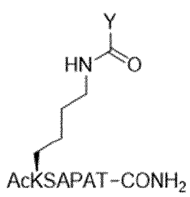

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVI)

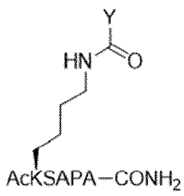

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVII)

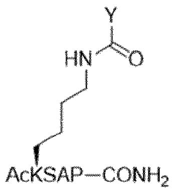

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVIII)

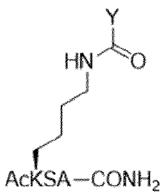

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIX)

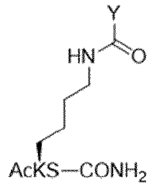

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXX)

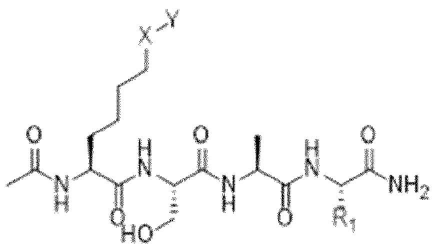

wherein $R_1$ is selected from

$R_1$ 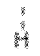 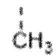 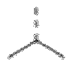 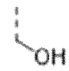 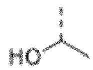 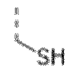 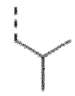

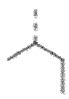 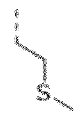 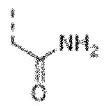 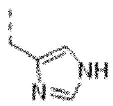 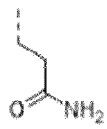 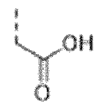 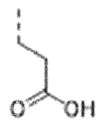

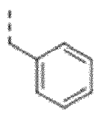 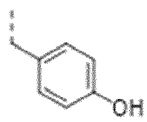 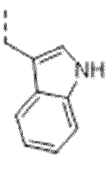 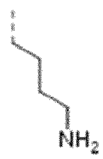 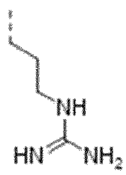

X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXI)

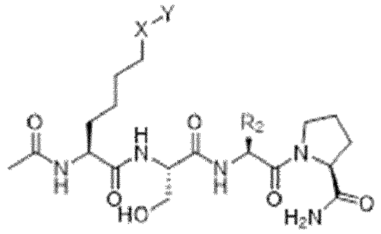

wherein $R_2$ is selected from

$R_2$ 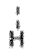 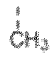 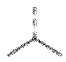 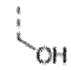 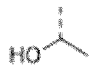 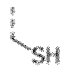 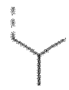

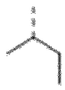 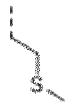 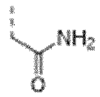 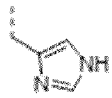 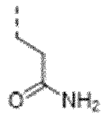 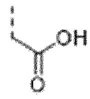 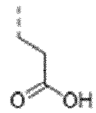

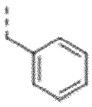 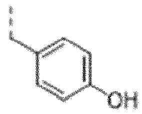 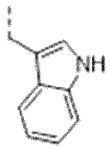 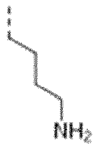 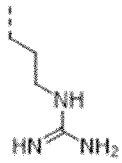

X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXII)

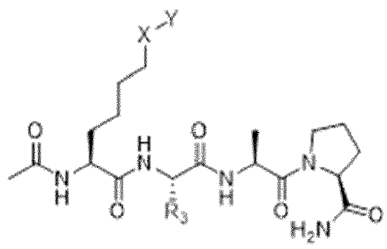

wherein $R_3$ is selected from

$R_3$ 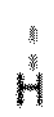 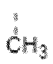 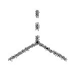 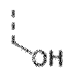 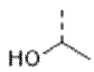 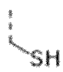 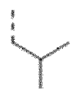

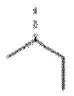 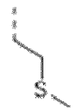 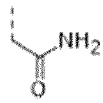 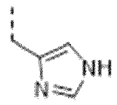 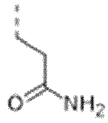 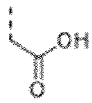 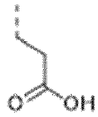

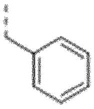 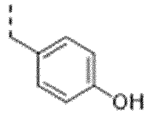 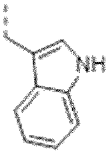 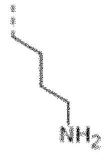 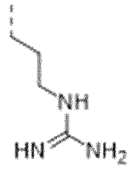

X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIII)

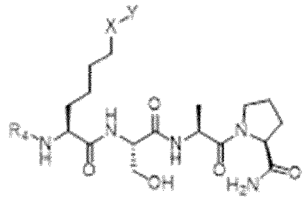

wherein $R_4$ is selected from

$R_4$ 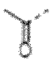 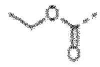 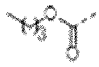 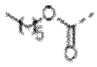 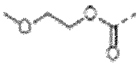 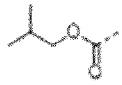 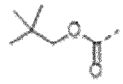

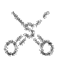 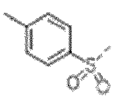 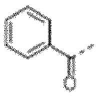 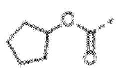 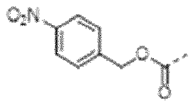 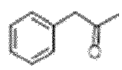 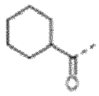

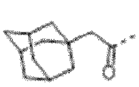 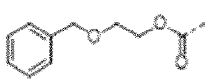 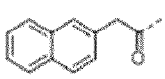 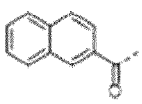 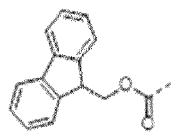

X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIV)

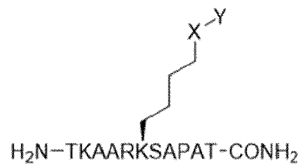

wherein X is selected from NH-C=O, NH-C=S, O-C=O, C=O-O, NH-$SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXV)

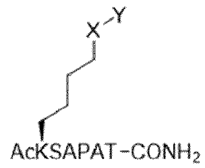

wherein X is selected from NH-C=O, NH-C=S, O-C=O, C=O-O, NH-$SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVI)

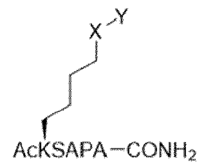

wherein X is selected from NH-C=O, NH-C=S, O-C=O, C=O-O, NH-$SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVII)

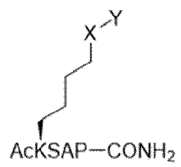

wherein X is selected from NH-C=O, NH-C=S, O-C=O, C=O-O, NH-$SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVIII)

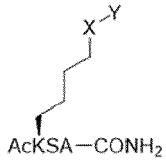

wherein X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIX)

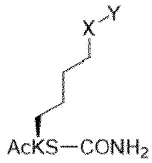

wherein X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XL)

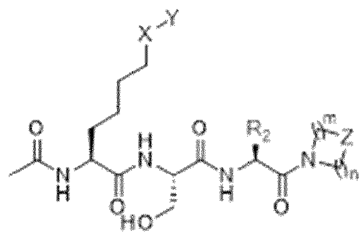

wherein $R_2$ is selected from

$R_2$ 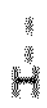 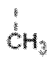 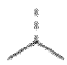 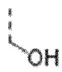 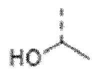 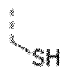 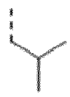

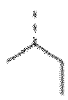 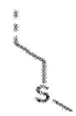 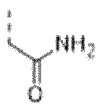 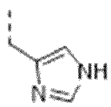 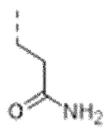 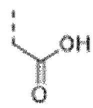 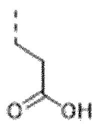

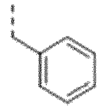
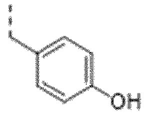
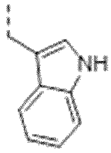
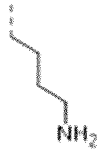
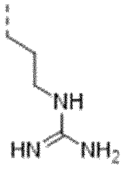

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
Z is selected from $CH_2$, O, NH, S, N-Me; and
m and n are each independently integers from 0 to 8, wherein at least one of m or n is not 0

Formula (XLI)

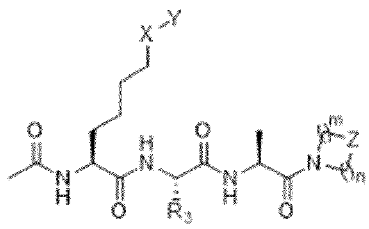

wherein $R_3$ is selected from

$R_3$ 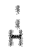 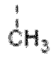 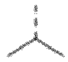 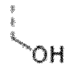 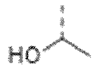 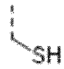 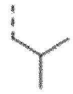

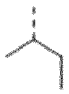 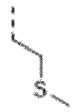 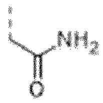 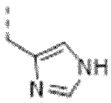 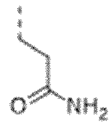 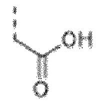 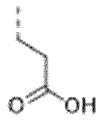

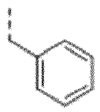 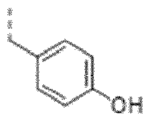 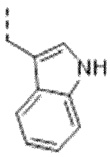 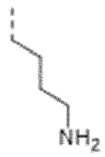 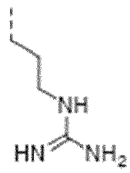

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
Z is selected from $CH_2$, O, NH, S, N-Me; and
m and n are each independently integers from 0 to 8, wherein at least one of m or n is not 0

Formula (XLII)

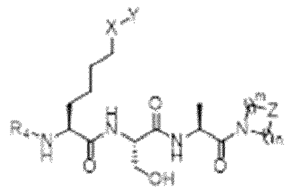

wherein $R_4$ is selected from

$R_4$ 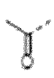 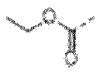 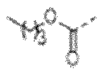 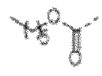 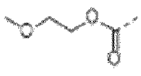 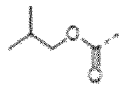 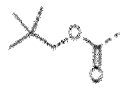

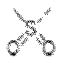 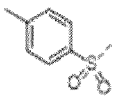 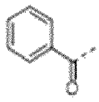 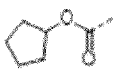 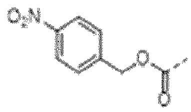 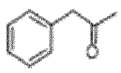 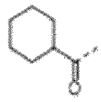

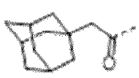 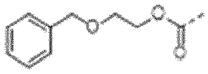 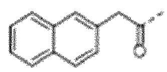 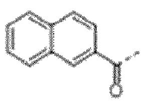 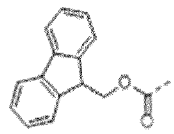

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
Z is selected from $CH_2$, O, NH, S, N-Me; and
m and n are each independently integers from 0 to 8, wherein at least one of m or n is not 0

Formula (XLIII)

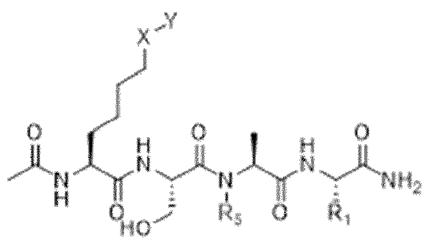

wherein $R_1$ is selected from

$R_1$ 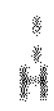 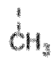 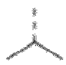 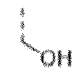 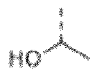 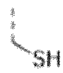 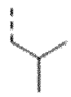

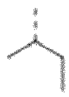 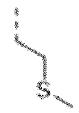 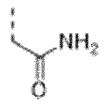 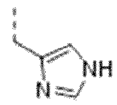 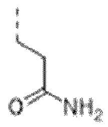 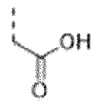 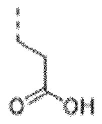

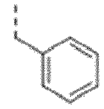 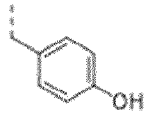 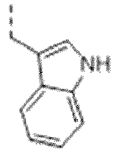 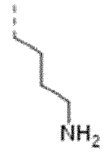 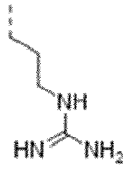

X is selected from NH-C=O, NH-C=S, O-C=O, C=O-O, $NH\text{-}SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N; and
$R_5$ is selected from H, Me, Et, Pr, or iPr

Formula (XLIV)

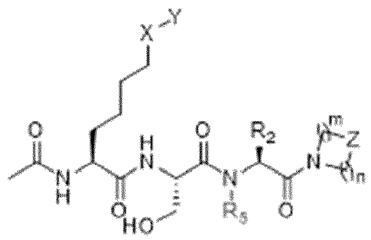

wherein $R_2$ is selected from

$R_2$ 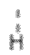 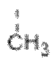 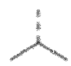 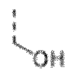 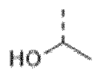 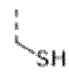 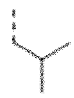

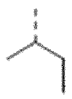 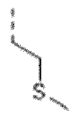 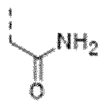 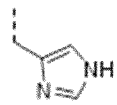 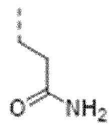 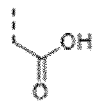 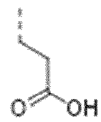

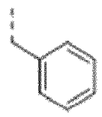 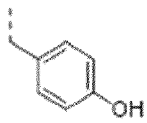 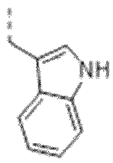 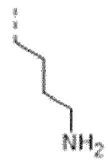 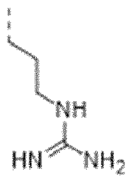

X is selected from NH-C=O, NH-C=S, O-C=O, C=O-O, $NH\text{-}SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;

Z is selected from $CH_2$, O, NH, S, C=O, N; and
$R_5$ is selected from H, Me, Et, Pr, or iPr

Formula (XLV)

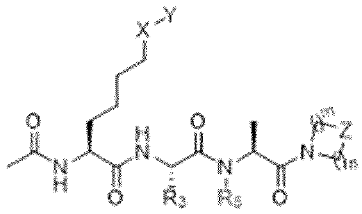

wherein $R_3$ is selected from

$R_3$ 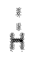 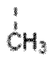 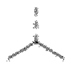 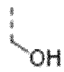 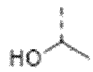 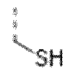 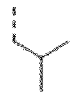

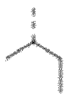 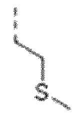 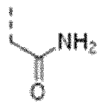 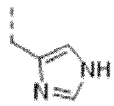 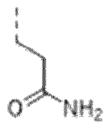 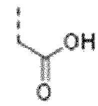 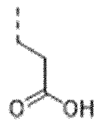

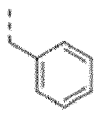 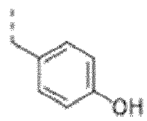 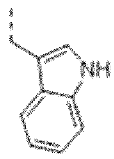 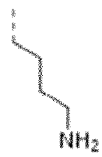 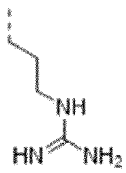

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N; and
$R_5$ is selected from H, Me, Et, Pr, or iPr

Formula (XLVI)

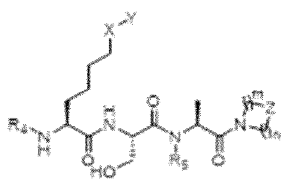

wherein $R_4$ is selected from

$R_4$ 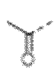 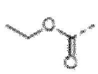 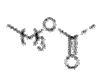 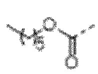 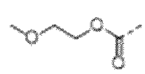 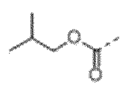 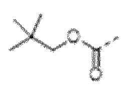

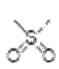 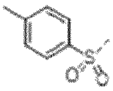 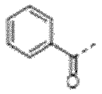 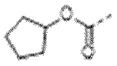 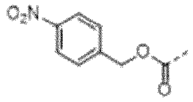 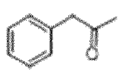 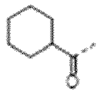

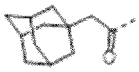 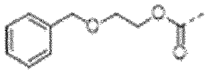 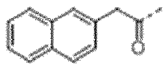 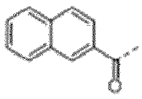 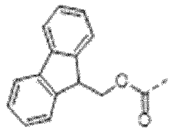

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N; and
$R_5$ is selected from H, Me, Et, Pr, or iPr

Formula (XLVII)

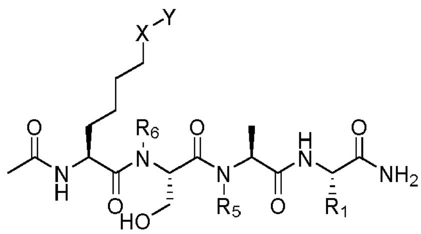

wherein $R_1$ is selected from

$R_1$ 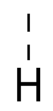 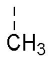 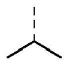 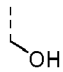 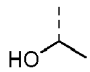 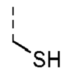 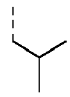

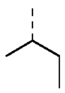 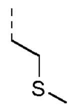 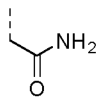 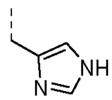 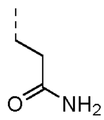 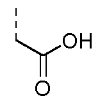 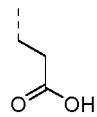

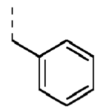 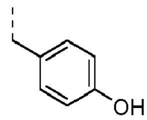 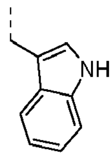 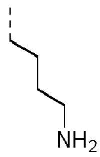 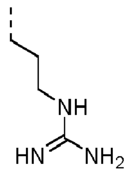

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (XLVIII)

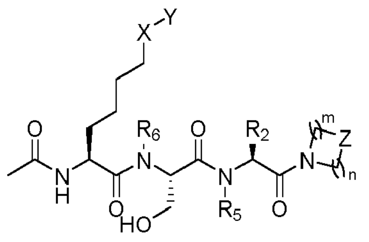

wherein $R_2$ is selected from

$R_2$ 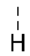 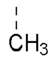 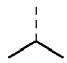 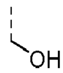 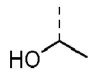 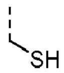 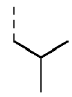

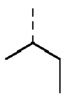 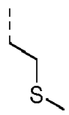 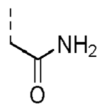 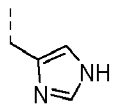 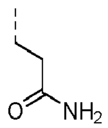 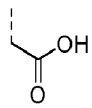 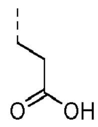

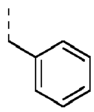 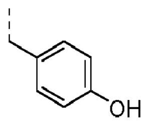 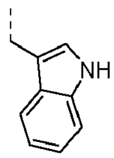 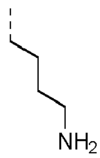 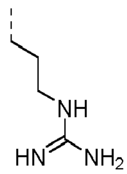

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (XLIX)

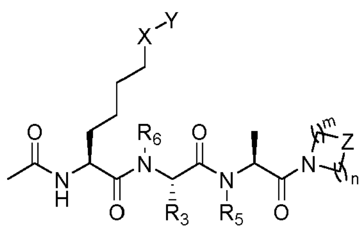

wherein $R_3$ is selected from

$R_3$ 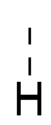 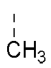 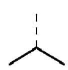 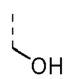 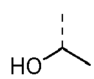 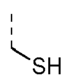 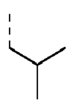

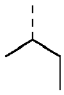 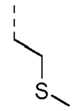 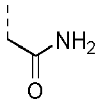 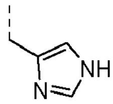 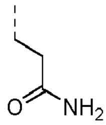 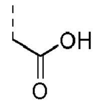 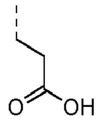

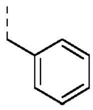 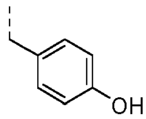 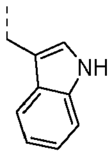 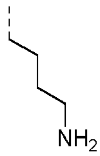 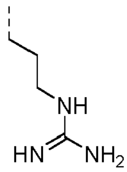

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (L)

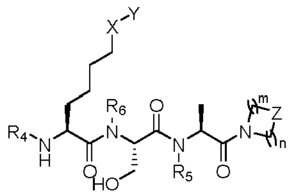

Wherein $R_4$ is selected from

$R_4$ 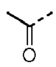 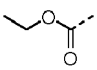 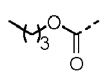 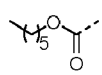 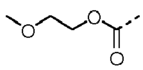 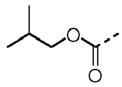 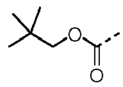

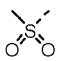 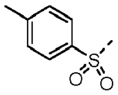 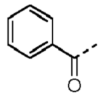 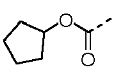 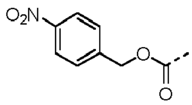 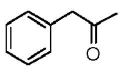 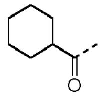

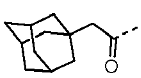 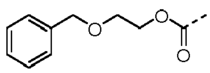 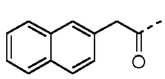 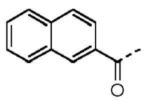 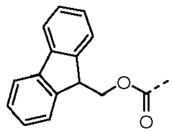

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (LI)

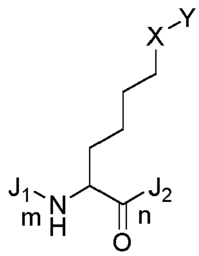

wherein X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
J1 and J2 is independently any $\alpha$ amino acid; and
m and n are each independently integers from 0 to 10, wherein at least one of m or n is not 0

Formula (LIII)

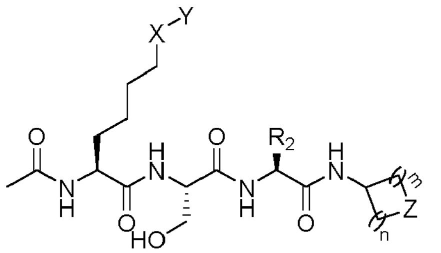

wherein X is NH-C=O, NH-C=S, O-C=O, C=O-O, or $NH-SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 8, wherein at least one of m or n is not 0; and
Z is $CH_2$, O, NH, S, or N-Me

$R_2$ 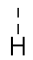 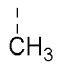 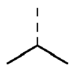 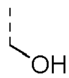 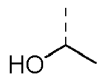 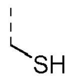 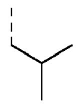

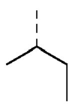 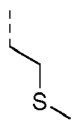 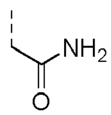 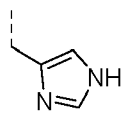 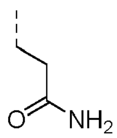 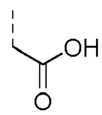 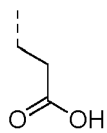

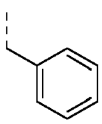 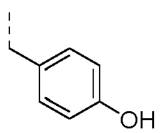 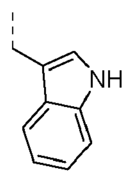 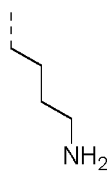 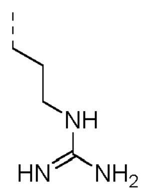

Formula (LIV)

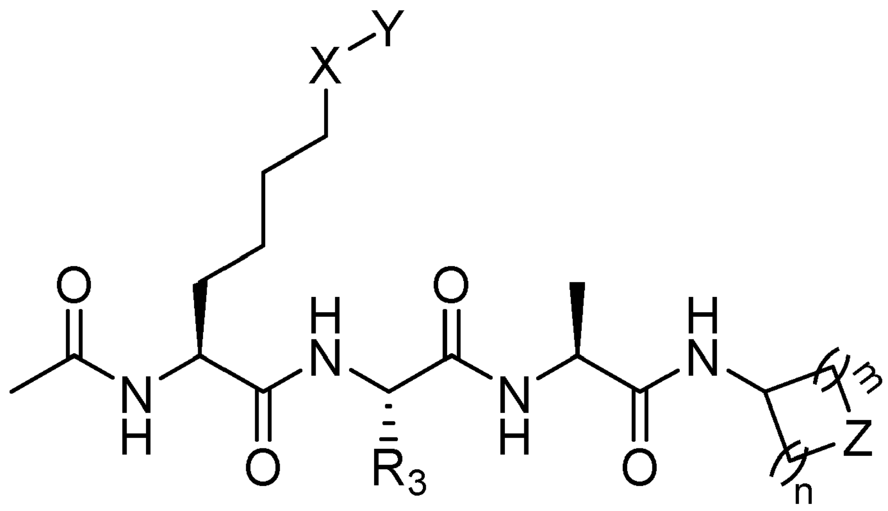

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 8, wherein at least one of m or n is not 0; and
Z is $CH_2$, O, NH, S, or N-Me

$R_3$ 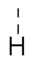 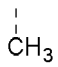 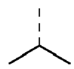 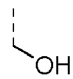 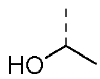 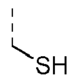 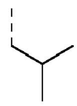

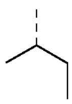 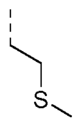 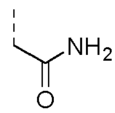 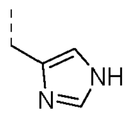 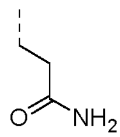 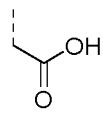 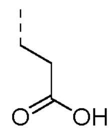

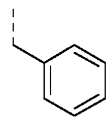 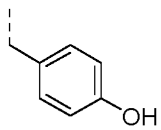 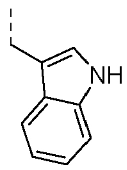 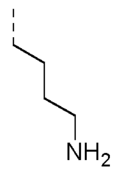 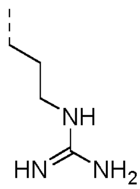

Formula (LV)

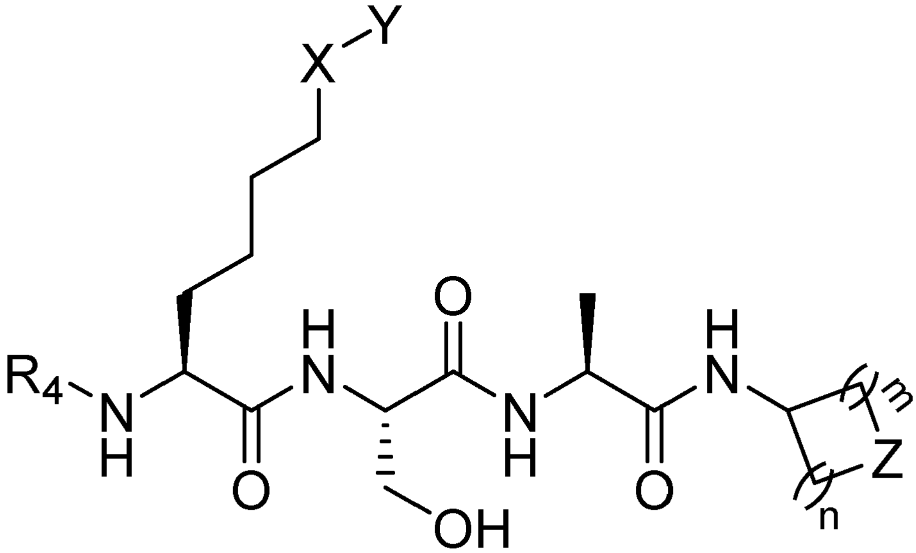

wherein X is NH-C=O, NH-C=S, O-C=O, C=O-O, or NH-$SO_2$;

Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0 to 8, wherein at least one of m or n is not 0; and

Z is $CH_2$, O, NH, S, or N-Me

$R_4$

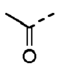 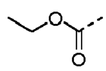 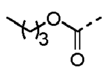 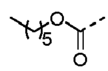 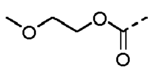 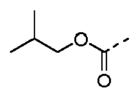 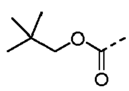

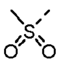 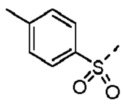 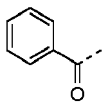 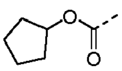 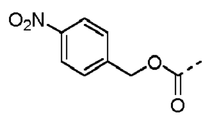 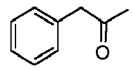 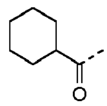

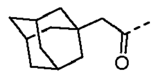 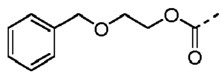 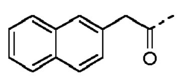 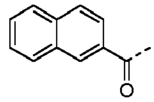 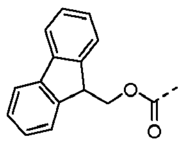

Formula (LVI)

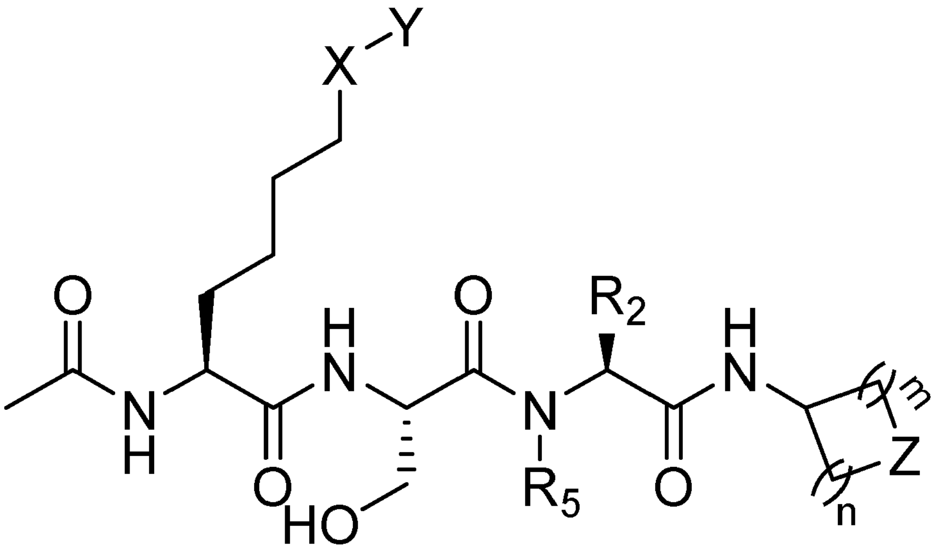

wherein X is NH-C=O, NH-C=S, O-C=O, C=O-O, or NH-$SO_2$;

Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0-10;

Z is $CH_2$, O, NH, S, C=O, or N; and

$R_5$ is H, Me, Et, Pr, or iPr

$R_2$ 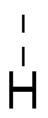 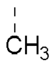 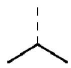 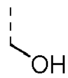 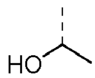 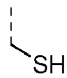 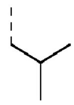

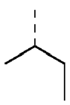 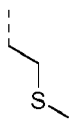 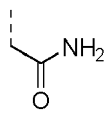 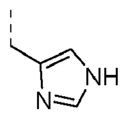 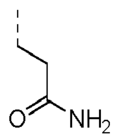 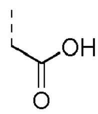 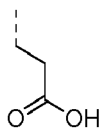

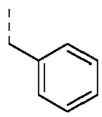 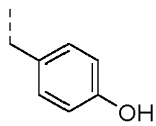 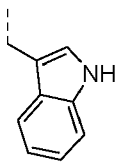 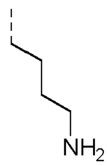 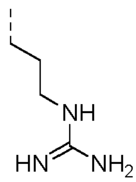

Formula (LVII)

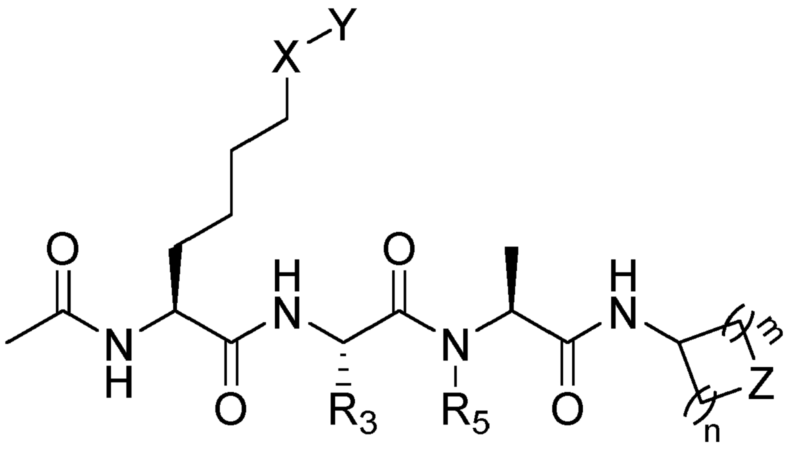

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S, C=O, or N; and
$R_5$ is H, Me, Et, Pr, or iPr

$R_3$ 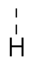 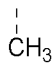 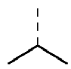 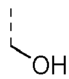 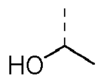 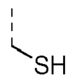 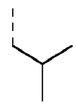

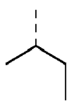 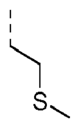 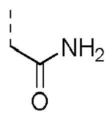 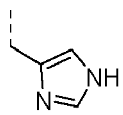 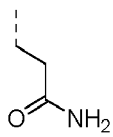 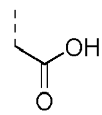 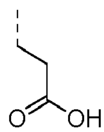

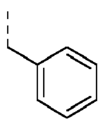 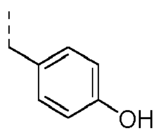 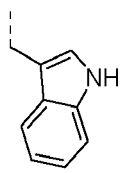 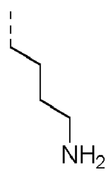 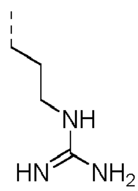

Formula (LVIII)

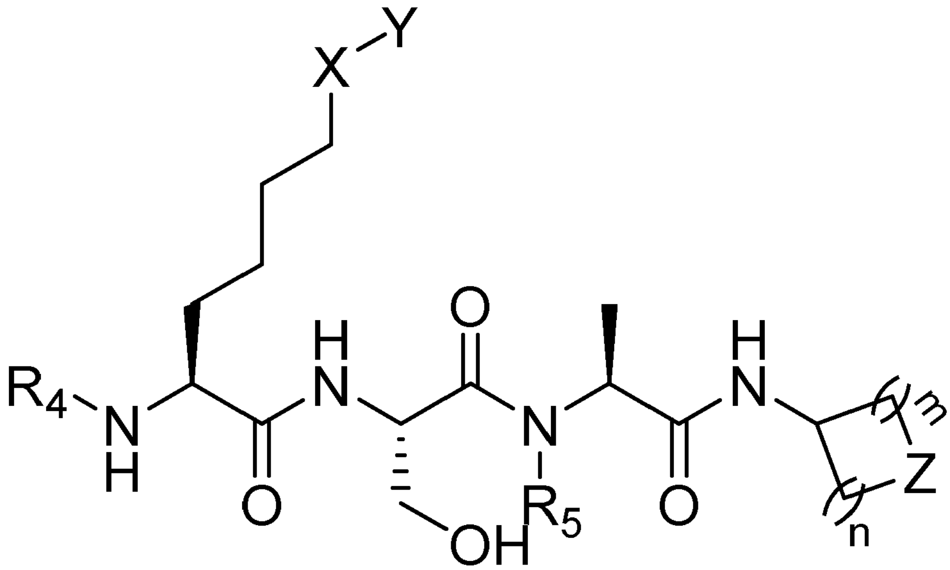

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S, C=O, or N; and
$R_5$ is H, Me, Et, Pr, or iPr

$R_4$

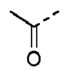 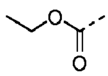 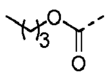 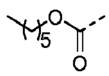 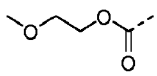 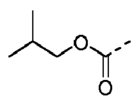 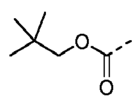

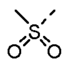 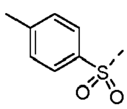 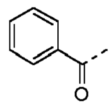 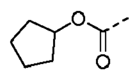 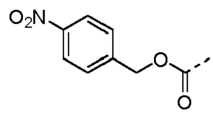 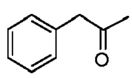 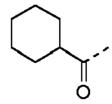

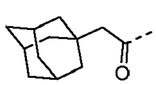 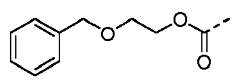 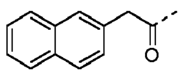 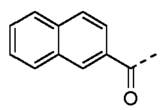 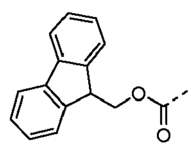

Formula (LIX)

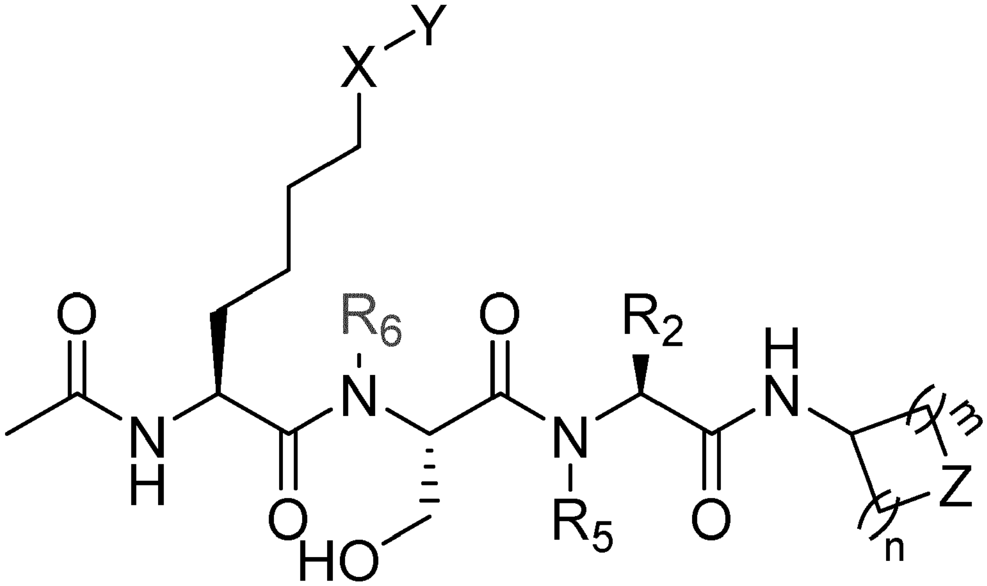

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S,C=O, or N;
$R_5$ is H, Me, Et, Pr, or iPr; and
$R_6$ is H, Me, Et, Pr, or iPr

$R_2$ 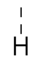 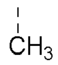 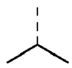 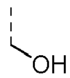 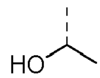 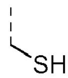 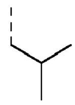

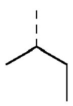 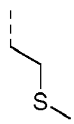 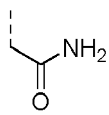 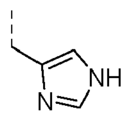 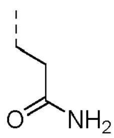 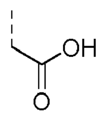 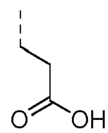

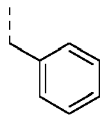 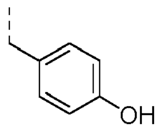 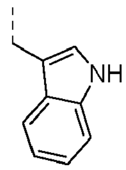 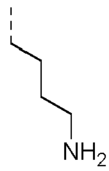 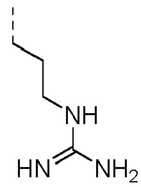

Formula (LX)

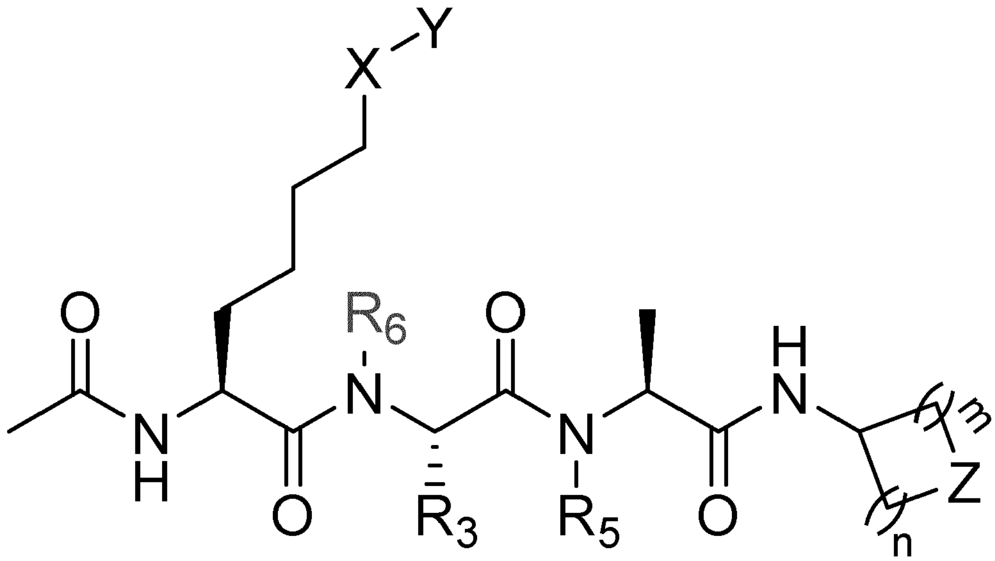

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S,C=O, or N;
$R_5$ is H, Me, Et, Pr, or iPr; and
$R_6$ is H, Me, Et, Pr, or iPr

$R_3$ 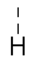 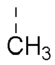 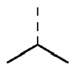 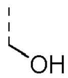 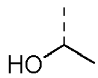 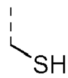 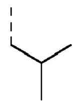

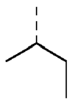 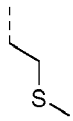 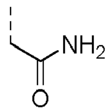 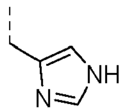 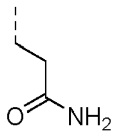 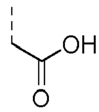 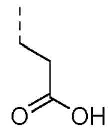

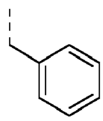 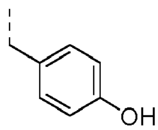 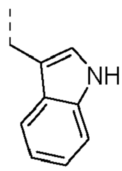 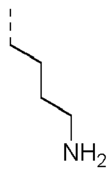 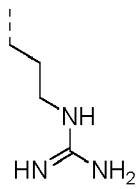

Formula (LXI)

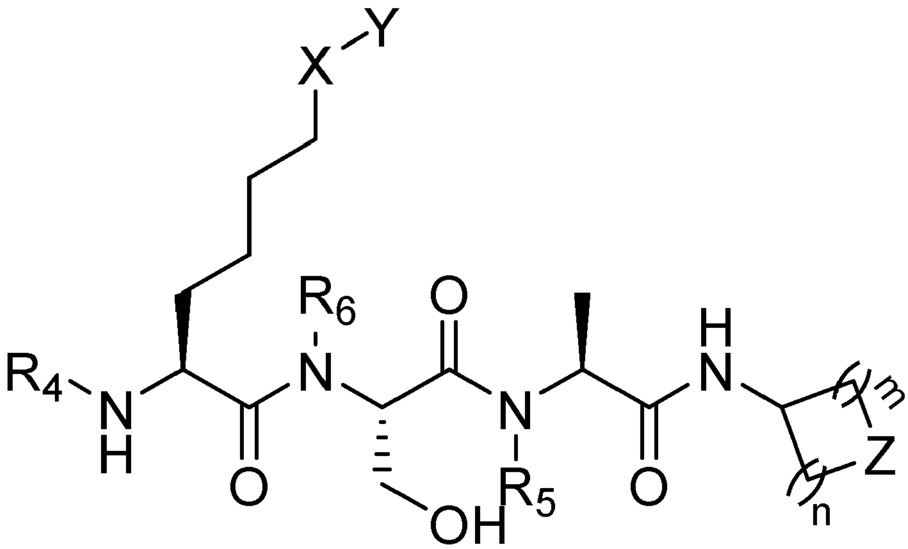

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S,C=O, or N;
$R_5$ is H, Me, Et, Pr, or iPr; and
$R_6$ is H, Me, Et, Pr, or iPr

$R_4$ 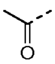 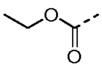 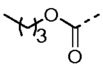 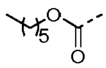 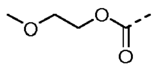 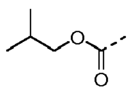 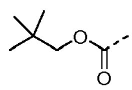

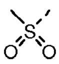 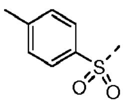 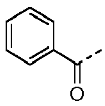 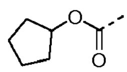 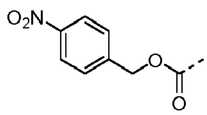 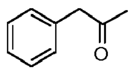 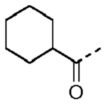

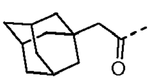 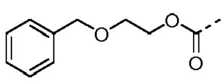 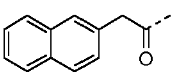 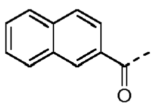 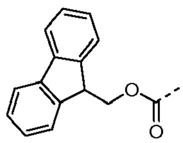

**9.** The compound of claim 7, wherein the compound is HC25b (formula (II)) or HC26b (formula (III)):

formula (II)

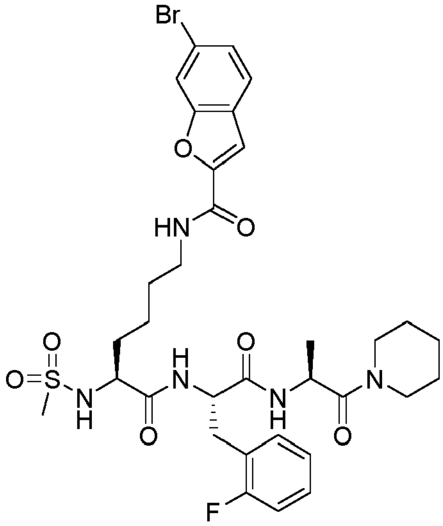

formula (III)

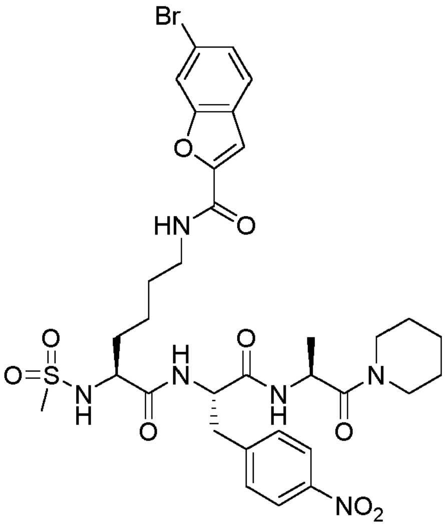

.

**10.** An anti-cancer composition comprising a YEATS2 YEATS inhibitor.

**11.** The composition of claim 10, further comprising pharmaceutically effective carriers and/or excipients.

**12.** The composition of claim 10, wherein the YEATS2 YEATS inhibitor is according to formula (I), formula (II), formula (III), formula (V), formula (VI), formula (VII), formula (VIII), formula (IX), formula (X), formula (XI), formula (XII), formula (XIII), formula (XIV), formula (XV), formula (XVI), formula (XVII), formula (XVIII), formula (XIX), formula (XX), formula (XXI), formula (XXII), formula (XXIII), formula (XXIV), formula (XXV), formula (XXVI), formula (XXVII), formula (XXVIII), formula (XXIX), formula (XXX), formula (XXXI), formula (XXXII), formula (XXXIII), formula (XXXIV), formula (XXXV), formula (XXXVI), formula (XXXVII), formula (XXXVIII), formula (XXXIX), formula (XL), formula (XLI), formula (XLII), formula (XLIII), formula (XLIV), formula (XLV), formula (XLVI), formula (XLVII), formula (XLVIII), formula (XLIX), formula (L), formula (LI), formula (LIII), formula (LIV), formula (LV), formula (LVI), formula (LVII), formula (LVIII), formula (LIX), formula (LX), or formula (LXI):

Formula (I)

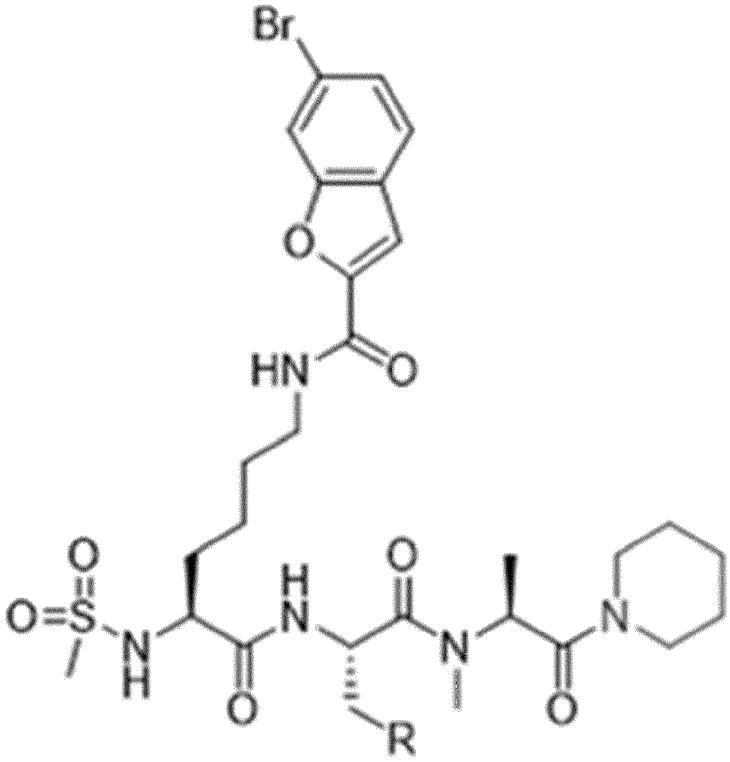

wherein R is selected from

R:

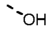
LS100

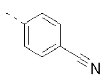
LS101

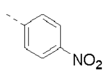
LS102

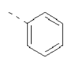
LS103

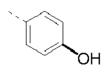
LS104

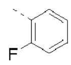
LS123

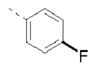
LS124

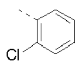
LS130

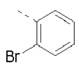
LS131

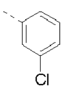
LS132

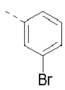
LS133

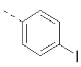
LS134

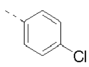
LS135

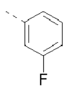
LS136

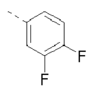
LS137

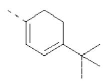
LS138

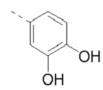
LS139

Formula (II)

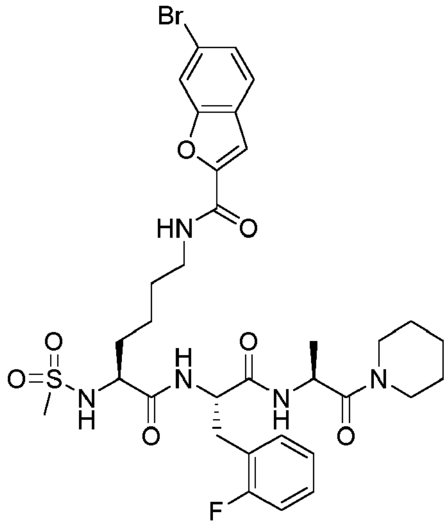

Formula (III)

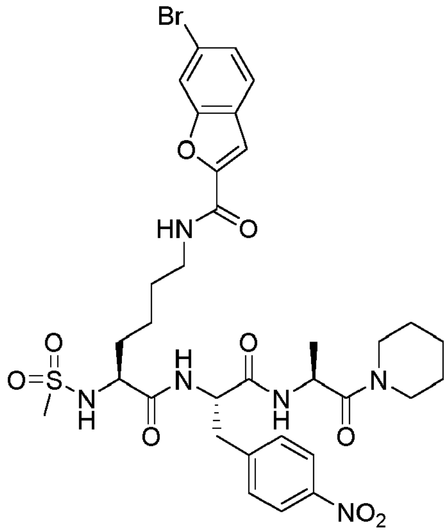

Formula (V)

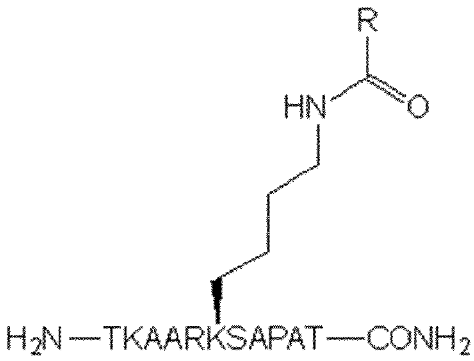

wherein R is selected from

R=

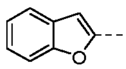
benfu

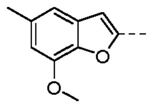
CA-1

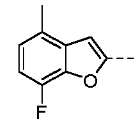
CA-2

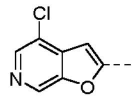
CA-3

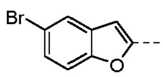
CA-8

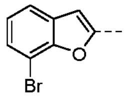
CA-9

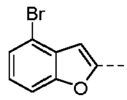
CA-10

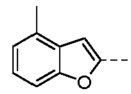
CA-11

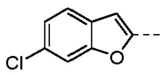
CA-16

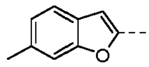
CA-17

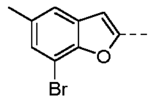
CA-18

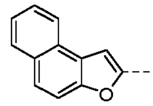
CA-19

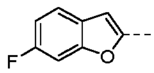
CA-24

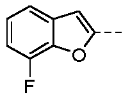
CA-25

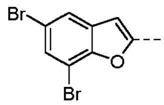
CA-26

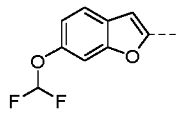
CA-27

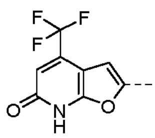
CA-32

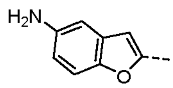
CA-33

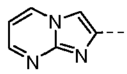
CA-34

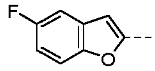
CA-35

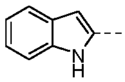
CA-40

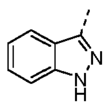
CA-41

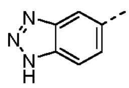
CA-42

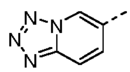
CA-43

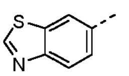
CA-48

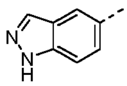
CA-49

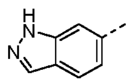
CA-50

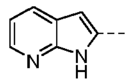
CA-51

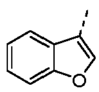
CA-56

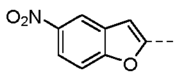
CA-57

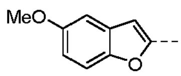
CA-58

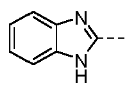
CA-59

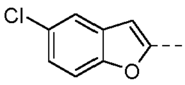
CA-4

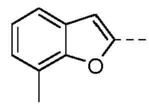
CA-5

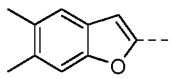
CA-6

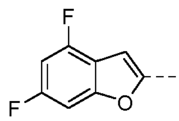
CA-7

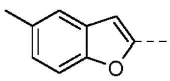
CA-12

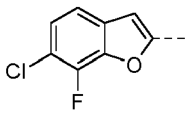
CA-13

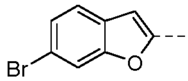
CA-14

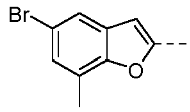
CA-15

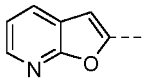
CA-20

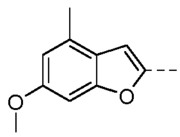
CA-21

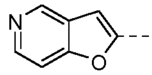
CA-22

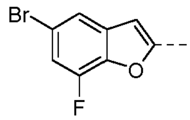
CA-23

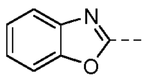
CA-28

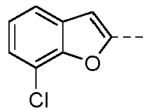
CA-29

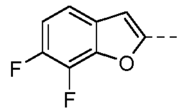
CA-30

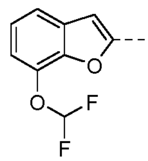
CA-31

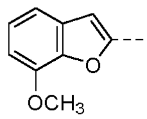
CA-36

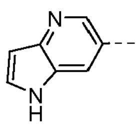
CA-37

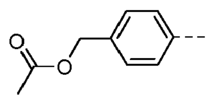
CA-38

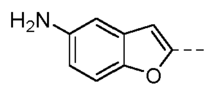
CA-39

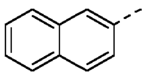
CA-44

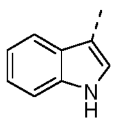
CA-45

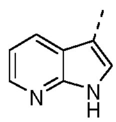
CA-46

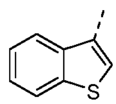
CA-47

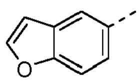
CA-52

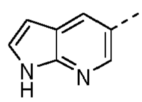
CA-53

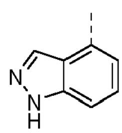
CA-54

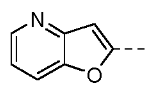
CA-55

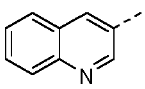
CA-60

Formula (VI)

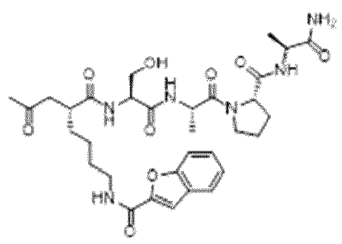

AcKbfSAPA

Formula (VII)

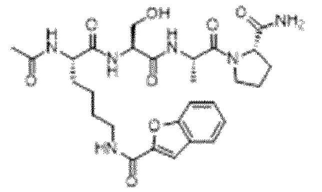

AcKbfSAP

Formula (VIII)

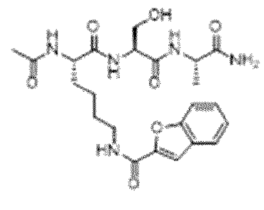

AcKbfSA

Formula (IX)

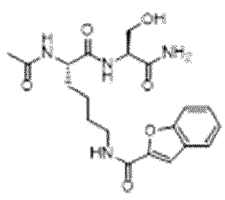

AcKbfS

Formula (X)

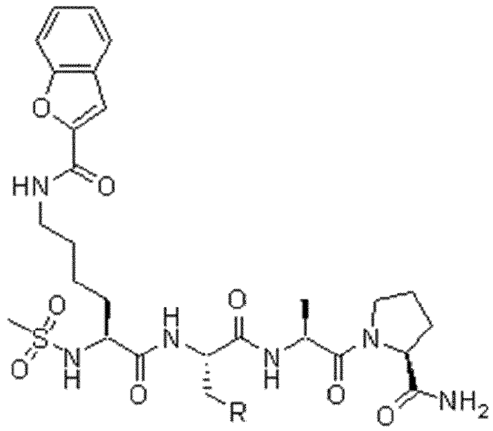

wherein R is selected from

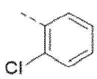 HC1 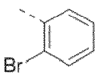 HC2 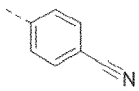 HC3 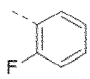 HC4 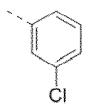 HC5 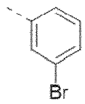 HC6 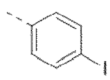 HC7

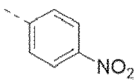 HC8 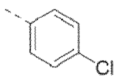 HC9 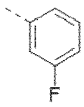 HC10 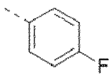 HC11 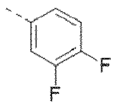 HC12 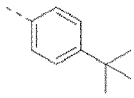 HC13 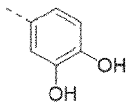 HC14

Formula (XI)

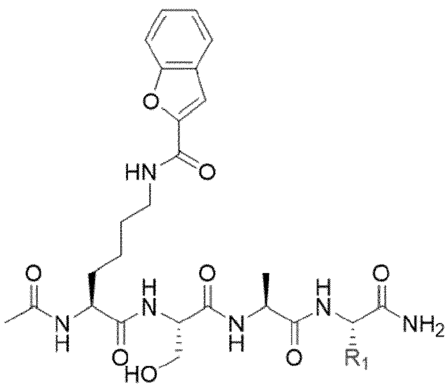

Wherein $R_1$ is selected from:

$R_1$ 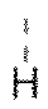 1-G 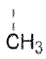 1-A 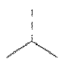 1-V 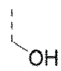 1-S 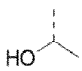 1-T 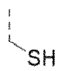 1-C 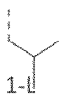 1-L

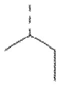 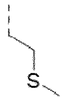 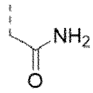 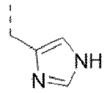 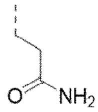 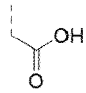 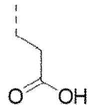

1-I 1-M 1-N 1-H 1-Q 1-D 1-E

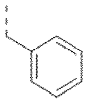 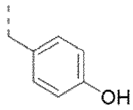 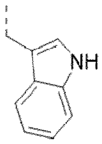 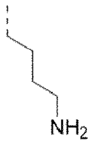 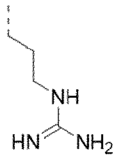

1-F 1-Y 1-W 1-K 1-R

Formula (XII)

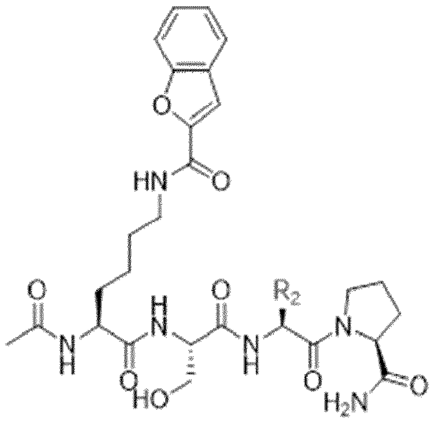

wherein $R_2$ is selected from

$R_2$ 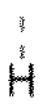 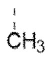 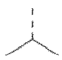 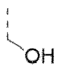 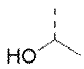 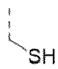 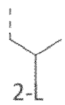

2-G 2-A 2-V 2-S 2-T 2-C 2-L

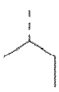 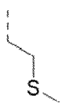 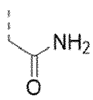 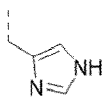 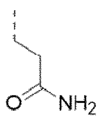 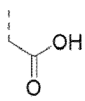 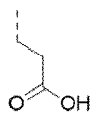

2-I 2-M 2-N 2-H 2-Q 2-D 2-E

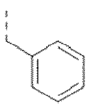 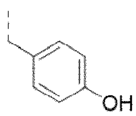 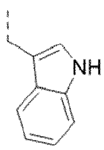 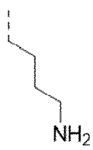 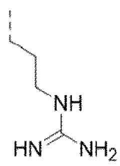

2-F 2-Y 2-W 2-K 2-R

Formula (XIII)

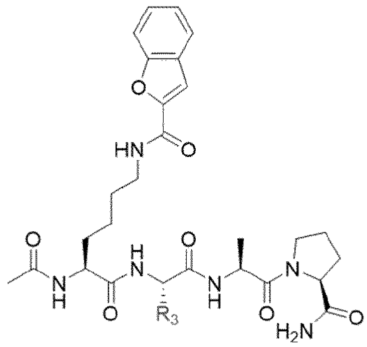

wherein $R_3$ is selected from:

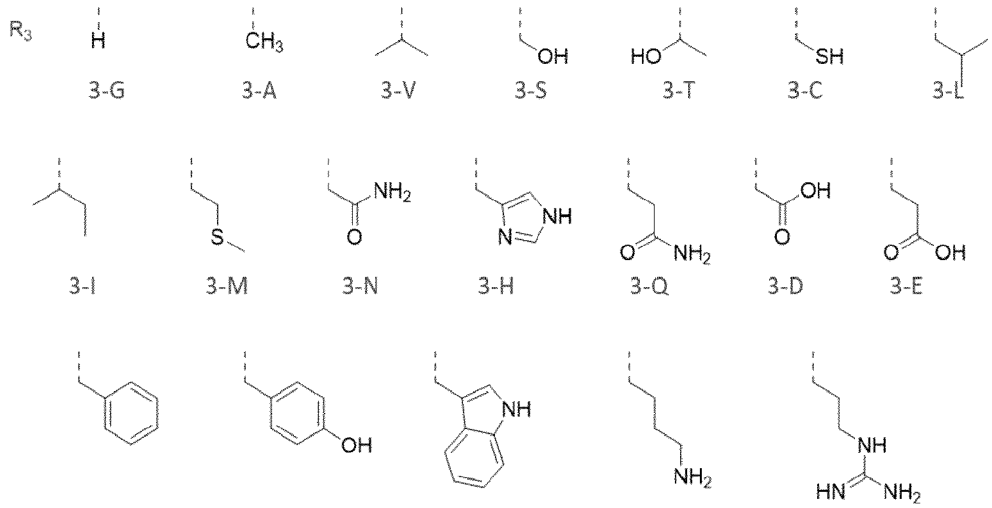

3-G 3-A 3-V 3-S 3-T 3-C 3-L

3-I 3-M 3-N 3-H 3-Q 3-D 3-E

3-F 3-Y 3-W 3-K 3-R

Formula (XIV)

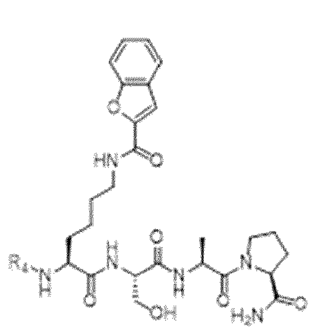

wherein $R_4$ is selected from:

$R_4$

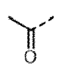
4-1

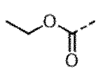
4-2

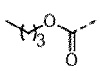
4-3

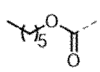
4-4

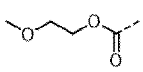
4-5

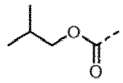
4-6

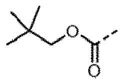
4-7

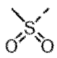
4-12

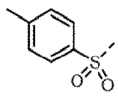
4-13

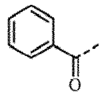
4-15

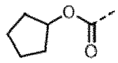
4-8

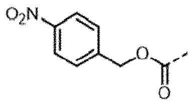
4-9

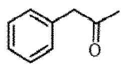
4-16

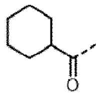
4-18

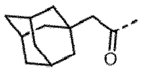
4-19

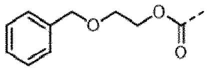
4-10

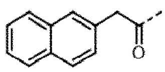
4-17

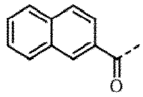
4-14

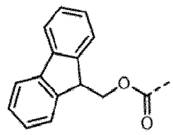
4-11

Formula (XV)

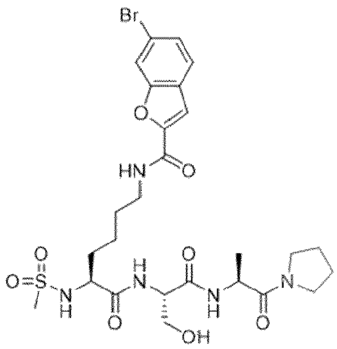

HC15a

Formula (XVI)

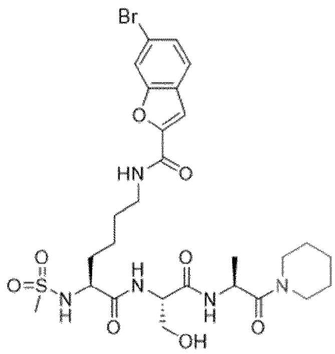

HC15b

Formula (XVII)

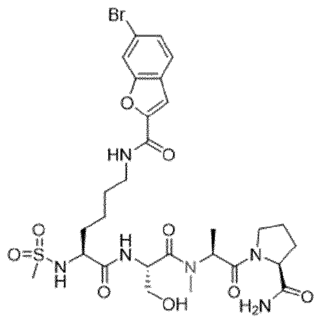

HC18

Formula (XVIII)

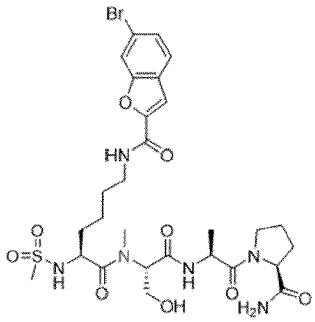

HC19

Formula (XIX)

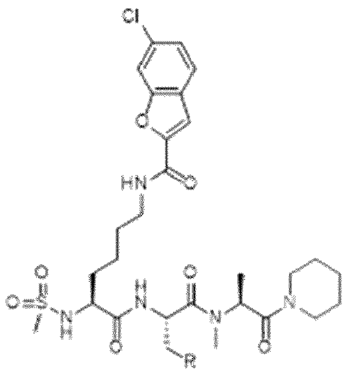

wherein R is selected from

R: 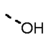 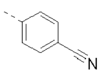 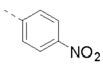 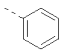 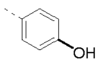 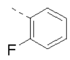 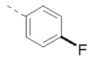

LS118 LS119 LS120 LS121 LS122 LS116 LS125

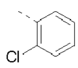
LS140
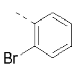
LS141
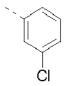
LS142
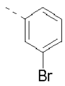
LS143
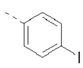
LS144
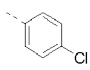
LS145
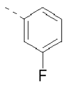
LS146
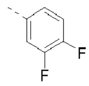
LS147
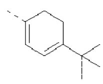
LS148
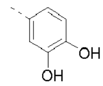
LS149

Formula (XX)

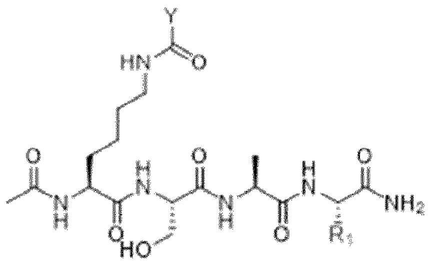

wherein $R_1$ is selected from

$R_1$

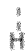
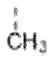
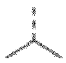
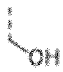
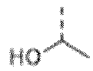
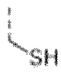
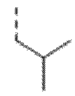
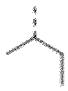
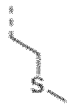
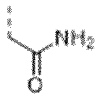
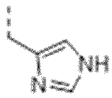
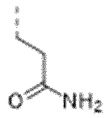
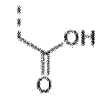
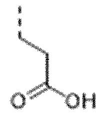
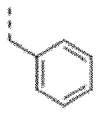
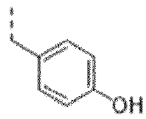
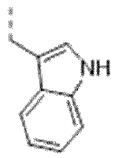
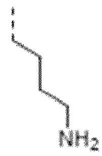
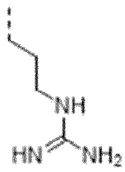

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXI)

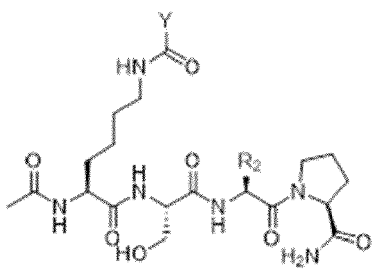

wherein $R_2$ is selected from

$R_2$

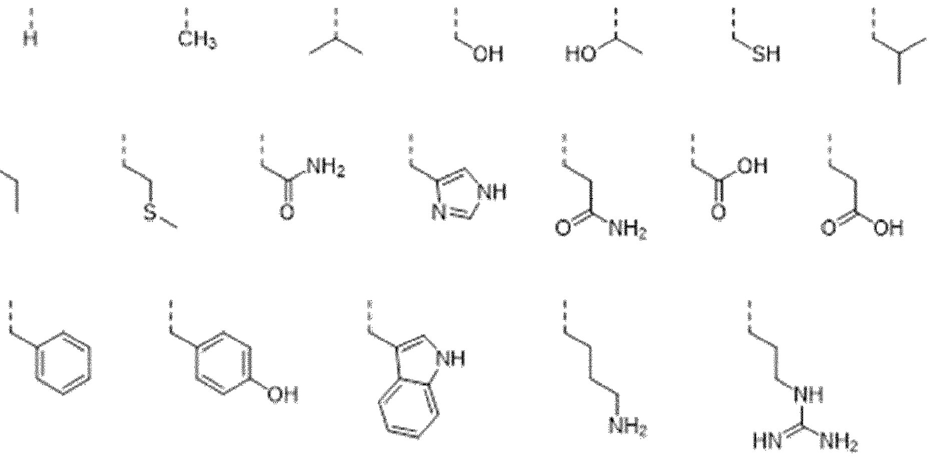

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXII)

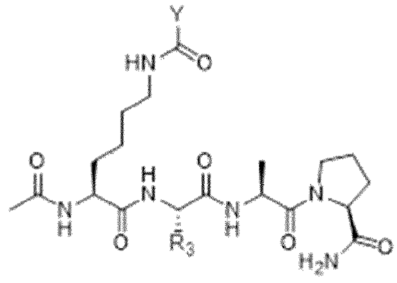

wherein $R_3$ is selected from

$R_3$

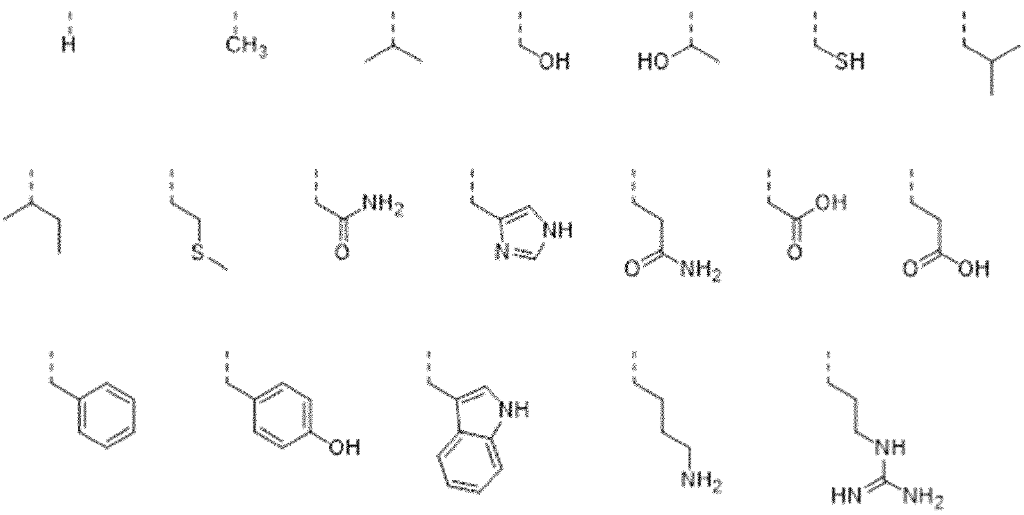

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIII)

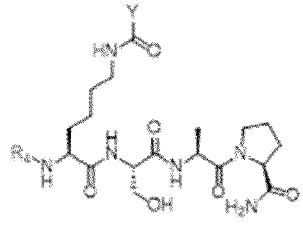

wherein $R_4$ is selected from

$R_4$

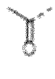 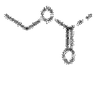 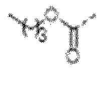 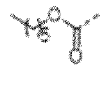 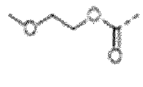 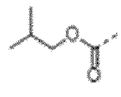 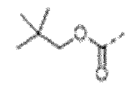

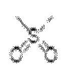 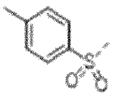 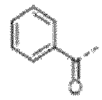 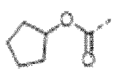 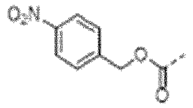 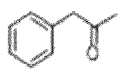 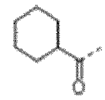

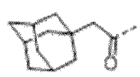 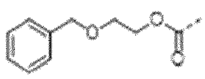 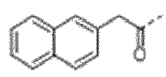 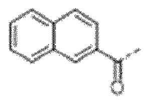 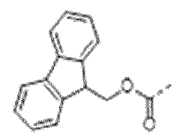

and Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIV)

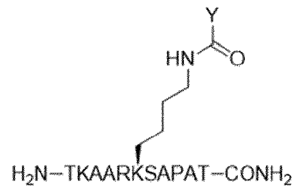

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXV)

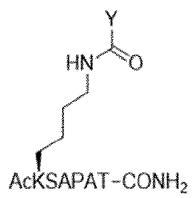

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVI)

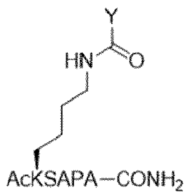

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVII)

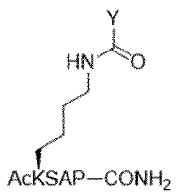

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXVIII)

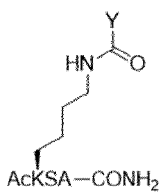

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXIX)

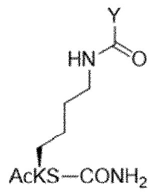

wherein Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized

Formula (XXX)

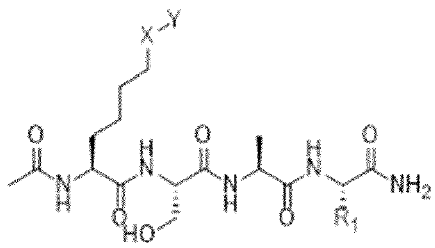

wherein $R_1$ is selected from

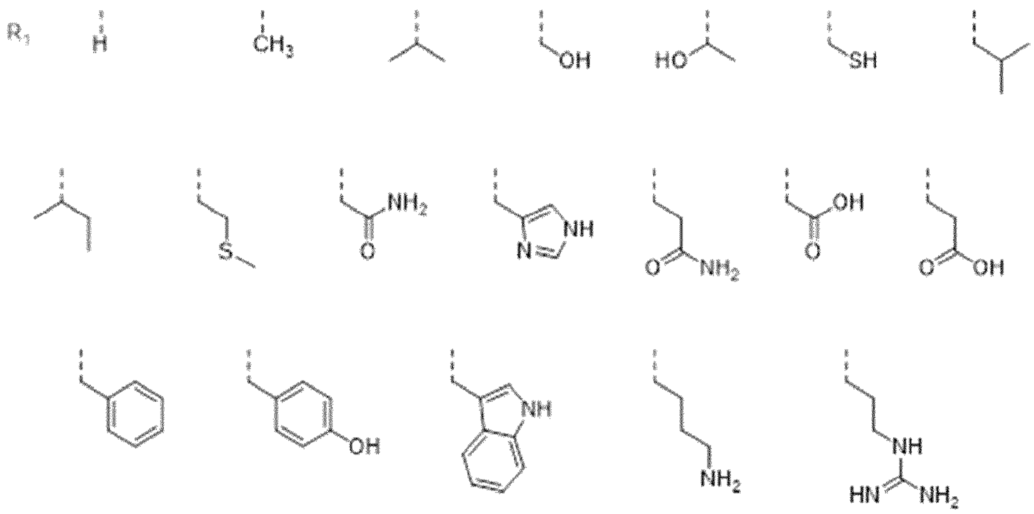

X is selected from NH-C=O, NH-C=S, O-C=O, C=O-O, NH-$SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXI)

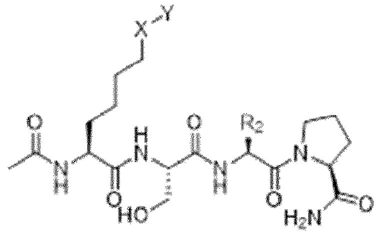

wherein $R_2$ is selected from

$R_2$ 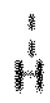 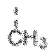 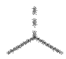 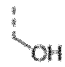 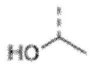 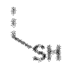 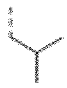

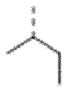 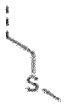 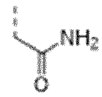 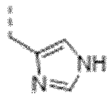 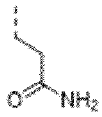 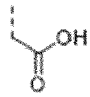 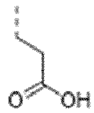

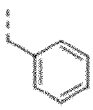 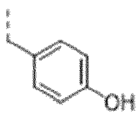 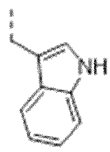 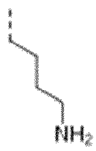 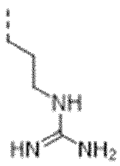

X is selected from NH-C=O, NH-C=S, O-C=O, C=O-O, NH-$SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXII)

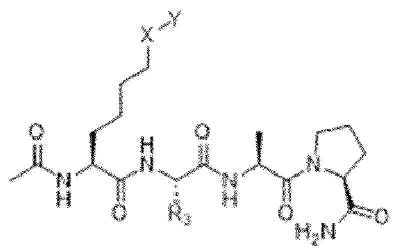

wherein $R_3$ is selected from

$R_3$ 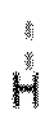 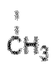 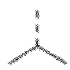 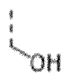 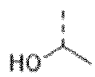 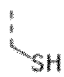 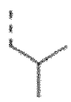

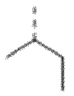 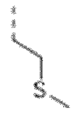 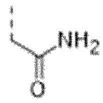 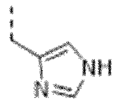 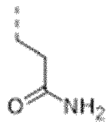 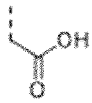 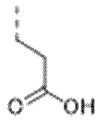

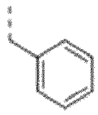 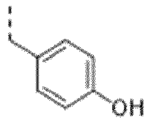 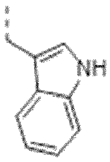 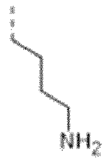 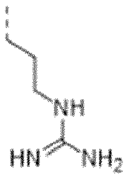

X is selected from NH-C=O, NH-C=S, O-C=O, C=O-O, $NH-SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIII)

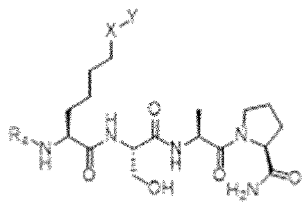

wherein $R_4$ is selected from

$R_4$ 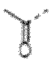 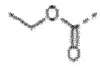 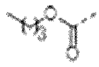 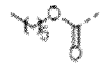 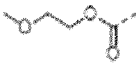 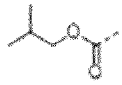 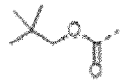

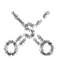 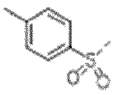 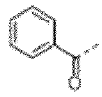 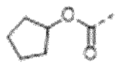 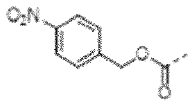 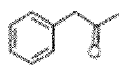 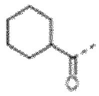

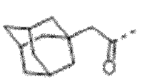 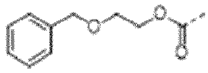 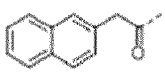 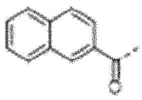 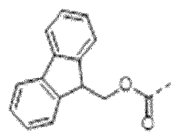

X is selected from NH-C=O, NH-C=S, O-C=O, C=O-O, $NH-SO_2$; and

Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIV)

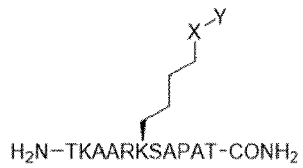

wherein X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXV)

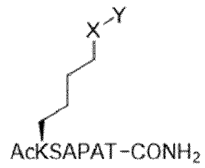

wherein X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVI)

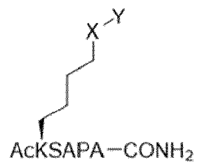

wherein X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVII)

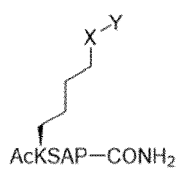

wherein X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXVIII)

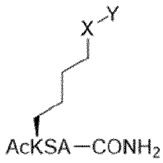

wherein X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XXXIX)

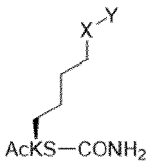

wherein X is selected from NH-C=O, NH-C=S, O-C=O, C=O-O, $NH-SO_2$; and
Y is selected from a conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons

Formula (XL)

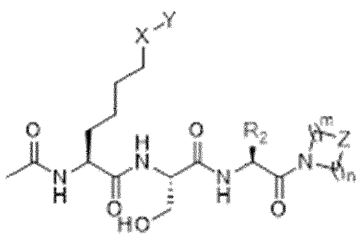

wherein $R_2$ is selected from

$R_2$ 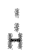 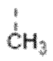 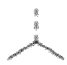 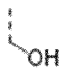 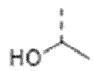 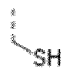 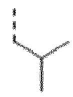

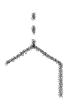 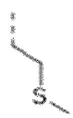 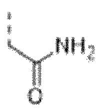 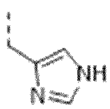 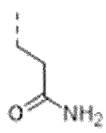 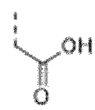 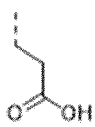

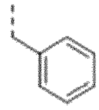 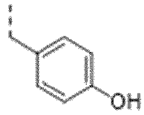 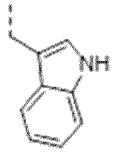 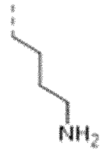 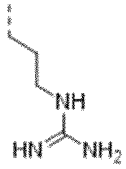

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
Z is selected from $CH_2$, O, NH, S, N-Me; and
m and n are each independently integers from 0 to 8, wherein at least one of m or n is not 0

Formula (XLI)

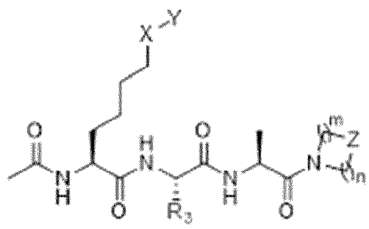

wherein $R_3$ is selected from

$R_3$ 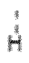 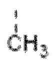 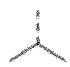 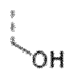 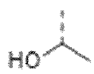 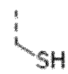 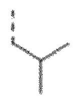

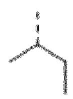 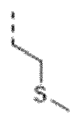 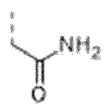 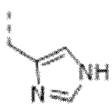 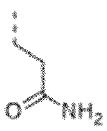 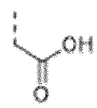 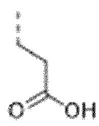

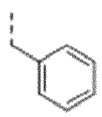 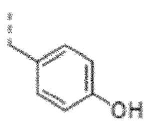 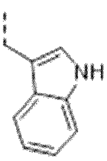 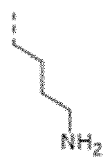 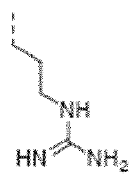

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
Z is selected from $CH_2$, O, NH, S, N-Me; and
m and n are each independently integers from 0 to 8, wherein at least one of m or n is not 0

Formula (XLII)

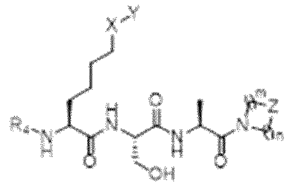

wherein $R_4$ is selected from

$R_4$ 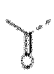 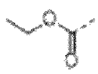 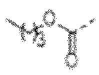 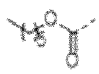 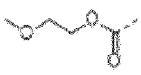 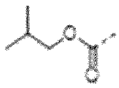 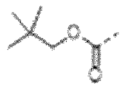

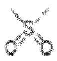 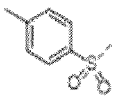 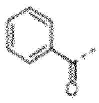 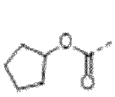 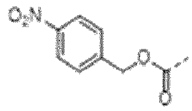 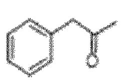 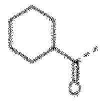

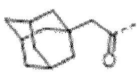 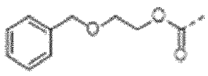 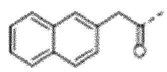 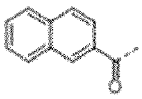 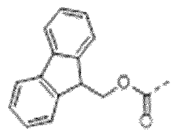

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized; Z is selected from $CH_2$, O, NH, S, N-Me; and
and m and n are each independently integers from 0 to 8, wherein at least one of m or n is not 0

Formula (XLIII)

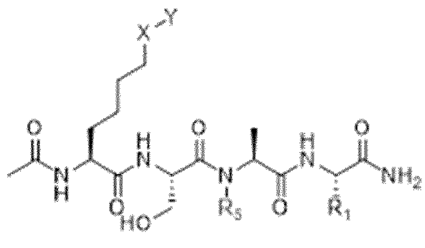

wherein $R_1$ is selected from

$R_1$ 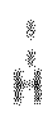 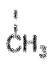 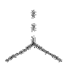 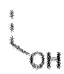 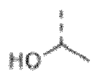 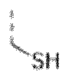 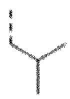

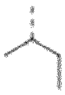 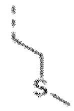 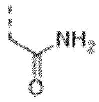 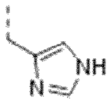 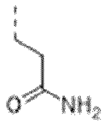 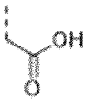 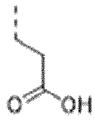

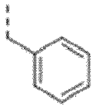 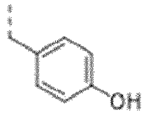 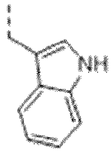 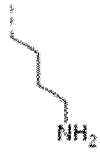 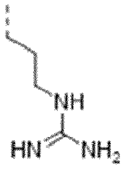

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N; and
$R_5$ is selected from H, Me, Et, Pr, or iPr

Formula (XLIV)

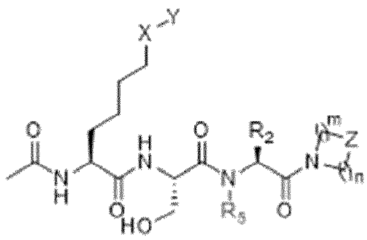

wherein $R_2$ is selected from

$R_2$ 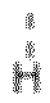 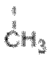 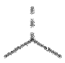 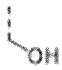 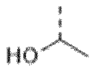 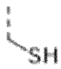 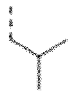

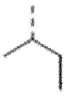 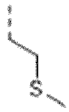 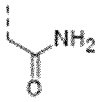 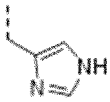 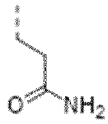 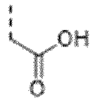 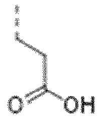

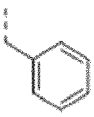 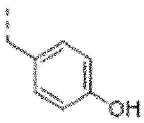 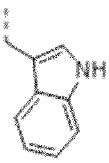 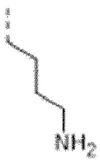 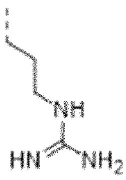

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;

Z is selected from $CH_2$, O, NH, S, C=O, N; and
$R_5$ is selected from H, Me, Et, Pr, or iPr

Formula (XLV)

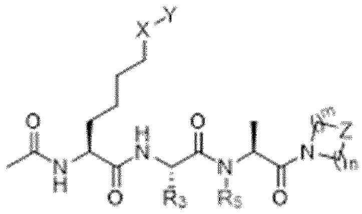

wherein $R_3$ is selected from

$R_3$ 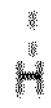 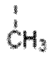 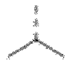 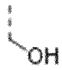 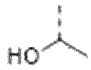 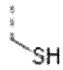 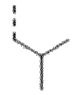

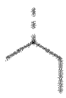 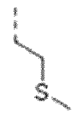 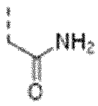 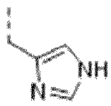 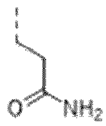 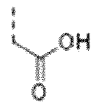 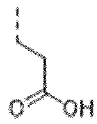

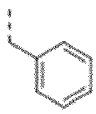 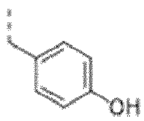 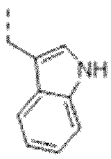 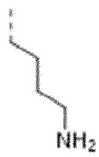 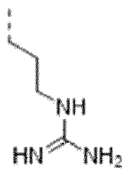

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N; and
$R_5$ is selected from H, Me, Et, Pr, or iPr

Formula (XLVI)

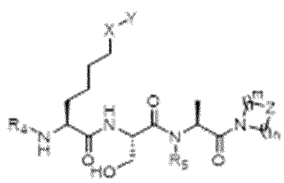

wherein $R_4$ is selected from

$R_4$ 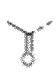 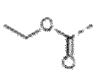 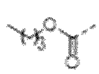 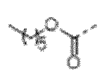 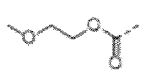 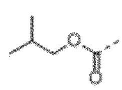 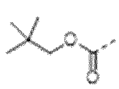

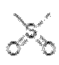
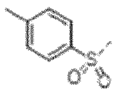
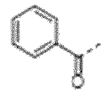
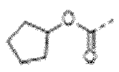
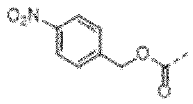
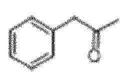
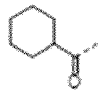

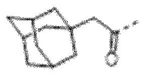
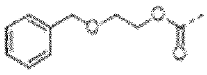
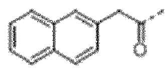
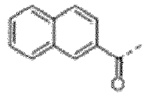
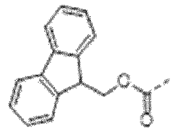

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N; and
$R_5$ is selected from H, Me, Et, Pr, or iPr

Formula (XLVII)

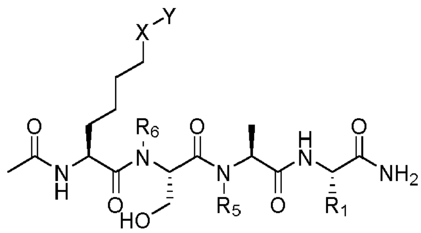

wherein $R_1$ is selected from

$R_1$ 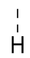
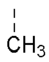
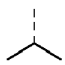
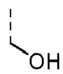
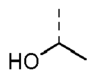
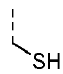
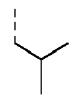

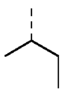
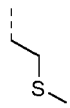
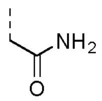
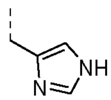
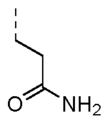
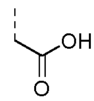
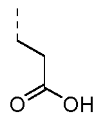

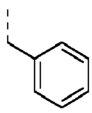
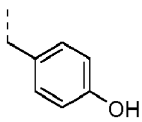
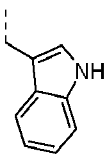
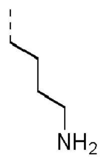
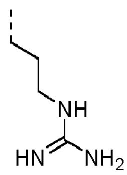

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (XLVIII)

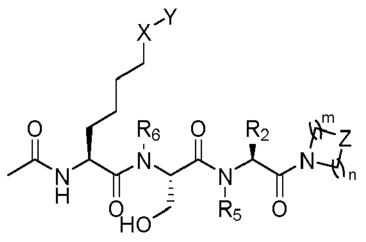

wherein $R_2$ is selected from

$R_2$ 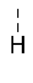 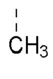 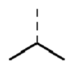 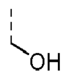 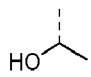 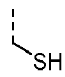 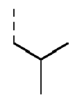

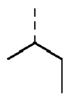 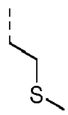 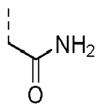 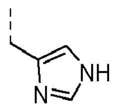 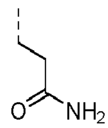 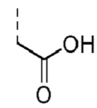 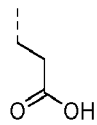

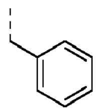 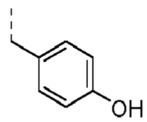 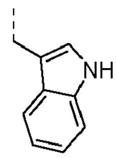 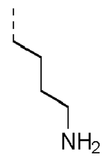 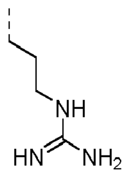

X is selected from NH-C=O, NH-C=S, O-C=O, C=O-O, NH-$SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (XLIX)

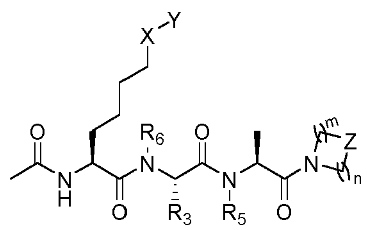

wherein $R_3$ is selected from

$R_3$ 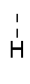 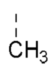 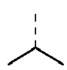 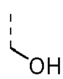 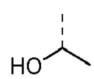 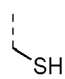 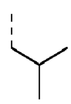

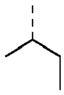 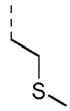 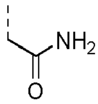 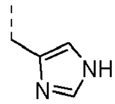 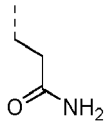 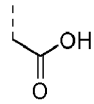 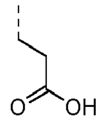

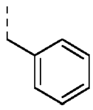 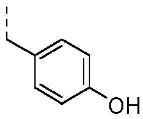 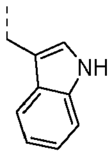 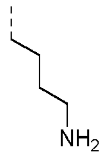 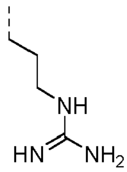

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (L)

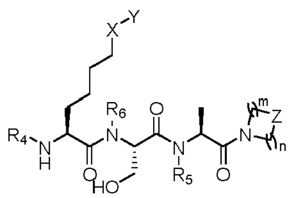

Wherein $R_4$ is selected from

$R_4$ 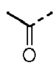 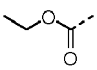 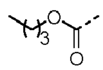 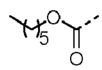 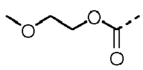 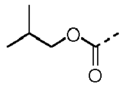 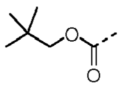

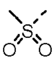 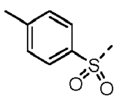 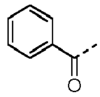 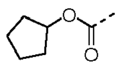 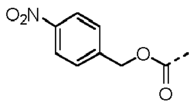 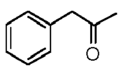 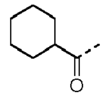

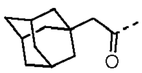 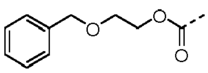 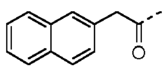 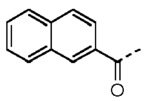 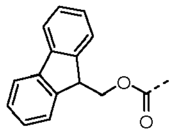

X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 10;
Z is selected from $CH_2$, O, NH, S, C=O, N;
$R_5$ is selected from H, Me, Et, Pr, or iPr; and
$R_6$ is selected from H, Me, Et, Pr, or iPr

Formula (LI)

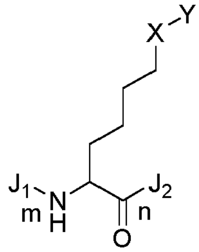

wherein X is selected from NH-C=O, NH-C=S, O-C=O, C=O-O, $NH-SO_2$;
Y is selected from conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
J1 and J2 is independently any $\alpha$ amino acid; and
m and n are each independently integers from 0 to 10, wherein at least one of m or n is not 0

Formula (LIII)

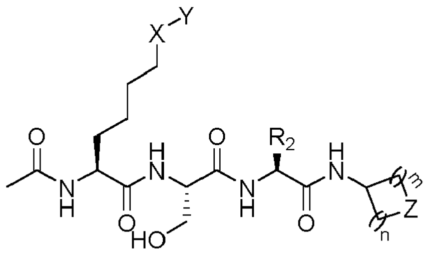

wherein X is NH-C=O, NH-C=S, O-C=O, C=O-O, or $NH-SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 8, wherein at least one of m or n is not 0; and Z is $CH_2$, O, NH, S, or N-Me

$R_2$ 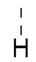 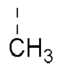 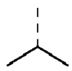 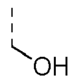 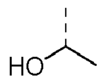 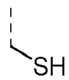 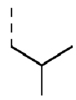

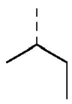 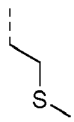 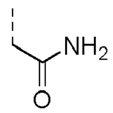 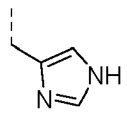 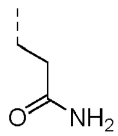 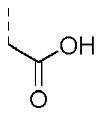 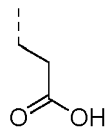

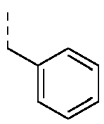 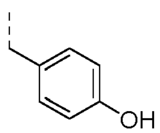 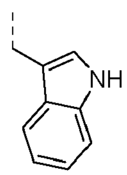 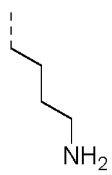 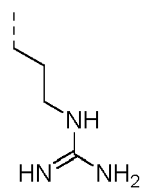

Formula (LIV)

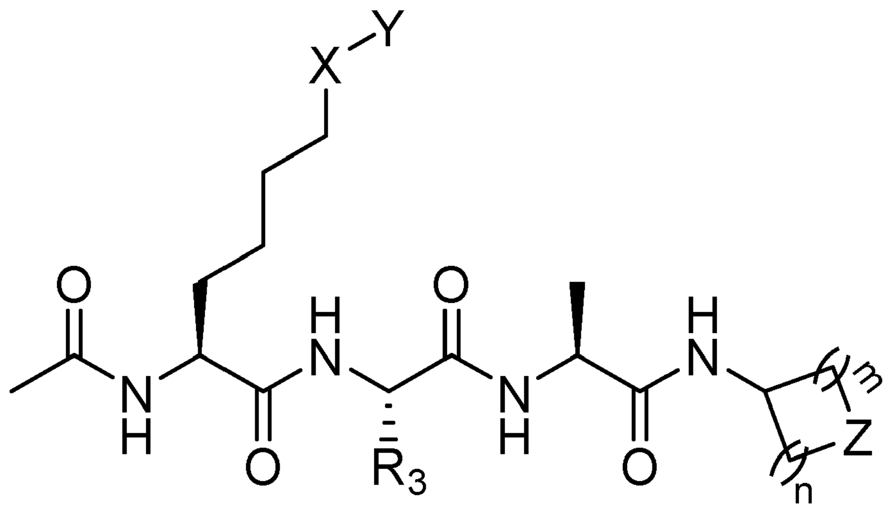

wherein X is NH-C=O, NH-C=S, O-C=O, C=O-O, or NH-$SO_2$;

Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;

m and n are each independently integers from 0 to 8, wherein at least one of m or n is not 0; and Z is $CH_2$, O, NH, S, or N-Me

$R_3$

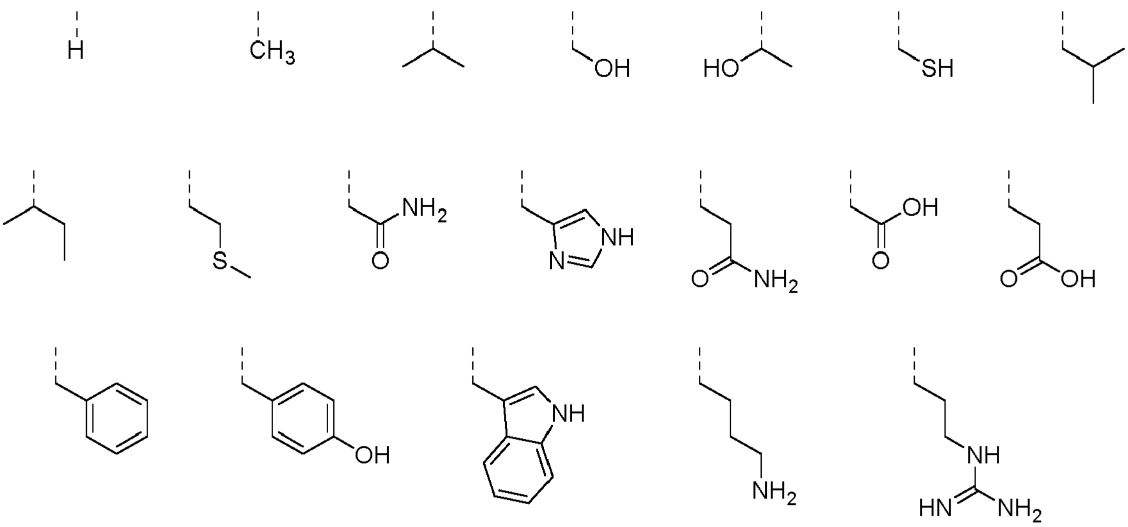

Formula (LV)

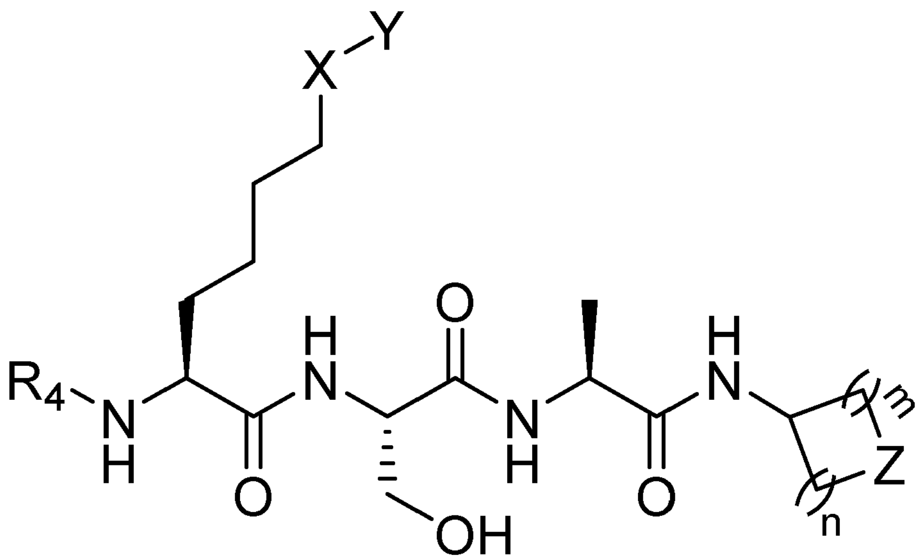

wherein X is NH-C=O, NH-C=S, O-C=O, C=O-O, or $NH\text{-}SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0 to 8, wherein at least one of m or n is not 0; and Z is $CH_2$, O, NH, S, or N-Me

$R_4$ 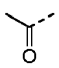 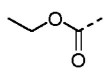 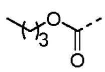 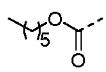 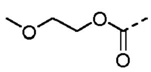 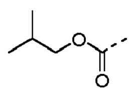 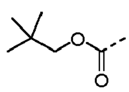

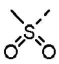 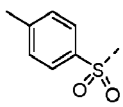 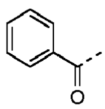 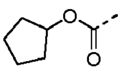 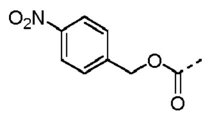 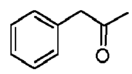 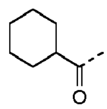

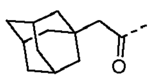 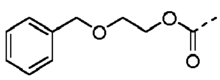 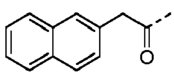 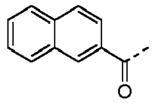 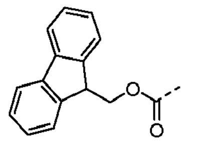

Formula (LVI)

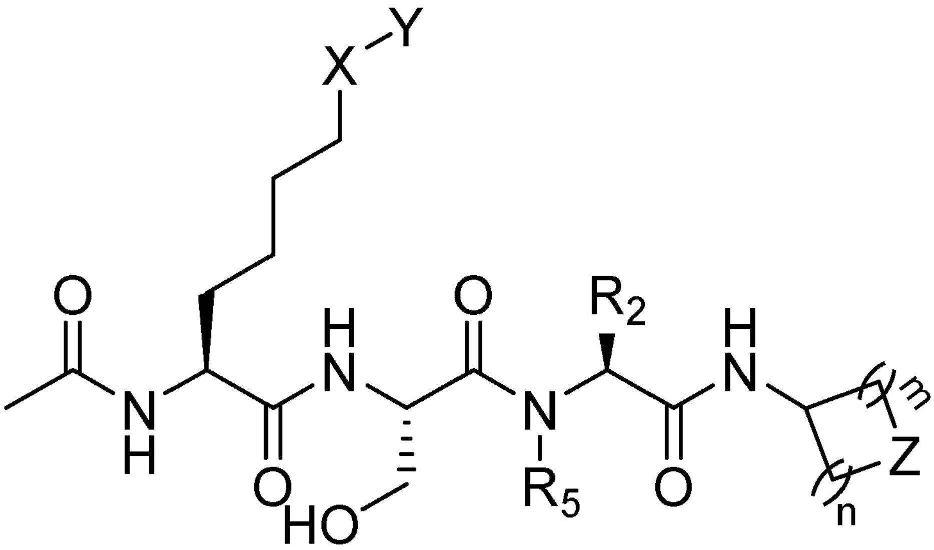

wherein X is NH-C=O, NH-C=S, O-C=O, C=O-O, or $NH-SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S, C=O, or N; and
$R_5$ is H, Me, Et, Pr, or iPr

$R_2$ 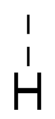 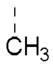 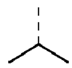 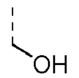 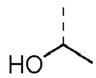 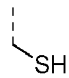 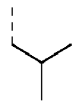

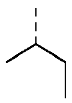 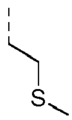 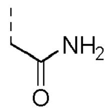 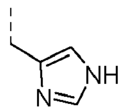 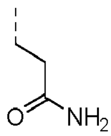 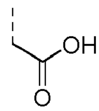 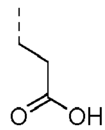

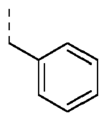 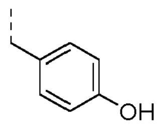 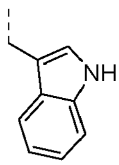 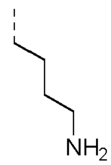 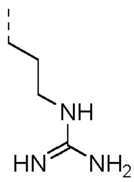

Formula (LVII)

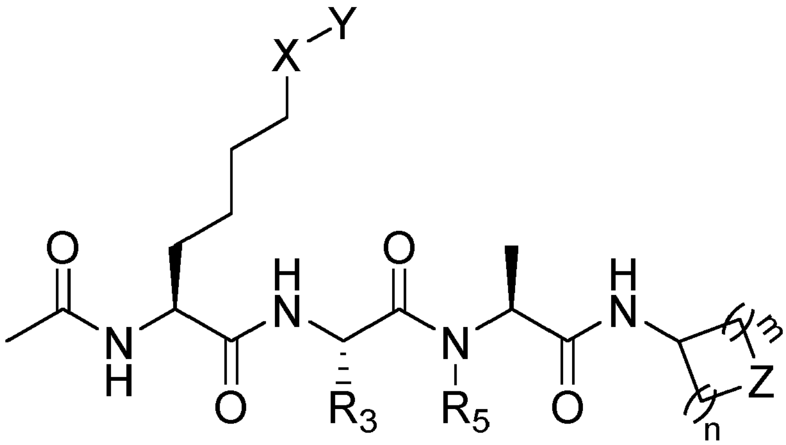

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S, C=O, or N; and
$R_5$ is H, Me, Et, Pr, or iPr

$R_3$ 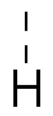 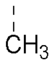 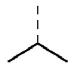 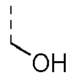 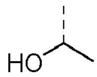 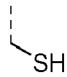 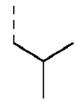

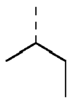 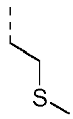 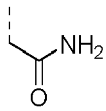 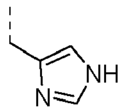 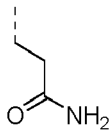 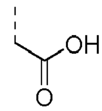 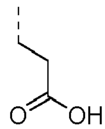

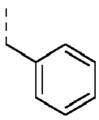 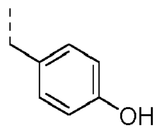 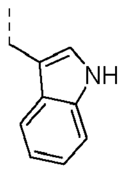 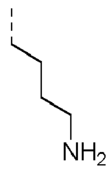 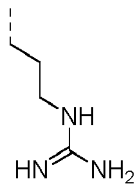

Formula (LVIII)

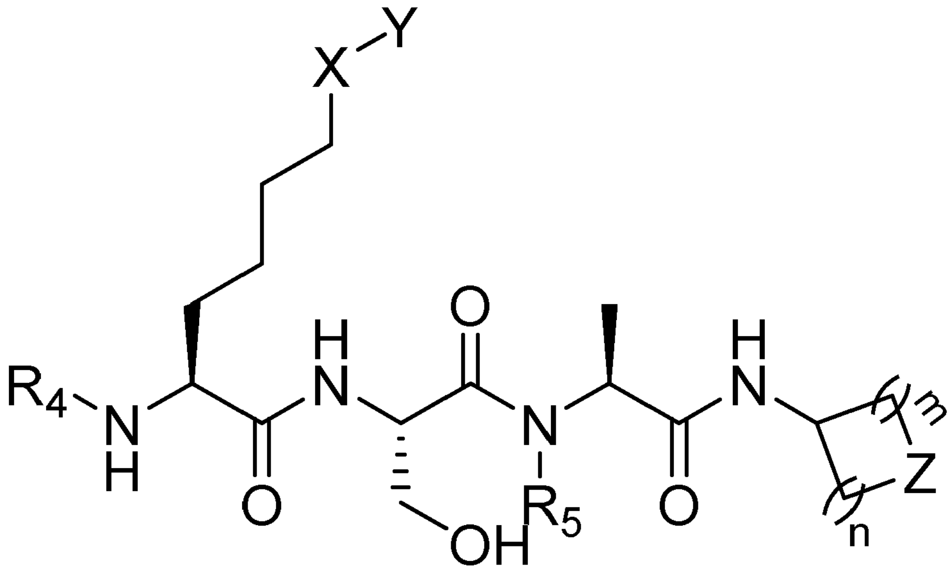

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S, C=O, or N; and
$R_5$ is H, Me, Et, Pr, or iPr

$R_4$

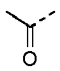 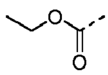 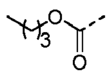 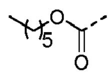 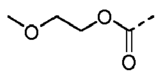 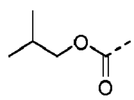 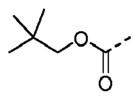

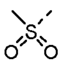 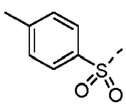 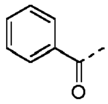 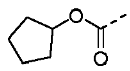 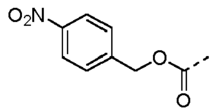 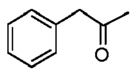 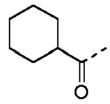

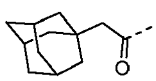 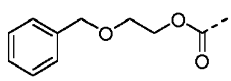 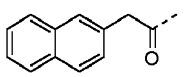 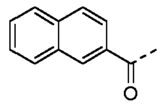 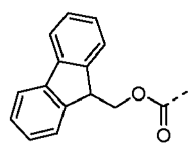

Formula (LIX)

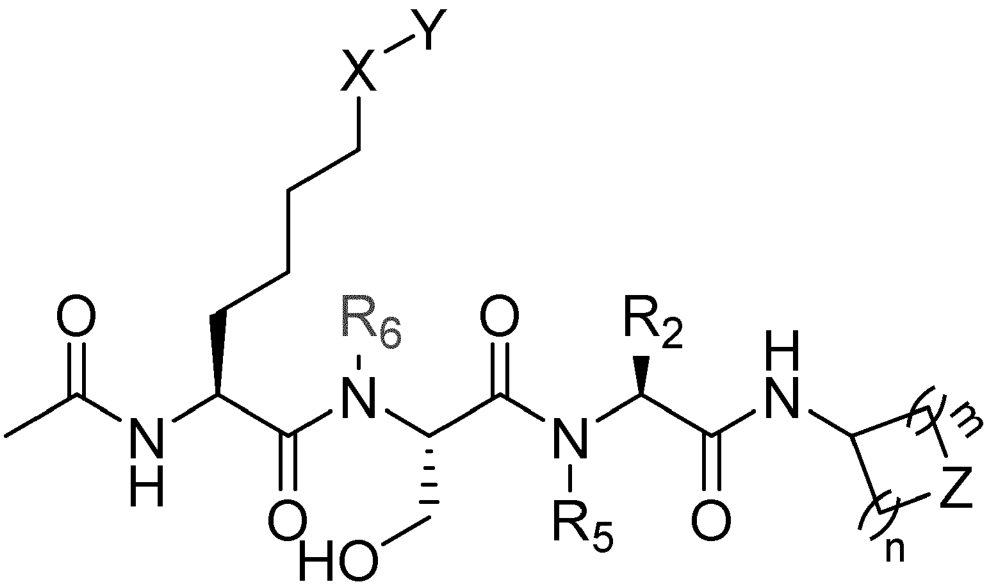

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S,C=O, or N;
$R_5$ is H, Me, Et, Pr, or iPr; and
$R_6$ is H, Me, Et, Pr, or iPr

$R_2$ 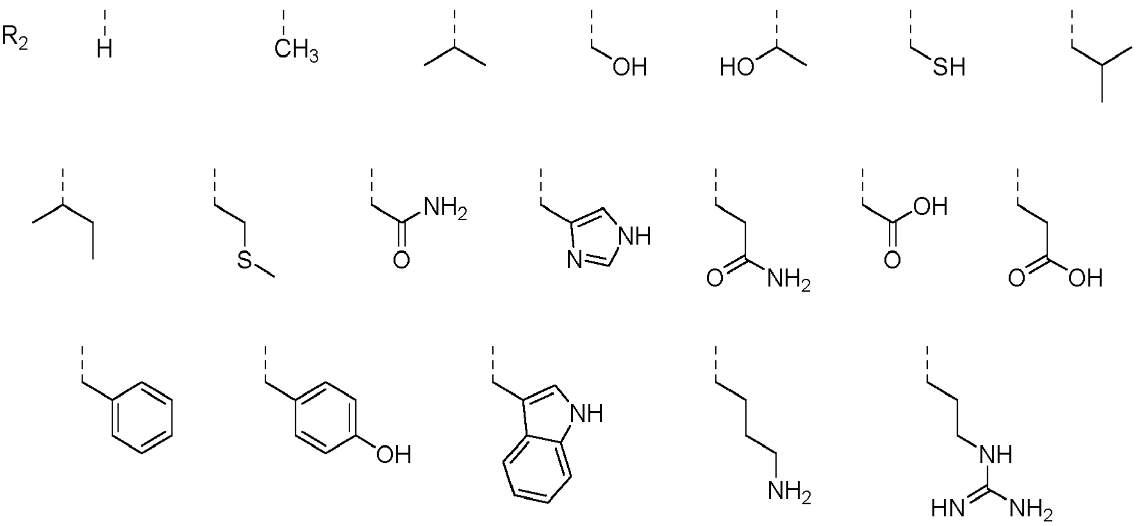

Formula (LX)

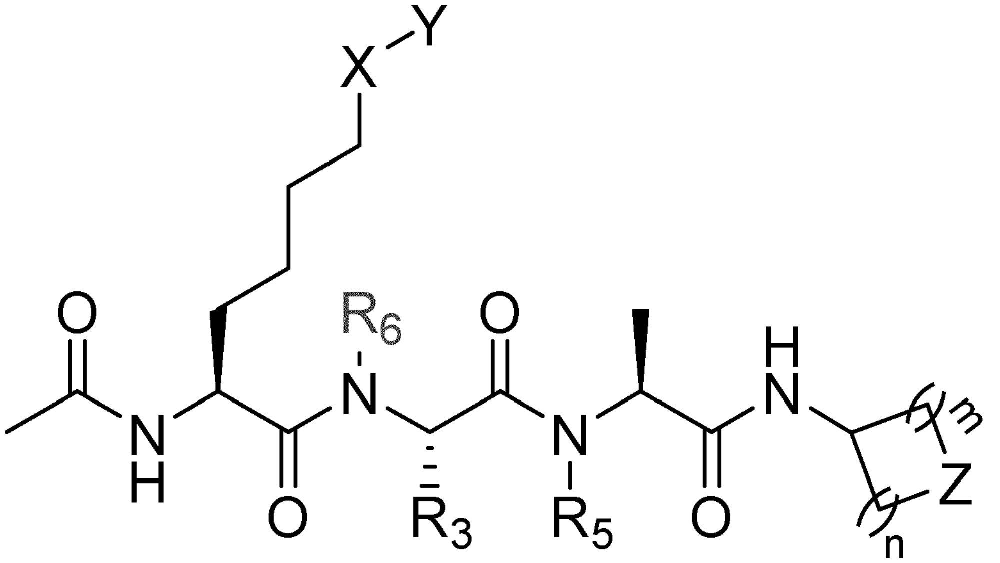

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S,C=O, or N;
$R_5$ is H, Me, Et, Pr, or iPr; and
$R_6$ is H, Me, Et, Pr, or iPr

$R_3$ 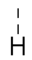 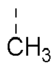 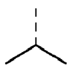 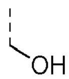 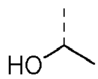 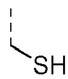 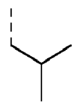

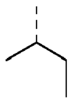 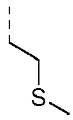 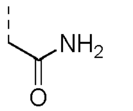 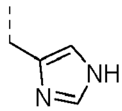 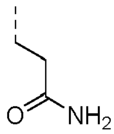 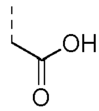 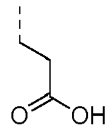

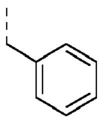 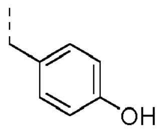 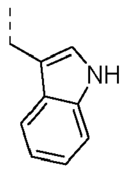 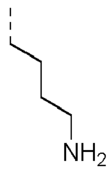 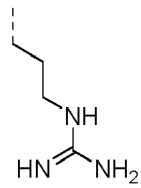

Formula (LXI)

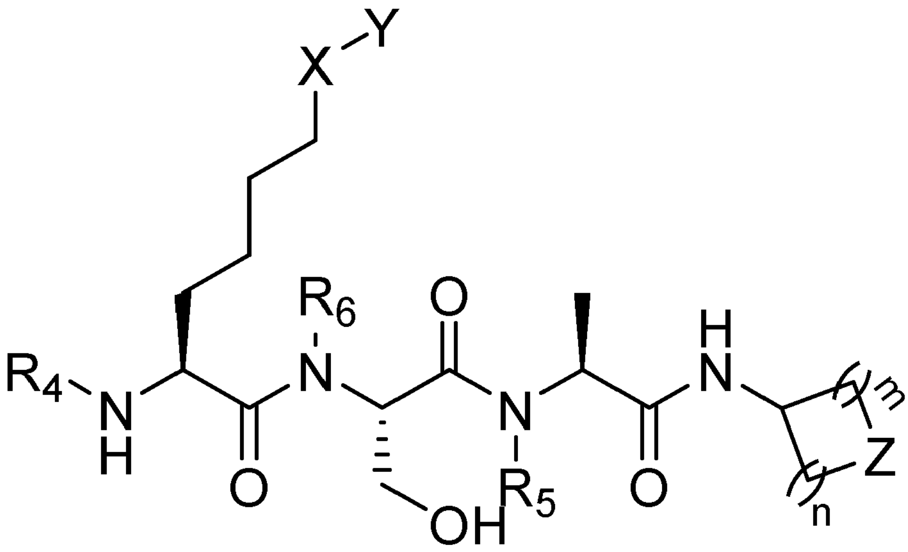

wherein X is NH-C=O, NH-C=S, O-C=O, C=O-O, or NH-$SO_2$;
Y is conjugated/delocalized group, comprising unsubstituted or substituted aromatic rings (monocyclic, bicyclic, tricyclic, tetracyclic), unsubstituted or substituted alkenyl or alkynyl, wherein the conjugated/delocalized group includes p-orbitals that allow for pi-electrons to become delocalized;
m and n are each independently integers from 0-10;
Z is $CH_2$, O, NH, S,C=O, or N;
$R_5$ is H, Me, Et, Pr, or iPr; and
$R_6$ is H, Me, Et, Pr, or iPr

$R_4$ 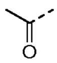 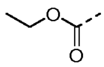 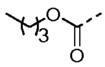 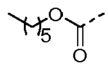 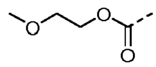 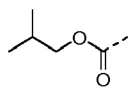 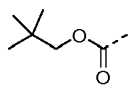

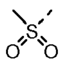 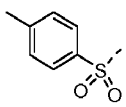 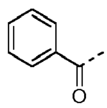 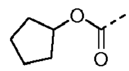 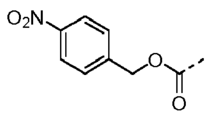 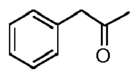 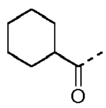

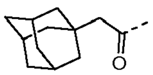 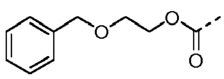 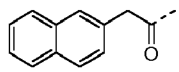 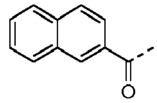 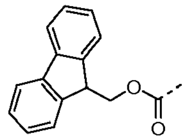

**13.** The composition of claim 12, wherein the YEATS2 YEATS inhibitor is HC25b (formula (II)) or HC26b (formula (III)):

Formula (II)

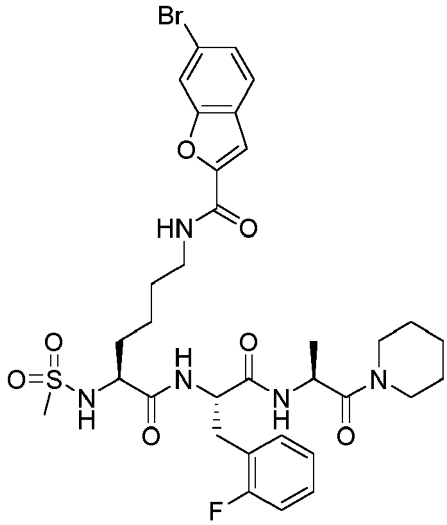

Formula (III)

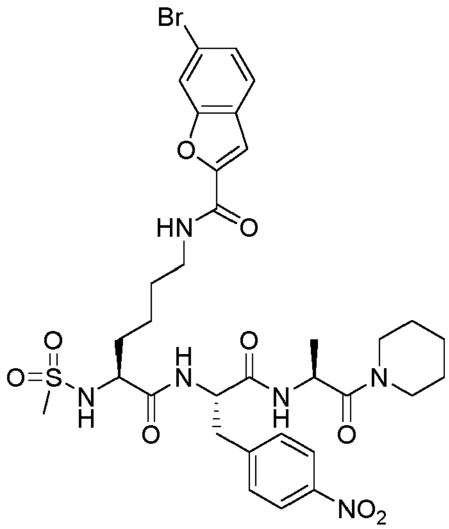

.

**14.** A compound according to formula (LII):

Formula (LII)

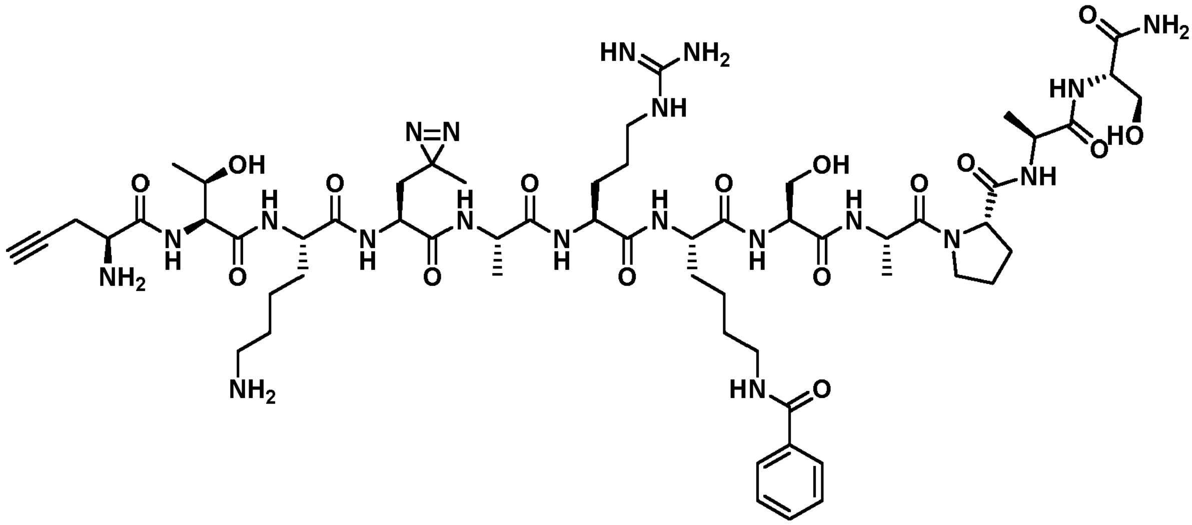

**15.** The compound of claim 14, wherein the compound is a probe bound to a YEATS2 protein.

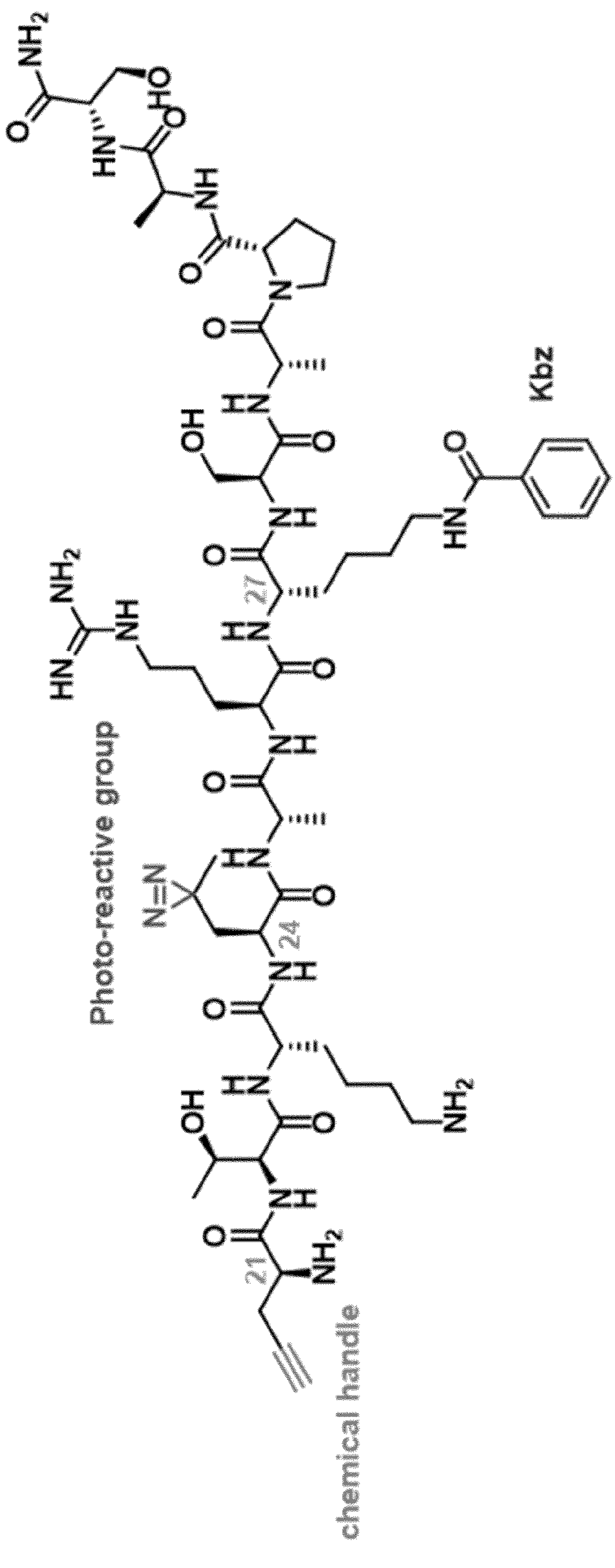


FIG. 1A

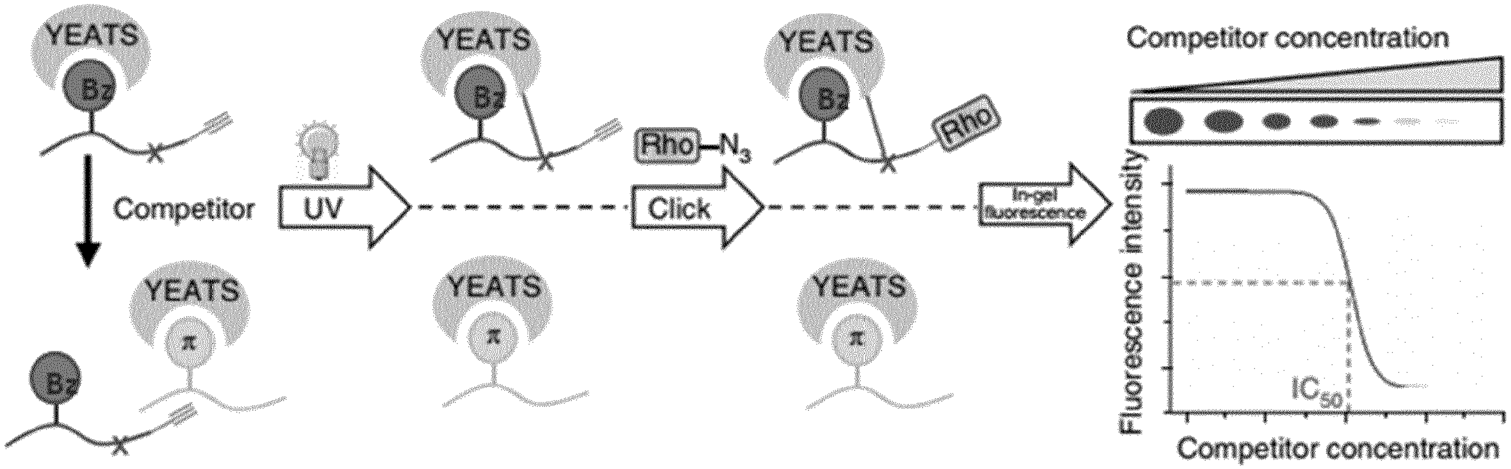
YEATS
Bz
Competitor
π
UV
Rho
$N_3$
Click
In-gel fluorescence
Competitor concentration
Fluorescence intensity
$IC_{50}$
Competitor concentration

FIG. 1B

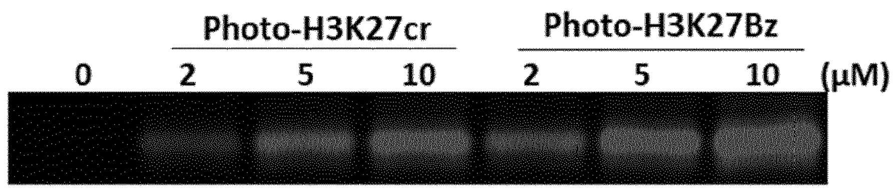
Photo-H3K27cr
Photo-H3K27Bz
0
2
5
10
2
5
10
(μM)

FIG. 1C

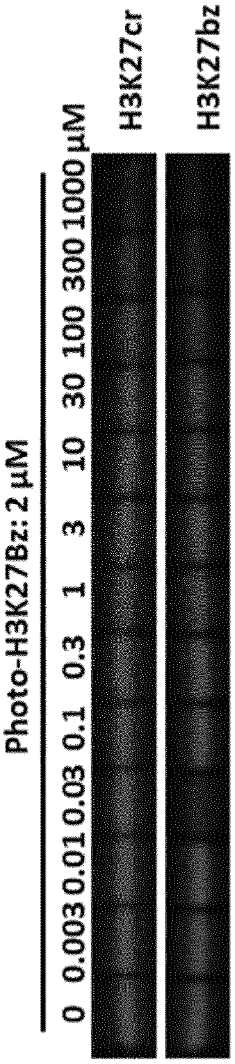
Photo-H3K27Bz: 2 μM
0 0.003 0.01 0.03 0.1 0.3 1 3 10 30 100 300 1000 μM
H3K27cr
H3K27bz

FIG. 1D

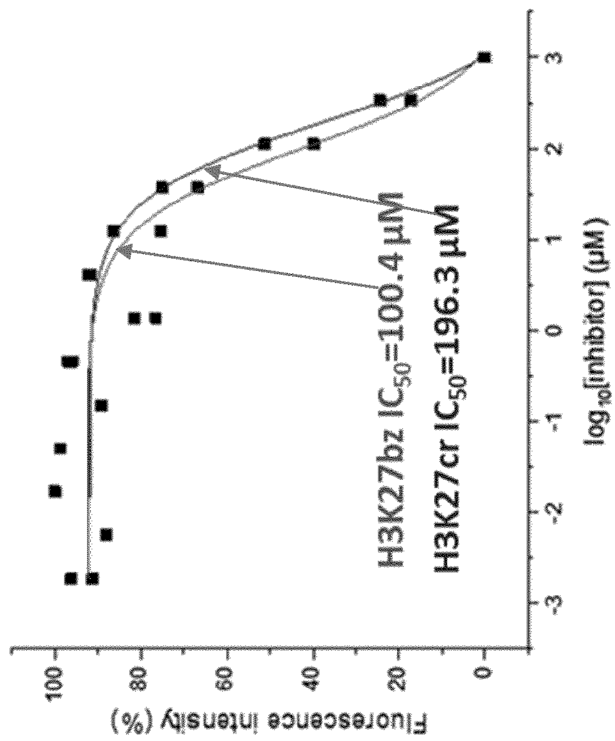
H3K27bz IC50=100.4 μM
H3K27cr IC50=196.3 μM
Fluorescence intensity (%)
log10[inhibitor] (μM)

FIG. 1E

(a)

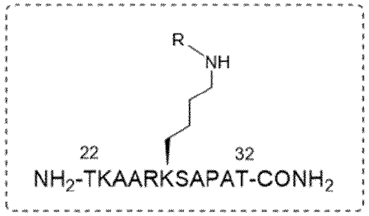

R =

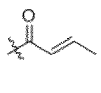
1 (Cr)

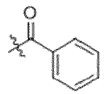
2 (Bz)

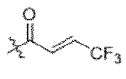
3 (TF)

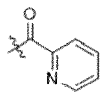
4 (2Py)

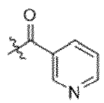
5 (3Py)

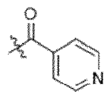
6 (4Py)

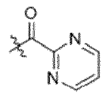
7 (Pm)

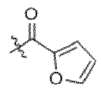
8 (2fu)

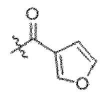
9 (3fu)

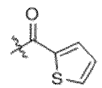
10 (2Tp)

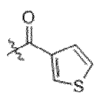
11 3Tp

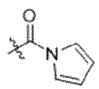
12 (N-Pr)

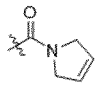
13 (N-Pr-2H)

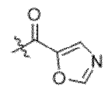
14 (5-oxa)

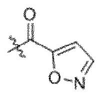
15 (isoxa)

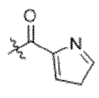
16 (4-oxa)

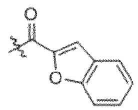
17 (benfu)

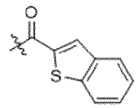
18 (benthio)

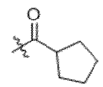
19 (cp)

FIG. 2A

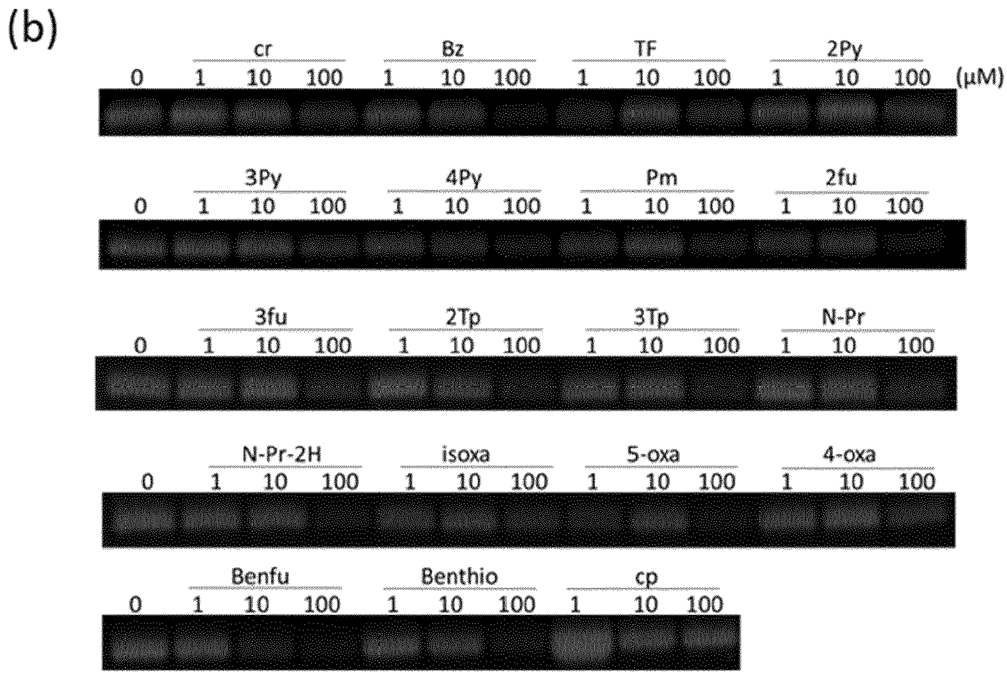
(b)
cr
Bz
TF
2Py
3Py
4Py
Pm
2fu
3fu
2Tp
3Tp
N-Pr
N-Pr-2H
isoxa
5-oxa
4-oxa
Benfu
Benthio
cp
0
1
10
100
(μM)

FIG. 2B

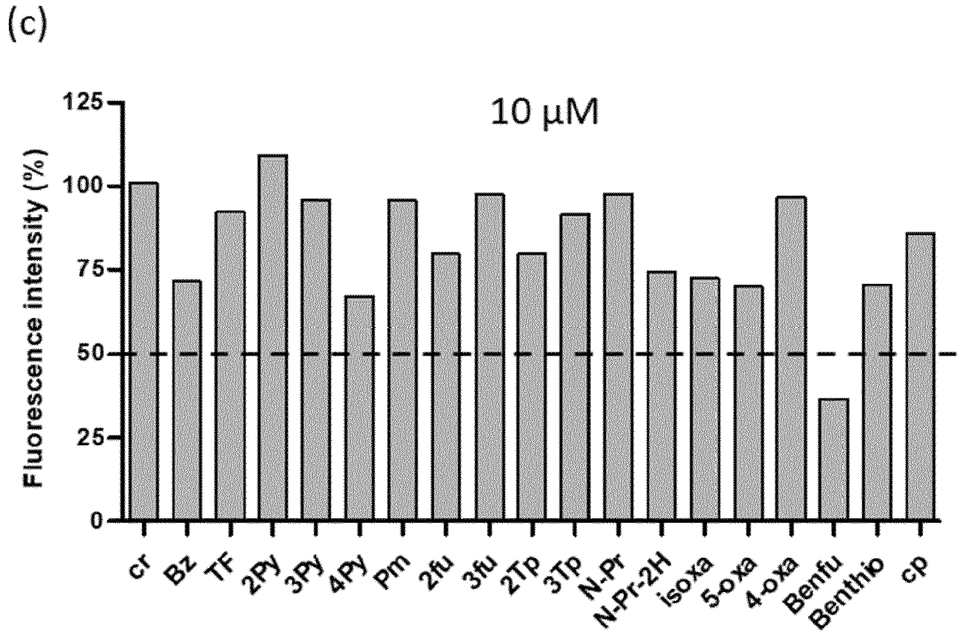
(c)
10 μM
Fluorescence intensity (%)
125
100
75
50
25
0
cr
Bz
TF
2Py
3Py
4Py
Pm
2fu
3fu
2Tp
3Tp
N-Pr
N-Pr-2H
isoxa
5-oxa
4-oxa
Benfu
Benthio
cp

FIG. 2C

(a)

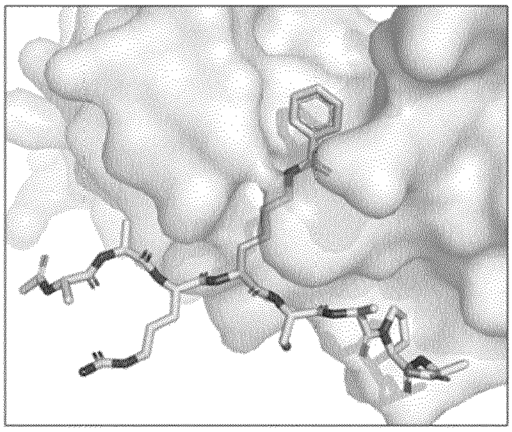

**YEATS2-H3K27Bz**

H3K27(Benfu): TKAAR $K_{bf}$SAPAT
Ac $K_{bf}$SAPA
Ac $K_{bf}$SAP
Ac $K_{bf}$SA
Ac $K_{bf}$S

FIG. 3A

(b)

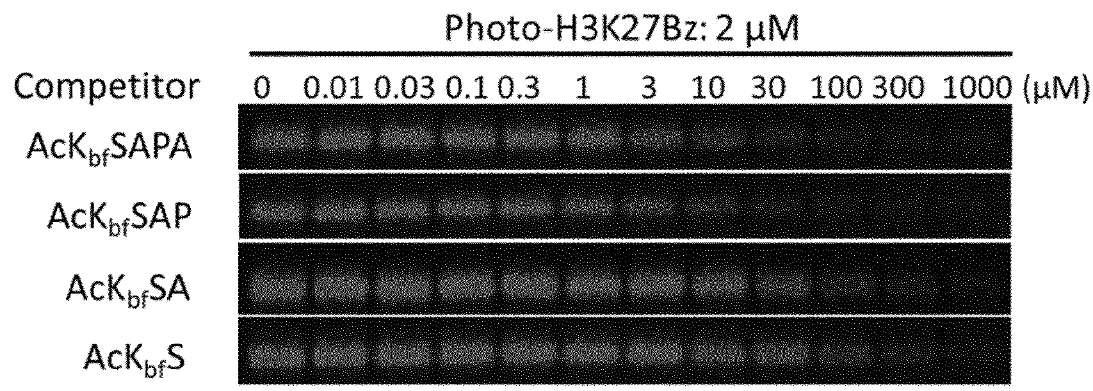


FIG. 3B

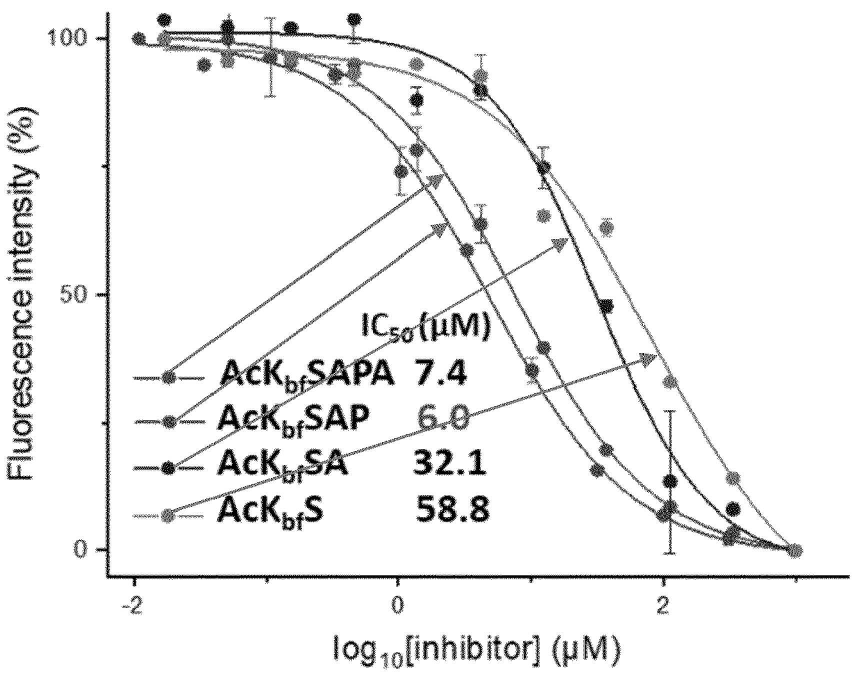
100
50
0
-2
0
2
Fluorescence intensity (%)
log10[inhibitor] (μM)
IC50 (μM)
AcKbfSAPA 7.4
AcKbfSAP 6.0
AcKbfSA 32.1
AcKbfS 58.8

FIG. 3C

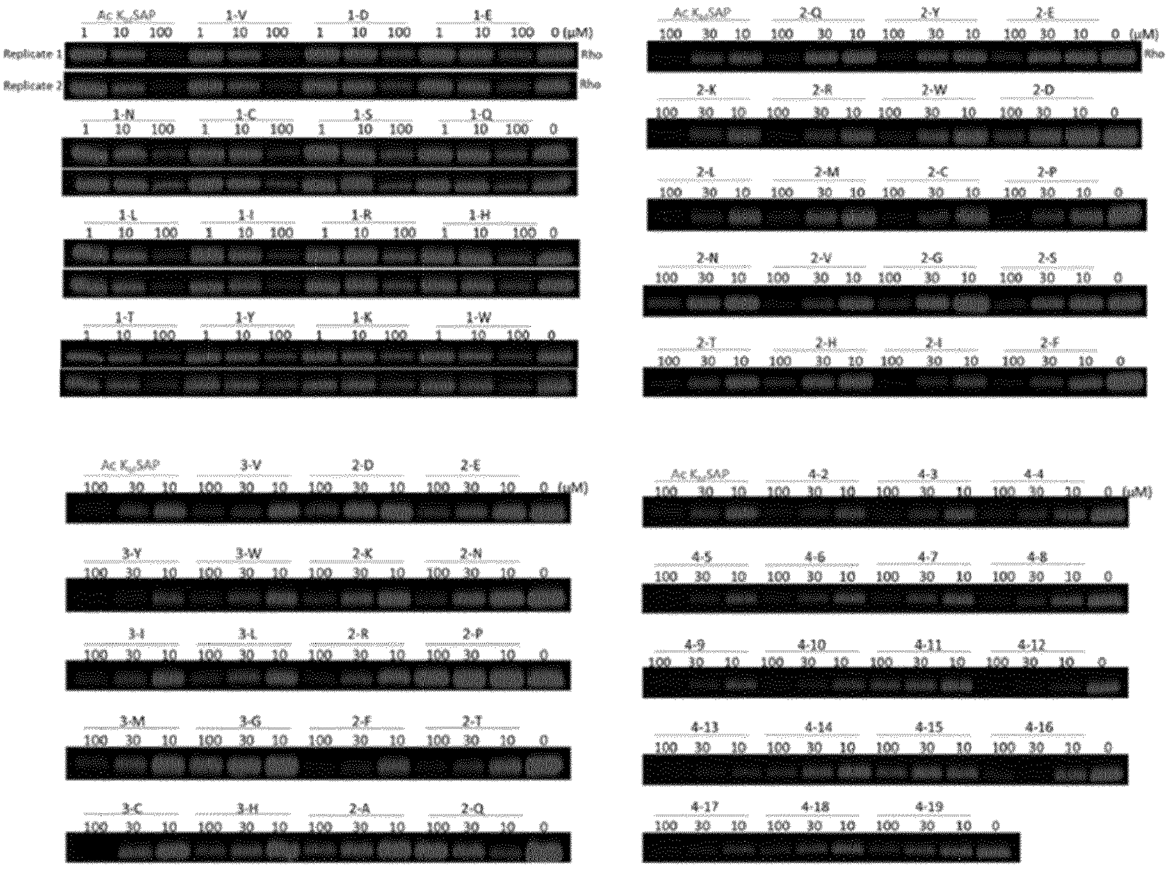


FIG. 4A

4 3 2 1

Ac $K_{bf}$ S A P

4-1 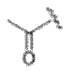

4-2 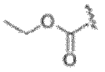

4-3 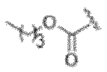

4-4 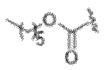

4-5 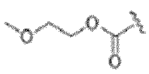

4-6 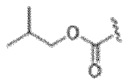

4-7 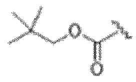

4-8 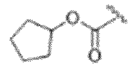

4-9 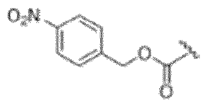

4-10 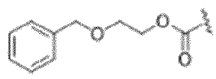

4-11 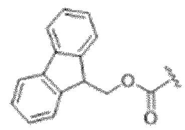

4-12 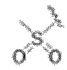

4-13 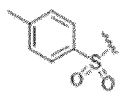

4-14 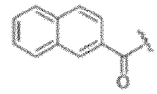

4-15 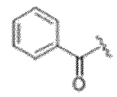

4-16 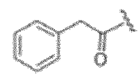

4-17 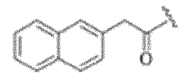

4-18 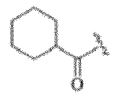

4-19 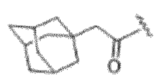

FIG. 4B

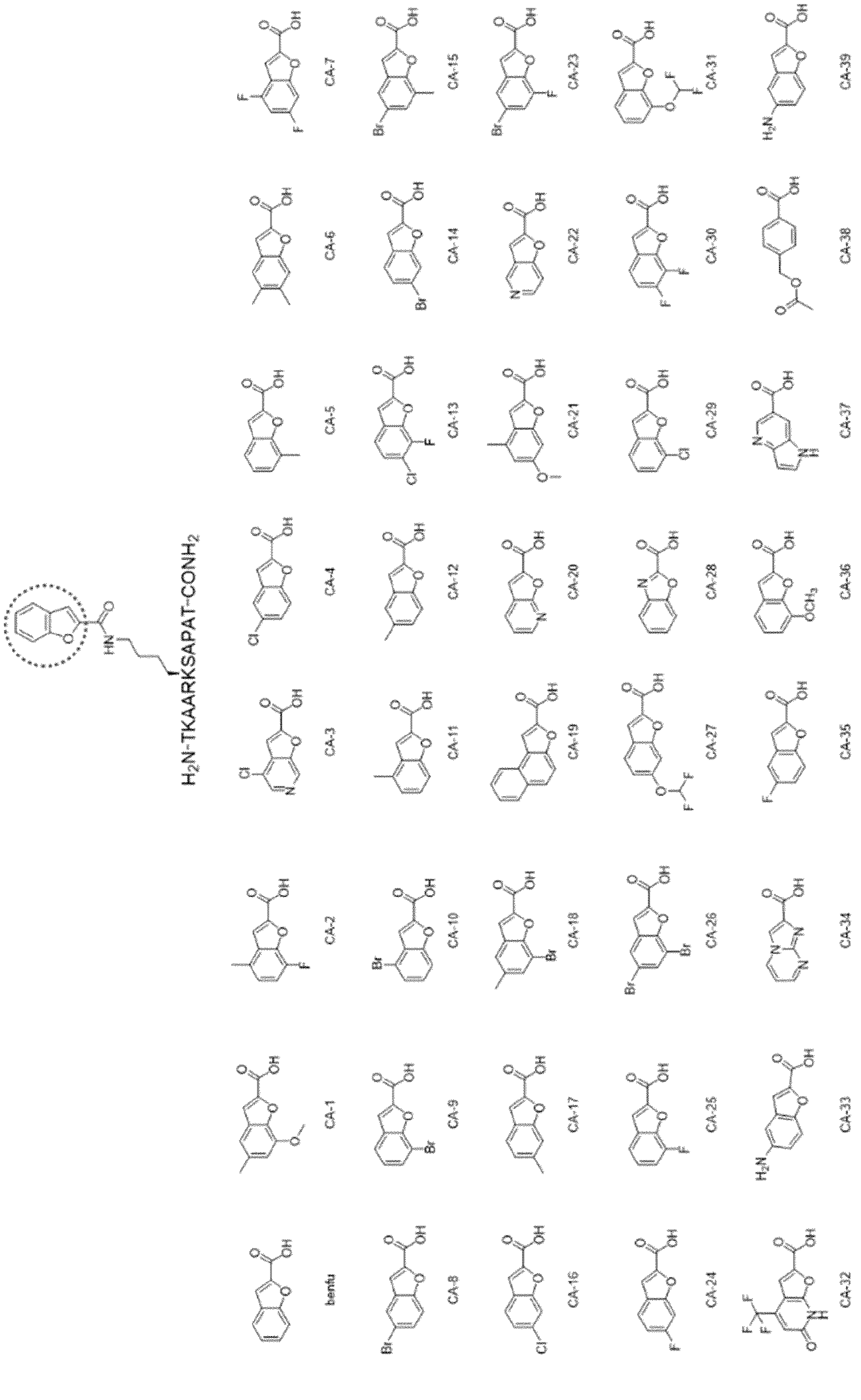

H$_2$N-TKAARKSAPAT-CONH$_2$

benfu, CA-1, CA-2, CA-3, CA-4, CA-5, CA-6, CA-7

CA-8, CA-9, CA-10, CA-11, CA-12, CA-13, CA-14, CA-15

CA-16, CA-17, CA-18, CA-19, CA-20, CA-21, CA-22, CA-23

CA-24, CA-25, CA-26, CA-27, CA-28, CA-29, CA-30, CA-31

CA-32, CA-33, CA-34, CA-35, CA-36, CA-37, CA-38, CA-39

FIG. 4C

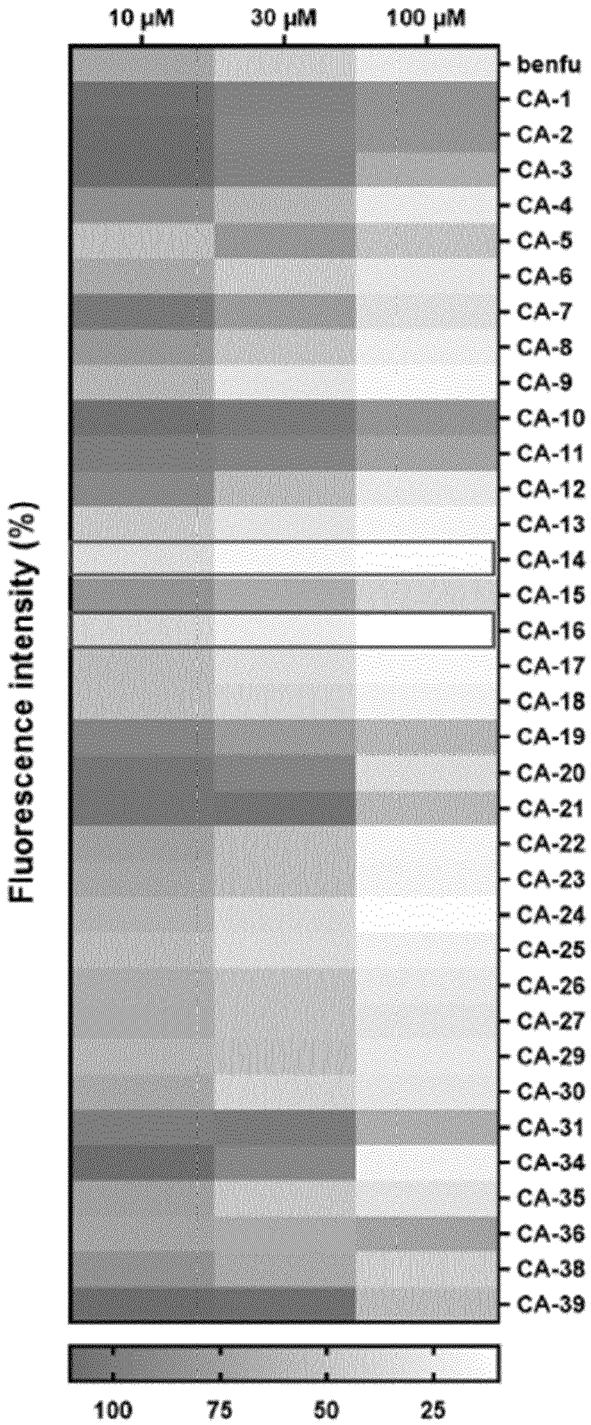
10 μM
30 μM
100 μM
Fluorescence intensity (%)
benfu
CA-1
CA-2
CA-3
CA-4
CA-5
CA-6
CA-7
CA-8
CA-9
CA-10
CA-11
CA-12
CA-13
CA-14
CA-15
CA-16
CA-17
CA-18
CA-19
CA-20
CA-21
CA-22
CA-23
CA-24
CA-25
CA-26
CA-27
CA-29
CA-30
CA-31
CA-34
CA-35
CA-36
CA-38
CA-39
100
75
50
25

FIG. 4D

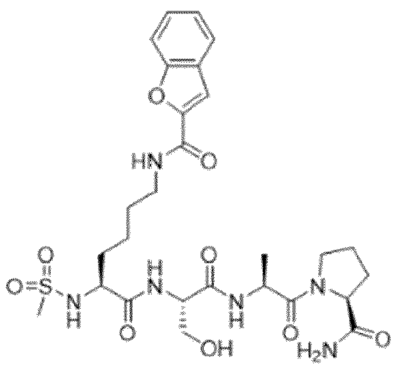

FIG. 5A

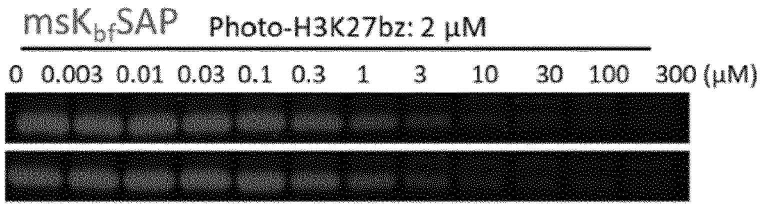


FIG. 5B

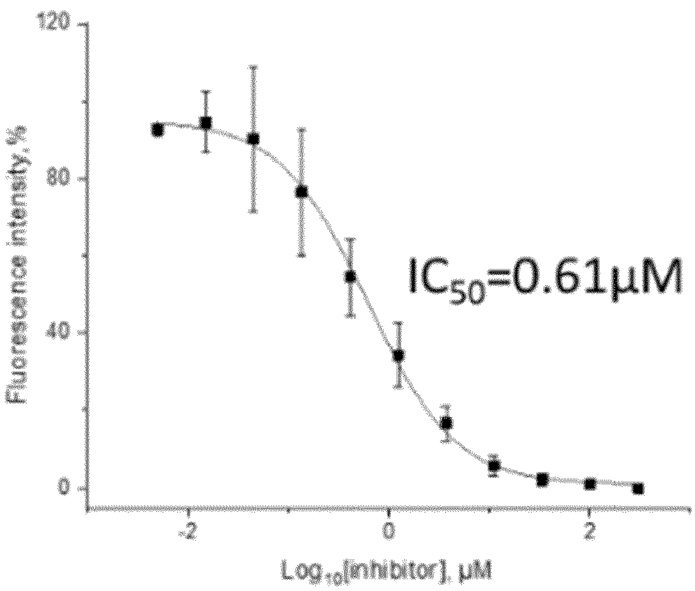


FIG. 5C

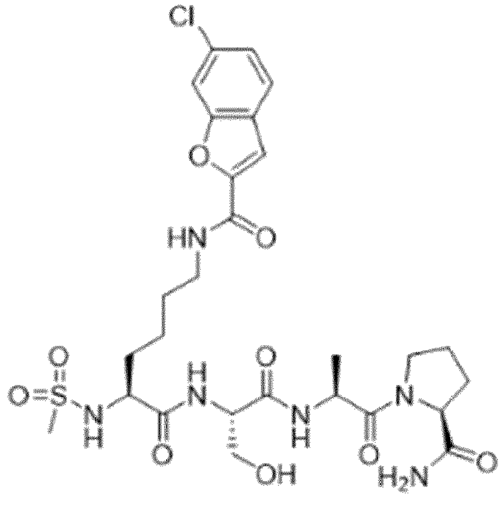

FIG. 5D

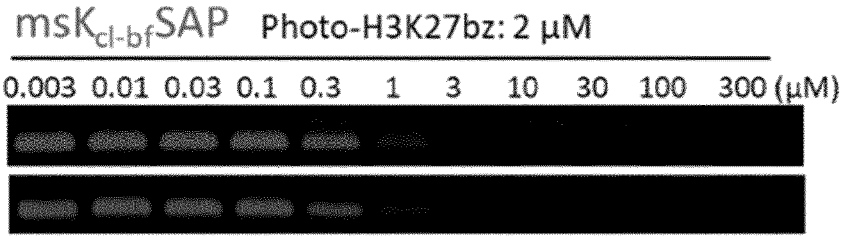


FIG. 5E

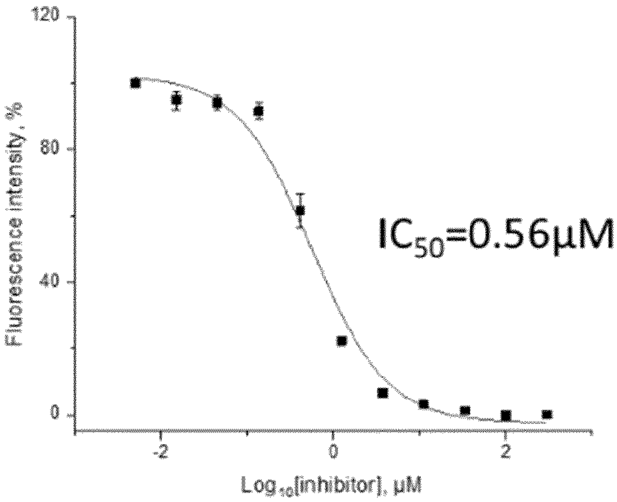


FIG. 5F

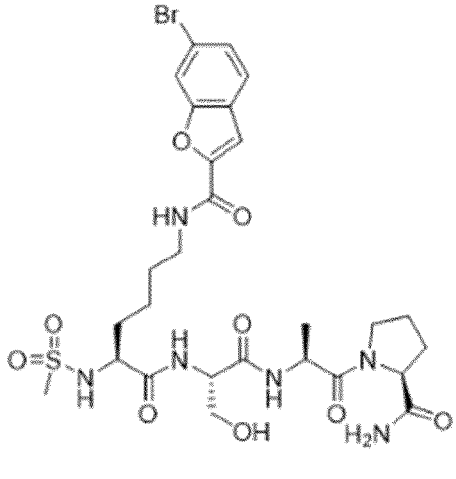

FIG. 5G

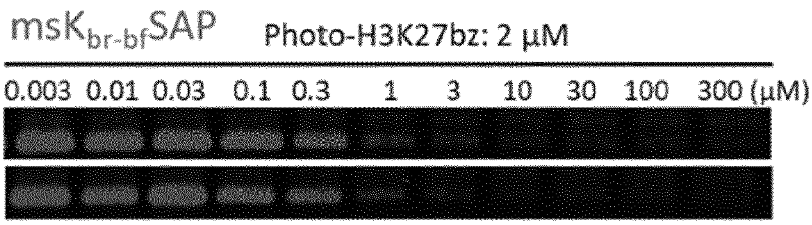


FIG. 5H

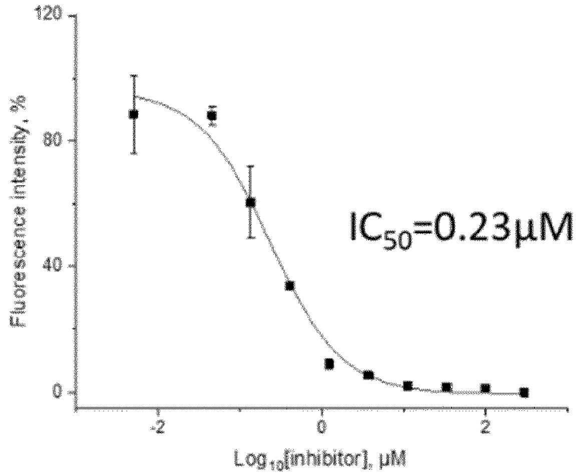


FIG. 5I

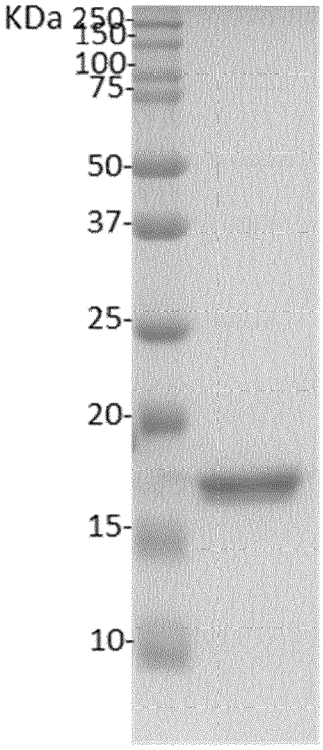


FIG. 6A

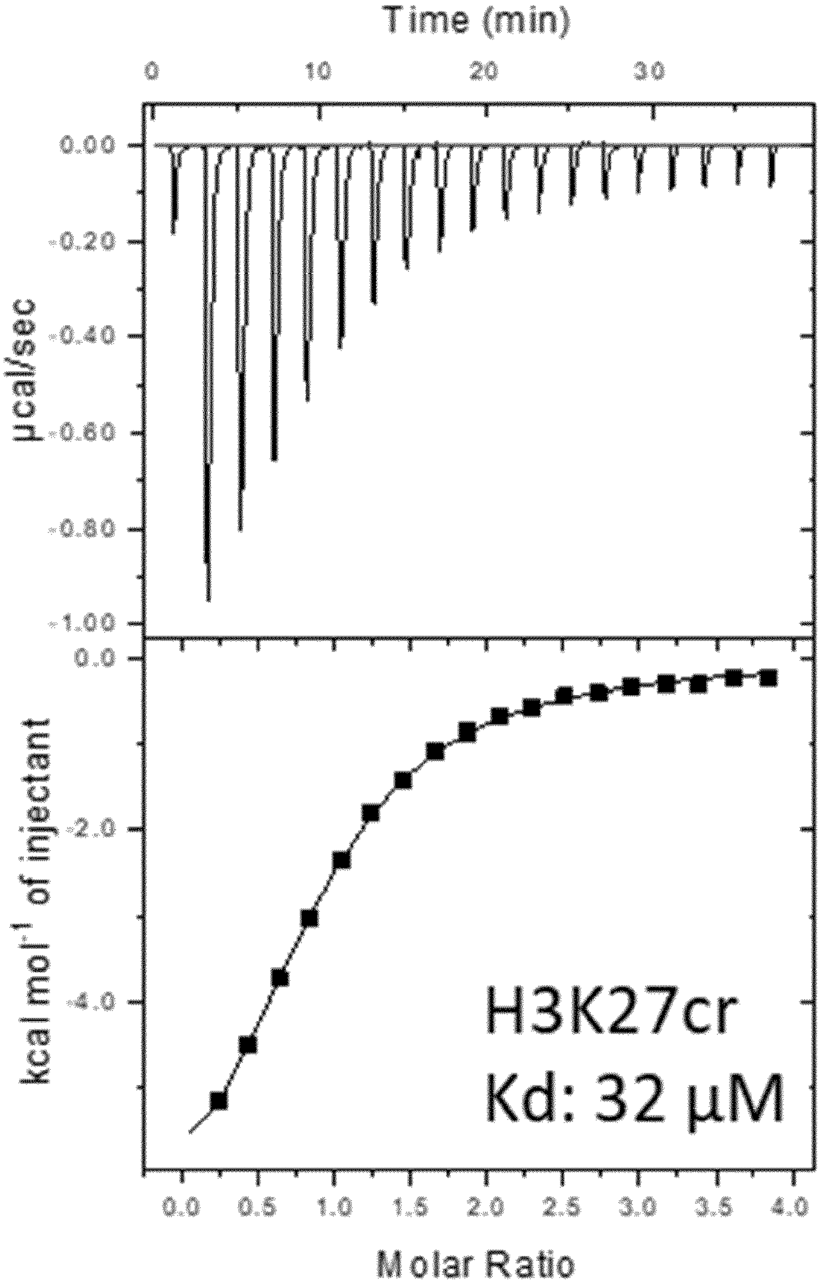
Time (min)
0
10
20
30
0.00
-0.20
-0.40
-0.60
-0.80
-1.00
µcal/sec
0.0
-2.0
-4.0
kcal mol-1 of injectant
H3K27cr
Kd: 32 µM
0.0
0.5
1.0
1.5
2.0
2.5
3.0
3.5
4.0
Molar Ratio

FIG. 6B

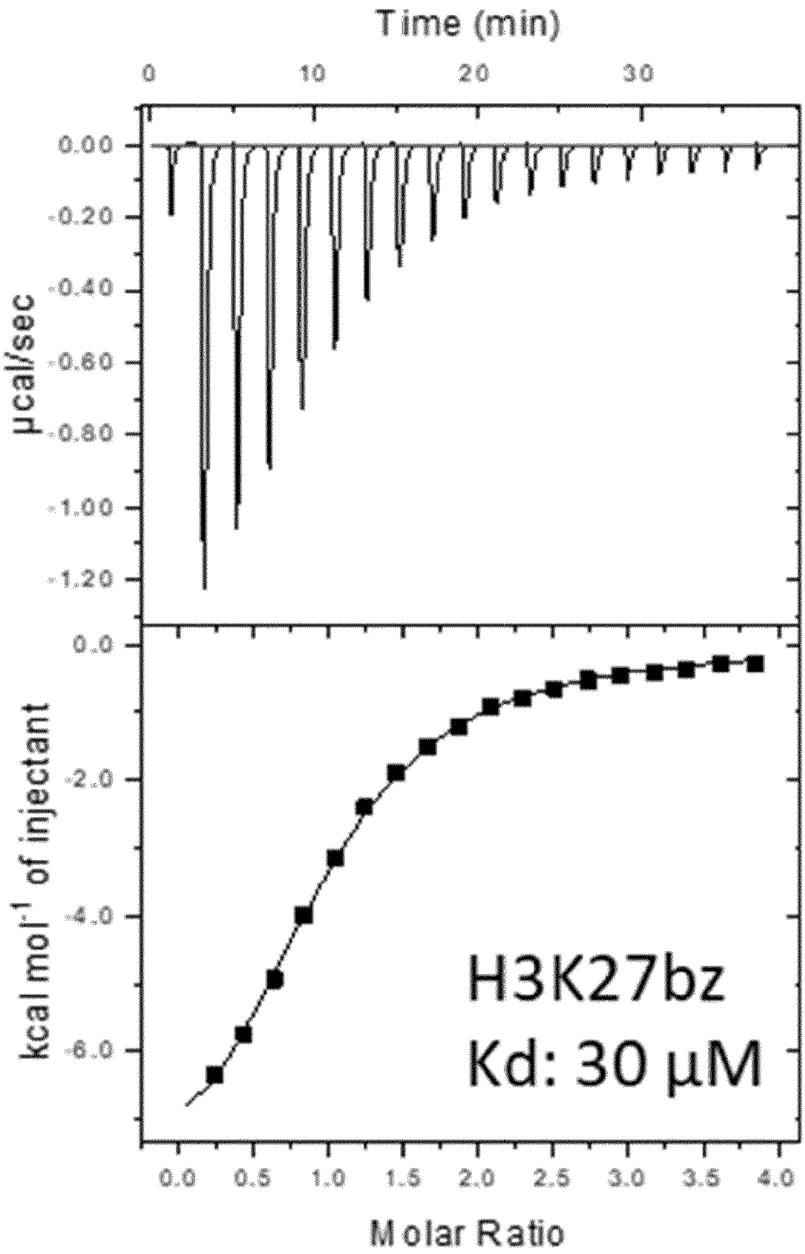
Time (min)
0
10
20
30
0.00
-0.20
-0.40
-0.60
-0.80
-1.00
-1.20
µcal/sec
0.0
-2.0
-4.0
-6.0
kcal mol-1 of injectant
H3K27bz
Kd: 30 µM
0.0
0.5
1.0
1.5
2.0
2.5
3.0
3.5
4.0
Molar Ratio

FIG. 6C

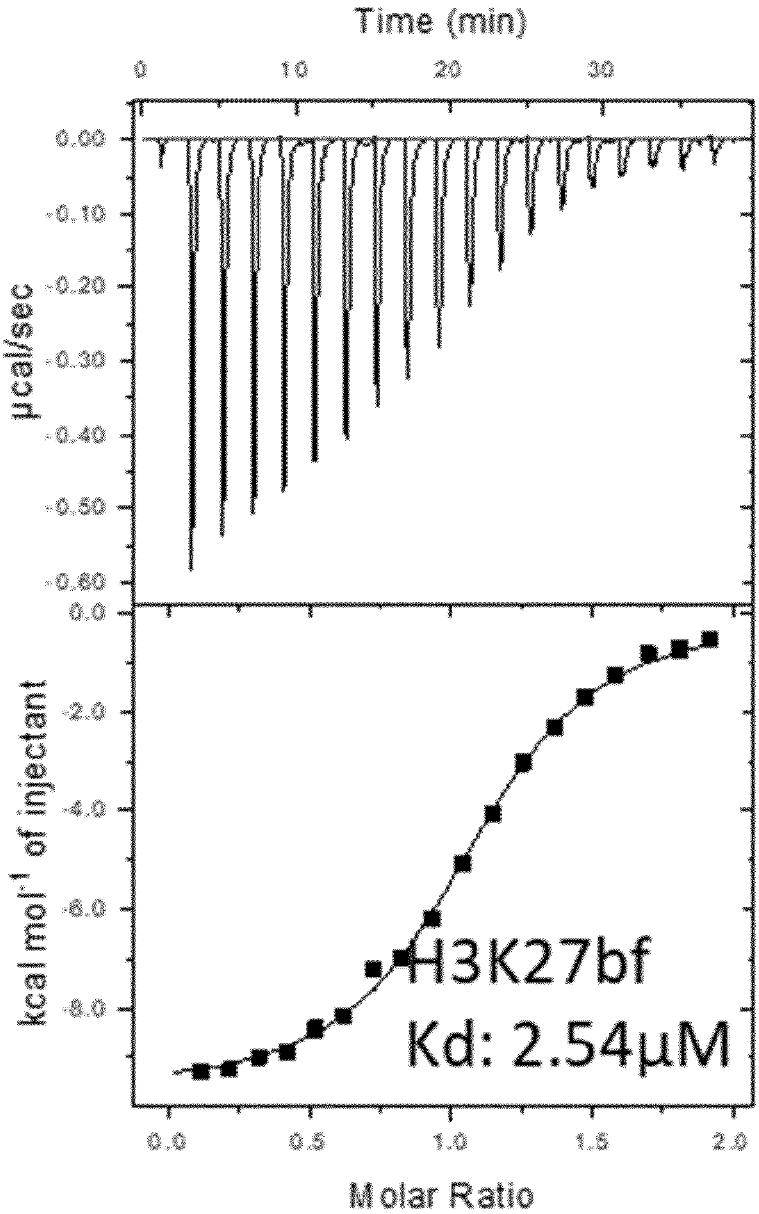
Time (min)
0
10
20
30
0.00
-0.10
-0.20
-0.30
-0.40
-0.50
-0.60
µcal/sec
0.0
-2.0
-4.0
-6.0
-8.0
kcal mol-1 of injectant
H3K27bf
Kd: 2.54µM
0.0
0.5
1.0
1.5
2.0
Molar Ratio

FIG. 6D

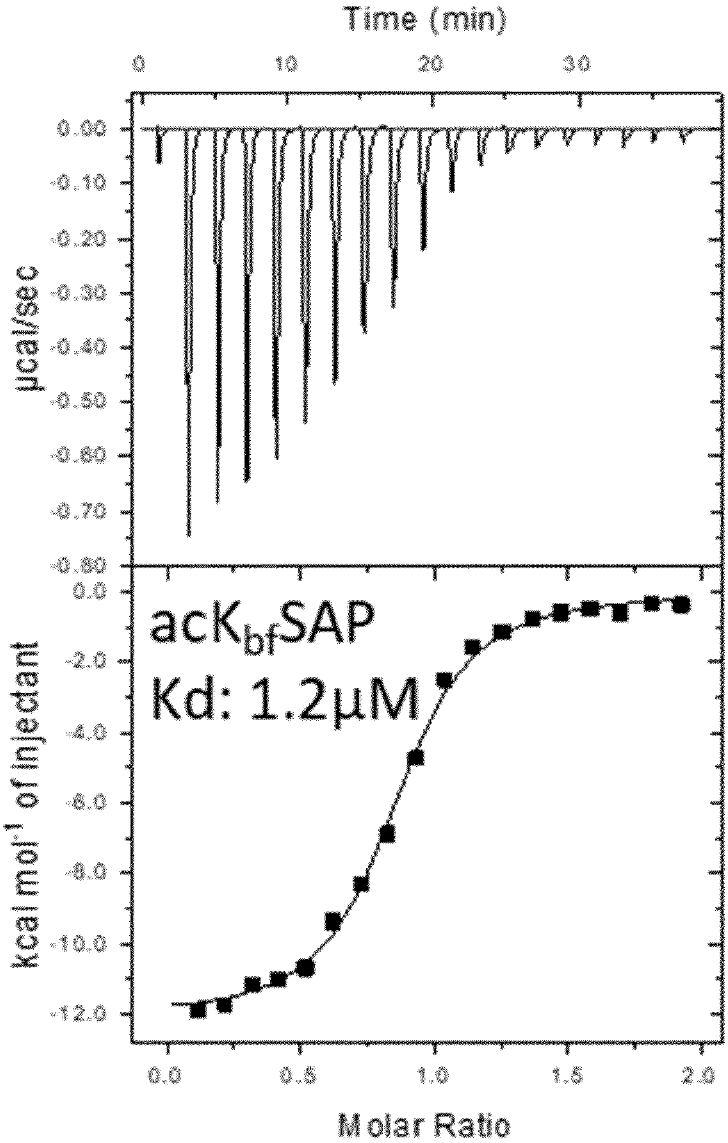
Time (min)
0
10
20
30
µcal/sec
0.00
-0.10
-0.20
-0.30
-0.40
-0.50
-0.60
-0.70
-0.80
acK$_{bf}$SAP
Kd: 1.2µM
kcal mol$^{-1}$ of injectant
0.0
-2.0
-4.0
-6.0
-8.0
-10.0
-12.0
0.0
0.5
1.0
1.5
2.0
Molar Ratio

FIG. 6E

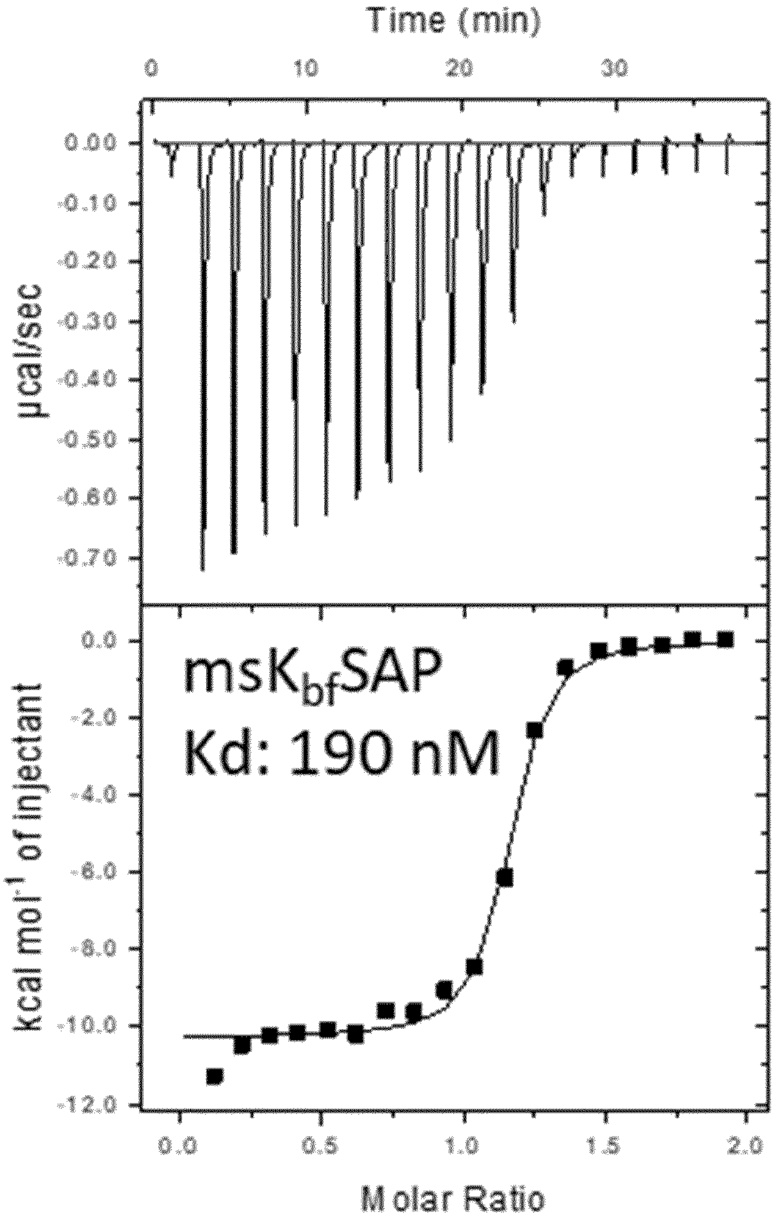
Time (min)
0
10
20
30
0.00
-0.10
-0.20
-0.30
-0.40
-0.50
-0.60
-0.70
µcal/sec
0.0
-2.0
-4.0
-6.0
-8.0
-10.0
-12.0
kcal mol-1 of injectant
msKbfSAP
Kd: 190 nM
0.0
0.5
1.0
1.5
2.0
Molar Ratio

FIG. 6F

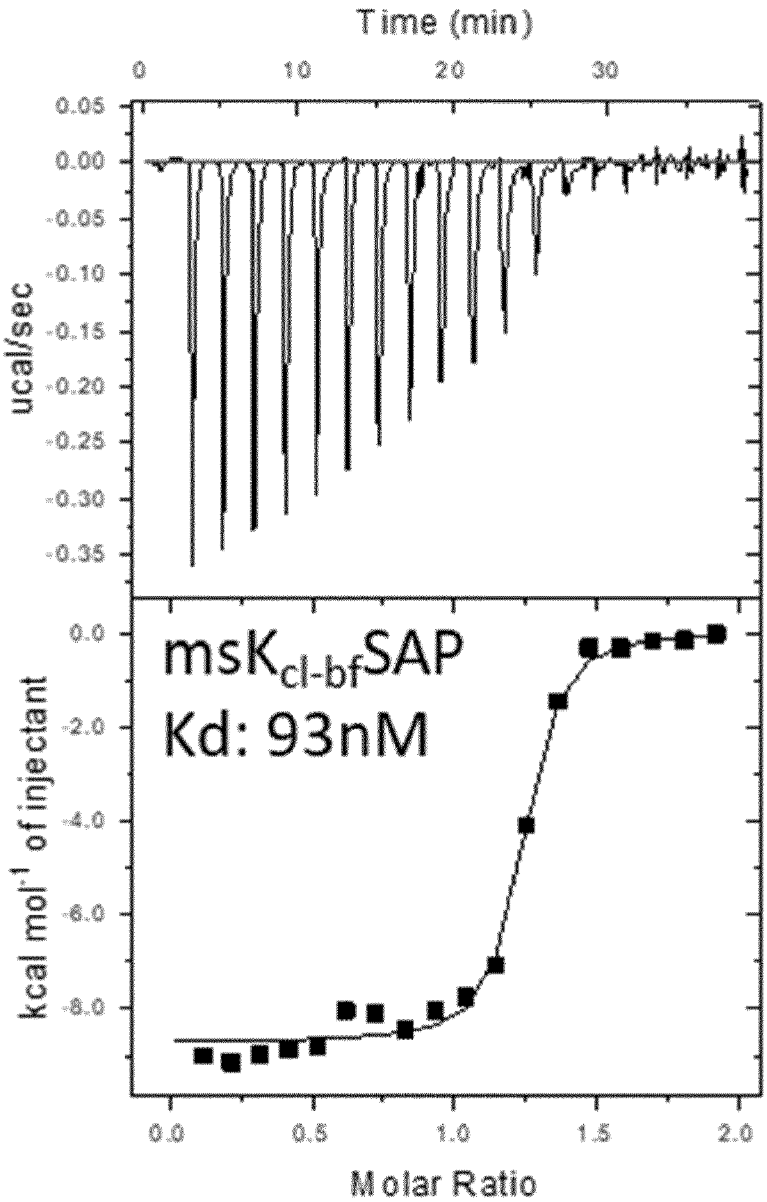
Time (min)
0
10
20
30
ucal/sec
0.05
0.00
-0.05
-0.10
-0.15
-0.20
-0.25
-0.30
-0.35
msK$_{cl-bf}$SAP
Kd: 93nM
kcal mol$^{-1}$ of injectant
0.0
-2.0
-4.0
-6.0
-8.0
0.0
0.5
1.0
1.5
2.0
Molar Ratio

FIG. 6G

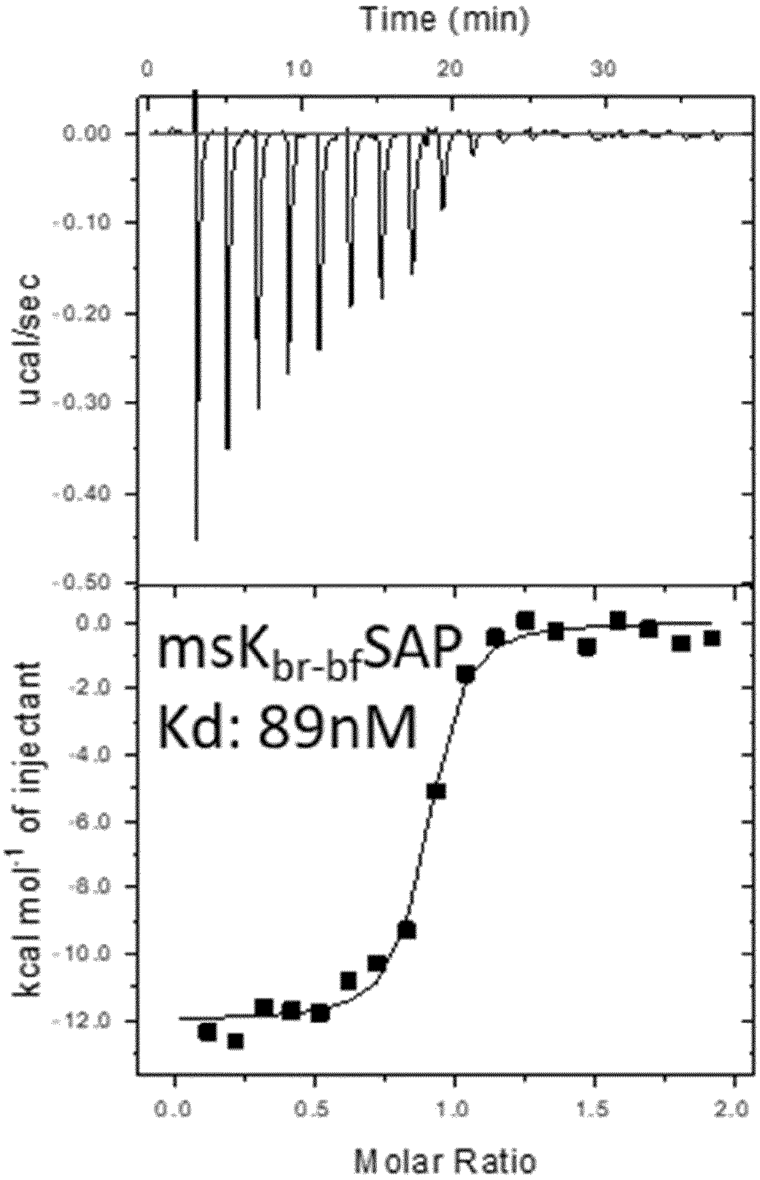
Time (min)
0
10
20
30
0.00
-0.10
-0.20
-0.30
-0.40
-0.50
ucal/sec
0.0
-2.0
-4.0
-6.0
-8.0
-10.0
-12.0
kcal mol-1 of injectant
msKbr-bfSAP
Kd: 89nM
0.0
0.5
1.0
1.5
2.0
Molar Ratio

FIG. 6H

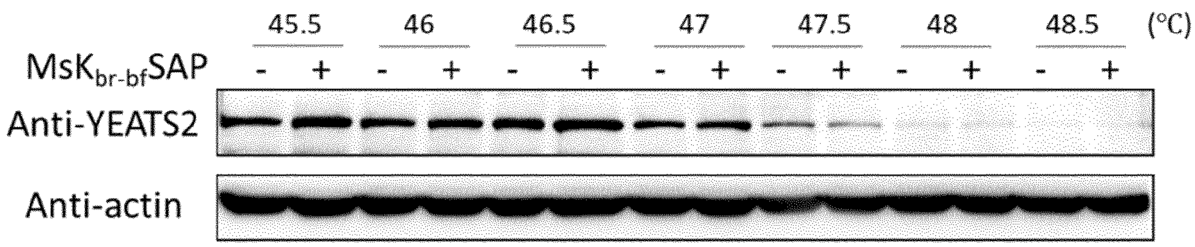
45.5
46
46.5
47
47.5
48
48.5
(°C)
MsKbr-bfSAP
- + - + - + - + - + - + - +
Anti-YEATS2
Anti-actin

FIG. 7A

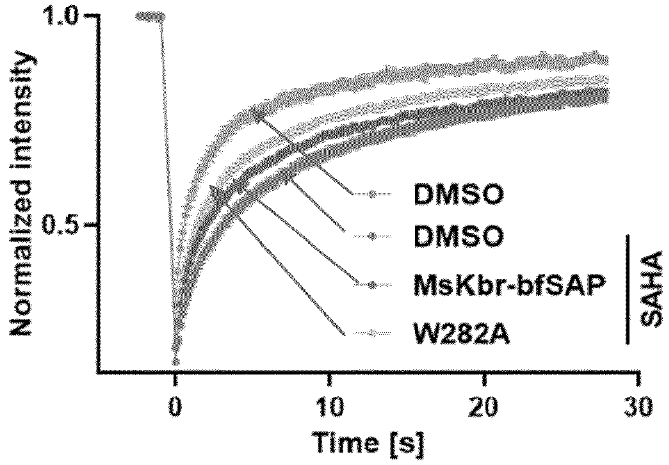
1.0
0.5
Normalized intensity
0
10
20
30
Time [s]
DMSO
DMSO
MsKbr-bfSAP
W282A
SAHA

FIG. 7B

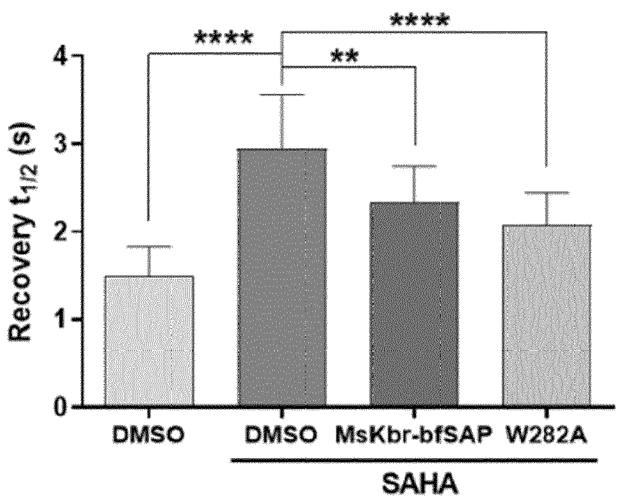
****
**
****
4
3
2
1
0
Recovery $t_{1/2}$ (s)
DMSO
DMSO
MsKbr-bfSAP
W282A
SAHA

FIG. 7C

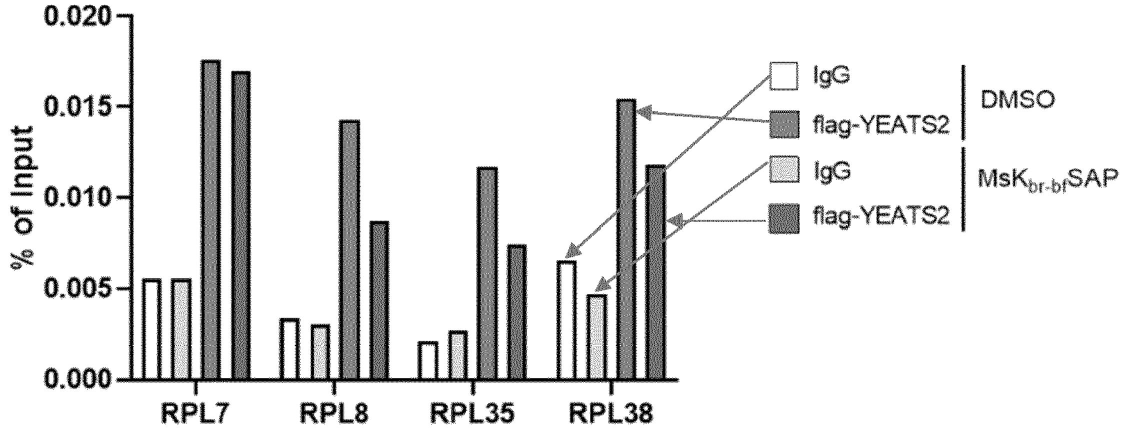
0.020
0.015
0.010
0.005
0.000
% of Input
RPL7
RPL8
RPL35
RPL38
IgG
flag-YEATS2
DMSO
IgG
flag-YEATS2
MsK$_{br-bf}$SAP

FIG. 7D

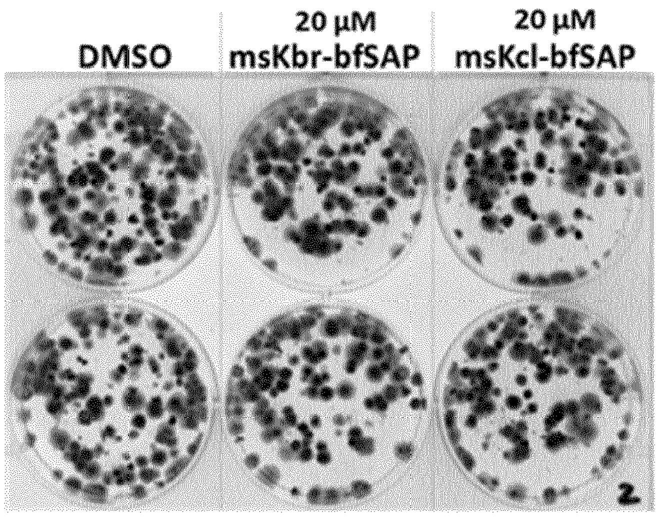
DMSO
20 µM
msKbr-bfSAP
20 µM
msKcl-bfSAP

FIG. 8A

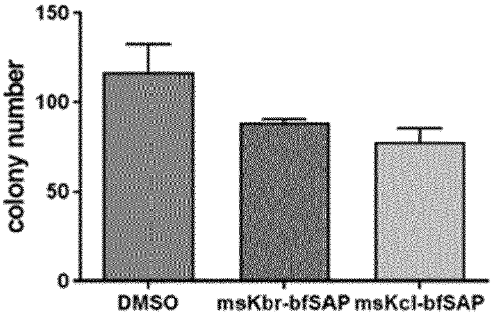


FIG. 8B

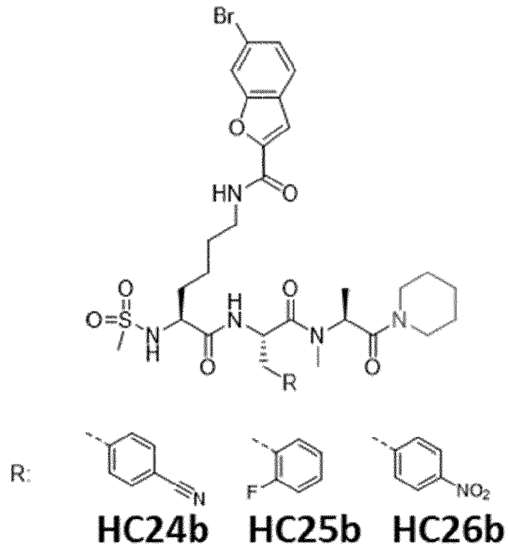

HC24b HC25b HC26b

FIG. 9A

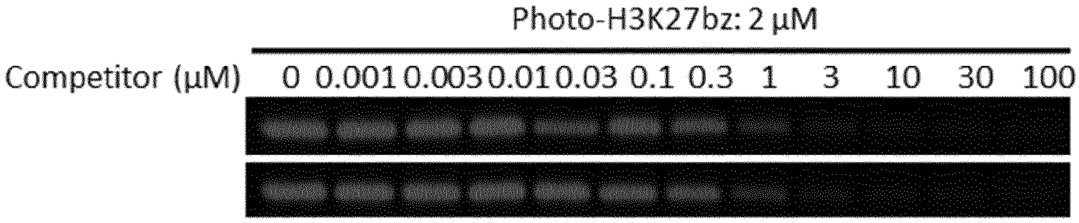


FIG. 9B

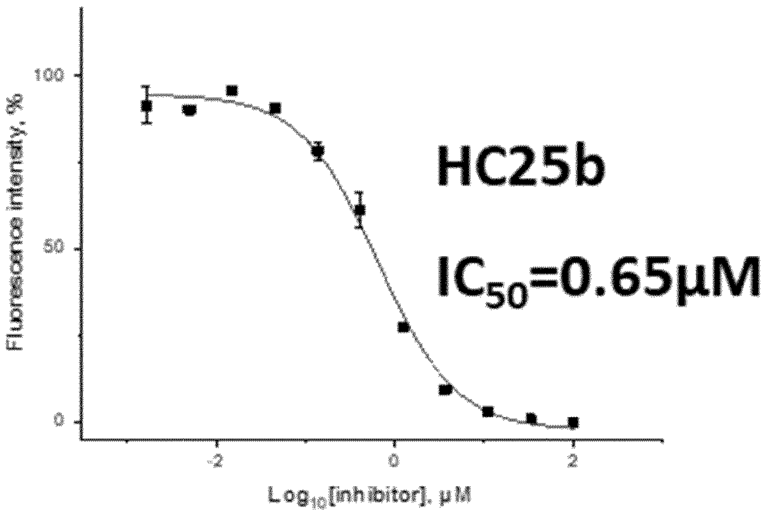


FIG. 9C

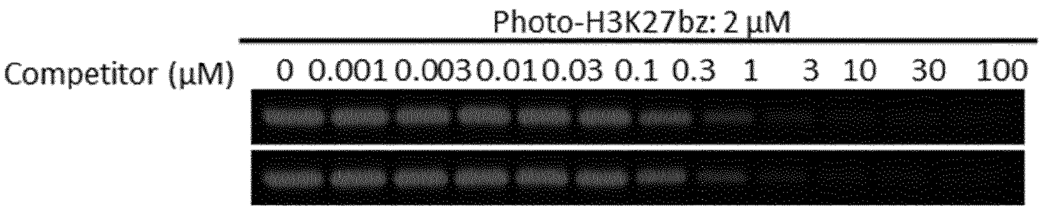


FIG. 9D

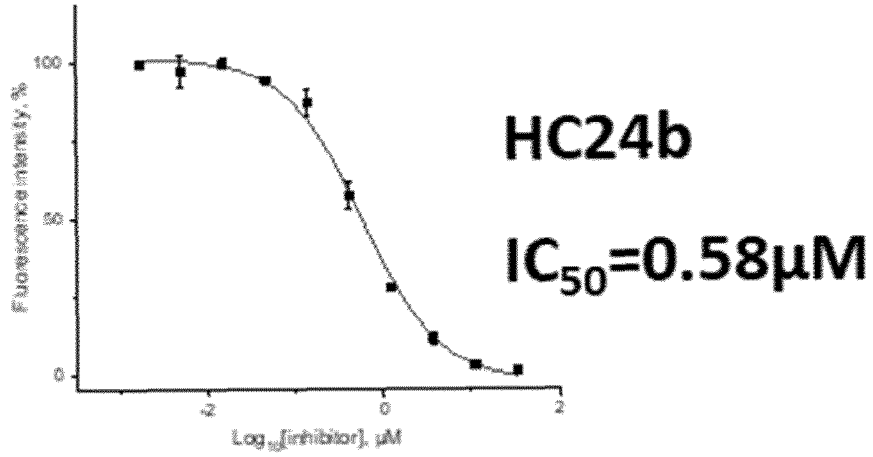


FIG. 9E

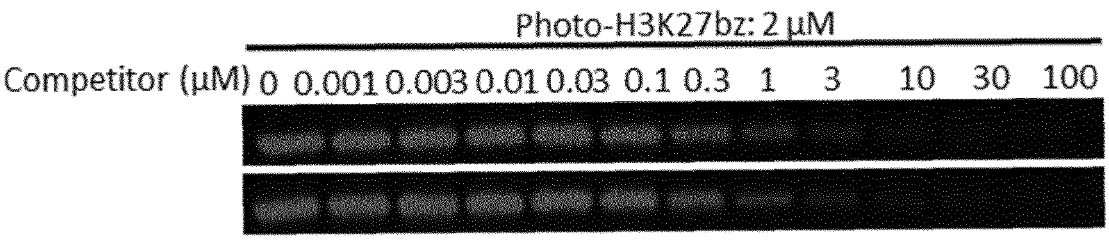


FIG. 9F

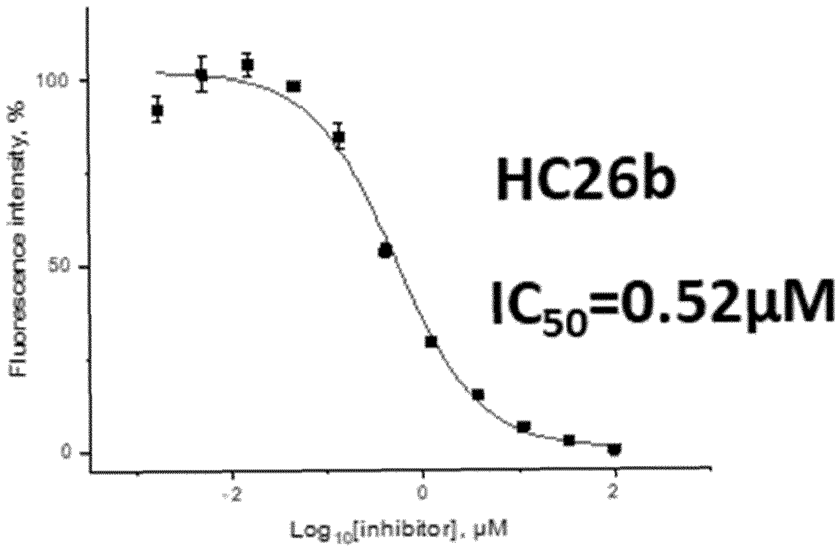


FIG. 9G

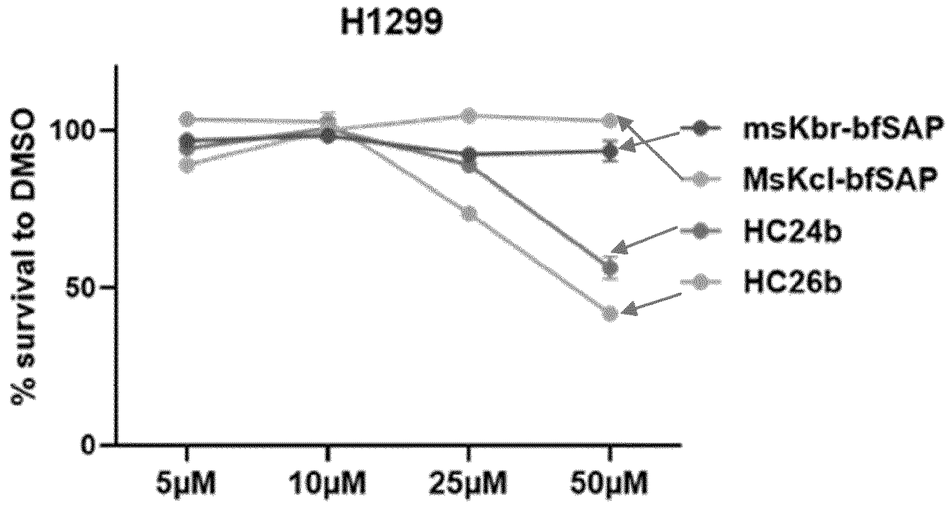


FIG. 10A

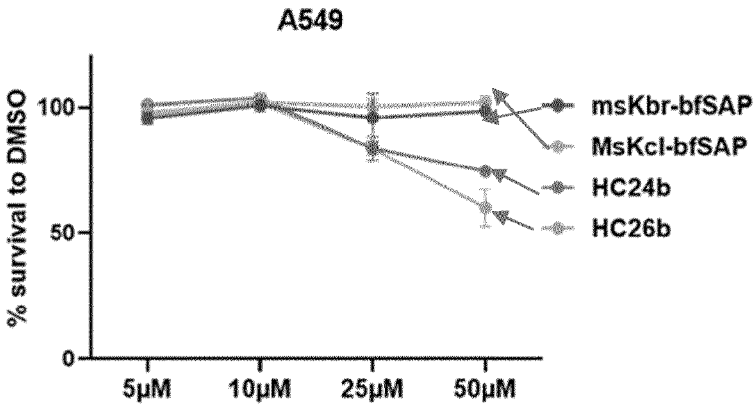


FIG. 10B

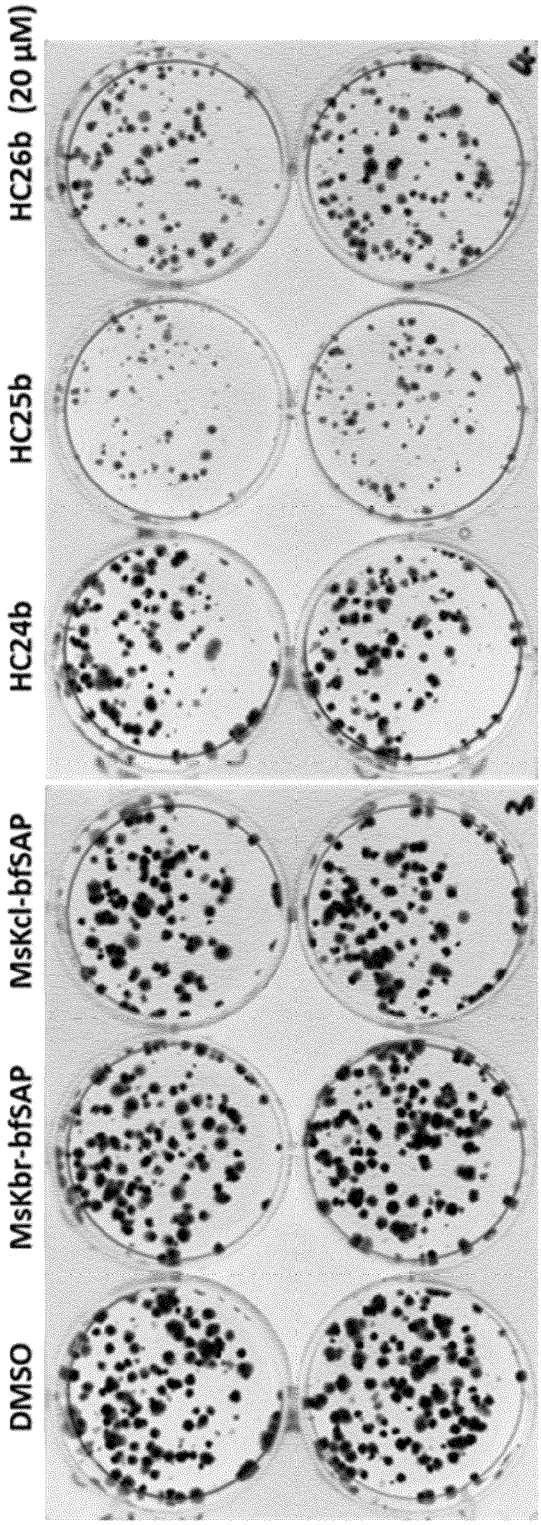


FIG. 10C

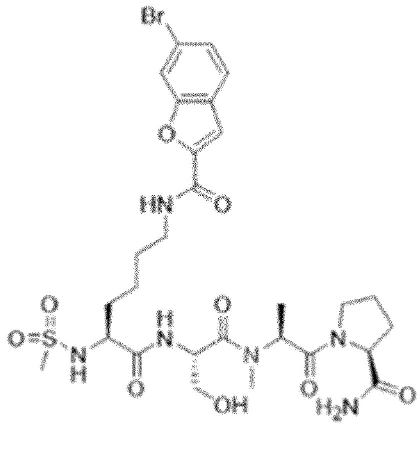

HC18

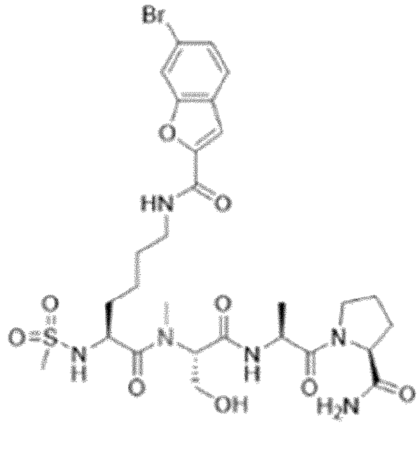

HC19

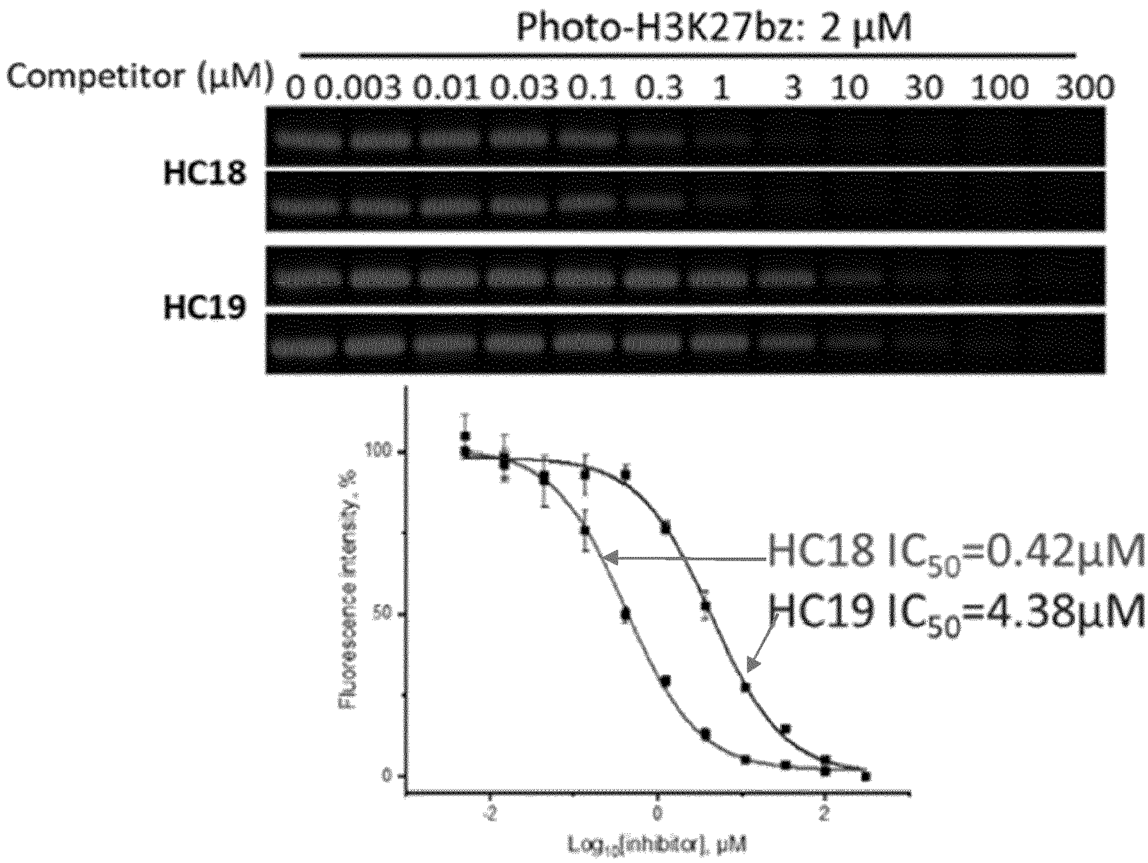


FIG. 11A

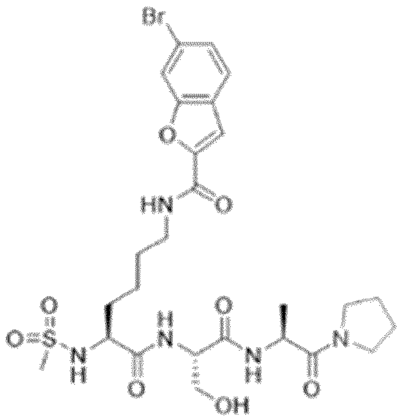

HC15a

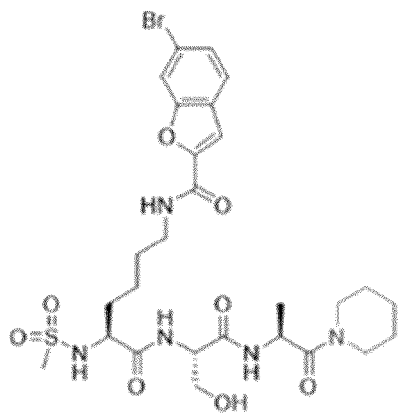

HC15b

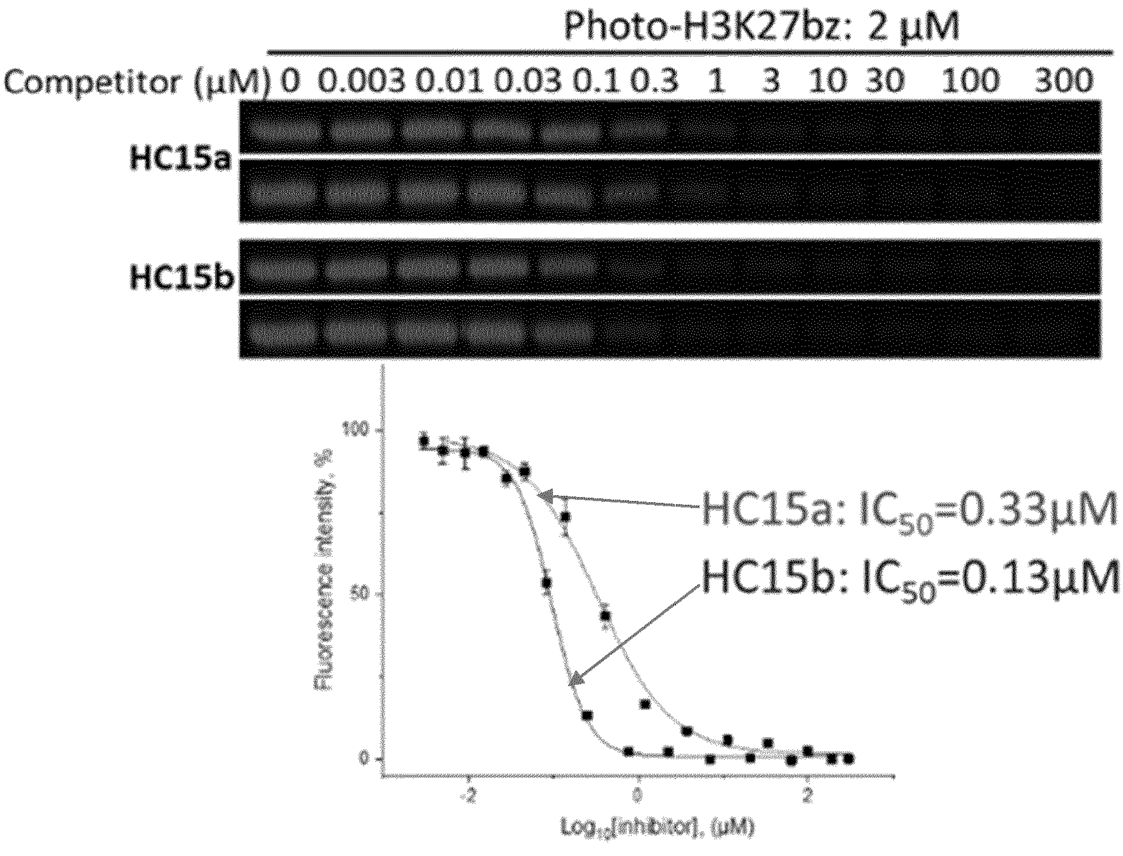


FIG. 11B

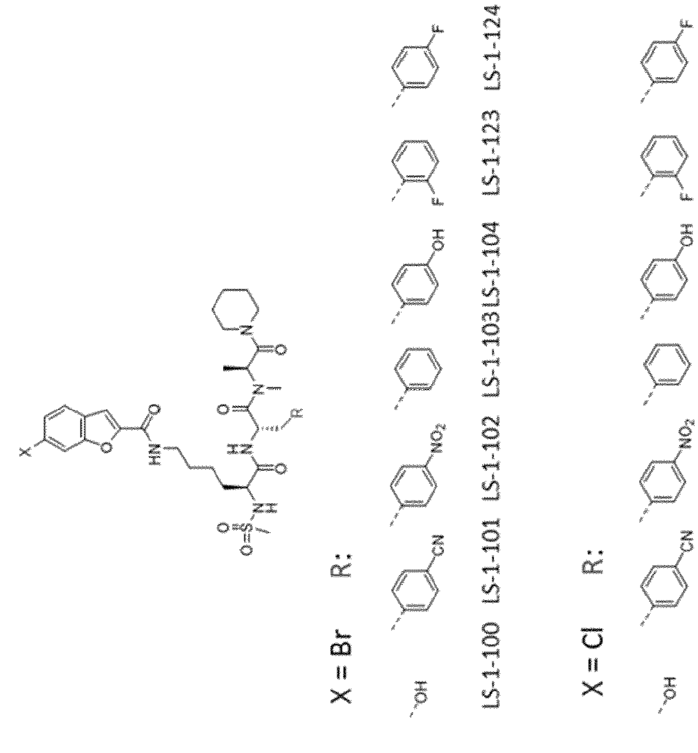

X = Br R:

LS-1-100 LS-1-101 LS-1-102 LS-1-103 LS-1-104 LS-1-123 LS-1-124

X = Cl R:

LS-1-118 LS-1-119 LS-1-120 LS-1-121 LS-1-122 LS-1-116 LS-1-125

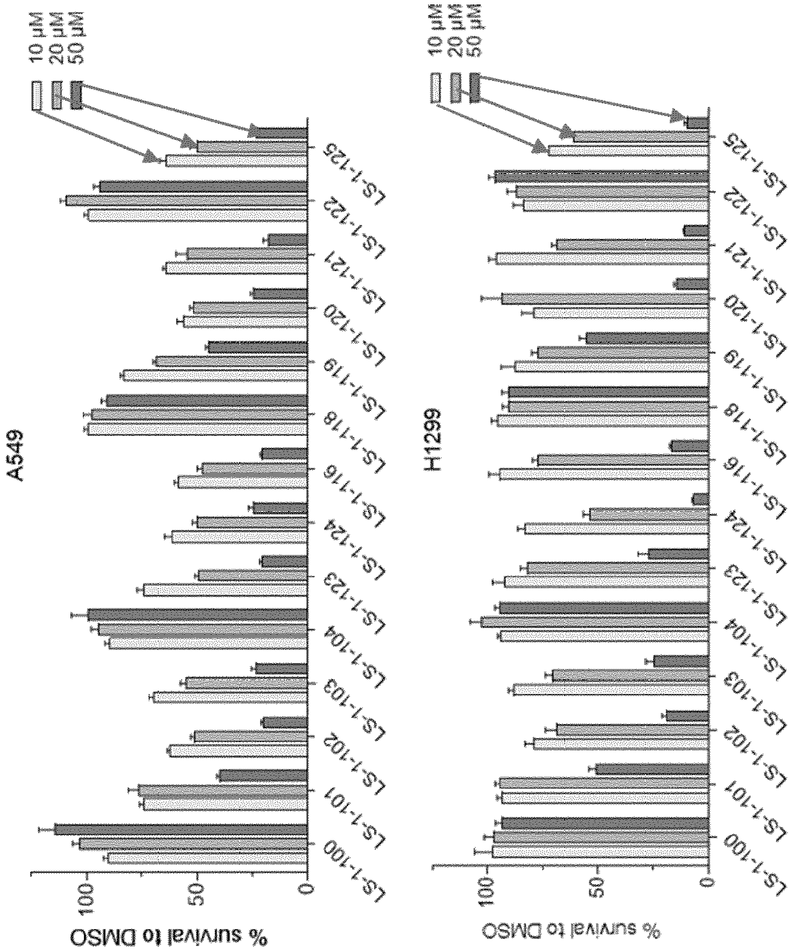


FIG. 12A

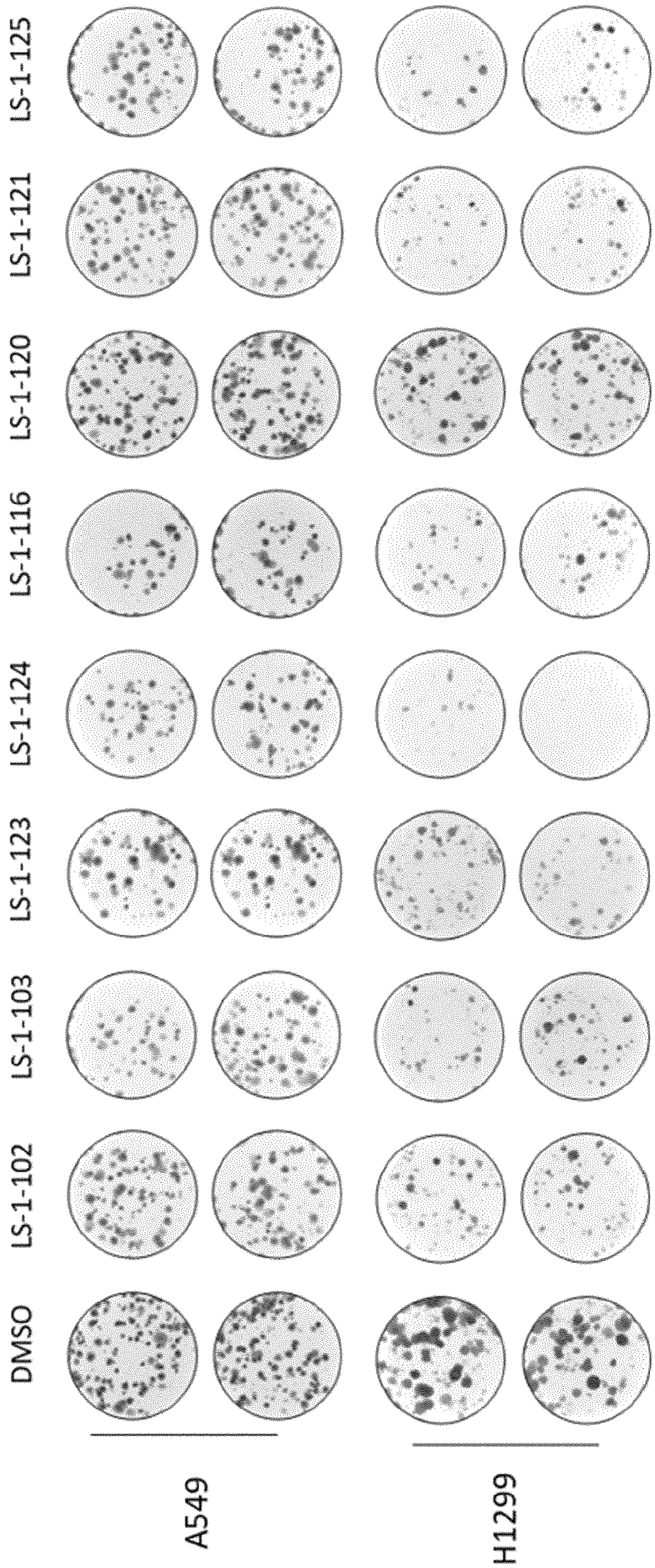
LS-1-125
LS-1-121
LS-1-120
LS-1-116
LS-1-124
LS-1-123
LS-1-103
LS-1-102
DMSO
A549
H1299

FIG. 12B

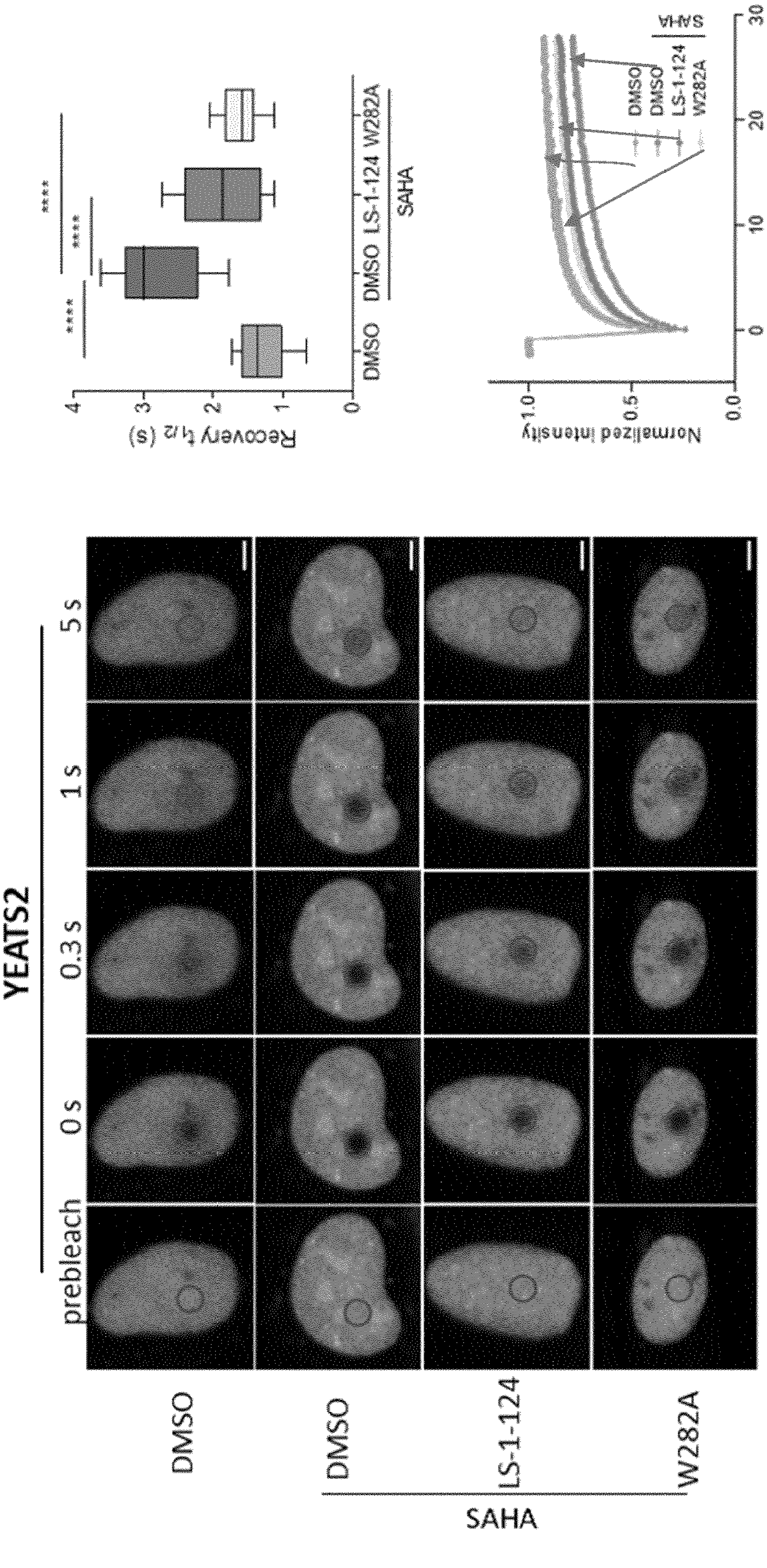
YEATS2
prebleach
0 s
0.3 s
1 s
5 s
DMSO
DMSO
LS-1-124
W282A
SAHA
Recovery t1/2 (s)
****
****
****
DMSO
DMSO
LS-1-124
W282A
SAHA
Normalized intensity
Time [s]
DMSO
DMSO
LS-1-124
W282A
SAHA

FIG. 13A

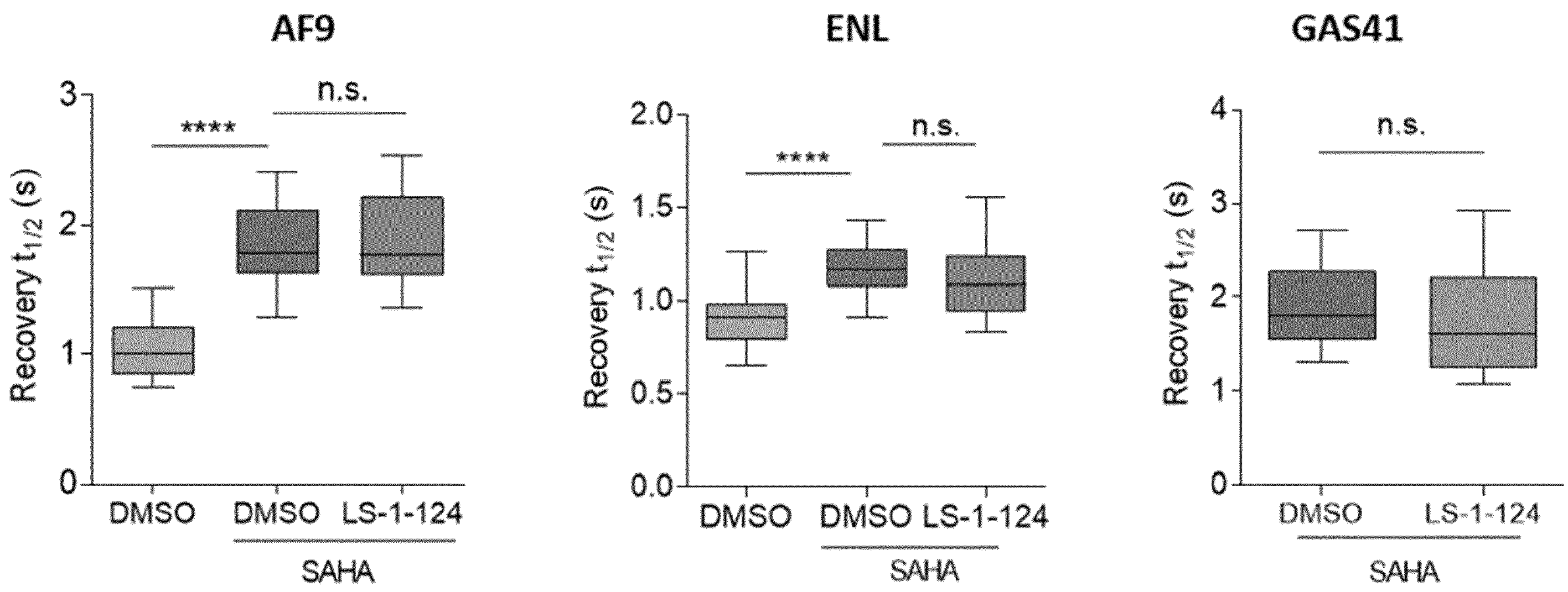


FIG. 13B

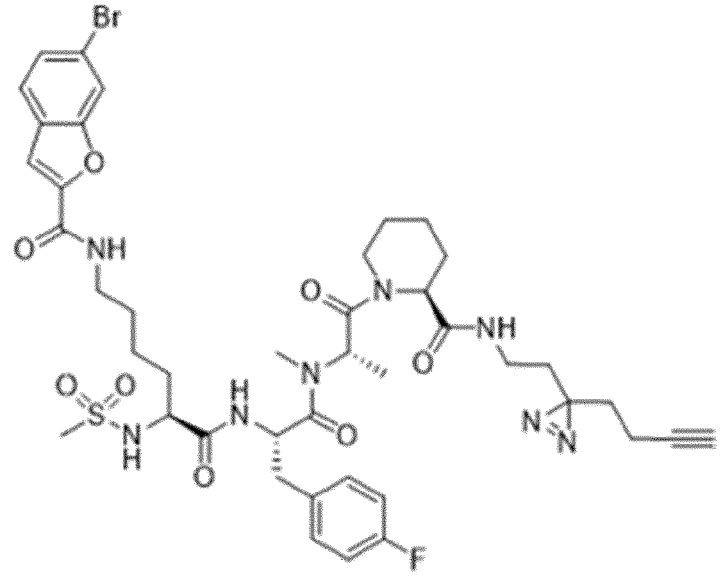

Probe 1

FIG. 14A

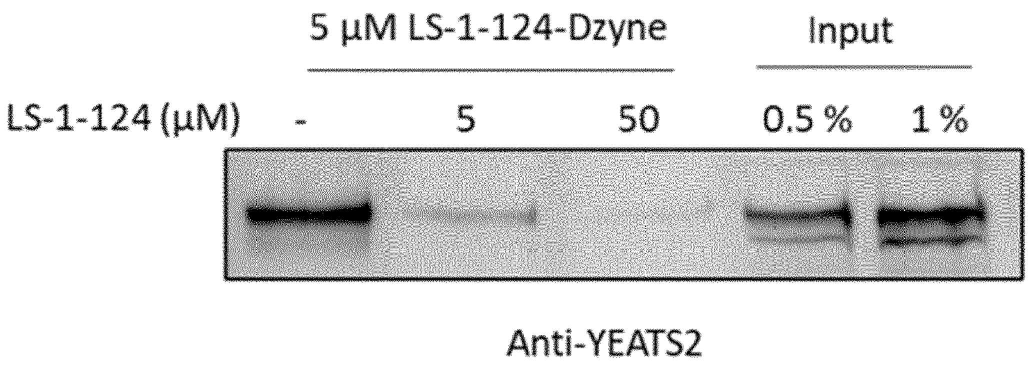


FIG. 14B

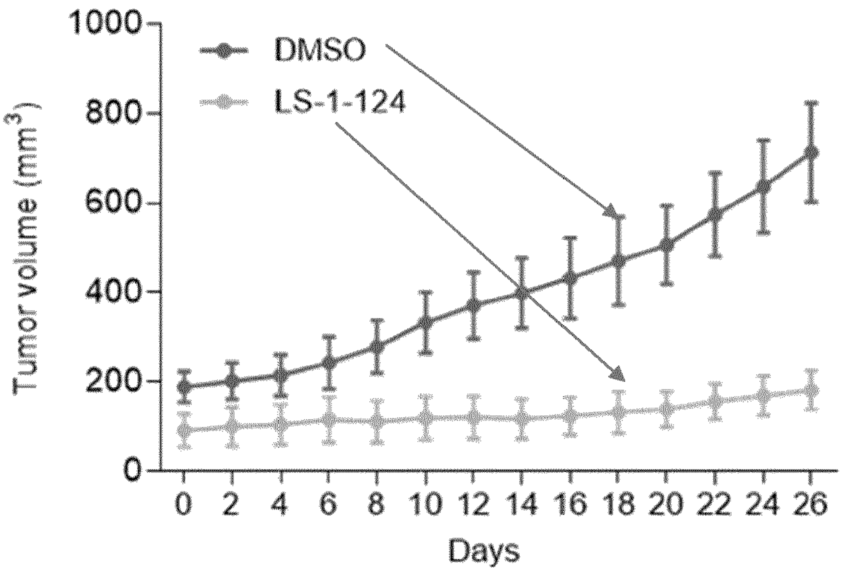


FIG. 15A

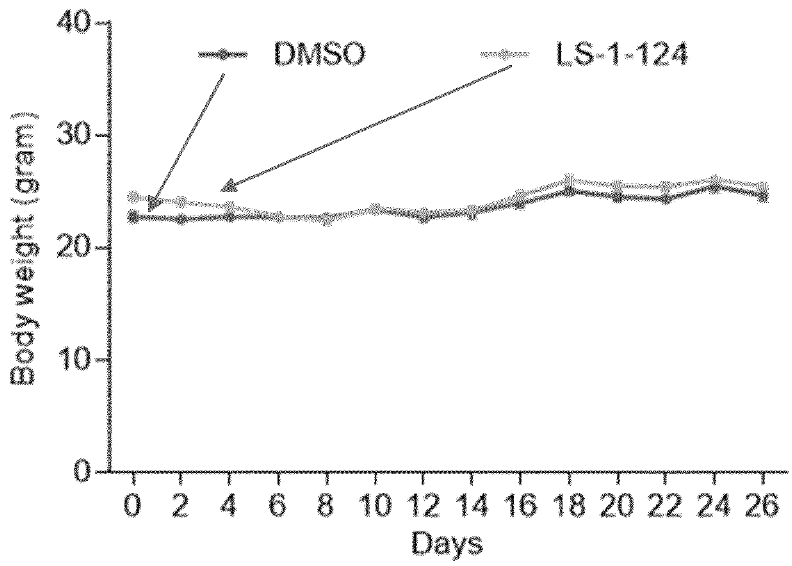


FIG. 15B

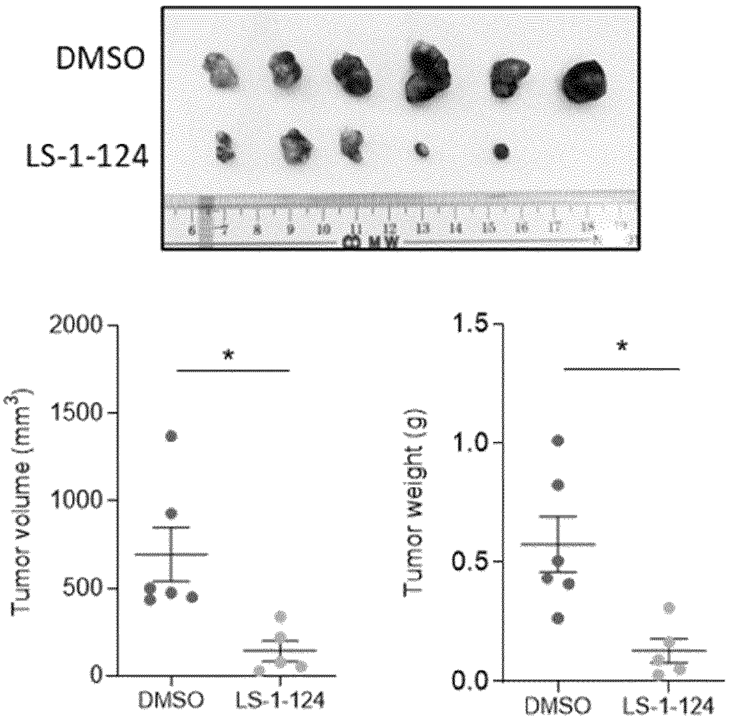


FIG. 15C

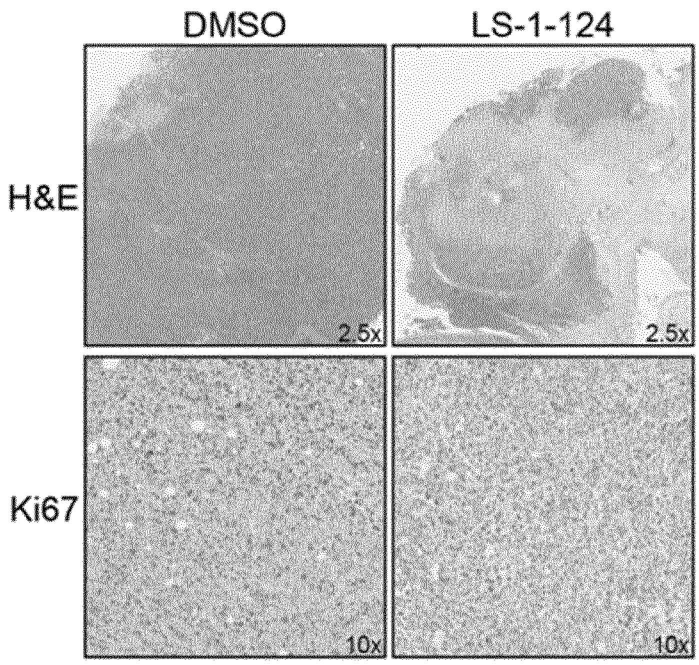


FIG. 15D

Europäisches Patentamt
European Patent Office
Office européen des brevets

# EUROPEAN SEARCH REPORT

Application Number
EP 23 16 2698

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Wong Yuen Kwan: "Development of YEATS2 Inhibitor as a Potential Non-Small Cell Lung Cancer Cure", , 5 November 2020 (2020-11-05), XP093076665, Retrieved from the Internet: URL:https://www.scifac.hku.hk/f/page/7014/13906/C9_Poster.pdf [retrieved on 2023-08-28] * the whole document * ----- | 8,10,12, 14,15 | INV. A61P35/00 A61K38/08 A61K38/04 |
| X | EP 3 717 504 A1 (UNIV HONG KONG [CN]) 7 October 2020 (2020-10-07) * claims 2, 10 page 106 claims 13, 16, 17 * ----- | 1-6,8, 10,12 | |
| A | ZHAO DAN ET AL: "YEATS2 is a selective histone crotonylation reader", CELL RESEARCH, vol. 26, no. 5, 22 April 2016 (2016-04-22), pages 629-632, XP093077539, Singapore ISSN: 1001-0602, DOI: 10.1038/cr.2016.49 Retrieved from the Internet: URL:https://www.ncbi.nlm.nih.gov/pmc/articles/PMC4856769/pdf/cr201649a.pdf> * the whole document * ----- | 1-15 | TECHNICAL FIELDS SEARCHED (IPC) A61P A61K |

The present search report has been drawn up for all claims

1

| Place of search | Date of completion of the search | Examiner |
|---|---|---|
| Munich | 31 August 2023 | Albayrak, Timur |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 16 2698

This annex lists the patent family members relating to the patent documents cited in the above-mentioned European search report. The members are as contained in the European Patent Office EDP file on
The European Patent Office is in no way liable for these particulars which are merely given for the purpose of information.

31-08-2023

| Patent document cited in search report | Publication date | Patent family member(s) | Publication date |
|---|---|---|---|
| EP 3717504 A1 | 07-10-2020 | CN 111615517 A | 01-09-2020 |
| | | EP 3717504 A1 | 07-10-2020 |
| | | JP 7239202 B2 | 14-03-2023 |
| | | JP 2021504371 A | 15-02-2021 |
| | | US 2021277061 A1 | 09-09-2021 |
| | | WO 2019101195 A1 | 31-05-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63269511


### Non-patent literature cited in the description


- **MI, W. ; GUAN, H. ; LYU, J. et al.** YEATS2 links histone acetylation to tumorigenesis of non-small cell lung cancer. *Nat Commun,* 2017, vol. 8, 1088
- **ERB MA ; SCOTT TG ; LI BE ; XIE H ; PAULK J ; SEO HS ; SOUZA A ; ROBERTS JM ; DASTJERDI S ; BUCKLEY DL.** Transcription control by the ENL YEATS domain in acute leukaemia. *Nature,* 09 March 2017, vol. 543 (7644), 270-274